(19) Europäisches Patentamt
European Patent Office
Office européen des brevets

(11) EP 4 223 743 A1

(12) **EUROPEAN PATENT APPLICATION**
published in accordance with Art. 153(4) EPC

(43) Date of publication:
**09.08.2023 Bulletin 2023/32**

(21) Application number: **21863054.9**

(22) Date of filing: **29.09.2021**

(51) International Patent Classification (IPC):
***C07D 209/86*** (2006.01) ***C07D 405/12*** (2006.01)
***H01L 51/50*** (2006.01)

(52) Cooperative Patent Classification (CPC):
**C07D 405/12; C07D 209/86; C09K 11/06; H10K 85/615; H10K 85/631; H10K 85/6572; H10K 85/6574;** H10K 50/156

(86) International application number:
**PCT/JP2021/035741**

(87) International publication number:
**WO 2022/071350 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2020 JP 2020166481**

(71) Applicant: **Idemitsu Kosan Co.,Ltd.**
**Tokyo 100-8321 (JP)**

(72) Inventors:
- **HAKETA, Tasuku**
  **Sodegaura-shi, Chiba 299-0293 (JP)**
- **TANAKA, Shota**
  **Sodegaura-shi, Chiba 299-0293 (JP)**
- **TAKAHASHI, Yusuke**
  **Sodegaura-shi, Chiba 299-0293 (JP)**

(74) Representative: **Vossius & Partner**
**Patentanwälte Rechtsanwälte mbB**
**Siebertstraße 3**
**81675 München (DE)**

(54) **COMPOUND, MATERIAL FOR ORGANIC ELECTROLUMINESCENT ELEMENTS, ORGANIC ELECTROLUMINESCENT ELEMENT, AND ELECTRONIC DEVICE**

(57) To provide a compound for further improving the capability of an organic EL device, an organic electroluminescent element having a further improved device capability, and an electronic device including the organic electroluminescent element. Disclosed are a compound represented by the following formula (1) or formula (2) (wherein each symbol in each formula is as defined in the description), an organic electroluminescent element containing the compound, and an electronic device including the organic electroluminescent element.

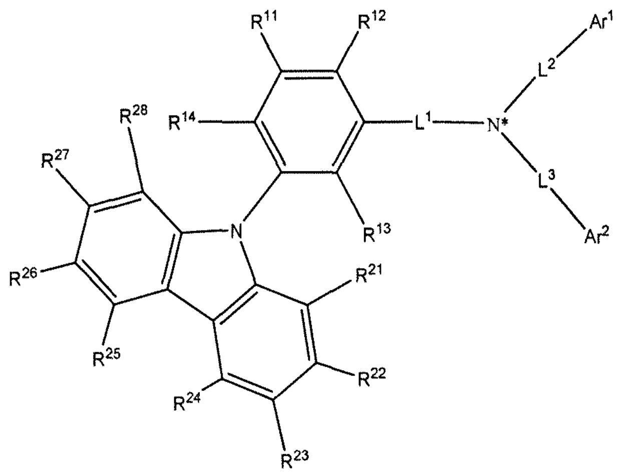

(1)

EP 4 223 743 A1

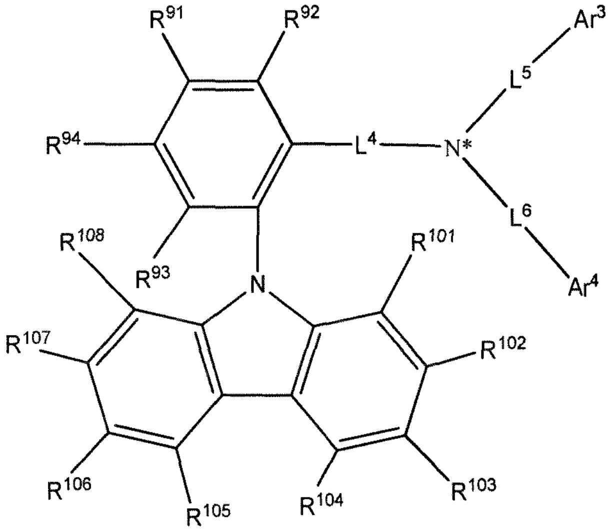

(2)

[Fig. 1]

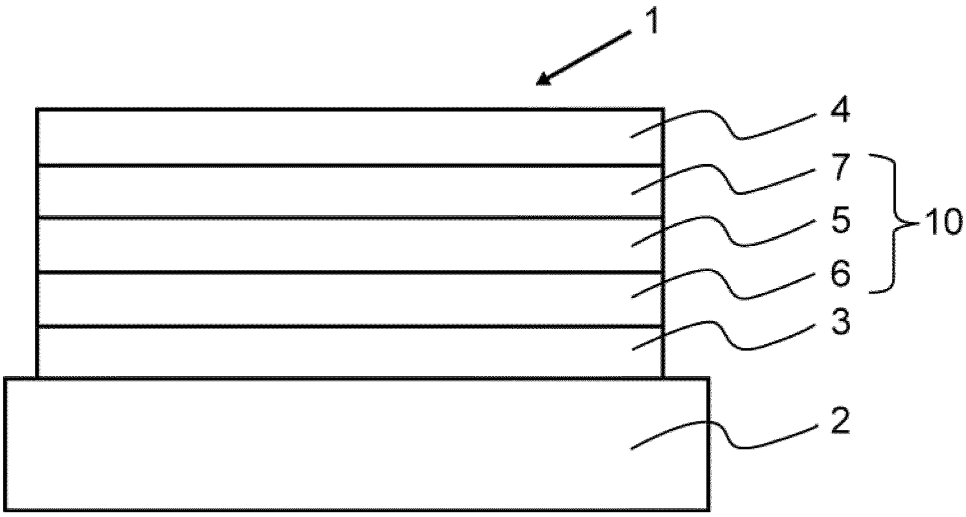




(Cont. next page)

[Fig. 2]

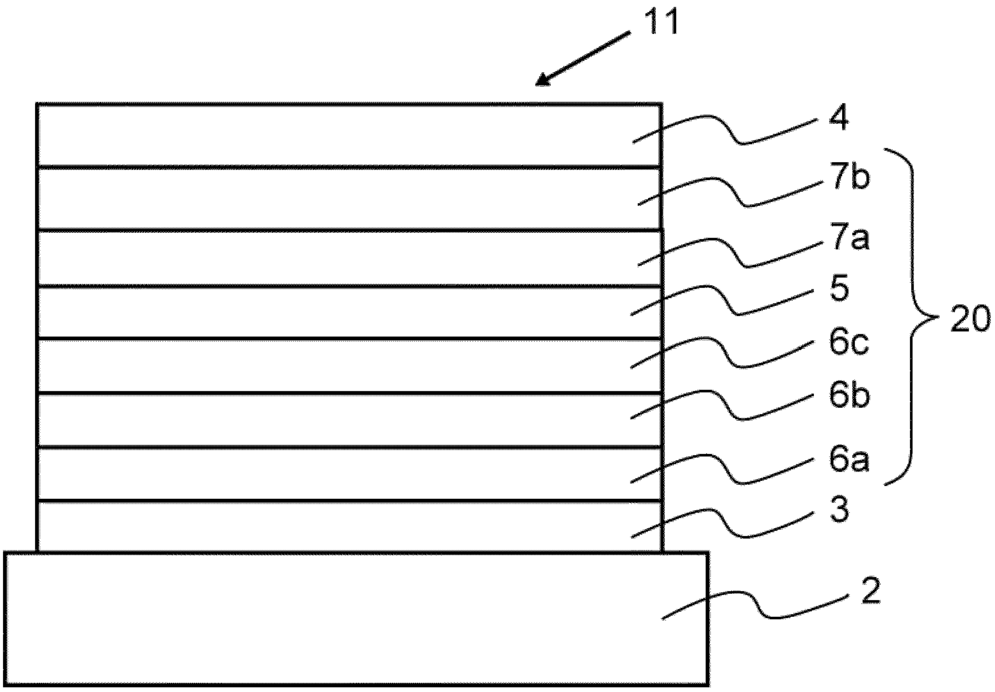

## Description

### Technical Field

The present invention relates to a compound, a material for organic electroluminescent elements, an organic electroluminescent element, and an electronic device including the organic luminescent device.

### Background Art

In general, an organic electroluminescent element (which may be hereinafter referred to as an "organic EL device") is constituted by an anode, a cathode, and an organic layer intervening between the anode and the cathode. In application of a voltage between both the electrodes, electrons from the cathode side and holes from the anode side are injected into a light emitting region, and the injected electrons and holes are recombined in the light emitting region to generate an excited state, which then returns to the ground state to emit light. Accordingly, development of a material that efficiently transports electrons or holes into the light emitting region, and promotes recombination of the electrons and holes is important for providing a high-performance organic EL device.

PTLs 1 to 17 describe compounds used for a material for organic electroluminescent elements.

### Citation List

### Patent Literature

PTL 1: WO 2011/024451 A
PTL 2: KR 10-2011-0064222 A
PTL 3: US 6,110,307 B
PTL 4: WO 2013/122082 A
PTL 5: WO 2014/007022 A
PTL 6: US 2014/0138633 A1
PTL 7: KR 10-2014-0103697 A
PTL 8: KR 10-2015-0031892 A
PTL 9: KR 10-2015-0116337 A
PTL 10: US 2018/0090688 A1
PTL 11: WO 2017/118238 A
PTL 12: KR 10-2017-0084917 A
PTL 13: US 2019/0189927 A1
PTL 14: US 2020/0106017 A1
PTL 15: KR 10-2018-0078177 A
PTL 16: KR 10-1978651 B
PTL 17: KR 10-2019-0084880 A

### Summary of Invention

### Technical Problem

Various compounds for organic EL devices have been reported, but a compound that further enhances the capability of an organic EL device has been still demanded.

The present invention has been made for solving the problem, and an object thereof is to provide a compound that further improves the capability of an organic EL device, an organic EL device having a further improved device capability, and an electronic device including the organic EL device.

### Solution to Problem

As a result of intensive studies on the capability of organic EL devices containing the compounds described in PTLs 1 to 17 and other compounds, the present inventors have found that: a monoamine having at least one deuterium atom in which one group having a substituted or unsubstituted m-(9-carbazolyl)phenyl group via a specific linker (including a single bond) and the remaining two groups each having a specific aryl group or a specific heterocyclic group via a

specific linker (including a single bond) are bonded to a central nitrogen atom; or a monoamine having at least one deuterium atom in which one group having a substituted or unsubstituted o-(9-carbazolyl)phenyl group via a specific linker (including a single bond) and the remaining two groups each having a specific aryl group or a specific heterocyclic group via a specific linker (including a single bond) are bonded to a central nitrogen atom; provides an organic EL device with more improved device capability.

In one embodiment, the present invention provides a compound represented by the following formula (1), which has at least one deuterium atom:

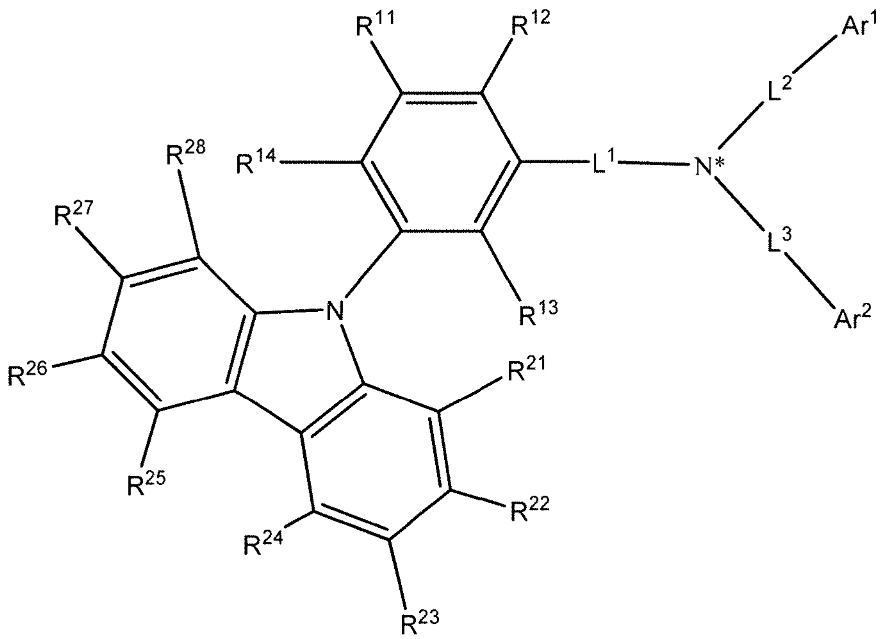

(1)

wherein

N* is a central nitrogen atom;

$Ar^1$ and $Ar^2$ are each independently a substituted or unsubstituted aryl group having 6 to 16 ring carbon atoms or a substituted or unsubstituted monovalent heterocyclic group having 5 to 30 ring atoms;

when both of $Ar^1$ and $Ar^2$ are the substituted or unsubstituted aryl groups having 6 to 16 ring carbon atoms, the total number of carbon atoms of the two substituted or unsubstituted aryl groups having 6 to 16 ring carbon atoms is 12 to 38;

$L^1$ is any one of a single bond, a substituted or unsubstituted arylene group having 6 to 30 ring carbon atoms, and a substituted or unsubstituted divalent heterocyclic group having 5 to 30 ring atoms;

the substituent of $L^1$ is a substituted or unsubstituted alkyl group having 1 to 50 carbon atoms, or a substituted or unsubstituted aryl group having 6 to 50 ring carbon atoms;

$L^2$ and $L^3$ are each independently any one of a single bond, a substituted or unsubstituted non-fused arylene group having 6 to 30 ring carbon atoms, and a substituted or unsubstituted divalent heterocyclic group having 5 to 30 ring atoms;

$Ar^1$ and $L^2$ are not crosslinked;

$Ar^2$ and $L^3$ are not crosslinked; and

$R^{11}$ to $R^{14}$ and $R^{21}$ to $R^{28}$ are each independently a hydrogen atom or a substituent, and the substituent is a substituted or unsubstituted alkyl group having 1 to 50 carbon atoms or a substituted or unsubstituted aryl group having 6 to 50 ring carbon atoms.

In another embodiment, the present invention provides a compound represented by the following formula (2), which has at least one deuterium atom:

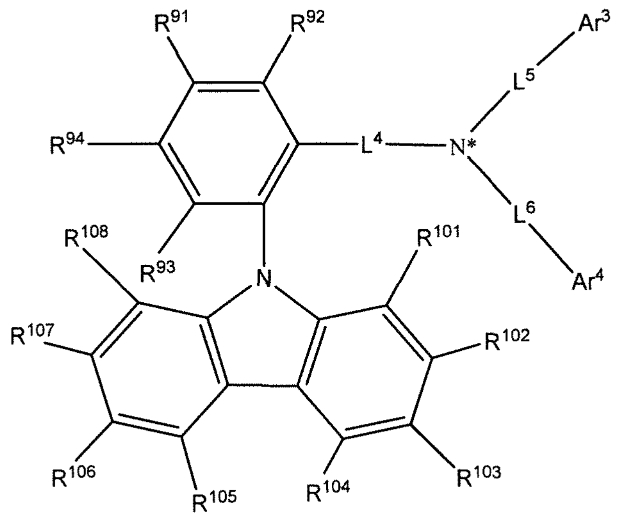

(2)

wherein

N* is a central nitrogen atom;

$Ar^3$ and $Ar^4$ are each independently a substituted or unsubstituted aryl group having 6 to 30 ring carbon atoms (excluding a phenyl group) or a substituted or unsubstituted monovalent heterocyclic group having 5 to 30 ring atoms;

$L^4$, $L^5$ and $L^6$ are each independently any one of a single bond, a substituted or unsubstituted arylene group having 6 to 30 ring carbon atoms, and a substituted or unsubstituted divalent heterocyclic group having 5 to 30 ring atoms; and

$R^{91}$ to $R^{94}$ and $R^{101}$ to $R^{108}$ are each independently a hydrogen atom or a substituent, and the substituent is a halogen atom; a nitro group; a cyano group; a substituted or unsubstituted alkyl group having 1 to 50 carbon atoms; a substituted or unsubstituted alkenyl group having 2 to 50 carbon atoms; a substituted or unsubstituted alkynyl group having 2 to 50 carbon atoms; a substituted or unsubstituted cycloalkyl group having 3 to 50 ring carbon atoms; a substituted or unsubstituted haloalkyl group having 1 to 50 carbon atoms; a substituted or unsubstituted alkoxy group having 1 to 50 carbon atoms; a substituted or unsubstituted haloalkoxy group having 1 to 50 carbon atoms; a substituted or unsubstituted alkylthio group having 1 to 50 carbon atoms; a substituted or unsubstituted aryl group having 6 to 50 ring carbon atoms; a substituted or unsubstituted aryloxy group having 6 to 50 ring carbon atoms; a substituted or unsubstituted arylthio group having 6 to 50 ring carbon atoms; a substituted or unsubstituted aralkyl group having 7 to 50 carbon atoms; a substituted or unsubstituted monovalent heterocyclic group having 5 to 50 ring atoms; or a mono-, di-, or tri-substituted silyl group having a substituent selected from a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, and a substituted or unsubstituted monovalent heterocyclic group having 5 to 50 ring atoms.

Further, in another embodiment, the present invention provides a material for an organic EL device containing the compound represented by the formula (1) or the formula (2).

In still another embodiment, the present invention provides an organic electroluminescent element including an anode, a cathode, and one or more organic layers intervening between the anode and the cathode, the organic layers including a light emitting layer, at least one layer of the organic layers containing the compound represented by the formula (1) or the formula (2).

In a further embodiment, the present invention provides an electronic device including the organic electroluminescent element.

## Advantageous Effects of Invention

An organic EL device containing the compound represented by the formula (1) or the formula (2) shows an improved device capability.

## Brief Description of Drawings

Fig. 1 is a schematic illustration showing an example of the layer configuration of the organic EL device according to one embodiment of the present invention.

Fig. 2 is a schematic illustration showing an example of the layer configuration of another organic EL device according to one embodiment of the present invention.

## Description of Embodiments

### [Definitions]

In the description herein, the hydrogen atom encompasses isotopes thereof having different numbers of neutrons, i.e., a light hydrogen atom (protium), a heavy hydrogen atom (deuterium), and tritium.

In the description herein, the bonding site where the symbol, such as "R", or "D" representing a deuterium atom is not shown is assumed to have a hydrogen atom, i.e., a protium atom, a deuterium atom, or a tritium atom, bonded thereto.

In the description herein, the number of ring carbon atoms shows the number of carbon atoms among the atoms constituting the ring itself of a compound having a structure including atoms bonded to form a ring (such as a monocyclic compound, a condensed ring compound, a bridged compound, a carbocyclic compound, and a heterocyclic compound). In the case where the ring is substituted by a substituent, the carbon atom contained in the substituent is not included in the number of ring carbon atoms. The same definition is applied to the "number of ring carbon atoms" described hereinafter unless otherwise indicated. For example, a benzene ring has 6 ring carbon atoms, a naphthalene ring has 10 ring carbon atoms, a pyridine ring has 5 ring carbon atoms, and a furan ring has 4 ring carbon atoms. For example, 9,9-diphenylfluorenyl group has 13 ring carbon atoms, and 9,9'-spirobifluorenyl group has 25 ring carbon atoms.

In the case where a benzene ring has, for example, an alkyl group substituted thereon as a substituent, the number of carbon atoms of the alkyl group is not included in the number of ring carbon atoms of the benzene ring. Accordingly, a benzene ring having an alkyl group substituted thereon has 6 ring carbon atoms. In the case where a naphthalene ring has, for example, an alkyl group substituted thereon as a substituent, the number of carbon atoms of the alkyl group is not included in the number of ring carbon atoms of the naphthalene ring. Accordingly, a naphthalene ring having an alkyl group substituted thereon has 10 ring carbon atoms.

In the description herein, the number of ring atoms shows the number of atoms constituting the ring itself of a compound having a structure including atoms bonded to form a ring (such as a monocyclic ring, a condensed ring, and a set of rings) (such as a monocyclic compound, a condensed ring compound, a bridged compound, a carbocyclic compound, and a heterocyclic compound). The atom that does not constitute the ring (such as a hydrogen atom terminating the bond of the atom constituting the ring) and, in the case where the ring is substituted by a substituent, the atom contained in the substituent are not included in the number of ring atoms. The same definition is applied to the "number of ring atoms" described hereinafter unless otherwise indicated. For example, a pyridine ring has 6 ring atoms, a quinazoline ring has 10 ring atoms, and a furan ring has 5 ring atoms. For example, the number of hydrogen atoms bonded to a pyridine ring or atoms constituting a substituent is not included in the number of ring atoms of the pyridine ring. Accordingly, a pyridine ring having a hydrogen atom or a substituent bonded thereto has 6 ring atoms. For example, the number of hydrogen atoms bonded to carbon atoms of a quinazoline ring or atoms constituting a substituent is not included in the number of ring atoms of the quinazoline ring. Accordingly, a quinazoline ring having a hydrogen atom or a substituent bonded thereto has 10 ring atoms.

In the description herein, the expression "having XX to YY carbon atoms" in the expression "substituted or unsubstituted ZZ group having XX to YY carbon atoms" means the number of carbon atoms of the unsubstituted ZZ group, and, in the case where the ZZ group is substituted, the number of carbon atoms of the substituent is not included. Herein, "YY" is larger than "XX", "XX" represents an integer of 1 or more, and "YY" represents an integer of 2 or more.

In the description herein, the expression "having XX to YY atoms" in the expression "substituted or unsubstituted ZZ group having XX to YY atoms" means the number of atoms of the unsubstituted ZZ group, and, in the case where the ZZ group is substituted, the number of atoms of the substituent is not included. Herein, "YY" is larger than "XX", "XX" represents an integer of 1 or more, and "YY" represents an integer of 2 or more.

In the description herein, an unsubstituted ZZ group means the case where the "substituted or unsubstituted ZZ group" is an "unsubstituted ZZ group", and a substituted ZZ group means the case where the "substituted or unsubstituted ZZ group" is a "substituted ZZ group".

In the description herein, the expression "unsubstituted" in the expression "substituted or unsubstituted ZZ group" means that hydrogen atoms in the ZZ group are not substituted by a substituent. The hydrogen atoms in the "unsubstituted ZZ group" each are a protium atom, a deuterium atom, or a tritium atom.

In the description herein, the expression "substituted" in the expression "substituted or unsubstituted ZZ group" means that one or more hydrogen atom in the ZZ group is substituted by a substituent. The expression "substituted" in the expression "BB group substituted by an AA group" similarly means that one or more hydrogen atom in the BB group is substituted by the AA group.

Substituents in Description

The substituents described in the description herein will be explained.

In the description herein, the number of ring carbon atoms of the "unsubstituted aryl group" is 6 to 50, preferably 6 to 30, and more preferably 6 to 18, unless otherwise indicated in the description.

In the description herein, the number of ring atoms of the "unsubstituted heterocyclic group" is 5 to 50, preferably 5 to 30, and more preferably 5 to 18, unless otherwise indicated in the description.

In the description herein, the number of carbon atoms of the "unsubstituted alkyl group" is 1 to 50, preferably 1 to 20, and more preferably 1 to 6, unless otherwise indicated in the description.

In the description herein, the number of carbon atoms of the "unsubstituted alkenyl group" is 2 to 50, preferably 2 to 20, and more preferably 2 to 6, unless otherwise indicated in the description.

In the description herein, the number of carbon atoms of the "unsubstituted alkynyl group" is 2 to 50, preferably 2 to 20, and more preferably 2 to 6, unless otherwise indicated in the description.

In the description herein, the number of ring carbon atoms of the "unsubstituted cycloalkyl group" is 3 to 50, preferably 3 to 20, and more preferably 3 to 6, unless otherwise indicated in the description.

In the description herein, the number of ring carbon atoms of the "unsubstituted arylene group" is 6 to 50, preferably 6 to 30, and more preferably 6 to 18, unless otherwise indicated in the description.

In the description herein, the number of ring atoms of the "unsubstituted divalent heterocyclic group" is 5 to 50, preferably 5 to 30, and more preferably 5 to 18, unless otherwise indicated in the description.

In the description herein, the number of carbon atoms of the "unsubstituted alkylene group" is 1 to 50, preferably 1 to 20, and more preferably 1 to 6, unless otherwise indicated in the description.

Substituted or Unsubstituted Aryl Group

In the description herein, specific examples (set of specific examples G1) of the "substituted or unsubstituted aryl group" include the unsubstituted aryl groups (set of specific examples G1A) and the substituted aryl groups (set of specific examples G1B) shown below. (Herein, the unsubstituted aryl group means the case where the "substituted or unsubstituted aryl group" is an "unsubstituted aryl group", and the substituted aryl group means the case where the "substituted or unsubstituted aryl group" is a "substituted aryl group".) In the description herein, the simple expression "aryl group" encompasses both the "unsubstituted aryl group" and the "substituted aryl group".

The "substituted aryl group" means a group formed by substituting one or more hydrogen atom of the "unsubstituted aryl group" by a substituent. Examples of the "substituted aryl group" include groups formed by one or more hydrogen atom of each of the "unsubstituted aryl groups" in the set of specific examples G1A by a substituent, and the examples of the substituted aryl groups in the set of specific examples G1B. The examples of the "unsubstituted aryl group" and the examples of the "substituted aryl group" enumerated herein are mere examples, and the "substituted aryl group" in the description herein encompasses groups formed by substituting a hydrogen atom bonded to the carbon atom of the aryl group itself of each of the "substituted aryl groups" in the set of specific examples G1B by a substituent, and groups formed by substituting a hydrogen atom of the substituent of each of the "substituted aryl groups" in the set of specific examples G1B by a substituent.

Unsubstituted Aryl Group (Set of Specific Examples G1A):

a phenyl group,
a p-biphenyl group,
a m-biphenyl group,
an o-biphenyl group,
a p-terphenyl-4-yl group,
a p-terphenyl-3-yl group,
a p-terphenyl-2-yl group,
a m-terphenyl-4-yl group,
a m-terphenyl-3-yl group,
a m-terphenyl-2-yl group,
an o-terphenyl-4-yl group,
an o-terphenyl-3-yl group,
an o-terphenyl-2-yl group,
a 1-naphthyl group,
a 2-naphthyl group,
an anthryl group,
a benzanthryl group,

a phenanthryl group,
a benzophenanthryl group,
a phenarenyl group,
a pyrenyl group,
a chrysenyl group,
a benzochrysenyl group,
a triphenylenyl group,
a benzotriphenylenyl group,
a tetracenyl group,
a pentacenyl group,
a fluorenyl group,
a 9,9'-spirobifluorenyl group,
a benzofluorenyl group,
a dibenzofluorenyl group,
a fluoranthenyl group,
a benzofluoranthenyl group,
a perylenyl group, and
monovalent aryl groups derived by removing one hydrogen atom from each of the ring structures represented by the following general formulae (TEMP-1) to (TEMP-15) :

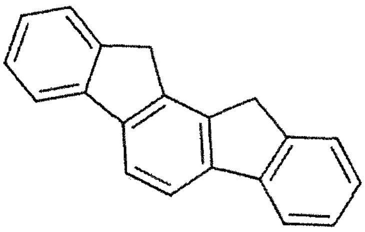
(TEMP-1)
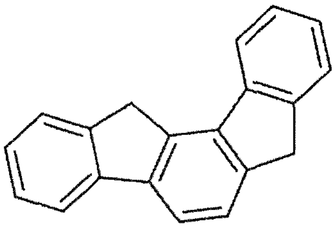
(TEMP-2)
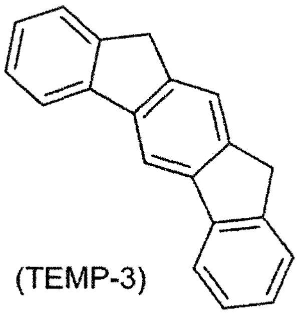
(TEMP-3)
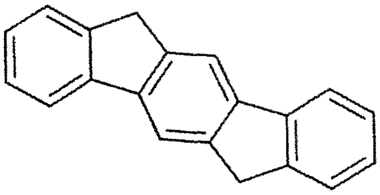
(TEMP-4)
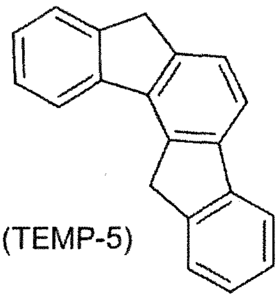
(TEMP-5)
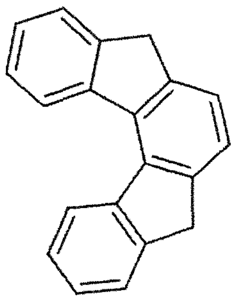
(TEMP-6)
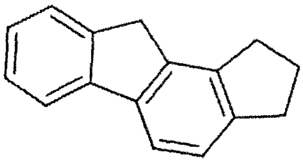
(TEMP-7)
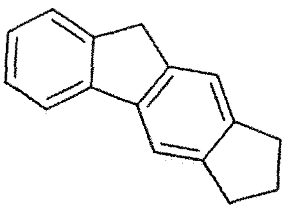
(TEMP-8)
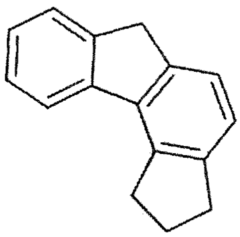
(TEMP-9)

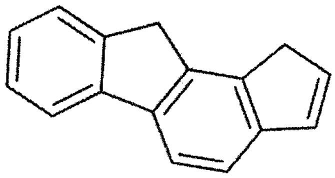

(TEMP-10)

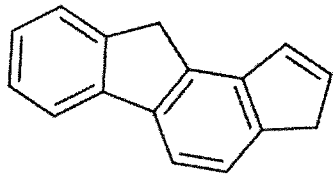

(TEMP-11)

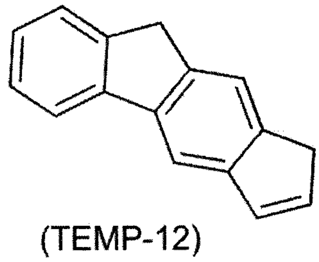

(TEMP-12)

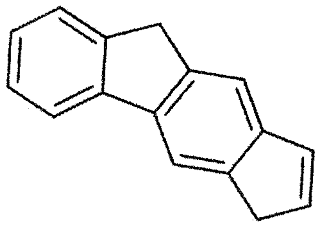

(TEMP-13)

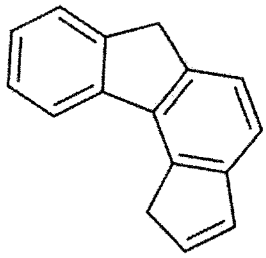

(TEMP-14)

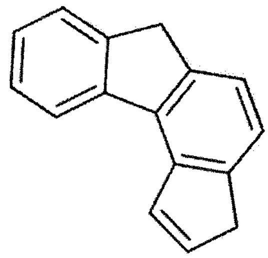

(TEMP-15)

Substituted Aryl Group (Set of Specific Examples G1B):

an o-tolyl group,
a m-tolyl group,
a p-tolyl group,
a p-xylyl group,
a m-xylyl group,
an o-xylyl group,
a p-isopropylphenyl group,
a m-isopropylphenyl group,
an o-isopropylphenyl group,
a p-t-butylphenyl group,
a m-t-butylphenyl group,
a o-t-butylphenyl group,
a 3,4,5-trimethylphenyl group,
a 9,9-dimethylfluorenyl group,
a 9,9-diphenylfluorenyl group,
a 9,9-bis(4-methylphenyl)fluorenyl group,
a 9,9-bis(4-isopropylphenyl)fluorenyl group,
a 9,9-bis(4-t-butylphenyl)fluorenyl group,
a cyanophenyl group,
a triphenylsilylphenyl group,
a trimethylsilylphenyl group,
a phenylnaphthyl group,
a naphthylphenyl group, and
groups formed by substituting one or more hydrogen atom of each of monovalent aryl groups derived from the ring structures represented by the general formulae (TEMP-1) to (TEMP-15) by a substituent.

Substituted or Unsubstituted Heterocyclic Group

In the description herein, the "heterocyclic group" means a cyclic group containing at least one hetero atom in the ring atoms. Specific examples of the hetero atom include a nitrogen atom, an oxygen atom, a sulfur atom, a silicon atom, a phosphorus atom, and a boron atom.
In the description herein, the "heterocyclic group" is a monocyclic group or a condensed ring group.
In the description herein, the "heterocyclic group" is an aromatic heterocyclic group or a non-aromatic heterocyclic group.

In the description herein, specific examples (set of specific examples G2) of the "substituted or unsubstituted heterocyclic group" include the unsubstituted heterocyclic groups (set of specific examples G2A) and the substituted heterocyclic groups (set of specific examples G2B) shown below. (Herein, the unsubstituted heterocyclic group means the case where the "substituted or unsubstituted heterocyclic group" is an "unsubstituted heterocyclic group", and the substituted heterocyclic group means the case where the "substituted or unsubstituted heterocyclic group" is a "substituted heterocyclic group".) In the description herein, the simple expression "heterocyclic group" encompasses both the "unsubstituted heterocyclic group" and the "substituted heterocyclic group".

The "substituted heterocyclic group" means a group formed by substituting one or more hydrogen atom of the "unsubstituted heterocyclic group" by a substituent. Specific examples of the "substituted heterocyclic group" include groups formed by substituting a hydrogen atom of each of the "unsubstituted heterocyclic groups" in the set of specific examples G2A by a substituent, and the examples of the substituted heterocyclic groups in the set of specific examples G2B. The examples of the "unsubstituted heterocyclic group" and the examples of the "substituted heterocyclic group" enumerated herein are mere examples, and the "substituted heterocyclic group" in the description herein encompasses groups formed by substituting a hydrogen atom bonded to the ring atom of the heterocyclic group itself of each of the "substituted heterocyclic groups" in the set of specific examples G2B by a substituent, and groups formed by substituting a hydrogen atom of the substituent of each of the "substituted heterocyclic groups" in the set of specific examples G2B by a substituent.

The set of specific examples G2A includes, for example, the unsubstituted heterocyclic group containing a nitrogen atom (set of specific examples G2A1), the unsubstituted heterocyclic group containing an oxygen atom (set of specific examples G2A2), the unsubstituted heterocyclic group containing a sulfur atom (set of specific examples G2A3), and monovalent heterocyclic groups derived by removing one hydrogen atom from each of the ring structures represented by the following general formulae (TEMP-16) to (TEMP-33) (set of specific examples G2A4).

The set of specific examples G2B includes, for example, the substituted heterocyclic groups containing a nitrogen atom (set of specific examples G2B1), the substituted heterocyclic groups containing an oxygen atom (set of specific examples G2B2), the substituted heterocyclic groups containing a sulfur atom (set of specific examples G2B3), and groups formed by substituting one or more hydrogen atom of each of monovalent heterocyclic groups derived from the ring structures represented by the following general formulae (TEMP-16) to (TEMP-33) by a substituent (set of specific examples G2B4).

Unsubstituted Heterocyclic Group containing Nitrogen Atom (Set of Specific Examples G2A1):

a pyrrolyl group,
an imidazolyl group,
a pyrazolyl group,
a triazolyl group,
a tetrazolyl group,
an oxazolyl group,
an isoxazolyl group,
an oxadiazolyl group,
a thiazolyl group,
an isothiazolyl group,
a thiadiazolyl group,
a pyridyl group,
a pyridazinyl group,
a pyrimidinyl group,
a pyrazinyl group,
a triazinyl group,
an indolyl group,
an isoindolyl group,
an indolizinyl group,
a quinolizinyl group,
a quinolyl group,
an isoquinolyl group,
a cinnolinyl group,
a phthalazinyl group,
a quinazolinyl group,
a quinoxalinyl group,
a benzimidazolyl group,

an indazolyl group,
a phenanthrolinyl group,
a phenanthridinyl group,
an acridinyl group,
a phenazinyl group,
a carbazolyl group,
a benzocarbazolyl group,
a morpholino group,
a phenoxazinyl group,
a phenothiazinyl group,
an azacarbazolyl group, and
a diazacarbazolyl group.

Unsubstituted Heterocyclic Group containing Oxygen Atom (Set of Specific Examples G2A2):

a furyl group,
an oxazolyl group,
an isoxazolyl group,
an oxadiazolyl group,
a xanthenyl group,
a benzofuranyl group,
an isobenzofuranyl group,
a dibenzofuranyl group,
a naphthobenzofuranyl group,
a benzoxazolyl group,
a benzisoxazolyl group,
a phenoxazinyl group,
a morpholino group,
a dinaphthofuranyl group,
an azadibenzofuranyl group,
a diazadibenzofuranyl group,
an azanaphthobenzofuranyl group, and
a diazanaphthobenzofuranyl group.

Unsubstituted Heterocyclic Group containing Sulfur Atom (Set of Specific Examples G2A3):

a thienyl group,
a thiazolyl group,
an isothiazolyl group,
a thiadiazolyl group,
a benzothiophenyl group (benzothienyl group),
an isobenzothiophenyl group (isobenzothienyl group),
a dibenzothiophenyl group (dibenzothienyl group),
a naphthobenzothiophenyl group (naphthobenzothienyl group),
a benzothiazolyl group,
a benzisothiazolyl group,
a phenothiazinyl group,
a dinaphthothiophenyl group (dinaphthothienyl group),
an azadibenzothiophenyl group (azadibenzothienyl group),
a diazadibenzothiophenyl group (diazadibenzothienyl group),
an azanaphthobenzothiophenyl group (azanaphthobenzothienyl group), and
a diazanaphthobenzothiophenyl group (diazanaphthobenzothienyl group).

Monovalent Heterocyclic Group derived by removing One Hydrogen Atom from Ring Structures represented by General Formulae (TEMP-16) to (TEMP-33) (Set of Specific Examples G2A4)

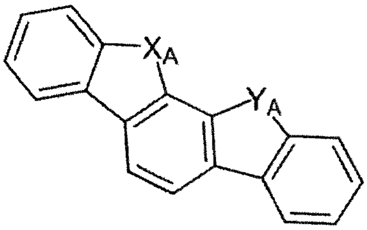

(TEMP-16)

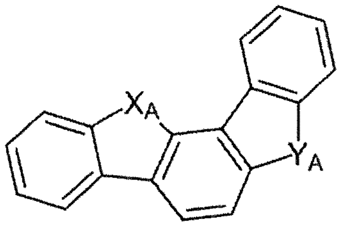

(TEMP-17)

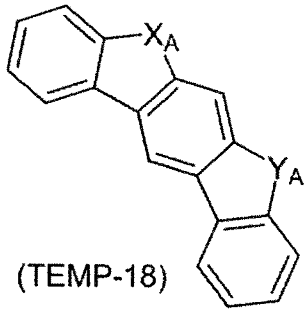

(TEMP-18)

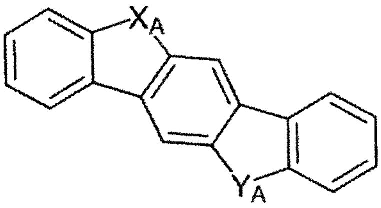

(TEMP-19)

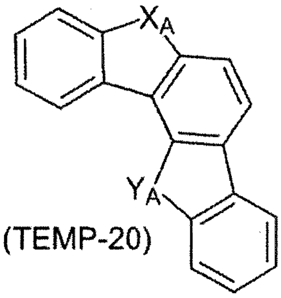

(TEMP-20)

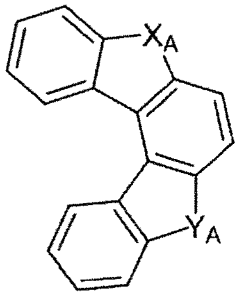

(TEMP-21)

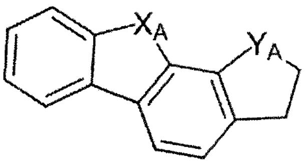

(TEMP-22)

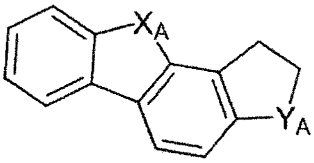

(TEMP-23)

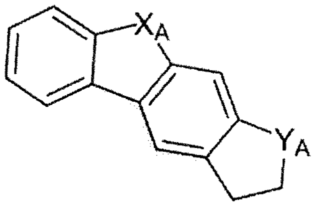

(TEMP-24)

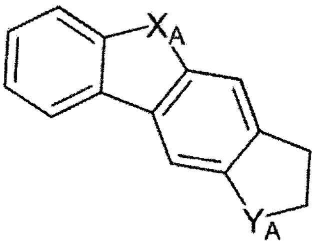

(TEMP-25)

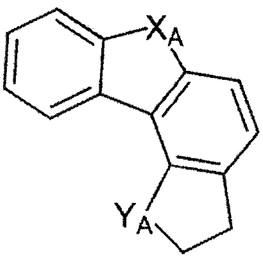

(TEMP-26)

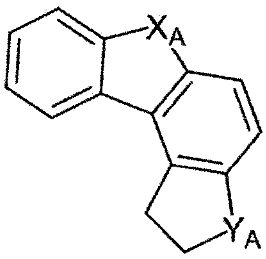

(TEMP-27)

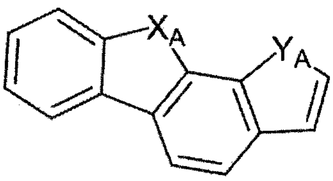

(TEMP-28)

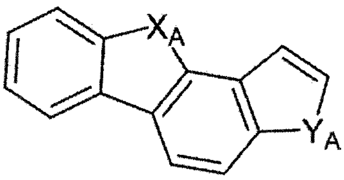

(TEMP-29)

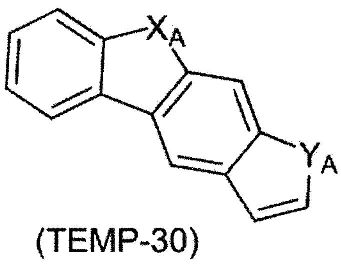

(TEMP-30)

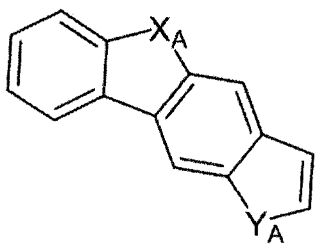

(TEMP-31)

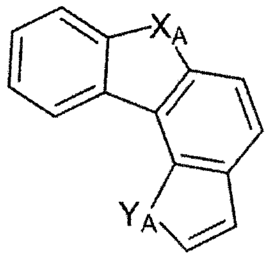

(TEMP-32)

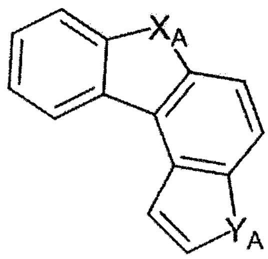

(TEMP-33)

In the general formulae (TEMP-16) to (TEMP-33), XA and YA each independently represent an oxygen atom, a sulfur atom, NH, or CH2, provided that at least one of XA and YA represents an oxygen atom, a sulfur atom, or NH.

In the general formulae (TEMP-16) to (TEMP-33), in the case where at least one of XA and YA represents NH or CH2, the monovalent heterocyclic groups derived from the ring structures represented by the general formulae (TEMP-16) to (TEMP-33) include monovalent groups formed by removing one hydrogen atom from the NH or CH2.

Substituted Heterocyclic Group containing Nitrogen Atom (Set of Specific Examples G2B1):

a (9-phenyl)carbazolyl group,
a (9-biphenylyl)carbazolyl group,
a (9-phenyl)phenylcarbazolyl group,
a (9-naphthyl)carbazolyl group,
a diphenylcarbazol-9-yl group,
a phenylcarbazol-9-yl group,
a methylbenzimidazolyl group,
an ethylbenzimidazolyl group,
a phenyltriazinyl group,
a biphenyltriazinyl group,
a diphenyltriazinyl group,
a phenylquinazolinyl group, and
a biphenylquinazolinyl group.

Substituted Heterocyclic Group containing Oxygen Atom (Set of Specific Examples G2B2):

a phenyldibenzofuranyl group,
a methyldibenzofuranyl group,
a t-butyldibenzofuranyl group, and
a monovalent residual group of spiro[9H-xanthene-9,9'-[9H]fluorene].

Substituted Heterocyclic Group containing Sulfur Atom (Set of Specific Examples G2B3):

a phenyldibenzothiophenyl group,
a methyldibenzothiophenyl group,
a t-butyldibenzothiophenyl group, and
a monovalent residual group of spiro[9H-thioxanthene-9,9'-[9H]fluorene].

Group formed by substituting one or more Hydrogen Atom of Monovalent Heterocyclic Group derived from Ring Structures represented by General Formulae (TEMP-16) to (TEMP-33) by Substituent (Set of Specific Examples G2B4)

The "one or more hydrogen atom of the monovalent heterocyclic group" means one or more hydrogen atom selected from the hydrogen atom bonded to the ring carbon atom of the monovalent heterocyclic group, the hydrogen atom bonded to the nitrogen atom in the case where at least one of XA and YA represents NH, and the hydrogen atom of the methylene group in the case where one of XA and YA represents CH2.

Substituted or Unsubstituted Alkyl Group

In the description herein, specific examples (set of specific examples G3) of the "substituted or unsubstituted

alkyl group" include the unsubstituted alkyl groups (set of specific examples G3A) and the substituted alkyl groups (set of specific examples G3B) shown below. (Herein, the unsubstituted alkyl group means the case where the "substituted or unsubstituted alkyl group" is an "unsubstituted alkyl group", and the substituted alkyl group means the case where the "substituted or unsubstituted alkyl group" is a "substituted alkyl group".) In the description herein, the simple expression "alkyl group" encompasses both the "unsubstituted alkyl group" and the "substituted alkyl group".

The "substituted alkyl group" means a group formed by substituting one or more hydrogen atom of the "unsubstituted alkyl group" by a substituent. Specific examples of the "substituted alkyl group" include groups formed by substituting one or more hydrogen atom of each of the "unsubstituted alkyl groups" (set of specific examples G3A) by a substituent, and the examples of the substituted alkyl groups (set of specific examples G3B). In the description herein, the alkyl group in the "unsubstituted alkyl group" means a chain-like alkyl group. Accordingly, the "unsubstituted alkyl group" encompasses an "unsubstituted linear alkyl group" and an "unsubstituted branched alkyl group". The examples of the "unsubstituted alkyl group" and the examples of the "substituted alkyl group" enumerated herein are mere examples, and the "substituted alkyl group" in the description herein encompasses groups formed by substituting a hydrogen atom of the alkyl group itself of each of the "substituted alkyl groups" in the set of specific examples G3B by a substituent, and groups formed by substituting a hydrogen atom of the substituent of each of the "substituted alkyl groups" in the set of specific examples G3B by a substituent.

Unsubstituted Alkyl Group (Set of Specific Examples G3A):

a methyl group,
an ethyl group,
a n-propyl group,
an isopropyl group,
a n-butyl group,
an isobutyl group,
a s-butyl group, and
a t-butyl group.

Substituted Alkyl Group (Set of Specific Examples G3B):

a heptafluoropropyl group (including isomers),
a pentafluoroethyl group,
a 2,2,2-trifluoroethyl group, and
a trifluoromethyl group.

Substituted or Unsubstituted Alkenyl Group

In the description herein, specific examples (set of specific examples G4) of the "substituted or unsubstituted alkenyl group" include the unsubstituted alkenyl groups (set of specific examples G4A) and the substituted alkenyl groups (set of specific examples G4B) shown below. (Herein, the unsubstituted alkenyl group means the case where the "substituted or unsubstituted alkenyl group" is an "unsubstituted alkenyl group", and the substituted alkenyl group means the case where the "substituted or unsubstituted alkenyl group" is a "substituted alkenyl group".) In the description herein, the simple expression "alkenyl group" encompasses both the "unsubstituted alkenyl group" and the "substituted alkenyl group".

The "substituted alkenyl group" means a group formed by substituting one or more hydrogen atom of the "unsubstituted alkenyl group" by a substituent. Specific examples of the "substituted alkenyl group" include the "unsubstituted alkenyl groups" (set of specific examples G4A) that each have a substituent, and the examples of the substituted alkenyl groups (set of specific examples G4B). The examples of the "unsubstituted alkenyl group" and the examples of the "substituted alkenyl group" enumerated herein are mere examples, and the "substituted alkenyl group" in the description herein encompasses groups formed by substituting a hydrogen atom of the alkenyl group itself of each of the "substituted alkenyl groups" in the set of specific examples G4B by a substituent, and groups formed by substituting a hydrogen atom of the substituent of each of the "substituted alkenyl groups" in the set of specific examples G4B by a substituent.

Unsubstituted Alkenyl Group (Set of Specific Examples G4A):

a vinyl group,
an allyl group,
a 1-butenyl group,
a 2-butenyl group, and

a 3-butenyl group.

Substituted Alkenyl Group (Set of Specific Examples G4B):

a 1,3-butanedienyl group,
a 1-methylvinyl group,
a 1-methylallyl group,
a 1,1-dimethylallyl group,
a 2-methylallyl group, and
a 1,2-dimethylallyl group.

Substituted or Unsubstituted Alkynyl Group

In the description herein, specific examples (set of specific examples G5) of the "substituted or unsubstituted alkynyl group" include the unsubstituted alkynyl group (set of specific examples G5A) shown below. (Herein, the unsubstituted alkynyl group means the case where the "substituted or unsubstituted alkynyl group" is an "unsubstituted alkynyl group".) In the description herein, the simple expression "alkynyl group" encompasses both the "unsubstituted alkynyl group" and the "substituted alkynyl group".

The "substituted alkynyl group" means a group formed by substituting one or more hydrogen atom of the "unsubstituted alkynyl group" by a substituent. Specific examples of the "substituted alkenyl group" include groups formed by substituting one or more hydrogen atom of the "unsubstituted alkynyl group" (set of specific examples G5A) by a substituent.

Unsubstituted Alkynyl Group (Set of Specific Examples G5A):
an ethynyl group.

Substituted or Unsubstituted Cycloalkyl Group

In the description herein, specific examples (set of specific examples G6) of the "substituted or unsubstituted cycloalkyl group" include the unsubstituted cycloalkyl groups (set of specific examples G6A) and the substituted cycloalkyl group (set of specific examples G6B) shown below. (Herein, the unsubstituted cycloalkyl group means the case where the "substituted or unsubstituted cycloalkyl group" is an "unsubstituted cycloalkyl group", and the substituted cycloalkyl group means the case where the "substituted or unsubstituted cycloalkyl group" is a "substituted cycloalkyl group".) In the description herein, the simple expression "cycloalkyl group" encompasses both the "unsubstituted cycloalkyl group" and the "substituted cycloalkyl group".

The "substituted cycloalkyl group" means a group formed by substituting one or more hydrogen atom of the "unsubstituted cycloalkyl group" by a substituent. Specific examples of the "substituted cycloalkyl group" include groups formed by substituting one or more hydrogen atom of each of the "unsubstituted cycloalkyl groups" (set of specific examples G6A) by a substituent, and the example of the substituted cycloalkyl group (set of specific examples G6B). The examples of the "unsubstituted cycloalkyl group" and the examples of the "substituted cycloalkyl group" enumerated herein are mere examples, and the "substituted cycloalkyl group" in the description herein encompasses groups formed by substituting one or more hydrogen atom bonded to the carbon atoms of the cycloalkyl group itself of the "substituted cycloalkyl group" in the set of specific examples G6B by a substituent, and groups formed by substituting a hydrogen atom of the substituent of the "substituted cycloalkyl group" in the set of specific examples G6B by a substituent.

Unsubstituted Cycloalkyl Group (Set of Specific Examples G6A):

a cyclopropyl group,
a cyclobutyl group,
a cyclopentyl group,
a cyclohexyl group,
a 1-adamantyl group,
a 2-adamantyl group,
a 1-norbornyl group, and
a 2-norbornyl group.

Substituted Cycloalkyl Group (Set of Specific Examples G6B):
a 4-methylcyclohexyl group.

Group represented by -Si(R901)(R902)(R903)

In the description herein, specific examples (set of specific examples G7) of the group represented by -Si(R901)(R902)(R903) include:

-Si(G1)(G1)(G1),
-Si(G1)(G2)(G2),
-Si(G1)(G1)(G2),
-Si(G2)(G2)(G2),
-Si(G3)(G3)(G3), and
-Si(G6)(G6)(G6).

Herein,

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Plural groups represented by G1 in -Si(G1)(G1)(G1) are the same as or different from each other.
Plural groups represented by G2 in -Si(G1)(G2)(G2) are the same as or different from each other.
Plural groups represented by G1 in -Si(G1)(G1)(G2) are the same as or different from each other.
Plural groups represented by G2 in -Si(G2)(G2)(G2) are the same as or different from each other.
Plural groups represented by G3 in -Si(G3)(G3)(G3) are the same as or different from each other.
Plural groups represented by G6 in -Si(G6)(G6)(G6) are the same as or different from each other.

Group represented by -O-(R904)

In the description herein, specific examples (set of specific examples G8) of the group represented by -O-(R904) include:

-O(G1),
-O(G2),
-O(G3), and
-O(G6).

Herein,

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Group represented by -S-(R905)

In the description herein, specific examples (set of specific examples G9) of the group represented by -S-(R905) include:

-S(G1),
-S(G2),
-S(G3), and
-S(G6).

Herein,

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and

G6 represents the "substituted or unsubstituted cycloalkyl group" described in the set of specific examples G6.

Group represented by -N(R906)(R907)

In the description herein, specific examples (set of specific examples G10) of the group represented by -N(R906)(R907) include:

-N(G1)(G1),
-N(G2)(G2),
-N(G1)(G2),
-N(G3)(G3), and
-N(G6)(G6).

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Plural groups represented by G1 in -N(G1)(G1) are the same as or different from each other.
Plural groups represented by G2 in -N(G2)(G2) are the same as or different from each other.
Plural groups represented by G3 in -N(G3)(G3) are the same as or different from each other.
Plural groups represented by G6 in -N(G6)(G6) are the same as or different from each other.

Halogen Atom

In the description herein, specific examples (set of specific examples G11) of the "halogen atom" include a fluorine atom, a chlorine atom, a bromine atom, and an iodine atom.

Substituted or Unsubstituted Fluoroalkyl Group

In the description herein, the "substituted or unsubstituted fluoroalkyl group" means a group formed by substituting at least one hydrogen atom bonded to the carbon atom constituting the alkyl group in the "substituted or unsubstituted alkyl group" by a fluorine atom, and encompasses a group formed by substituting all the hydrogen atoms bonded to the carbon atoms constituting the alkyl group in the "substituted or unsubstituted alkyl group" by fluorine atoms (i.e., a perfluoroalkyl group). The number of carbon atoms of the "unsubstituted fluoroalkyl group" is 1 to 50, preferably 1 to 30, and more preferably 1 to 18, unless otherwise indicated in the description. The "substituted fluoroalkyl group" means a group formed by substituting one or more hydrogen atom of the "fluoroalkyl group" by a substituent. In the description herein, the "substituted fluoroalkyl group" encompasses a group formed by substituting one or more hydrogen atom bonded to the carbon atom of the alkyl chain in the "substituted fluoroalkyl group" by a substituent, and a group formed by substituting one or more hydrogen atom of the substituent in the "substituted fluoroalkyl group" by a substituent. Specific examples of the "unsubstituted fluoroalkyl group" include examples of groups formed by substituting one or more hydrogen atom in each of the "alkyl group" (set of specific examples G3) by a fluorine atom.

Substituted or Unsubstituted Haloalkyl Group

In the description herein, the "substituted or unsubstituted haloalkyl group" means a group formed by substituting at least one hydrogen atom bonded to the carbon atom constituting the alkyl group in the "substituted or unsubstituted alkyl group" by a halogen atom, and encompasses a group formed by substituting all the hydrogen atoms bonded to the carbon atoms constituting the alkyl group in the "substituted or unsubstituted alkyl group" by halogen atoms. The number of carbon atoms of the "unsubstituted haloalkyl group" is 1 to 50, preferably 1 to 30, and more preferably 1 to 18, unless otherwise indicated in the description. The "substituted haloalkyl group" means a group formed by substituting one or more hydrogen atom of the "haloalkyl group" by a substituent. In the description herein, the "substituted haloalkyl group" encompasses a group formed by substituting one or more hydrogen atom bonded to the carbon atom of the alkyl chain in the "substituted haloalkyl group" by a substituent, and a group formed by substituting one or more hydrogen atom of the substituent in the "substituted haloalkyl group" by a substituent. Specific examples of the "unsubstituted haloalkyl group" include examples of groups formed by substituting one or more hydrogen atom in each of the "alkyl group" (set of specific examples G3) by a halogen atom. A haloalkyl group may be referred to as a halogenated alkyl group in some cases.

Substituted or Unsubstituted Alkoxy Group

In the description herein, specific examples of the "substituted or unsubstituted alkoxy group" include a group represented by -O(G3), wherein G3 represents the "substituted or unsubstituted alkyl group" described in the set of specific examples G3. The number of carbon atoms of the "unsubstituted alkoxy group" is 1 to 50, preferably 1 to 30, and more preferably 1 to 18, unless otherwise indicated in the description.

Substituted or Unsubstituted Alkylthio Group

In the description herein, specific examples of the "substituted or unsubstituted alkylthio group" include a group represented by -S(G3), wherein G3 represents the "substituted or unsubstituted alkyl group" described in the set of specific examples G3. The number of carbon atoms of the "unsubstituted alkylthio group" is 1 to 50, preferably 1 to 30, and more preferably 1 to 18, unless otherwise indicated in the description.

Substituted or Unsubstituted Aryloxy Group

In the description herein, specific examples of the "substituted or unsubstituted aryloxy group" include a group represented by -O(G1), wherein G1 represents the "substituted or unsubstituted aryl group" described in the set of specific examples G1. The number of ring carbon atoms of the "unsubstituted aryloxy group" is 6 to 50, preferably 6 to 30, and more preferably 6 to 18, unless otherwise indicated in the description.

Substituted or Unsubstituted Arylthio Group

In the description herein, specific examples of the "substituted or unsubstituted arylthio group" include a group represented by -S(G1), wherein G1 represents the "substituted or unsubstituted aryl group" described in the set of specific examples G1. The number of ring carbon atoms of the "unsubstituted arylthio group" is 6 to 50, preferably 6 to 30, and more preferably 6 to 18, unless otherwise indicated in the description.

Substituted or Unsubstituted Trialkylsilyl Group

In the description herein, specific examples of the "trialkylsilyl group" include a group represented by -Si(G3)(G3)(G3), wherein G3 represents the "substituted or unsubstituted alkyl group" described in the set of specific examples G3. Plural groups represented by G3 in -Si(G3)(G3)(G3) are the same as or different from each other. The number of carbon atoms of each of alkyl groups of the "substituted or unsubstituted trialkylsilyl group" is 1 to 50, preferably 1 to 20, and more preferably 1 to 6, unless otherwise indicated in the description.

Substituted or Unsubstituted Aralkyl Group

In the description herein, specific examples of the "substituted or unsubstituted aralkyl group" include a group represented by -(G3)-(G1), wherein G3 represents the "substituted or unsubstituted alkyl group" described in the set of specific examples G3, and G1 represents the "substituted or unsubstituted aryl group" described in the set of specific examples G1. Accordingly, the "aralkyl group" is a group formed by substituting a hydrogen atom of an "alkyl group" by an "aryl group" as a substituent, and is one embodiment of the "substituted alkyl group". The "unsubstituted aralkyl group" is an "unsubstituted alkyl group" that is substituted by an "unsubstituted aryl group", and the number of carbon atoms of the "unsubstituted aralkyl group" is 7 to 50, preferably 7 to 30, and more preferably 7 to 18, unless otherwise indicated in the description.

Specific examples of the "substituted or unsubstituted aralkyl group" include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, and a 2-β-naphthylisopropyl group.

In the description herein, the substituted or unsubstituted aryl group is preferably a phenyl group, a p-biphenyl group, a m-biphenyl group, an o-biphenyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group, a 9,9-diphenylfluorenyl group, and the like, unless otherwise indicated in the description.

In the description herein, the substituted or unsubstituted heterocyclic group is preferably a pyridyl group, a

pyrimidinyl group, a triazinyl group, a quinolyl group, an isoquinolyl group, a quinazolinyl group, a benzimidazolyl group, a phenanthrolinyl group, a carbazolyl group (e.g., a 1-carbazolyl, group, a 2-carbazolyl, group, a 3-carbazolyl, group, a 4-carbazolyl, group, or a 9-carbazolyl, group), a benzocarbazolyl group, an azacarbazolyl group, a diazacarbazolyl group, a dibenzofuranyl group, a naphthobenzofuranly group, an azadibenzofuranyl group, a diazadibenzofuranyl group, a dibenzothiophenyl group, a naphthobenzothiophenyl group, an azadibenzothiophenyl group, a diazadibenzothiophenyl group, a (9-phenyl)carbazolyl group (e.g., a (9-phenyl)carbazol-1-yl group, a (9-phenyl)carbazol-2-yl group, a (9-phenyl)carbazol-3-yl group, or a (9-phenyl)carbazol-4-yl group), a (9-biphenylyl)carbazolyl group, a (9-phenyl)phenylcarbazolyl group, a diphenylcarbazol-9-yl group, a phenylcarbazol-9-yl group, a phenyltriazinyl group, a biphenylyltriazinyl group, a diphenyltriazinyl group, a phenyldibenzofuranyl group, a phenyldibenzothiophenyl group, and the like, unless otherwise indicated in the description.

In the description herein, the carbazolyl group is specifically any one of the following groups unless otherwise indicated in the description.

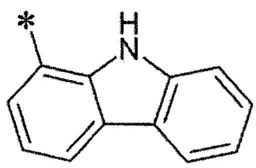
(TEMP-Cz1)

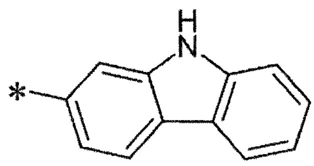
(TEMP-Cz2)

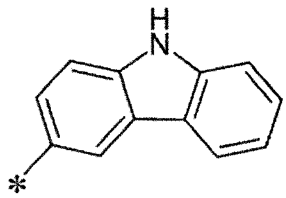
(TEMP-Cz3)

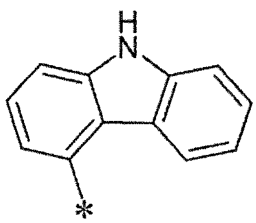
(TEMP-Cz4)

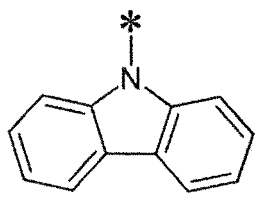
(TEMP-Cz5)

In the description herein, the (9-phenyl)carbazolyl group is specifically any one of the following groups unless otherwise indicated in the description.

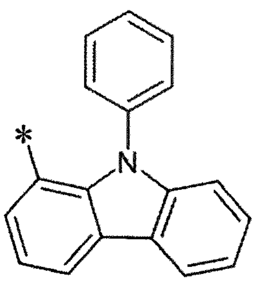
(TEMP-Cz6)

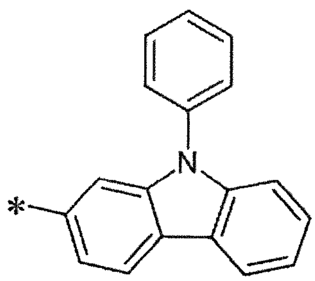
(TEMP-Cz7)

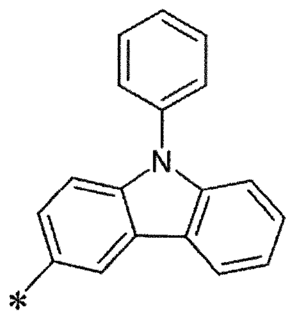
(TEMP-Cz8)

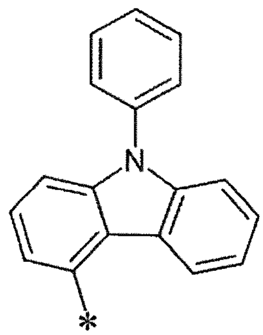
(TEMP-Cz9)

In the general formulae (TEMP-Cz1) to (TEMP-Cz9), * represents a bonding site.

In the description herein, the dibenzofuranyl group and the dibenzothiophenyl group are specifically any one of the following groups unless otherwise indicated in the description.

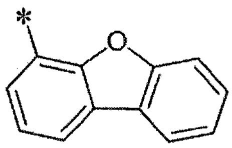 (TEMP-34) 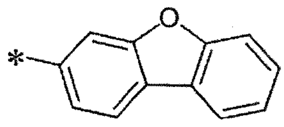 (TEMP-35) 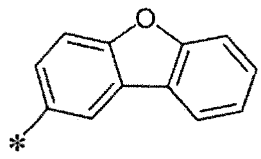 (TEMP-36) 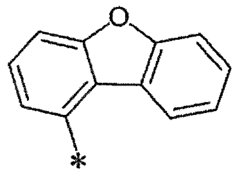 (TEMP-37)

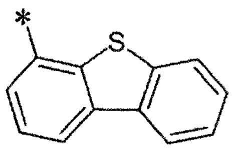 (TEMP-38) 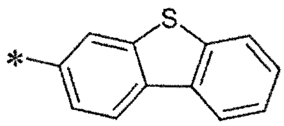 (TEMP-39) 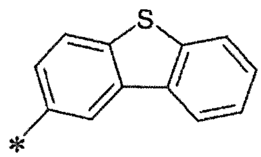 (TEMP-40) 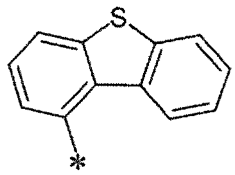 (TEMP-41)

In the general formulae (TEMP-34) to (TEMP-41), * represents a bonding site.

In the description herein, the substituted or unsubstituted alkyl group is preferably a methyl group, an ethyl group, a propyl group, an isopropyl group, a n-butyl group, an isobutyl group, a t-butyl group, or the like unless otherwise indicated in the description.

Substituted or Unsubstituted Arylene Group

In the description herein, the "substituted or unsubstituted arylene group" is a divalent group derived by removing one hydrogen atom on the aryl ring from the "substituted or unsubstituted aryl group" described above unless otherwise indicated in the description. Specific examples (set of specific examples G12) of the "substituted or unsubstituted arylene group" include divalent groups derived by removing one hydrogen atom on the aryl ring from the "substituted or unsubstituted aryl groups" described in the set of specific examples G1.

Substituted or Unsubstituted Divalent Heterocyclic Group

In the description herein, the "substituted or unsubstituted divalent heterocyclic group" is a divalent group derived by removing one hydrogen atom on the heterocyclic ring from the "substituted or unsubstituted heterocyclic group" described above unless otherwise indicated in the description. Specific examples (set of specific examples G13) of the "substituted or unsubstituted divalent heterocyclic group" include divalent groups derived by removing one hydrogen atom on the heterocyclic ring from the "substituted or unsubstituted heterocyclic groups" described in the set of specific examples G2.

Substituted or Unsubstituted Alkylene Group

In the description herein, the "substituted or unsubstituted alkylene group" is a divalent group derived by removing one hydrogen atom on the alkyl chain from the "substituted or unsubstituted alkyl group" described above unless otherwise indicated in the description. Specific examples (set of specific examples G14) of the "substituted or unsubstituted alkylene group" include divalent groups derived by removing one hydrogen atom on the alkyl chain from the "substituted or unsubstituted alkyl groups" described in the set of specific examples G3.

In the description herein, the substituted or unsubstituted arylene group is preferably any one of the groups represented by the following general formulae (TEMP-42) to (TEMP-68) unless otherwise indicated in the description.

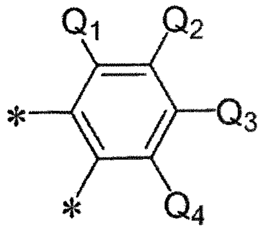

(TEMP-42)

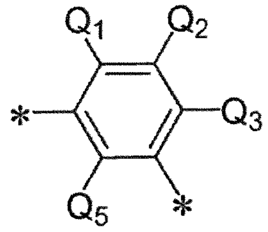

(TEMP-43)

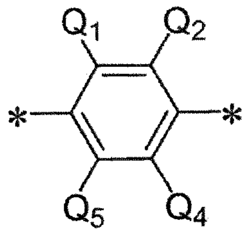

(TEMP-44)

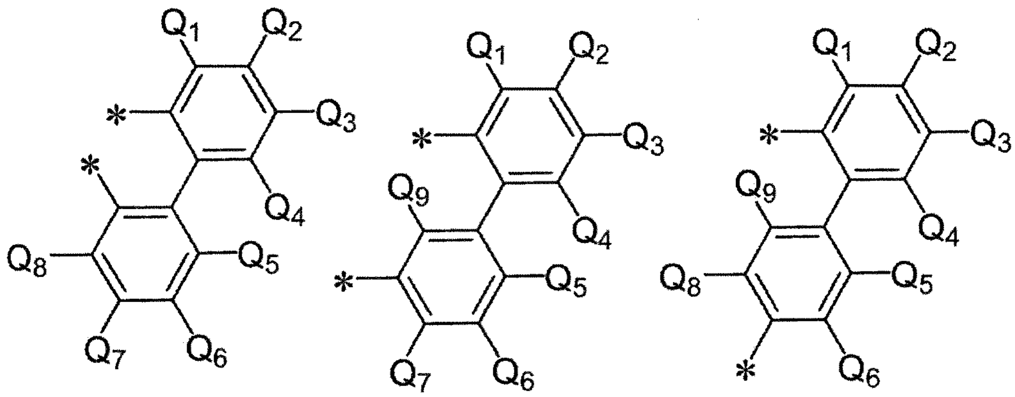

(TEMP-45) (TEMP-46) (TEMP-47)

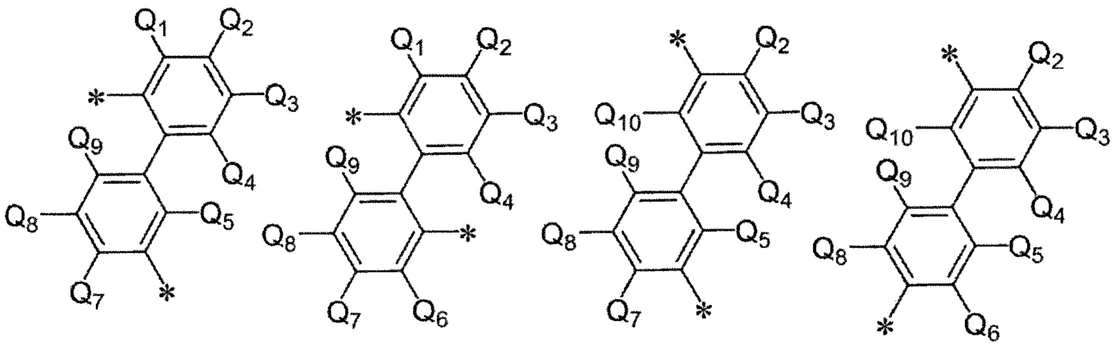

(TEMP-48) (TEMP-49) (TEMP-50) (TEMP-51)

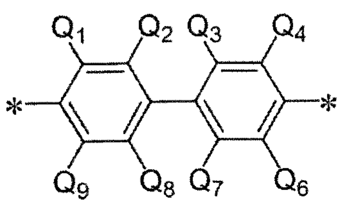

(TEMP-52)

In the general formulae (TEMP-42) to (TEMP-52), Q1 to Q10 each independently represent a hydrogen atom or a substituent.

In the general formulae (TEMP-42) to (TEMP-52), * represents a bonding site.

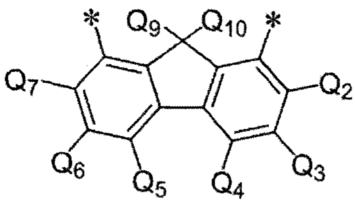

(TEMP-53)

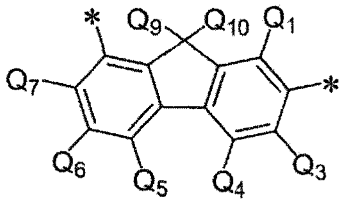

(TEMP-54)

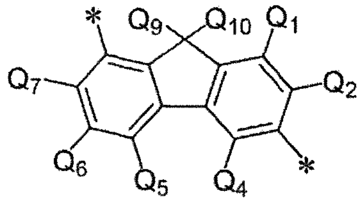

(TEMP-55)

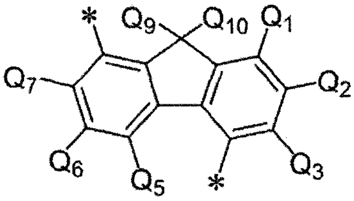

(TEMP-56)

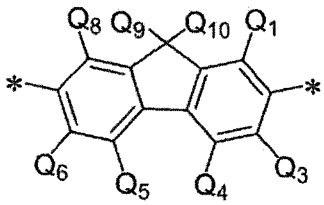

(TEMP-57)

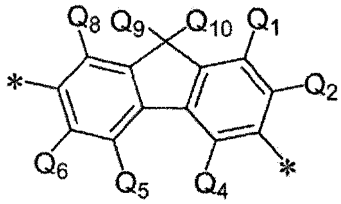

(TEMP-58)

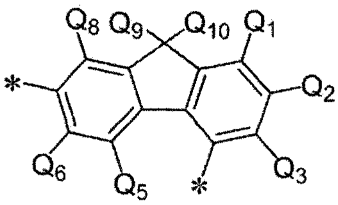

(TEMP-59)

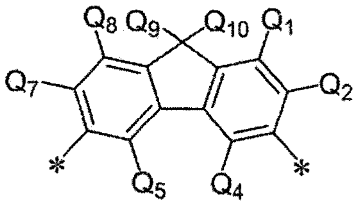

(TEMP-60)

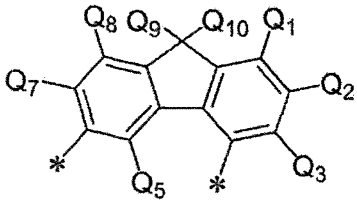

(TEMP-61)

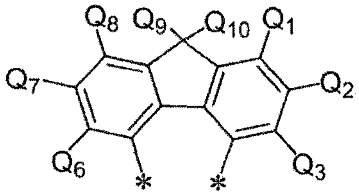

(TEMP-62)

In the general formulae (TEMP-53) to (TEMP-62), Q1 to Q10 each independently represent a hydrogen atom or a substituent.

The formulae Q9 and Q10 may be bonded to each other to form a ring via a single bond.

In the general formulae (TEMP-53) to (TEMP-62), * represents a bonding site.

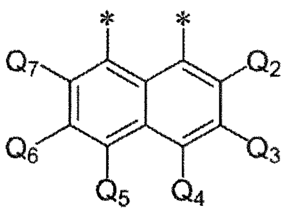

(TEMP-63)

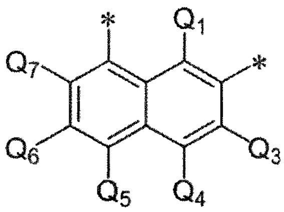

(TEMP-64)

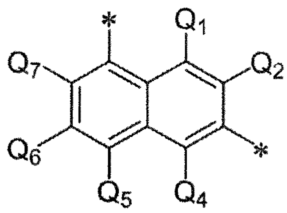

(TEMP-65)

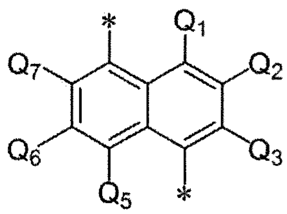
(TEMP-66)

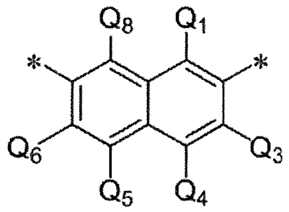
(TEMP-67)

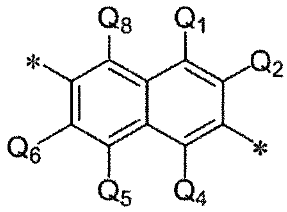
(TEMP-68)

In the general formulae (TEMP-63) to (TEMP-68), Q1 to Q8 each independently represent a hydrogen atom or a substituent.

In the general formulae (TEMP-63) to (TEMP-68), * represents a bonding site.

In the description herein, the substituted or unsubstituted divalent heterocyclic group is preferably the groups represented by the following general formulae (TEMP-69) to (TEMP-102) unless otherwise indicated in the description.

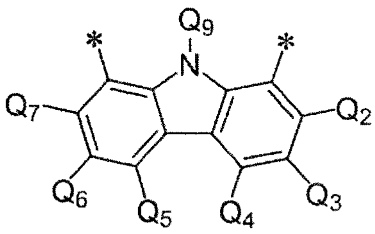
(TEMP-69)

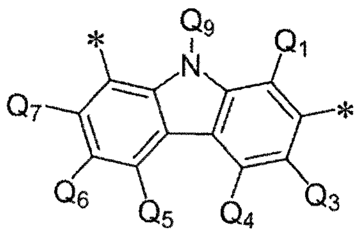
(TEMP-70)

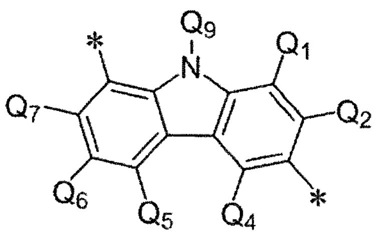
(TEMP-71)

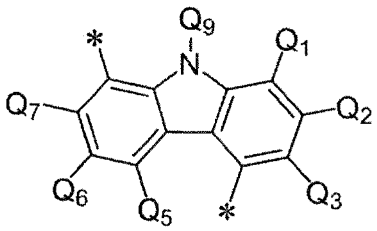
(TEMP-72)

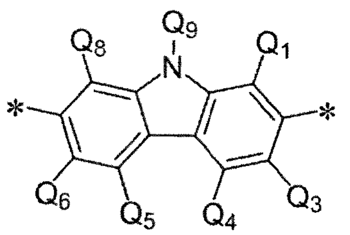
(TEMP-73)

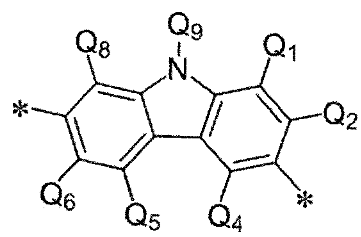
(TEMP-74)

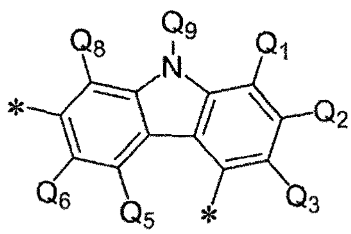
(TEMP-75)

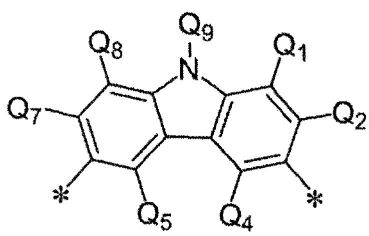
(TEMP-76)

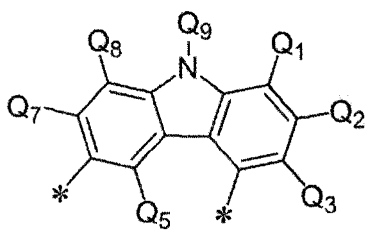
(TEMP-77)

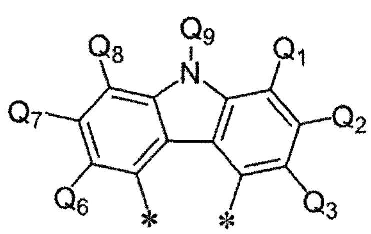
(TEMP-78)

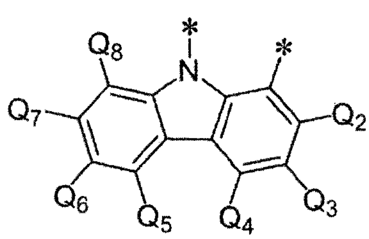
(TEMP-79)

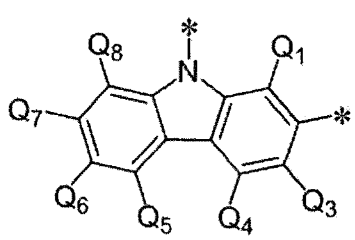
(TEMP-80)

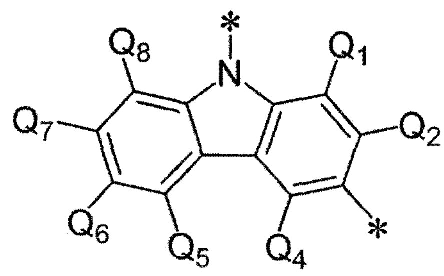

(TEMP-81)

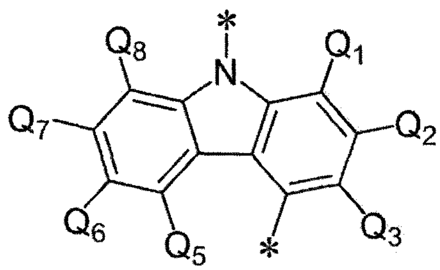

(TEMP-82)

In the general formulae (TEMP-69) to (TEMP-82), Q1 to Q9 each independently represent a hydrogen atom or a substituent.

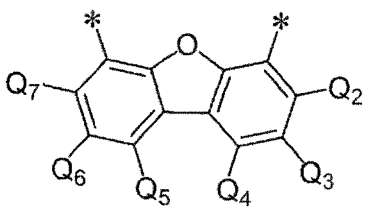

(TEMP-83)

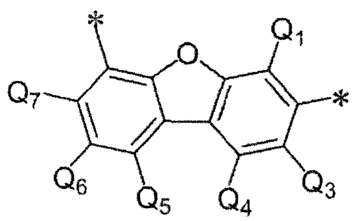

(TEMP-84)

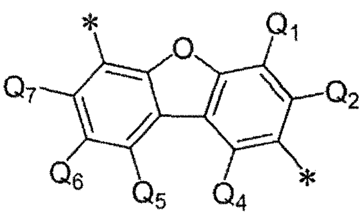

(TEMP-85)

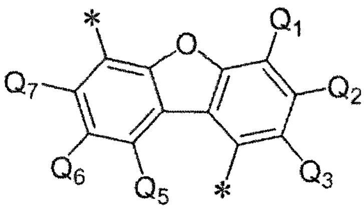

(TEMP-86)

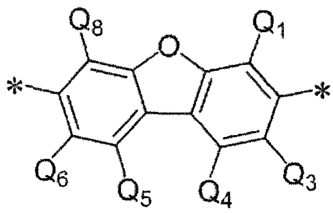

(TEMP-87)

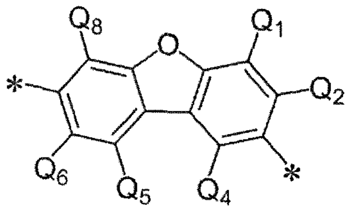

(TEMP-88)

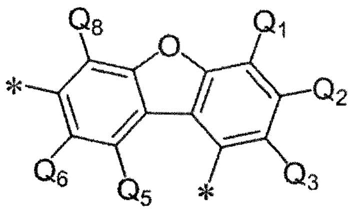

(TEMP-89)

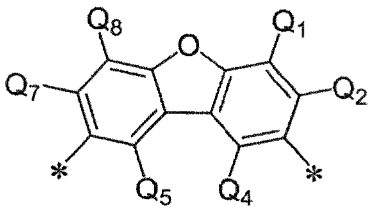

(TEMP-90)

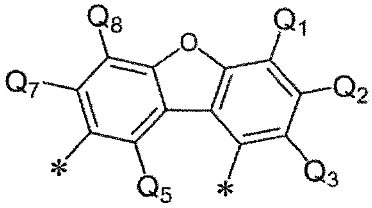

(TEMP-91)

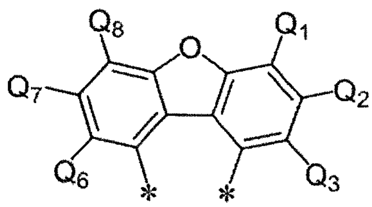

(TEMP-92)

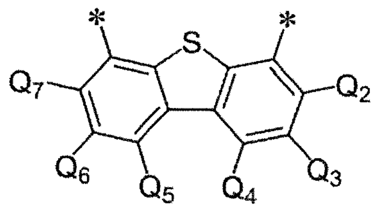

(TEMP-93)

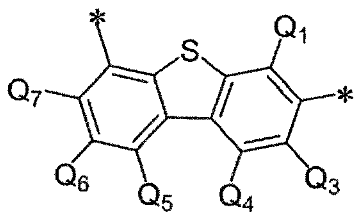

(TEMP-94)

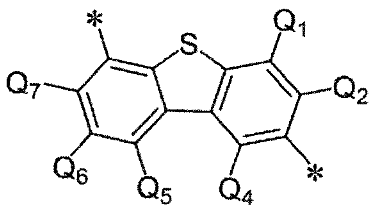

(TEMP-95)

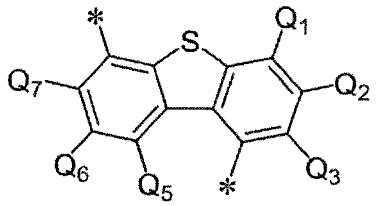

(TEMP-96)

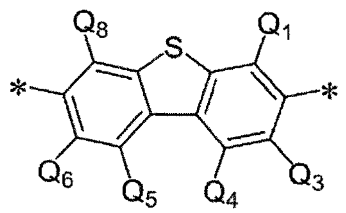

(TEMP-97)

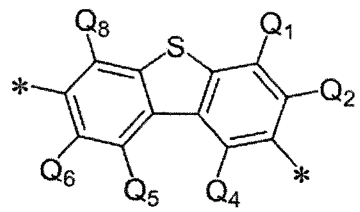

(TEMP-98)

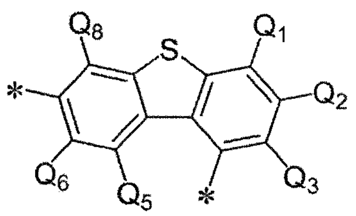

(TEMP-99)

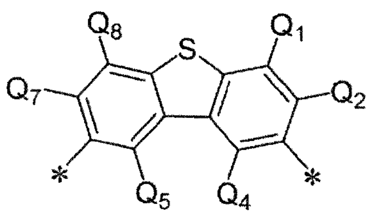

(TEMP-100)

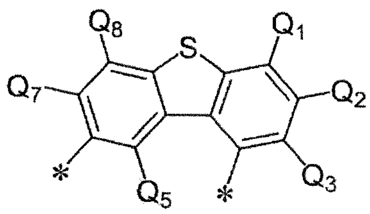

(TEMP-101)

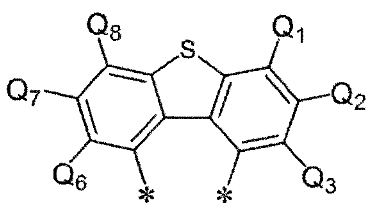

(TEMP-102)

In the general formulae (TEMP-83) to (TEMP-102), Q1 to Q8 each independently represent a hydrogen atom or a substituent.

The above are the explanation of the "substituents in the description herein".

Case forming Ring by bonding

In the description herein, the case where "one or more combinations of combinations each including adjacent two or more each are bonded to each other to form a substituted or unsubstituted monocyclic ring, or each are bonded to each other to form a substituted or unsubstituted condensed ring, or each are not bonded to each other" means a case where "one or more combinations of combinations each including adjacent two or more each are bonded to each other to form a substituted or unsubstituted monocyclic ring", a case where "one or more combinations of combinations each including adjacent two or more each are bonded to each other to form a substituted or unsubstituted condensed ring", and a case where "one or more combinations of combinations each including adjacent two or more each are not bonded to each other".

In the description herein, the case where "one or more combinations of combinations each including adjacent two or more each are bonded to each other to form a substituted or unsubstituted monocyclic ring" and the case where "one or more combinations of combinations each including adjacent two or more each are bonded to each other to form a substituted or unsubstituted condensed ring" (which may be hereinafter collectively referred to as a "case forming a

ring by bonding") will be explained below. The cases will be explained for the anthracene compound represented by the following general formula (TEMP-103) having an anthracene core skeleton as an example.

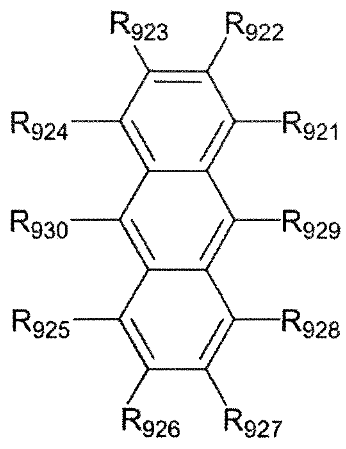

(TEMP-103)

For example, in the case where "one or more combinations of combinations each including adjacent two or more each are bonded to each other to form a ring" among R921 to R930, the combinations each including adjacent two as one combination include a combination of R921 and R922, a combination of R922 and R923, a combination of R923 and R924, a combination of R924 and R930, a combination of R930 and R925, a combination of R925 and R926, a combination of R926 and R927, a combination of R927 and R928, a combination of R928 and R929, and a combination of R929 and R921.

The "one or more combinations" mean that two or more combinations each including adjacent two or more may form rings simultaneously. For example, in the case where R921 and R922 are bonded to each other to form a ring QA, and simultaneously R925 and R926 are bonded to each other to form a ring QB, the anthracene compound represented by the general formula (TEMP-103) is represented by the following general formula (TEMP-104).

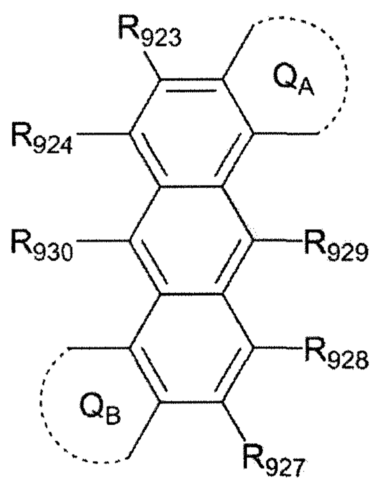

(TEMP-104)

The case where the "combination including adjacent two or more forms rings" encompasses not only the case where adjacent two included in the combination are bonded as in the aforementioned example, but also the case where adjacent three or more included in the combination are bonded. For example, this case means that R921 and R922 are bonded to each other to form a ring QA, R922 and R923 are bonded to each other to form a ring QC, and adjacent three (R921, R922, and R923) included in the combination are bonded to each other to form rings, which are condensed to the anthracene core skeleton, and in this case, the anthracene compound represented by the general formula (TEMP-103) is represented by the following general formula (TEMP-105). In the following general formula (TEMP-105), the ring QA and the ring QC share R922.

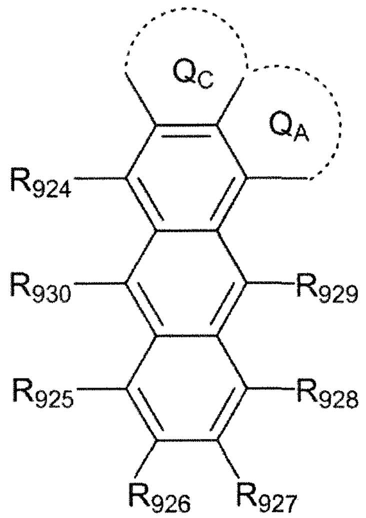

(TEMP-105)

The formed "monocyclic ring" or "condensed ring" may be a saturated ring or an unsaturated ring in terms of structure of the formed ring itself. In the case where the "one combination including adjacent two" forms a "monocyclic ring" or a "condensed ring", the "monocyclic ring" or the "condensed ring" may form a saturated ring or an unsaturated ring. For example, the ring QA and the ring QB formed in the general formula (TEMP-104) each are a "monocyclic ring" or a "condensed ring". The ring QA and the ring QC formed in the general formula (TEMP-105) each are a "condensed ring". The ring QA and the ring QC in the general formula (TEMP-105) form a condensed ring through condensation of the ring QA and the ring QC. In the case where the ring QA in the general formula (TMEP-104) is a benzene ring, the ring QAis a monocyclic ring. In the case where the ring QA in the general formula (TMEP-104) is a naphthalene ring, the ring QA is a condensed ring.

The "unsaturated ring" means an aromatic hydrocarbon ring or an aromatic heterocyclic ring. The "saturated ring" means an aliphatic hydrocarbon ring or a non-aromatic heterocyclic ring.

Specific examples of the aromatic hydrocarbon ring include the structures formed by terminating the groups exemplified as the specific examples in the set of specific examples G1 with a hydrogen atom.

Specific examples of the aromatic heterocyclic ring include the structures formed by terminating the aromatic heterocyclic groups exemplified as the specific examples in the set of specific examples G2 with a hydrogen atom.

Specific examples of the aliphatic hydrocarbon ring include the structures formed by terminating the groups exemplified as the specific examples in the set of specific examples G6 with a hydrogen atom.

The expression "to form a ring" means that the ring is formed only with the plural atoms of the core structure or with the plural atoms of the core structure and one or more arbitrary element. For example, the ring QA formed by bonding R921 and R922 each other shown in the general formula (TEMP-104) means a ring formed with the carbon atom of the anthracene skeleton bonded to R921, the carbon atom of the anthracene skeleton bonded to R922, and one or more arbitrary element. As a specific example, in the case where the ring QAis formed with R921 and R922, and in the case where a monocyclic unsaturated ring is formed with the carbon atom of the anthracene skeleton bonded to R921, the carbon atom of the anthracene skeleton bonded to R922, and four carbon atoms, the ring formed with R921 and R922 is a benzene ring.

Herein, the "arbitrary element" is preferably at least one kind of an element selected from the group consisting of a carbon element, a nitrogen element, an oxygen element, and a sulfur element, unless otherwise indicated in the description. For the arbitrary element (for example, for a carbon element or a nitrogen element), a bond that does not form a ring may be terminated with a hydrogen atom or the like, and may be substituted by an "arbitrary substituent" described later. In the case where an arbitrary element other than a carbon element is contained, the formed ring is a heterocyclic ring.

The number of the "one or more arbitrary element" constituting the monocyclic ring or the condensed ring is preferably 2 or more and 15 or less, more preferably 3 or more and 12 or less, and further preferably 3 or more and 5 or less, unless otherwise indicated in the description.

What is preferred between the "monocyclic ring" and the "condensed ring" is the "monocyclic ring" unless otherwise indicated in the description.

What is preferred between the "saturated ring" and the "unsaturated ring" is the "unsaturated ring" unless otherwise indicated in the description.

The "monocyclic ring" is preferably a benzene ring unless otherwise indicated in the description.

The "unsaturated ring" is preferably a benzene ring unless otherwise indicated in the description.

In the case where the "one or more combinations of combinations each including adjacent two or more" each are "bonded to each other to form a substituted or unsubstituted monocyclic ring", or each are "bonded to each other to form a substituted or unsubstituted condensed ring", it is preferred that the one or more combinations of combinations each including adjacent two or more each are bonded to each other to form a substituted or unsubstituted "unsaturated ring" containing the plural atoms of the core skeleton and 1 or more and 15 or less at least one kind of an element selected from the group consisting of a carbon element, a nitrogen element, an oxygen element, and a sulfur element, unless otherwise indicated in the description.

In the case where the "monocyclic ring" or the "condensed ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

In the case where the "saturated ring" or the "unsaturated ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

The above are the explanation of the case where "one or more combinations of combinations each including adjacent two or more" each are "bonded to each other to form a substituted or unsubstituted monocyclic ring", and the case where "one or more combinations of combinations each including adjacent two or more" each are "bonded to each other to form a substituted or unsubstituted condensed ring" (i.e., the "case forming a ring by bonding").

Substituent for "Substituted or Unsubstituted"

In one embodiment in the description herein, the substituent for the case of "substituted or unsubstituted" (which may be hereinafter referred to as an "arbitrary substituent") is, for example, a group selected from the group consisting of

an unsubstituted alkyl group having 1 to 50 carbon atoms,
an unsubstituted alkenyl group having 2 to 50 carbon atoms,
an unsubstituted alkynyl group having 2 to 50 carbon atoms,
an unsubstituted cycloalkyl group having 3 to 50 ring carbon atoms,
-Si(R901)(R902)(R903),
-O-(R904),
-S-(R905),
-N(R906)(R907),
a halogen atom, a cyano group, a nitro group,
an unsubstituted aryl group having 6 to 50 ring carbon atoms, and
an unsubstituted heterocyclic group having 5 to 50 ring atoms,
wherein R901 to R907 each independently represent
a hydrogen atom,
a substituted or unsubstituted alkyl group having 1 to 50 carbon atoms
a substituted or unsubstituted cycloalkyl group having 3 to 50 ring carbon atoms,
a substituted or unsubstituted aryl group having 6 to 50 ring carbon atoms, or
a substituted or unsubstituted heterocyclic group having 5 to 50 ring atoms.

In the case where two or more groups each represented by R901 exist, the two or more groups each represented by R901 are the same as or different from each other,

in the case where two or more groups each represented by R902 exist, the two or more groups each represented by R902 are the same as or different from each other,
in the case where two or more groups each represented by R903 exist, the two or more groups each represented by R903 are the same as or different from each other,
in the case where two or more groups each represented by R904 exist, the two or more groups each represented by R904 are the same as or different from each other,
in the case where two or more groups each represented by R905 exist, the two or more groups each represented by R905 are the same as or different from each other,
in the case where two or more groups each represented by R906 exist, the two or more groups each represented by R906 are the same as or different from each other, and
in the case where two or more groups each represented by R907 exist, the two or more groups each represented by R907 are the same as or different from each other.

In one embodiment, the substituent for the case of "substituted or unsubstituted" may be a group selected from the group consisting of

an alkyl group having 1 to 50 carbon atoms,
an aryl group having 6 to 50 ring carbon atoms, and
a heterocyclic group having 5 to 50 ring atoms.

In one embodiment, the substituent for the case of "substituted or unsubstituted" may be a group selected from the group consisting of

an alkyl group having 1 to 18 carbon atoms,
an aryl group having 6 to 18 ring carbon atoms, and
a heterocyclic group having 5 to 18 ring atoms.

The specific examples of the groups for the arbitrary substituent described above are the specific examples of the substituent described in the section "Substituents in Description" described above.
In the description herein, the arbitrary adjacent substituents may form a "saturated ring" or an "unsaturated ring", preferably form a substituted or unsubstituted saturated 5-membered ring, a substituted or unsubstituted saturated 6-membered ring, a substituted or unsubstituted unsaturated 5-membered ring, or a substituted or unsubstituted unsaturated 6-membered ring, and more preferably form a benzene ring, unless otherwise indicated.
In the description herein, the arbitrary substituent may further have a substituent unless otherwise indicated in the description. The definition of the substituent that the arbitrary substituent further has may be the same as the arbitrary substituent.
In the description herein, a numerical range shown by "AA to BB" means a range including the numerical value AA as the former of "AA to BB" as the lower limit value and the numerical value BB as the latter of "AA to BB" as the upper limit value.

<Compound>

The compound of the present invention will be described below.
A compound according to one embodiment of the present invention is represented by the following formula (1) and has at least one deuterium atom. However, hereinafter, the compound of the present invention represented by the formula (1) and each formula included in the formula (1) described later may be simply referred to as "inventive compound A".
The compound according to one embodiment of the present invention is represented by formula (2) described below and has at least one deuterium atom. Hereinafter, the compound of the present invention represented by the formula (2) described later and formulae included in the formula (2) described later may be simply referred to as "inventive compound B".
Further, when both "inventive compound A" and "inventive compound B" are meant, they may be simply referred to as "inventive compound".

<Inventive Compound A>

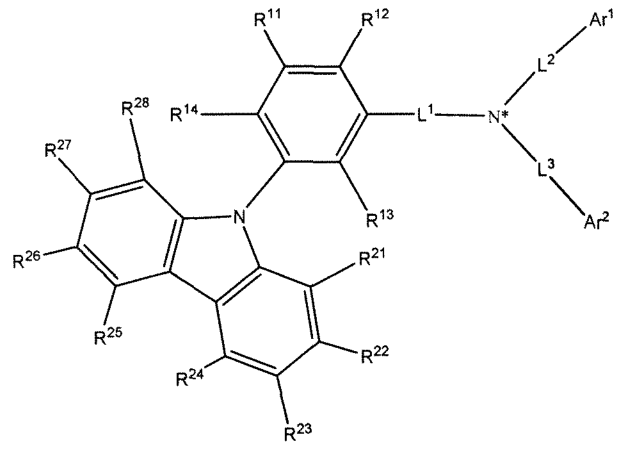

(1)

Hereinafter, symbols in the formula (1) and formulae included in the formula (1) described later will be described. Note that the same symbols have the same meaning.
In the formula (1), N* is a central nitrogen atom.

($Ar^1$, $Ar^2$)

In the formula (1), $Ar^1$ and $Ar^2$ are each independently a substituted or unsubstituted aryl group having 6 to 16 ring carbon atoms or a substituted or unsubstituted monovalent heterocyclic group having 5 to 30 ring atoms.
When both of $Ar^1$ and $Ar^2$ are the substituted or unsubstituted aryl groups having 6 to 16 ring carbon atoms, the total number of carbon atoms of the two substituted or unsubstituted aryl groups having 6 to 16 ring carbon atoms is 12 to 38.
In the description herein, the "total number of carbon atoms" includes the number of carbon atoms of the substituent.
The substituent of $Ar^1$ and the substituent of $Ar^2$ are both unsubstituted substituents. That is, substitution of the substituent of $Ar^1$ and substitution of the substituent of $Ar^2$ are not allowed.
When the substituted or unsubstituted aryl group having 6 to 16 ring carbon atoms represented by $Ar^1$ and $Ar^2$ includes a substituted or unsubstituted fluorenyl group, at least one of the substituted or unsubstituted fluorenyl groups is preferably represented by the following formula (A).

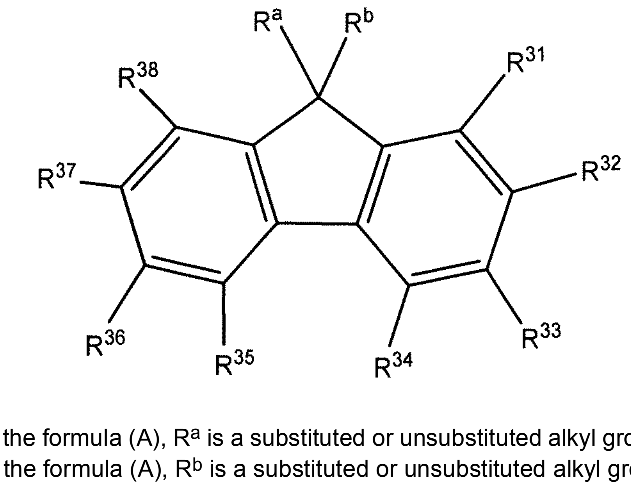

(A)

In the formula (A), $R^a$ is a substituted or unsubstituted alkyl group or a substituted or unsubstituted aryl group.
In the formula (A), $R^b$ is a substituted or unsubstituted alkyl group.
In the formula (A), one of $R^{31}$ to $R^{38}$ is a single bond bonded to $L^2$ or $L^3$.
$R^{31}$ to $R^{38}$ which are not single bonds are each independently a hydrogen atom or a substituent, and the substituent is the same as the substituent of $Ar^1$ and the substituent of $Ar^2$ in the formula (1) described above, and preferred embodiments thereof are also the same.
The details of the substituted or unsubstituted alkyl group represented by $R^a$ and $R^b$ are as described above in the section of "Substituents in Description".
The substituents of the aforementioned substituted alkyl groups represented by $R^a$ and $R^b$ are all unsubstituted

substituents. That is, substitution of the aforementioned substituted alkyl groups represented by $R^a$ and $R^b$ is not allowed.

The unsubstituted alkyl group represented by $R^a$ and $R^b$ is preferably a methyl group, an ethyl group, an n-propyl group, an isopropyl group, an n-butyl group, an isobutyl group, an s-butyl group, or a t-butyl group, more preferably a methyl group, an ethyl group, an isopropyl group, or a t-butyl group, and still more preferably a methyl group or a t-butyl group.

The details of the substituted or unsubstituted aryl group represented by $R^a$ are as described above in the section of "Substituents in Description".

The substituent of the aforementioned substituted aryl group represented by $R^a$ is an unsubstituted substituent. That is, substitution of the aforementioned substituted aryl group represented by $R^a$ is not allowed.

The unsubstituted aryl group represented by $R^a$ is more preferably selected from the group consisting of a phenyl group, a biphenyl group, a naphthyl group, and a phenanthryl group.

(Aryl group represented by $Ar^1$ and $Ar^2$)

The details of the substituted or unsubstituted aryl group having 6 to 16 ring carbon atoms represented by $Ar^1$ and $Ar^2$ are as described above in the section of "Substituents in Description".

The unsubstituted aryl group represented by $Ar^1$ and $Ar^2$ is preferably composed of only a benzene ring, and more preferably selected from the group consisting of a phenyl group, a biphenyl group, a naphthyl group, and a phenanthryl group.

(Heterocyclic group represented by $Ar^1$ and $Ar^2$)

The details of the substituted or unsubstituted monovalent heterocyclic group having 5 to 30 ring atoms represented by $Ar^1$ and $Ar^2$ are as described above in the section of "Substituents in Description".

The unsubstituted monovalent heterocyclic group represented by $Ar^1$ and $Ar^2$ is preferably a heterocyclic group selected from a pyridyl group, a pyrimidinyl group, a triazinyl group, a quinolyl group, an isoquinolyl group, a quinazolinyl group, a benzimidazolyl group, a phenanthrolinyl group, a carbazolyl group (1-carbazolyl group, 2-carbazolyl group, 3-carbazolyl group, 4-carbazolyl group, or 9-carbazolyl group), a benzocarbazolyl group, a dibenzofuranyl group, a naphthobenzofuranyl group, a dibenzothiophenyl group, and a naphthobenzothiophenyl group, and more preferably a heterocyclic group selected from a pyridyl group, a dibenzofuranyl group, and a dibenzothiophenyl group.

(Substituent of $Ar^1$ and Substituent of $Ar^2$)

The substituent of $Ar^1$ and the substituent of $Ar^2$ are each independently a halogen atom; a nitro group; a cyano group; an unsubstituted alkyl group having 1 to 50 carbon atoms; an unsubstituted alkenyl group having 2 to 50 carbon atoms; an unsubstituted alkynyl group having 2 to 50 carbon atoms; an unsubstituted cycloalkyl group having 3 to 50 ring carbon atoms; an unsubstituted haloalkyl group having 1 to 50 carbon atoms; an unsubstituted alkoxy group having 1 to 50 carbon atoms; an unsubstituted haloalkoxy group having 1 to 50 carbon atoms; an unsubstituted alkylthio group having 1 to 50 carbon atoms; an unsubstituted aryl group having 6 to 50 ring carbon atoms; an unsubstituted aryloxy group having 6 to 50 ring carbon atoms; an unsubstituted arylthio group having 6 to 50 ring carbon atoms; an unsubstituted aralkyl group having 7 to 50 carbon atoms; an unsubstituted monovalent heterocyclic group having 5 to 50 ring atoms; or a mono-, di-, or tri-substituted silyl group having a substituent selected from an unsubstituted alkyl group having 1 to 50 carbon atoms, an unsubstituted cycloalkyl group having 3 to 50 ring carbon atoms, an unsubstituted aryl group having 6 to 50 ring carbon atoms, and an unsubstituted monovalent heterocyclic group having 5 to 50 ring atoms.

(Halogen atom)

The details of the halogen atom as the substituent of $Ar^1$ and the substituent of $Ar^2$ are as described above in the section of "Substituents in Description".

(Alkyl group)

The details of the unsubstituted alkyl group having 1 to 50 carbon atoms as the substituent of $Ar^1$ and the substituent of $Ar^2$ are as described above in the section of "Substituents in Description".

(Alkenyl group)

The details of the unsubstituted alkenyl group having 2 to 50 carbon atoms as the substituent of $Ar^1$ and the

substituent of $Ar^2$ are as described above in the section of "Substituents in Description".

(Alkynyl group)

The details of the unsubstituted alkynyl group having 2 to 50 carbon atoms as the substituent of $Ar^1$ and the substituent of $Ar^2$ are as described above in the section of "Substituents in Description".

(Cycloalkyl group)

The details of the unsubstituted cycloalkyl group having 3 to 50 ring carbon atoms as the substituent of $Ar^1$ and the substituent of $Ar^2$ are as described above in the section of "Substituents in Description".

(Haloalkyl group)

The details of the unsubstituted haloalkyl group having 1 to 50 carbon atoms as the substituent of $Ar^1$ and the substituent of $Ar^2$ are as described above in the section of "Substituents in Description".

(Alkoxy group)

The details of the unsubstituted alkoxy group having 1 to 50 carbon atoms as the substituent of $Ar^1$ and the substituent of $Ar^2$ are as described above in the section of "Substituents in Description".

(Haloalkoxy group)

The unsubstituted haloalkoxy group having 1 to 50 carbon atoms as the substituent of $Ar^1$ and the substituent of $Ar^2$ is a group represented by -O(G12), and G12 is the aforementioned unsubstituted haloalkyl group.

(Alkylthio group)

The details of the unsubstituted alkylthio group having 1 to 50 carbon atoms as the substituent of $Ar^1$ and the substituent of $Ar^2$ are as described above in the section of "Substituents in Description".

(Aryl group)

The details of the unsubstituted aryl group having 6 to 50 ring carbon atoms as the substituent of $Ar^1$ and the substituent of $Ar^2$ are as described above in the section of "Substituents in Description".
The unsubstituted aryl group as the substituent of $Ar^1$ and the substituent of $Ar^2$ is more preferably selected from the group consisting of a phenyl group, a naphthyl group, and a phenanthryl group.
The unsubstituted aryl group having 6 to 50 ring carbon atoms as the substituent of $Ar^1$ and the substituent of $Ar^2$ includes, for example, a fused aryl group such as a fluorenyl group, a phenanthryl group, or an anthracenyl group; and the like, but does not include a ring assembly such as a biphenyl group, a terphenyl group, or a naphthylphenyl group.

(Aryloxy group)

The details of the unsubstituted aryloxy group having 6 to 50 ring carbon atoms as the substituent of $Ar^1$ and the substituent of $Ar^2$ are as described above in the section of "Substituents in Description".

(Arylthio group)

The details of the unsubstituted arylthio group having 6 to 50 ring carbon atoms as the substituent of $Ar^1$ and the substituent of $Ar^2$ are as described above in the section of "Substituents in Description".

(Aralkyl group)

The details of the unsubstituted aralkyl group having 7 to 50 carbon atoms as the substituent of $Ar^1$ and the substituent of $Ar^2$ are as described above in the section of "Substituents in Description".

(Heterocyclic group)

The details of the unsubstituted monovalent heterocyclic group having 5 to 50 ring atoms as the substituent of $Ar^1$ and the substituent of $Ar^2$ are as described above in the section of "Substituents in Description".

(Substituted silyl group)

The details of the mono-, di-, or tri-substituted silyl group as the substituent of $Ar^1$ and the substituent of $Ar^2$ are as described above in the section of "Substituents in Description".

($L^1$)

In the formula (1), $L^1$ is any one of a single bond, a substituted or unsubstituted arylene group having 6 to 30 ring carbon atoms, and a substituted or unsubstituted divalent heterocyclic group having 5 to 30 ring atoms.

(Arylene group represented by $L^1$)

The details of the substituted or unsubstituted arylene group having 6 to 30 ring carbon atoms represented by $L^1$ are as described above in the section of "Substituents in Description".
The substituted or unsubstituted arylene group represented by $L^1$ is preferably a phenylene group, a biphenylene group, or a terphenylene group.

(Heterocyclic group represented by $L^1$)

The details of the substituted or unsubstituted divalent heterocyclic group having 5 to 30 ring atoms represented by $L^1$ are as described above in the section of "Substituents in Description".

(Substituent of $L^1$)

The substituent of $L^1$ is a substituted or unsubstituted alkyl group having 1 to 50 carbon atoms, or a substituted or unsubstituted aryl group having 6 to 50 ring carbon atoms.

(Alkyl group)

The details of the substituted or unsubstituted alkyl group having 1 to 50 carbon atoms as the substituent of $L^1$ are as described above in the section of "Substituents in Description".

(Aryl group)

The details of the substituted or unsubstituted aryl group having 6 to 50 ring carbon atoms as the substituent of $L^1$ are as described above in the section of "Substituents in Description".

($L^2$, $L^3$)

In the formula (1), $L^2$ and $L^3$ are each independently any one of a single bond, a substituted or unsubstituted non-fused arylene group having 6 to 30 ring carbon atoms, and a substituted or unsubstituted divalent heterocyclic group having 5 to 30 ring atoms.
In the formula (1), $Ar^1$ and $L^2$ are not crosslinked, and $Ar^2$ and $L^3$ are not crosslinked.

(Non-fused Arylene group represented by $L^2$ and $L^3$)

The details of the substituted or unsubstituted non-fused arylene group having 6 to 30 ring carbon atoms represented by $L^2$ and $L^3$ are those obtained by selecting a non-fused one from the substituted or unsubstituted arylene groups described above in the section of "Substituents in Description".
The unsubstituted non-fused arylene group represented by $L^2$ and $L^3$ is preferably a phenylene group, a biphenylene group, or a terphenylene group.

(Heterocyclic group represented by $L^2$ and $L^3$)

The details of the substituted or unsubstituted divalent heterocyclic group having 5 to 30 ring atoms represented by $L^2$ and $L^3$ are as described above in the section of "Substituents in Description".

(Substituent of $L^2$ and Substituent of $L^3$)

The substituent of $L^2$ and the substituent of $L^3$ are each independently the same as the substituent of $Ar^1$ and the substituent of $Ar^2$ in the formula (1) described above, and preferred embodiments thereof are also the same.

($R^{11}$ to $R^{14}$, $R^{21}$ to $R^{28}$)

In the formula (1), $R^{11}$ to $R^{14}$ and $R^{21}$ to $R^{28}$ are each independently a hydrogen atom or a substituent, and the substituent is the same as the substituent of $L^1$, and preferred embodiments thereof are also the same.

(Structural Formula of Preferred Examples of Inventive Compound A)

Preferred examples of the inventive compound A include compounds represented by the following formulae (1-1) to (1-10). The compounds represented by the formulae (1-1) to (1-10) have at least one deuterium atom.

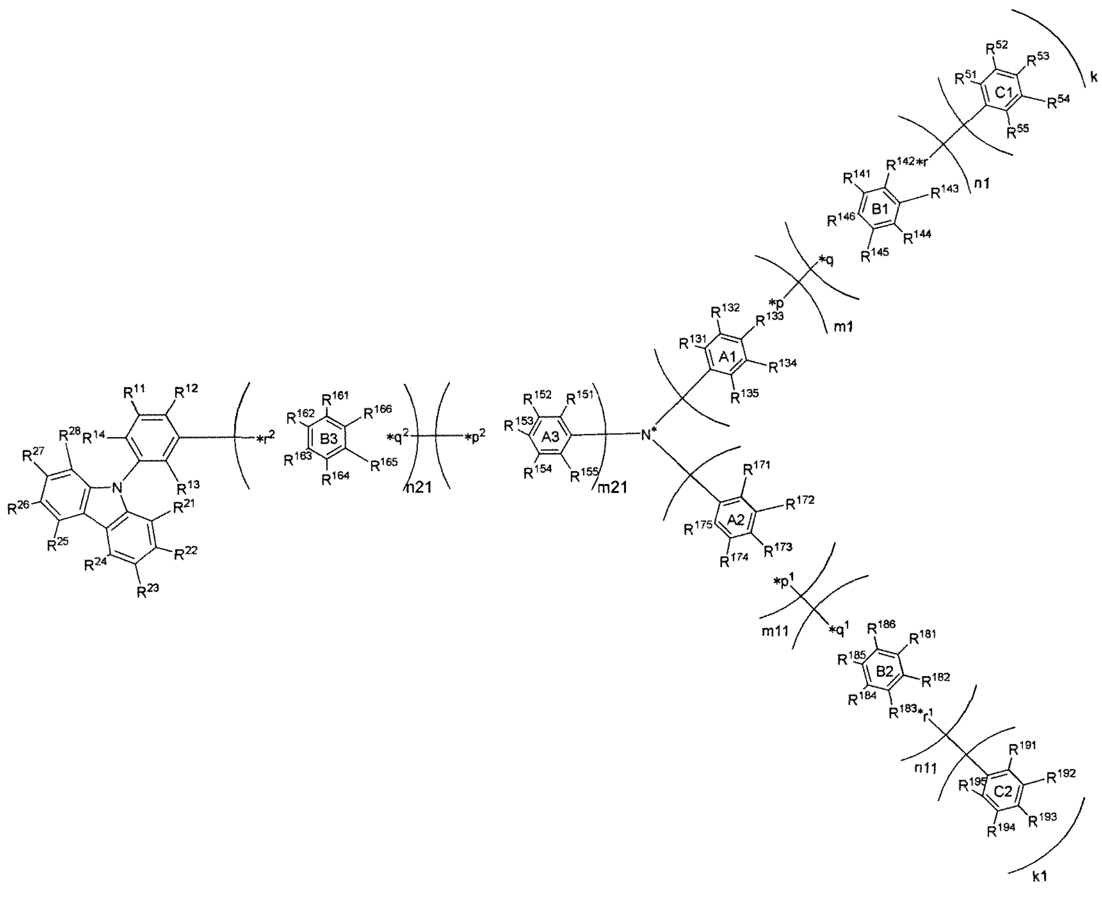

(1-1)

In the formula (1-1), $R^{11}$ to $R^{14}$ and $R^{21}$ to $R^{28}$ are as described above.

In the formula (1-1), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to $*p^2$, one selected from $R^{161}$ to $R^{166}$ is a single bond bonded to $*q^2$, and the other one selected from $R^{161}$ to $R^{166}$ is a single bond bonded to $*r^2$.

In the formula (1-1), $R^{131}$ to $R^{135}$, $R^{141}$ to $R^{146}$ , $R^{171}$ to $R^{175}$, $R^{181}$ to $R^{186}$, $R^{51}$ to $R^{55}$, and $R^{191}$ to $R^{195}$ are each independently a hydrogen atom or a substituent, and the substituent is the same substituent as the substituent of $Ar^1$ and the substituent of $Ar^2$ in the formula (1), provided that one selected from $R^{131}$ to $R^{135}$ is a single bond bonded

to *p, one selected from $R^{141}$ to $R^{146}$ is a single bond bonded to *q, the other one or two selected from $R^{141}$ to $R^{146}$ is a single bond bonded to *r, one selected from $R^{171}$ to $R^{175}$ is a single bond bonded to $*p^1$, one selected from $R^{181}$ to $R^{186}$ is a single bond bonded to $*q^1$, and the other one or two selected from $R^{181}$ to $R^{186}$ is a single bond bonded to $*r^1$.

In the formula (1-1), m1, m11, and m21 are each independently 0 or 1, n1, n11, and n21 are each independently 0 or 1, and

when m1 is 0 and n1 is 0, *r is bonded to a nitrogen atom N*,
when m1 is 0 and n1 is 1, *p is bonded to a nitrogen atom N*, and
when m1 is 1 and n1 is 0, one selected from $R^{131}$ to $R^{135}$ is a single bond bonded to *r,
when m11 is 0 and n11 is 0, $*r^1$ is bonded to a nitrogen atom N*,
when m11 is 0 and n11 is 1, $*p^1$ is bonded to a nitrogen atom N*, and
when m11 is 1 and n11 is 0, one selected from $R^{171}$ to $R^{175}$ is a single bond bonded to $*r^1$,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$ , and
k and $k^1$ are each independently 1 or 2.

In the formula (1-1), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{171}$ to $R^{175}$ that are not single bonds, adjacent two selected from $R^{181}$ to $R^{186}$ that are not single bonds, adjacent two selected from $R^{51}$ to $R^{55}$, and adjacent two selected from $R^{191}$ to $R^{195}$ are not independently bonded to each other and thus do not form a ring structure, and the benzene ring A1 and benzene ring B1, the benzene ring A1 and benzene ring C1, the benzene ring B1 and benzene ring C1, the benzene ring A2 and benzene ring B2, the benzene ring A2 and benzene ring C2, the benzene ring B2 and benzene ring C2, and the benzene ring A3 and benzene ring B3 are not crosslinked.

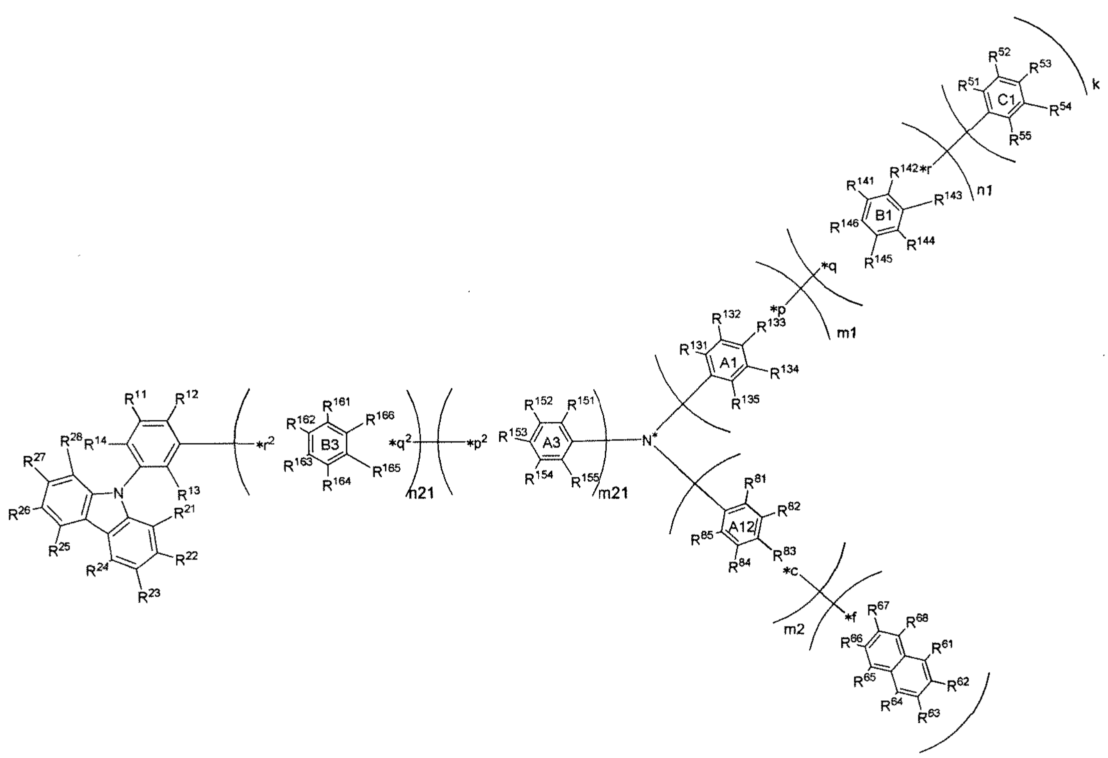

(1-2)

In the formula (1-2), $R^{11}$ to $R^{14}$ and $R^{21}$ to $R^{28}$ are as described above.

In the formula (1-2), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to $*p^2$, one selected from $R^{161}$ to $R^{166}$ is a single bond bonded to $*q^2$, and the other one selected from $R^{161}$ to $R^{166}$ is a single bond bonded to $*r^2$.

In the formula (1-2), $R^{131}$ to $R^{135}$, $R^{141}$ to $R^{146}$, $R^{81}$ to $R^{85}$, $R^{51}$ to $R^{55}$, and $R^{61}$ to $R^{68}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1)

described above, and preferred embodiments thereof are also the same, provided that one selected from $R^{131}$ to $R^{135}$ is a single bond bonded to *p, one selected from $R^{141}$ to $R^{146}$ is a single bond bonded to *q, the other one or two selected from $R^{141}$ to $R^{146}$ is a single bond bonded to *r, and one selected from $R^{81}$ to $R^{85}$ is a single bond bonded to *c.

In the formula (1-2), m1 and m21 are each independently 0 or 1, n1 and n21 are each independently 0 or 1, and

when m1 is 0 and n1 is 0, *r is bonded to a nitrogen atom N*,
when m1 is 0 and n1 is 1, *p is bonded to a nitrogen atom N*, and
when m1 is 1 and n1 is 0, one selected from $R^{131}$ to $R^{135}$ is a single bond bonded to *r,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$, and
k is 1 or 2.

In the formula (1-2), one selected from $R^{61}$ to $R^{68}$ is a single bond bonded to *f, m2 is 0 or 1, and when m2 is 0, *c is bonded to a nitrogen atom N*.

In the formula (1-2), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{81}$ to $R^{85}$ that are not single bonds, adjacent two selected from $R^{51}$ to $R^{55}$, and adjacent two selected from $R^{61}$ to $R^{68}$ are not independently bonded to each other and thus do not form a ring structure, and the benzene ring A1 and benzene ring B1, the benzene ring A1 and benzene ring C1, the benzene ring B1 and benzene ring C1, and the benzene ring A3 and benzene ring B3 are not crosslinked.

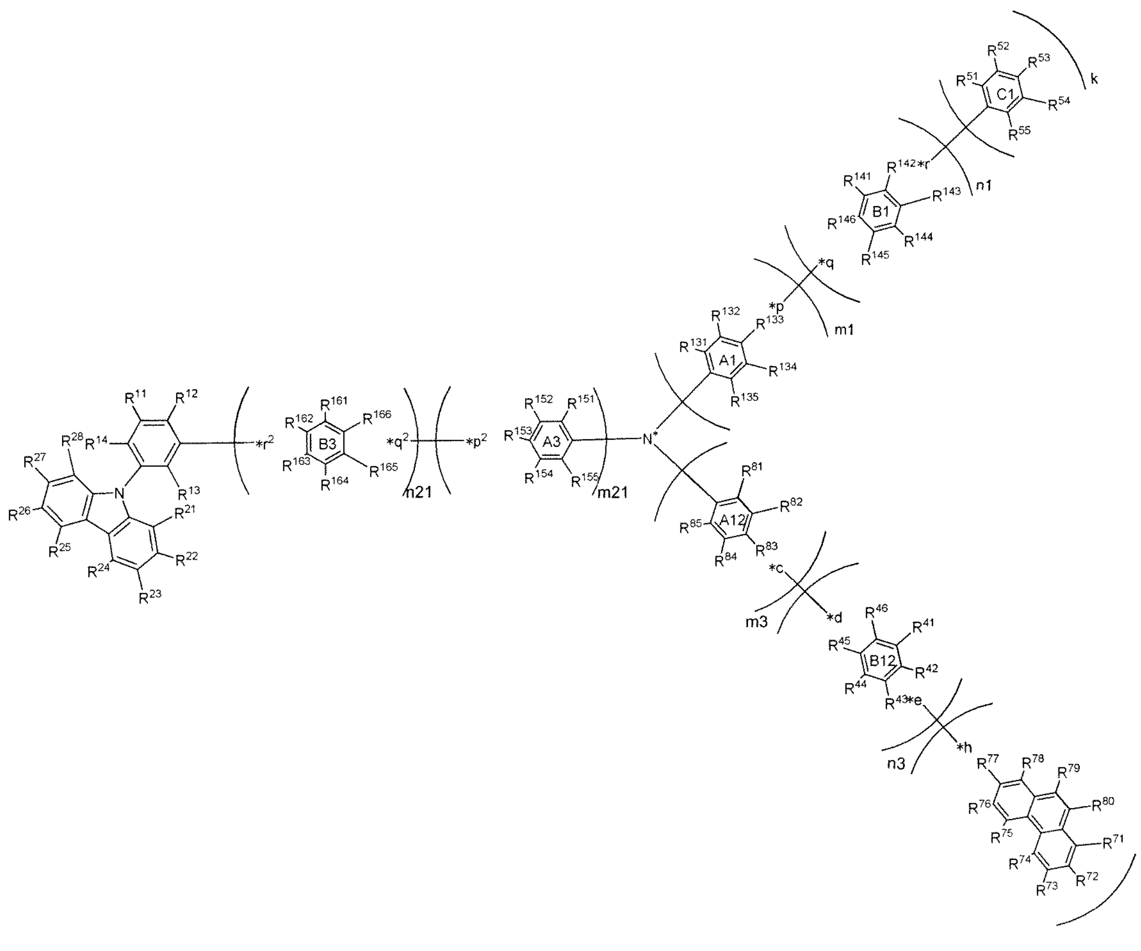

(1-3)

In the formula (1-3), $R^{11}$ to $R^{14}$ and $R^{21}$ to $R^{28}$ are as described above.

In the formula (1-3), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to $*p^2$, one selected from $R^{161}$ to

$R^{166}$ is a single bond bonded to $*q^2$, and the other one selected from $R^{161}$ to $R^{166}$ is a single bond bonded to $*r^2$.

In the formula (1-3), $R^{131}$ to $R^{135}$, $R^{141}$ to $R^{146}$, $R^{81}$ to $R^{85}$, $R^{41}$ to $R^{46}$, $R^{51}$ to $R^{55}$, and $R^{71}$ to $R^{80}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{131}$ to $R^{135}$ is a single bond bonded to *p, one selected from $R^{141}$ to $R^{146}$ is a single bond bonded to *q, the other one or two selected from $R^{141}$ to $R^{146}$ is a single bond bonded to *r, one selected from $R^{81}$ to $R^{85}$ is a single bond bonded to

*c, one selected from $R^{41}$ to $R^{46}$ is a single bond bonded to *d, and the other one selected from $R^{41}$ to $R^{46}$ is a single bond bonded to *e.

In the formula (1-3), m1, m3, and m21 are each independently 0 or 1, n1, n3, and n21 are each independently 0 or 1, and

when m1 is 0 and n1 is 0, *r is bonded to a nitrogen atom N*,
when m1 is 0 and n1 is 1, *p is bonded to a nitrogen atom N*, and
when m1 is 1 and n1 is 0, one selected from $R^{131}$ to $R^{135}$ is a single bond bonded to *r,
when m3 is 0 and n3 is 0, *e is bonded to a nitrogen atom N*,
when m3 is 0 and n3 is 1, *c is bonded to a nitrogen atom N*, and
when m3 is 1 and n3 is 0, one selected from $R^{81}$ to $R^{85}$ is a single bond bonded to *e,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$ , and
k is 1 or 2.

In the formula (1-3), one selected from $R^{71}$ to $R^{80}$ is a single bond bonded to *h.

In the formula (1-3), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{81}$ to $R^{85}$ that are not single bonds, adjacent two selected from $R^{41}$ to $R^{46}$ that are not single bonds, adjacent two selected from $R^{51}$ to $R^{55}$, and adjacent two selected from $R^{71}$ to $R^{80}$ are not independently bonded to each other and thus do not form a ring structure, and the benzene ring A1 and benzene ring B1, the benzene ring A1 and benzene ring C1, the benzene ring B1 and benzene ring C1, the benzene ring A12 and benzene ring B12, and the benzene ring A3 and benzene ring B3 are not crosslinked.

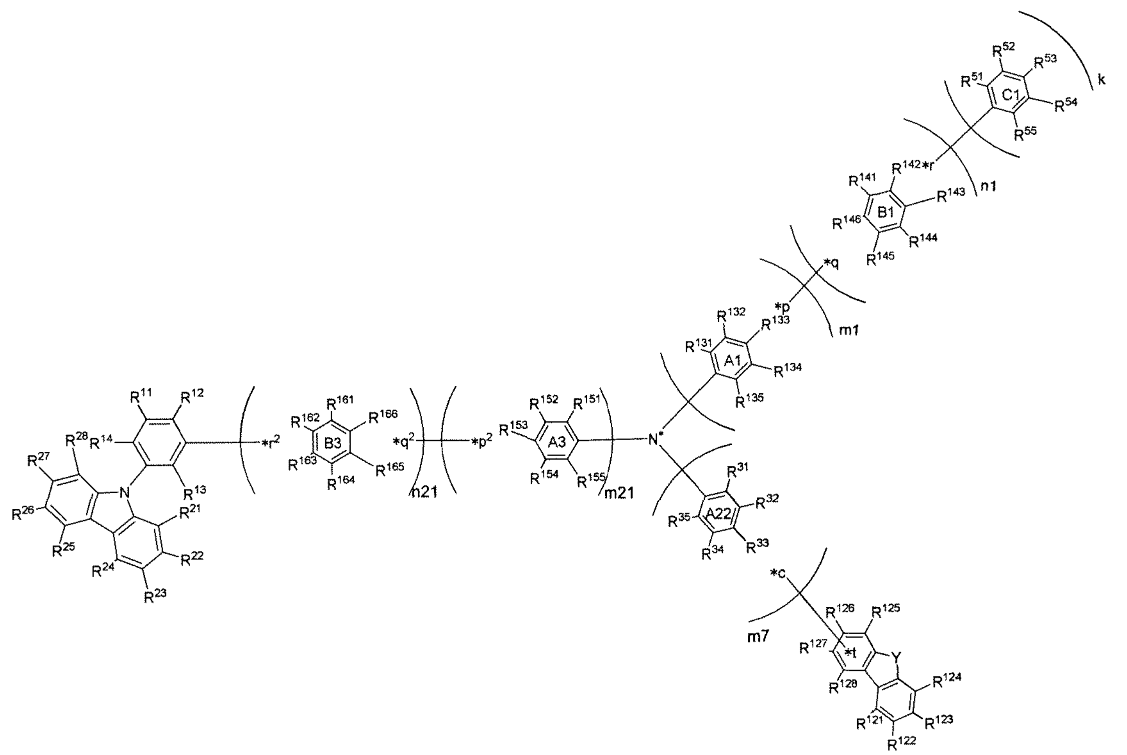

(1-4)

In the formula (1-4), $R^{11}$ to $R^{14}$ and $R^{21}$ to $R^{28}$ are as described above.

In the formula (1-4), $R^{151}$ to $R^{155}$ and $R^{161}$ to $R^{166}$ are each independently a hydrogen atom, a substituted or

unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to $*p^2$, one selected from $R^{161}$ to $R^{166}$ is a single bond bonded to $*q^2$, and the other one selected from $R^{161}$ to $R^{166}$ is a single bond bonded to $*r^2$.

In the formula (1-4), $R^{131}$ to $R^{135}$, $R^{141}$ to $R^{146}$, $R^{31}$ to $R^{35}$, $R^{51}$ to $R^{55}$, and $R^{121}$ to $R^{128}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{131}$ to $R^{135}$ is a single bond bonded to *p, one selected from $R^{141}$ to $R^{146}$ is a single bond bonded to *q, the other one or two selected from $R^{141}$ to $R^{146}$ is a single bond bonded to *r, one selected from $R^{31}$ to $R^{35}$ is a single bond bonded to *c, and one selected from $R^{121}$ to $R^{128}$ is a single bond bonded to *t.

In the formula (1-4), m1 and m21 are each independently 0 or 1, n1 and n21 are each independently 0 or 1, and

when m1 is 0 and n1 is 0, *r is bonded to a nitrogen atom N*,
when m1 is 0 and n1 is 1, *p is bonded to a nitrogen atom N*, and
when m1 is 1 and n1 is 0, one selected from $R^{131}$ to $R^{135}$ is a single bond bonded to *r,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$, and
k is 1 or 2.

In the formula (1-4), m7 is 0 or 1, and when m7 is 0, *c is bonded to a nitrogen atom N*.

In the formula (1-4), Y is an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group having 6 to 50 ring carbon atoms. In the case where both of $R^c$ and $R^d$ are aryl groups, $R^c$ and $R^d$ may be bonded to each other to form a spiro ring.

In the formula (1-4), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{31}$ to $R^{35}$ that are not single bonds, adjacent two selected from $R^{51}$ to $R^{55}$, and adjacent two selected from $R^{121}$ to $R^{124}$ and $R^{125}$ and $R^{128}$ are not independently bonded to each other and thus do not form a ring structure, and the benzene ring A1 and benzene ring B1, the benzene ring A1 and benzene ring C1, the benzene ring B1 and benzene ring C1, and the benzene ring A3 and benzene ring B3 are not crosslinked.

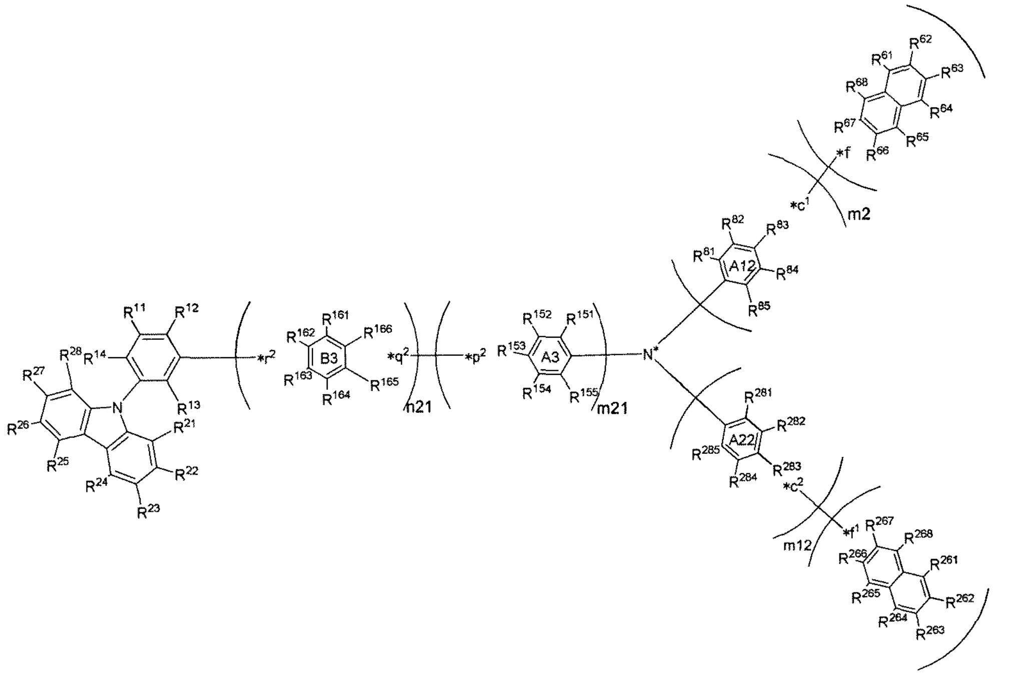

(1-5)

In the formula (1-5), $R^{11}$ to $R^{14}$ and $R^{21}$ to $R^{28}$ are as described above.

In the formula (1-5), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring

carbon atoms, provided that one selected from $R^{151}$ to $R^{155}$ is a single bond bonded to *$p^2$, one selected from $R^{161}$ to $R^{166}$ is a single bond bonded to *$q^2$, and the other one selected from $R^{161}$ to $R^{166}$ is a single bond bonded to *$r^2$.

In the formula (1-5), $R^{81}$ to $R^{85}$, $R^{281}$ to $R^{285}$, $R^{61}$ to R68, and $R^{261}$ to $R^{268}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1), and preferred embodiments thereof are also the same, provided that one selected from $R^{81}$ to $R^{85}$ is a single bond bonded to *$c^1$, one selected from $R^{281}$ to $R^{285}$ is a single bond bonded to *$C^2$, one selected from $R^{61}$ to $R^{68}$ is a single bond bonded to *f, and one selected from $R^{261}$ to $R^{268}$ is a single bond bonded to *$f^1$.

In the formula (1-5), m21 is 0 or 1, n21 is 0 or 1, and

when m21 is 0 and n21 is 0, *$r^2$ is bonded to a nitrogen atom N*,
when m21 is 0 and n21 is 1, *$p^2$ is bonded to a nitrogen atom N*, and
when m21 is 1 and n21 is 0, one selected from $R^{151}$ to $R^{155}$ is a single bond bonded to *$r^2$.

In the formula (1-5), m2 is 0 or 1 and when m2 is 0, *$c^1$ is bonded to a nitrogen atom N*, and m12 is 0 or 1 and when m12 is 0, *$c^2$ is bonded to a nitrogen atom N*.

In the formula (1-5), adjacent two selected from $R^{81}$ to $R^{85}$ that are not single bonds, adjacent two selected from $R^{151}$ to $R^{155}$ that are not single bonds, adjacent two selected from $R^{161}$ to $R^{166}$ that are not single bonds, adjacent two selected from $R^{281}$ to $R^{285}$ that are not single bonds, adjacent two selected from $R^{61}$ to $R^{68}$, and adjacent two selected from $R^{261}$ to $R^{268}$ are not independently bonded to each other and thus do not form a ring structure, and the benzene ring A3 and benzene ring B3 are not crosslinked.

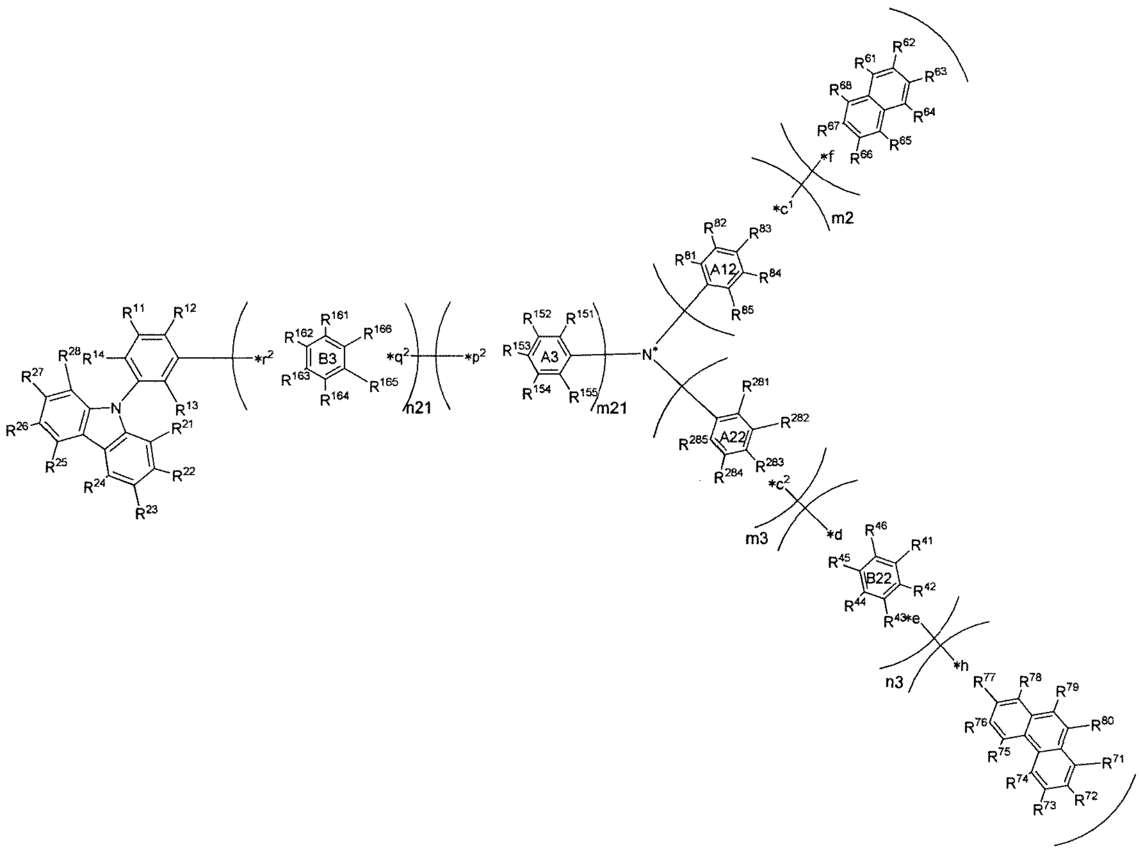

(1-6)

In the formula (1-6), $R^{11}$ to $R^{14}$ and $R^{21}$ to $R^{28}$ are as described above.

In the formula (1-6), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to *$p^2$, one selected from $R^{161}$ to $R^{166}$ is a single bond bonded to *$q^2$, and the other one selected from $R^{161}$ to $R^{166}$ is a single bond bonded to *$r^2$.

In the formula (1-6), $R^{81}$ to $R^{85}$, $R^{281}$ to $R^{285}$, $R^{41}$ to $R^{46}$, $R^{61}$ to $R^{68}$, and $R^{71}$ to $R^{80}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{81}$ to $R^{85}$ is

a single bond bonded to *$c^1$, one selected from $R^{281}$ to $R^{285}$ is a single bond bonded to *$c^2$, one selected from $R^{41}$ to $R^{46}$ is a single bond bonded to *d, and the other one selected from $R^{41}$ to $R^{46}$ is a single bond bonded to *e, one selected from $R^{61}$ to $R^{68}$ is a single bond bonded to *f, and one selected from $R^{71}$ to $R^{80}$ is a single bond bonded to *h.

In the formula (1-6), m21 and m3 are each independently 0 or 1, n21 and n3 are each independently 0 or 1, and

when m21 is 0 and n21 is 0, *$r^2$ is bonded to a nitrogen atom N*,
when m21 is 0 and n21 is 1, *$p^2$ is bonded to a nitrogen atom N*, and
when m21 is 1 and n21 is 0, one selected from $R^{151}$ to $R^{155}$ is a single bond bonded to *$r^2$,
when m3 is 0 and n3 is 0, *e is bonded to a nitrogen atom N*,
when m3 is 0 and n3 is 1, *$c^2$ is bonded to a nitrogen atom N*, and
when m3 is 1 and n3 is 0, one selected from $R^{281}$ to $R^{285}$ is a single bond bonded to *e.

In the formula (1-6), m2 is 0 or 1, and when m2 is 0, *$c^1$ is bonded to a nitrogen atom N*.

In the formula (1-6), adjacent two selected from $R^{81}$ to $R^{85}$ that are not single bonds, adjacent two selected from $R^{151}$ to $R^{155}$ that are not single bonds, adjacent two selected from $R^{161}$ to $R^{166}$ that are not single bonds, adjacent two selected from $R^{281}$ to $R^{285}$ that are not single bonds, adjacent two selected from $R^{41}$ to $R^{46}$ that are not single bonds, adjacent two selected from $R^{61}$ to $R^{68}$, and adjacent two selected from $R^{71}$ to $R^{80}$ are not independently bonded to each other and thus do not form a ring structure, and the benzene ring A22 and benzene ring B22, and the benzene ring A3 and benzene ring B3 are not crosslinked.

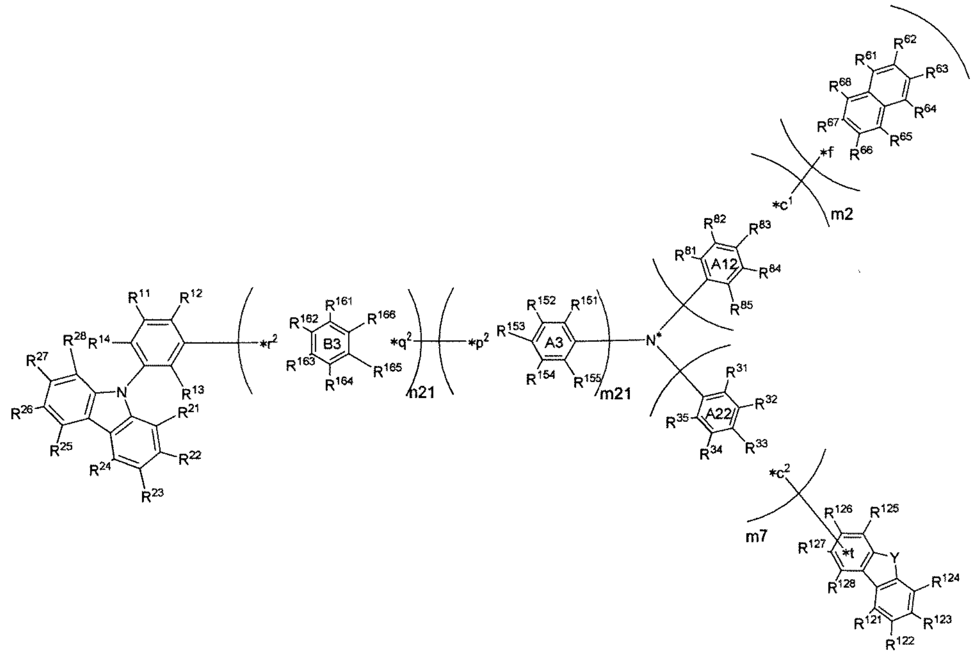

(1-7)

In the formula (1-7), $R^{11}$ to $R^{14}$ and $R^{21}$ to $R^{28}$ are as described above.

In the formula (1-7), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to *$p^2$, one selected from $R^{161}$ to $R^{166}$ is a single bond bonded to *$q^2$, and the other one selected from $R^{161}$ to $R^{166}$ is a single bond bonded to *$r^2$.

In the formula (1-7), $R^{81}$ to $R^{85}$, $R^{31}$ to $R^{35}$, $R^{61}$ to $R^{68}$, and $R^{121}$ to $R^{128}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1), and preferred embodiments thereof are also the same, provided that one selected from $R^{81}$ to $R^{85}$ is a single bond bonded to *$c^1$, one selected from $R^{31}$ to $R^{35}$ is a single bond bonded to *$c^2$, one selected from $R^{61}$ to $R^{65}$ is a single bond bonded to *f, and one selected from $R^{121}$ to $R^{128}$ is a single bond bonded to *t.

In the formula (1-7), m21 is 0 or 1, n21 is 0 or 1, and

when m21 is 0 and n21 is 0, *$r^2$ is bonded to a nitrogen atom N*,
when m21 is 0 and n21 is 1, *$p^2$ is bonded to a nitrogen atom N*, and
when m21 is 1 and n21 is 0, one selected from $R^{151}$ to $R^{155}$ is a single bond bonded to *$r^2$.

In the formula (1-7), m2 is 0 or 1 and when m2 is 0, *$c^1$ is bonded to a nitrogen atom N*, and m7 is 0 or 1 and when m7 is 0, *$c^2$ is bonded to a nitrogen atom N*.

In the formula (1-7), Y is an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group having 6 to 50 ring carbon atoms. In the case where both of $R^c$ and $R^d$ are aryl groups, $R^c$ and $R^d$ may be bonded to each other to form a spiro ring.

In the formula (1-7), adjacent two selected from $R^{81}$ to $R^{85}$ that are not single bonds, adjacent two selected from $R^{151}$ to $R^{155}$ that are not single bonds, adjacent two selected from $R^{161}$ to $R^{166}$ that are not single bonds, adjacent two selected from $R^{31}$ to $R^{35}$ that are not single bonds, adjacent two selected from $R^{61}$ to $R^{68}$, and adjacent two selected from $R^{121}$ to $R^{128}$ are not independently bonded to each other and thus do not form a ring structure, and the benzene ring A3 and benzene ring B3 are not crosslinked.

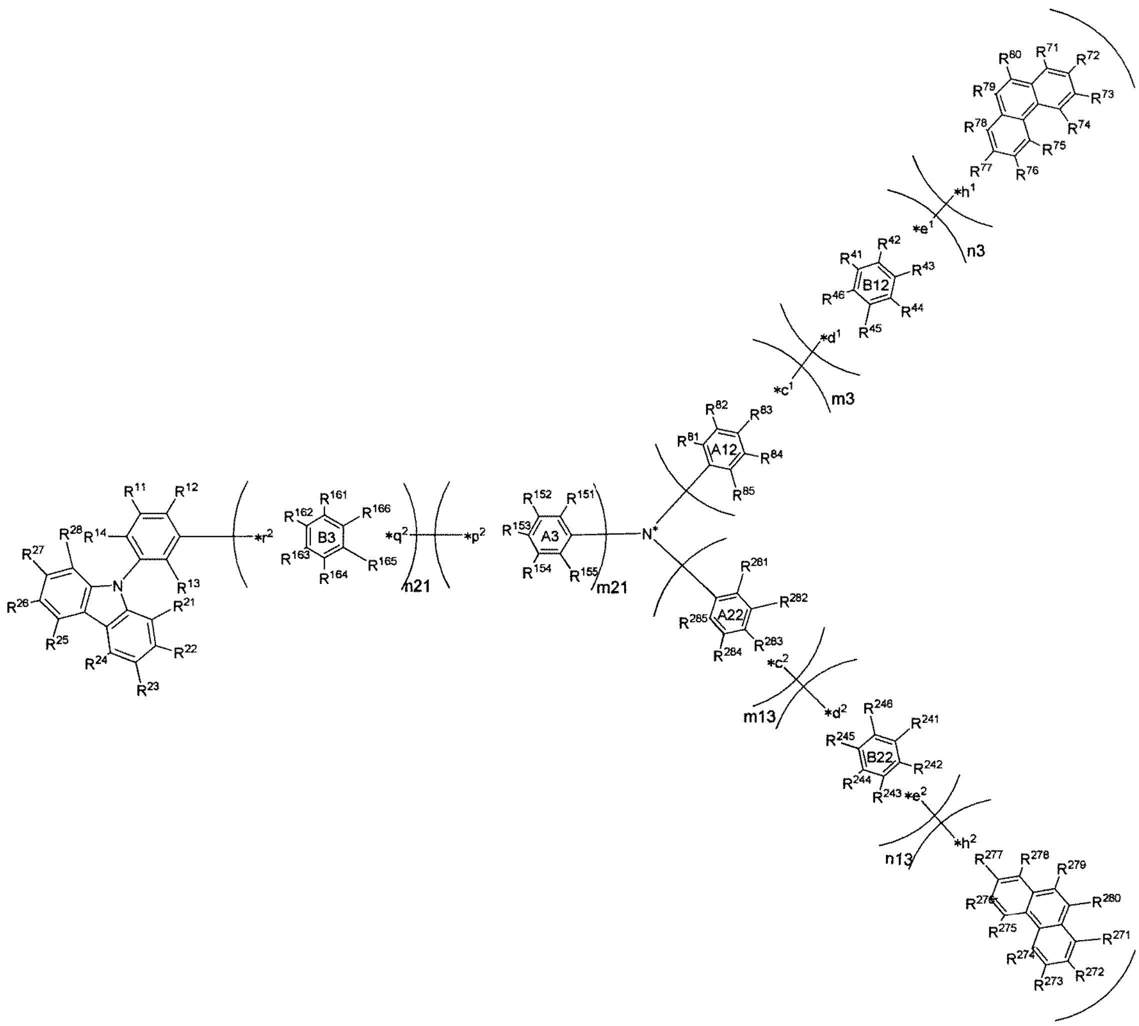

(1-8)

In the formula (1-8), $R^{11}$ to $R^{14}$ and $R^{21}$ to $R^{28}$ are as described above.

In the formula (1-8), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to *$p^2$, one selected from $R^{161}$ to $R^{166}$ is a single bond bonded to *$q^2$, and the other one selected from $R^{161}$ to $R^{166}$ is a single bond bonded to *$r^2$.

In the formula (1-8), $R^{81}$ to $R^{85}$, $R^{41}$ to $R^{46}$, $R^{281}$ to $R^{285}$, $R^{241}$ to $R^{246}$, $R^{71}$ to $R^{80}$, and $R^{271}$ to $R^{280}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{81}$ to $R^{85}$ is a single bond bonded to *$c^1$, one selected from $R^{41}$ to $R^{46}$ is a single bond bonded to *$d^1$ and the other one selected from $R^{41}$ to $R^{46}$ is a single bond bonded to *$e^1$, one selected from $R^{281}$ to $R^{285}$ is a single bond bonded to *$c^2$, one selected from $R^{241}$ to $R^{246}$ is a single bond bonded to *$d^2$ and the other one selected from $R^{241}$ to $R^{246}$ is a single bond bonded to *$e^2$, one selected from $R^{71}$ to $R^{80}$ is a single bond bonded to *$h^1$, and one selected from $R^{271}$ to

$R^{280}$ is a single bond bonded to $*h^2$.

In the formula (1-8), m3, m13, and m21 are each independently 0 or 1, n3, n13, and n21 are each independently 0 or 1, and

when m3 is 0 and n3 is 0, $*e^1$ is bonded to a nitrogen atom N*,
when m3 is 0 and n3 is 1, $*c^1$ is bonded to a nitrogen atom N*, and
when m3 is 1 and n3 is 0, one selected from $R^{81}$ to $R^{85}$ is a single bond bonded to $*e^1$,
when m13 is 0 and n13 is 0, $*e^2$ is bonded to a nitrogen atom N*,
when m13 is 0 and n13 is 1, $*c^2$ is bonded to a nitrogen atom N*, and
when m13 is 1 and n13 is 0, one selected from $R^{281}$ to $R^{285}$ is a single bond bonded to $*e^2$,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (1-8), adjacent two selected from $R^{81}$ to $R^{85}$ that are not single bonds, adjacent two selected from $R^{41}$ to $R^{46}$ that are not single bonds, adjacent two selected from $R^{151}$ to $R^{155}$ that are not single bonds, adjacent two selected from $R^{161}$ to $R^{166}$ that are not single bonds, adjacent two selected from $R^{281}$ to $R^{285}$ that are not single bonds, adjacent two selected from $R^{241}$ to $R^{246}$, adjacent two selected from $R^{71}$ to $R^{80}$, and adjacent two selected from $R^{271}$ to $R^{280}$ are not independently bonded to each other and thus do not form a ring structure, and the benzene ring A12 and benzene ring B12, the benzene ring A22 and benzene ring B22, and the benzene ring A3 and benzene ring B3 are not crosslinked.

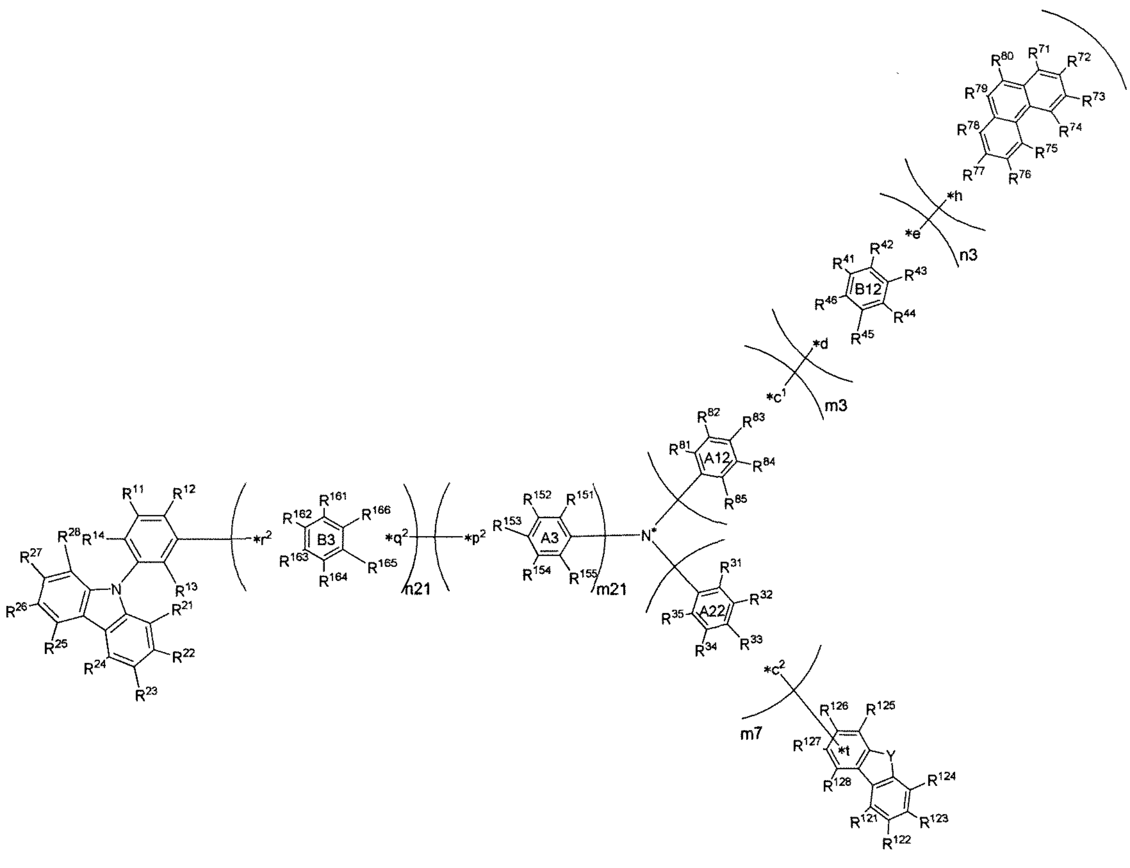

(1-9)

In the formula (1-9), $R^{11}$ to $R^{14}$ and $R^{21}$ to $R^{28}$ are as described above.

In the formula (1-9), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to $*p^2$, one selected from $R^{161}$ to $R^{166}$ is a single bond bonded to $*q^2$, and the other one selected from $R^{161}$ to $R^{166}$ is a single bond bonded to $*r^2$.

In the formula (1-9), $R^{81}$ to $R^{85}$, $R^{41}$ to $R^{46}$, $R^{31}$ to $R^{35}$, $R^{71}$ to $R^{80}$, and $R^{121}$ to $R^{128}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1)

described above, and preferred embodiments thereof are also the same, provided that one selected from $R^{81}$ to $R^{85}$ is a single bond bonded to $*c^1$, one selected from $R^{41}$ to $R^{46}$ is a single bond bonded to *d and the other one selected from $R^{41}$ to $R^{46}$ is a single bond bonded to *e, one selected from $R^{31}$ to $R^{35}$ is a single bond bonded to $*c^2$, one selected from $R^{71}$ to $R^{80}$ is a single bond bonded to *h, and one selected from $R^{121}$ to $R^{128}$ is a single bond bonded to *t.

In the formula (1-9), m3 and m21 are each independently 0 or 1, n3 and n21 are each independently 0 or 1, and

when m3 is 0 and n3 is 0, *e is bonded to a nitrogen atom N*,
when m3 is 0 and n3 is 1, $*c^1$ is bonded to a nitrogen atom N*, and
when m3 is 1 and n3 is 0, one selected from $R^{81}$ to $R^{85}$ is a single bond bonded to *e,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (1-9), m7 is 0 or 1, and when m7 is 0, $*c^2$ is bonded to a nitrogen atom N*.

In the formula (1-9), Y is an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group having 6 to 50 ring carbon atoms. However, the case where both of $R^c$ and $R^d$ are substituted or unsubstituted aryl groups having 6 to 50 ring carbon atoms is excluded.

In the formula (1-9), adjacent two selected from $R^{81}$ to $R^{85}$ that are not single bonds, adjacent two selected from $R^{41}$ to $R^{46}$ that are not single bonds, adjacent two selected from $R^{151}$ to $R^{155}$ that are not single bonds, adjacent two selected from $R^{161}$ to $R^{166}$ that are not single bonds, adjacent two selected from $R^{31}$ to $R^{35}$ that are not single bonds, adjacent two selected from $R^{71}$ to $R^{80}$, and adjacent two selected from $R^{121}$ to $R^{128}$ are not independently bonded to each other and thus do not form a ring structure, and the benzene ring A12 and benzene ring B12, and the benzene ring A3 and benzene ring B3 are not crosslinked.

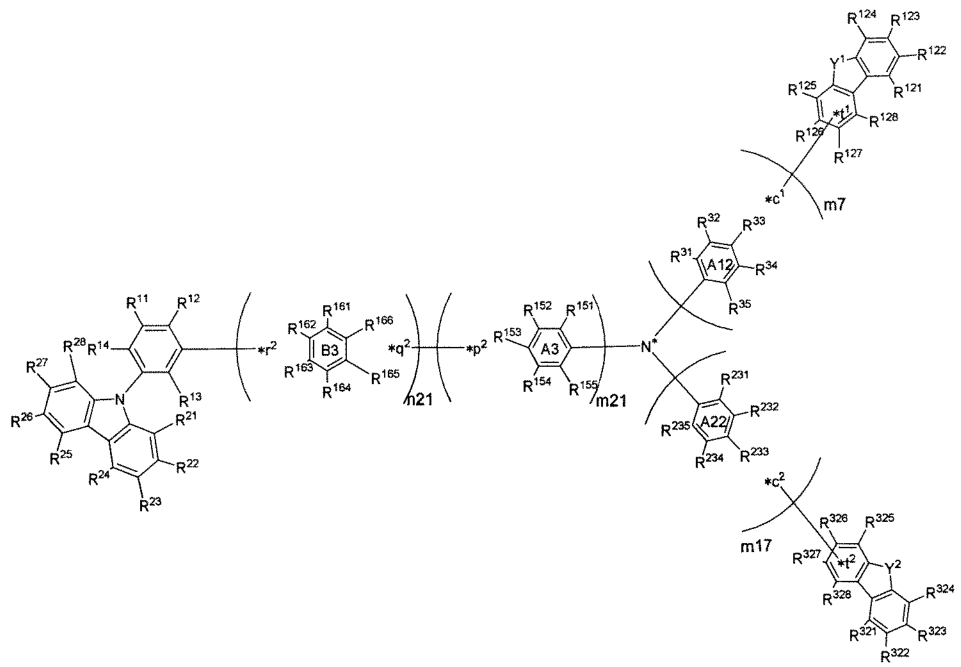

(1-10)

In the formula (1-10), $R^{11}$ to $R^{14}$ and $R^{21}$ to $R^{28}$ are as described above.

In the formula (1-10), $R^{151}$ to $R^{155}$ and $R^{161}$ to $R^{166}$ are each independently a hydrogen atom, a substituted or unsubstituted alkyl group having 1 to 50 carbon atoms, or a substituted or unsubstituted aryl group having 6 to 50 ring carbon atoms, provided that one selected from $R^{151}$ to $R^{155}$ is a single bond bonded to $*p^2$, one selected from $R^{161}$ to $R^{166}$ is a single bond bonded to $*q^2$, and the other one selected from $R^{161}$ to $R^{166}$ is a single bond bonded to $*r^2$.

In the formula (1-10), $R^{31}$ to $R^{35}$, $R^{231}$ to $R^{235}$, $R^{121}$ to $R^{128}$, and $R^{321}$ to $R^{328}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1), and preferred embodiments thereof are also the same, provided that one selected from $R^{31}$ to $R^{35}$ is a single bond bonded to $*c^1$, one selected from $R^{231}$ to $R^{235}$ is a single bond bonded to $*c^2$, one selected from $R^{121}$ to $R^{128}$ is a single bond bonded to $*t^1$, and one selected from $R^{321}$ to $R^{328}$ is a single bond bonded to $*t^2$.

In the formula (1-10), m21 is each independently 0 or 1, n21 is each independently 0 or 1, and

when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (1-10), m7 is 0 or 1 and when m7 is 0, $*c^1$ is bonded to a nitrogen atom N*, and m17 is 0 or 1 and when m17 is 0, $*c^2$ is bonded to a nitrogen atom N*.

In the formula (1-10), $Y^1$ and $Y^2$ are each independently an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group having 6 to 50 ring carbon atoms. In the case where both of $R^c$ and $R^d$ are aryl groups, $R^c$ and $R^d$ may be bonded to each other to form a spiro ring.

In the formula (1-10), adjacent two selected from $R^{31}$ to $R^{35}$ that are not single bonds, adjacent two selected from $R^{151}$ to $R^{155}$ that are not single bonds, adjacent two selected from $R^{161}$ to $R^{166}$ that are not single bonds, adjacent two selected from $R^{231}$ to $R^{235}$ that are not single bonds, adjacent two selected from $R^{121}$ to $R^{128}$, and adjacent two selected from $R^{321}$ to $R^{328}$ are not independently bonded to each other and thus do not form a ring structure, and the benzene ring A3 and benzene ring B3 are not crosslinked.

(Deuterium Atom Contained in Inventive Compound A)

The inventive compound A contains at least one deuterium atom.

A deuterium atom may be intentionally introduced into the inventive compound A by using a deuterated compound as a part or the whole of the raw material compound.

Here, examples of the raw material compound in which a part or the whole thereof is deuterated include a compound forming a 9-carbazolyl group in the formula (1), a compound forming a phenylene group in the formula (1), a compound forming a linker ($L^1$, $L^2$, $L^3$) in the formula (1), and a compound forming a terminal ($Ar^1$, $Ar^2$) in the formula (1).

In the formula (1), it is preferable that at least one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, a hydrogen atom directly bonded to a monovalent heterocyclic group represented by $Ar^2$, a hydrogen atom represented by $R^{11}$ to $R^{14}$, and a hydrogen atom represented by $R^{21}$ to $R^{28}$ is a deuterium atom.

That is, in the following formula (1-11), the sum of a, b, c, d, e, f, and g is preferably 1 or more.

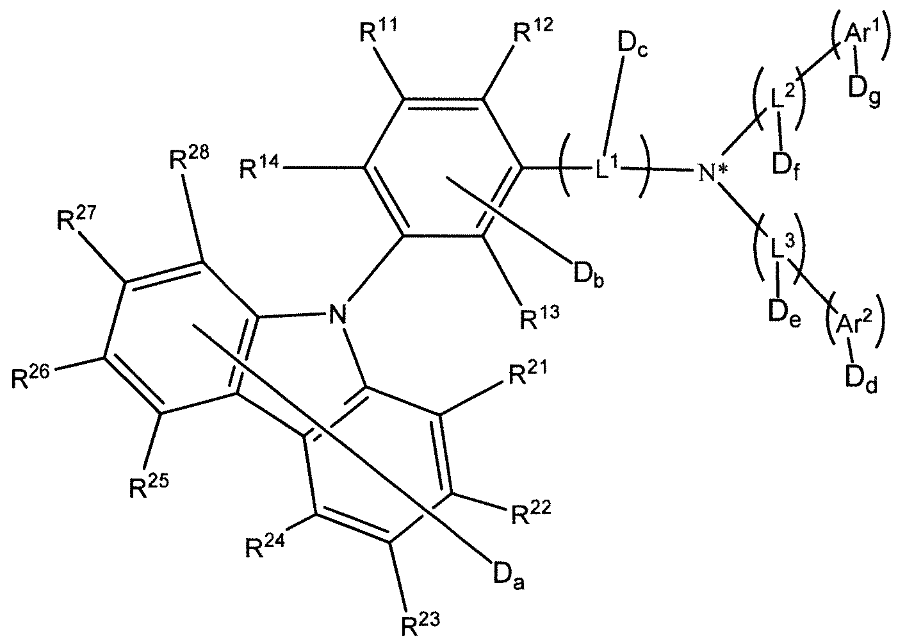

(1-11)

In the formula (1-11), "Da" represents that "a" of the hydrogen atoms represented by $R^{21}$ to $R^{28}$ indicates the number of deuterium atoms, "Db" represents that "b" of the hydrogen atoms represented by $R^{11}$ to $R^{14}$ indicates the number of deuterium atoms, "Dc" represents that "c" of the hydrogen atoms directly bonded to the arylene group or divalent heterocyclic group represented by $L^1$ indicates the number of deuterium atoms, "Dd" represents that "d" of the hydrogen atoms directly bonded to the aryl group or the hydrogen atoms directly bonded to the monovalent heterocyclic group represented by $Ar^2$ indicates the number of deuterium atoms, "De" represents that "e" of the hydrogen atoms directly bonded to the non-fused arylene group or divalent heterocyclic group represented by $L^3$ indicates the number of deuterium atoms, "Df" represents that "f" of the hydrogen atoms directly bonded to the non-fused arylene group or divalent heterocyclic group represented by $L^2$ indicates the number of deuterium atoms, and "Dg" represents that "g" of the hydrogen atoms directly bonded to the aryl group or monovalent heterocyclic group represented by $Ar^1$ indicates the number of deuterium atoms.

More preferably, in the formula (1), at least one deuterium atom contained in the inventive compound A is at least any one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, a hydrogen atom directly bonded to a monovalent heterocyclic group represented by $Ar^2$, a hydrogen atom represented by $R^{11}$ to $R^{14}$, and a hydrogen atom represented by $R^{21}$ to $R^{28}$ (that is, when $Ar^1$, $Ar^2$, $L^1$ to $L^3$, $R^{11}$ to $R^{14}$, and $R^{21}$ to $R^{28}$ are substituted in the formula (1), the substituents do not contain a deuterium atom).

In the formula (1), it is preferable that at least one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^2$, a hydrogen atom directly bonded to a monovalent heterocyclic group represented by $Ar^2$, a hydrogen atom represented by $R^{11}$ to $R^{14}$, and a hydrogen atom represented by $R^{21}$ to $R^{28}$ is a deuterium atom.

That is, in the formula (1-11), the sum of a, b, c, d, e, and f is preferably 1 or more.

More preferably, in the formula (1), at least one deuterium atom contained in the inventive compound A is at least any one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^2$, a hydrogen atom directly bonded to a monovalent heterocyclic group represented by $Ar^2$, a hydrogen atom represented by $R^{11}$ to $R^{14}$, and a hydrogen atom represented by $R^{21}$ to $R^{28}$ (that is, when $Ar^1$, $Ar^2$, $L^1$ to $L^3$, $R^{11}$ to $R^{14}$, and $R^{21}$ to $R^{28}$ are substituted in the formula (1), the substituents do not contain a deuterium atom, and the hydrogen atom directly bonded to the aryl group represented by $Ar^1$ and the hydrogen atom directly bonded to the monovalent heterocyclic group represented by $Ar^1$ do not contain a deuterium atom).

In the formula (1), it is more preferable that at least one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, a hydrogen atom directly bonded to a monovalent heterocyclic group represented by $Ar^2$, and a hydrogen atom represented by $R^{11}$ to $R^{14}$ is a deuterium atom.

That is, in the formula (1-11), the sum of b, c, d, e, f, and g is preferably 1 or more.

More preferably, in the formula (1), at least one deuterium atom contained in the inventive compound A is at least any one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, a hydrogen atom directly bonded to a monovalent heterocyclic group represented by $Ar^2$, and a hydrogen atom represented by $R^{11}$ to $R^{14}$ (that is, when $Ar^1$, $Ar^2$, $L^1$ to $L^3$, $R^{11}$ to $R^{14}$, and $R^{21}$ to $R^{28}$ are substituted

in the formula (1), the substituents do not contain a deuterium atom, and the hydrogen atom represented by $R^{21}$ to $R^{28}$ does not contain a deuterium atom).

In the formula (1), it is preferable that at least one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, and a hydrogen atom represented by $R^{11}$ to $R^{14}$ is a deuterium atom.

That is, in the formula (1-11), the sum of b, c, e, and f is preferably 1 or more.

More preferably, in the formula (1), at least one deuterium atom contained in the inventive compound A is at least any one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, and a hydrogen atom represented by $R^{11}$ to $R^{14}$ (that is, when $Ar^1$, $Ar^2$, $L^1$ to $L^3$, $R^{11}$ to $R^{14}$, and $R^{21}$ to $R^{28}$ are substituted in the formula (1), the substituents do not contain a deuterium atom, and the hydrogen atom directly bonded to the aryl group represented by $Ar^1$, the hydrogen atom directly bonded to the monovalent heterocyclic group represented by $Ar^1$, the hydrogen atom directly bonded to the aryl group represented by $Ar^2$, the hydrogen atom directly bonded to the monovalent heterocyclic group represented by $Ar^2$, and the hydrogen atom represented by $R^{21}$ to $R^{28}$ do not contain a deuterium atom).

In the formula (1), it is preferable that at least one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, and a hydrogen atom directly bonded to a monovalent heterocyclic group represented by $Ar^2$ is a deuterium atom.

That is, in the formula (1-11), the sum of c, d, e, f, and g is preferably 1 or more.

More preferably, in the formula (1), at least one deuterium atom contained in the inventive compound A is at least any one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, and a hydrogen atom directly bonded to a monovalent heterocyclic group represented by $Ar^2$ (that is, when $Ar^1$, $Ar^2$, $L^1$ to $L^3$, $R^{11}$ to $R^{14}$, and $R^{21}$ to $R^{28}$ are substituted in the formula (1), the substituents do not contain a deuterium atom, and the hydrogen atom represented by $R^{11}$ to $R^{14}$ and the hydrogen atom represented by $R^{21}$ to $R^{28}$ do not contain a deuterium atom).

In the formula (1), it is preferable that at least one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, and a hydrogen atom directly bonded to a divalent heterocyclic group represented by $L^3$ is a deuterium atom.

That is, in the formula (1-11), the sum of c, e, and f is preferably 1 or more.

More preferably, in the formula (1), at least one deuterium atom contained in the inventive compound A is at least any one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, and a hydrogen atom directly bonded to a divalent heterocyclic group represented by $L^3$ (that is, when $Ar^1$, $Ar^2$, $L^1$ to $L^3$, $R^{11}$ to $R^{14}$, and $R^{21}$ to $R^{28}$ are substituted in the formula (1), the substituents do not contain a deuterium atom, and the hydrogen atom directly bonded to the aryl group represented by $Ar^1$, the hydrogen atom directly bonded to the monovalent heterocyclic group represented by $Ar^1$, the hydrogen atom directly bonded to the aryl group represented by $Ar^2$, the hydrogen atom directly bonded to the monovalent heterocyclic group represented by $Ar^2$, the hydrogen atom represented by $R^{11}$ to $R^{14}$, and the hydrogen atom represented by $R^{21}$ to $R^{28}$ do not contain a deuterium atom).

In the formula (1), it is preferable that at least one of a hydrogen atom directly bonded to a non-fused arylene

group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, and a hydrogen atom directly bonded to a monovalent heterocyclic group represented by $Ar^2$ is a deuterium atom.

That is, in the formula (1-11), the sum of d, e, f, and g is preferably 1 or more.

More preferably, in the formula (1), at least one deuterium atom contained in the inventive compound A is at least any one of a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, and a hydrogen atom directly bonded to a monovalent heterocyclic group represented by $Ar^2$ (that is, when $Ar^1$, $Ar^2$, $L^1$ to $L^3$, $R^{11}$ to $R^{14}$, and $R^{21}$ to $R^{28}$ are substituted in the formula (1), the substituents do not contain a deuterium atom, and the hydrogen atom directly bonded to the arylene group represented by $L^1$, the hydrogen atom directly bonded to the divalent heterocyclic group represented by $L^1$, the hydrogen atom represented by $R^{11}$ to $R^{14}$, and the hydrogen atom represented by $R^{21}$ to $R^{28}$ do not contain a deuterium atom).

In the formula (1), it is preferable that at least one of a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, and a hydrogen atom directly bonded to a divalent heterocyclic group represented by $L^3$ is a deuterium atom.

That is, in the formula (1-11), the sum of e and f is preferably 1 or more.

More preferably, in the formula (1), at least one deuterium atom contained in the inventive compound A is at least any one of a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, and a hydrogen atom directly bonded to a divalent heterocyclic group represented by $L^3$ (that is, when $Ar^1$, $Ar^2$, $L^1$ to $L^3$, $R^{11}$ to $R^{14}$, and $R^{21}$ to $R^{28}$ are substituted in the formula (1), the substituents do not contain a deuterium atom, and the hydrogen atom directly bonded to the arylene group represented by $L^1$, the hydrogen atom directly bonded to the divalent heterocyclic group represented by $L^1$, the hydrogen atom directly bonded to the aryl group represented by $Ar^1$, the hydrogen atom directly bonded to the monovalent heterocyclic group represented by $Ar^1$, the hydrogen atom directly bonded to the aryl group represented by $Ar^2$, the hydrogen atom directly bonded to the monovalent heterocyclic group represented by $Ar^2$, the hydrogen atom represented by $R^{11}$ to $R^{14}$, and the hydrogen atom represented by $R^{21}$ to $R^{28}$ do not contain a deuterium atom).

The deuteration rate of the inventive compound A depends on the deuteration rate of the raw material compound used. Even if a raw material having a predetermined deuteration rate is used, a naturally derived light hydrogen isotope can be contained at a certain ratio. Accordingly, the aspect of the deuteration rate of the inventive compound A shown below includes a ratio obtained by simply counting the number of deuterium atoms represented by the chemical formula in consideration of a trace amount of naturally derived isotope.

The deuteration rate of the inventive compound A is preferably 1% or more, more preferably 3% or more, still more preferably 5% or more, even more preferably 10% or more, and particularly preferably 50% or more.

The inventive compound A may be a mixture of a deuterated compound (i.e., a compound having deuterium atoms intentionally introduced thereto) and a non-deuterated compound, or a mixture of two or more compounds having different deuteration rates from each other. The deuteration rate of such a mixture (the proportion of the number of deuterium atoms with respect to the total number of hydrogen atoms in the inventive compound A contained in the mixture) is preferably 1% or more, more preferably 3% or more, still more preferably 5% or more, even more preferably 10% or more, particularly preferably 50% or more, and less than 100%.

In the inventive compound A, the proportion of the number of deuterium atoms with respect to the total number of hydrogen atoms in $L^1$ to $L^3$ and $R^{11}$ to $R^{14}$ is preferably 10% or more, more preferably 20% or more, still more preferably 30% or more, even more preferably 40% or more, and particularly preferably 50% or more.

Preferred examples of the inventive compound A include compounds represented by the following formulae (1-11-1) to (1-11-16).

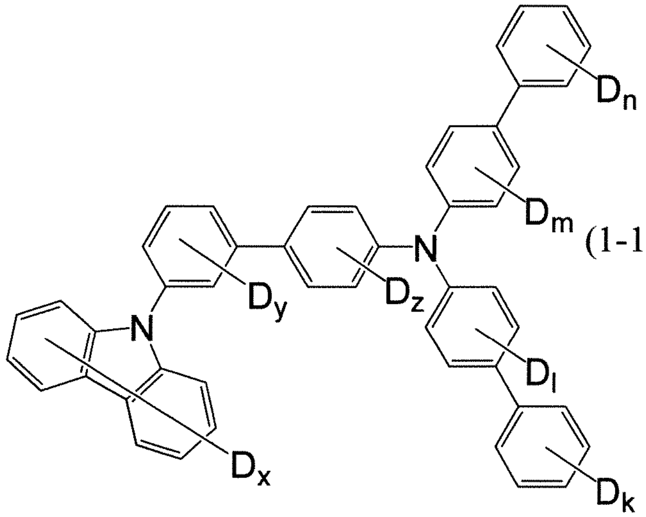

(1-11-1)

(In the formula (1-11-1), x + y + z + k + 1 + m + n = 1 to 34, and each R is omitted.)

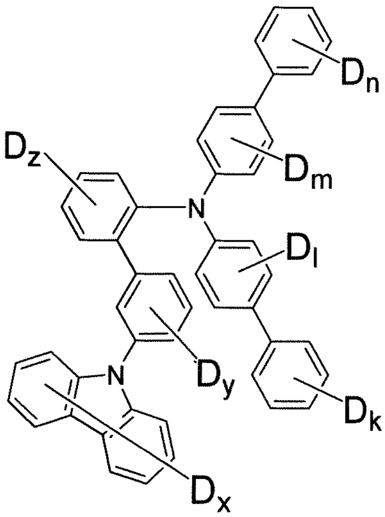

(1-11-2)

(In the formula (1-11-2), x + y + z + k + l + m + n = 1 to 34, and each R is omitted.)

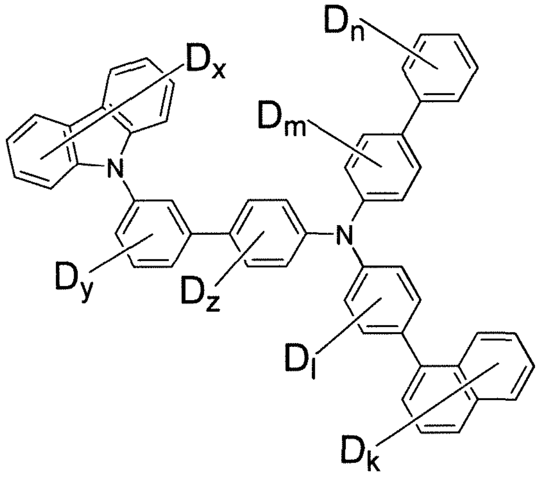

(1-11-3)

(In the formula (1-11-3), x + y + z + k + l + m + n = 1 to 36, and each R is omitted.)

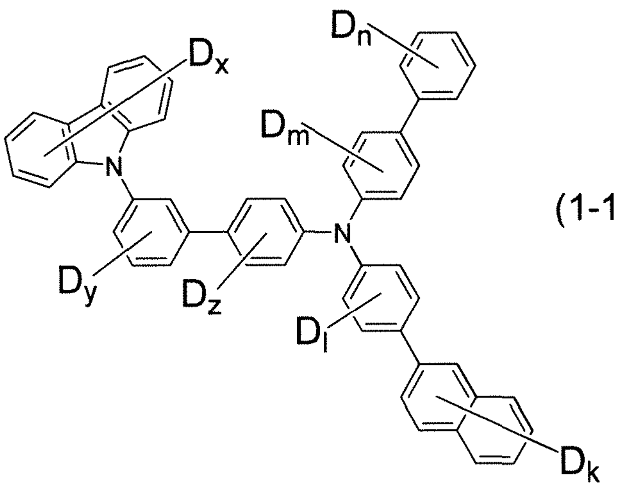

(1-11-4)

(In the formula (1-11-4), x + y + z + k + l + m + n = 1 to 36, and each R is omitted.)

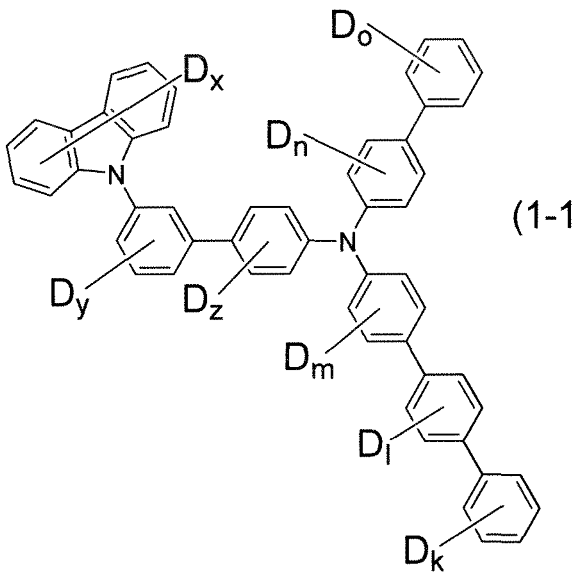

(1-11-5)

(In the formula (1-11-5), x + y + z + k + l + m + n + o = 1 to 38, and each R is omitted.)

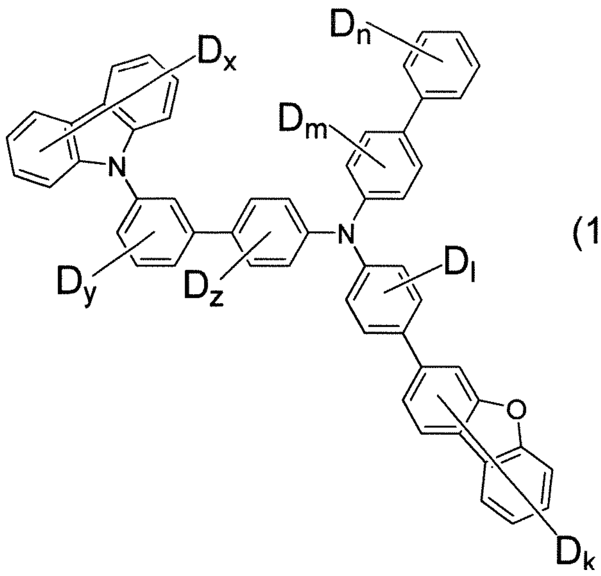

(1-11-6)

(In the formula (1-11-6), x + y + z + k + l + m + n = 1 to 36, and each R is omitted.)

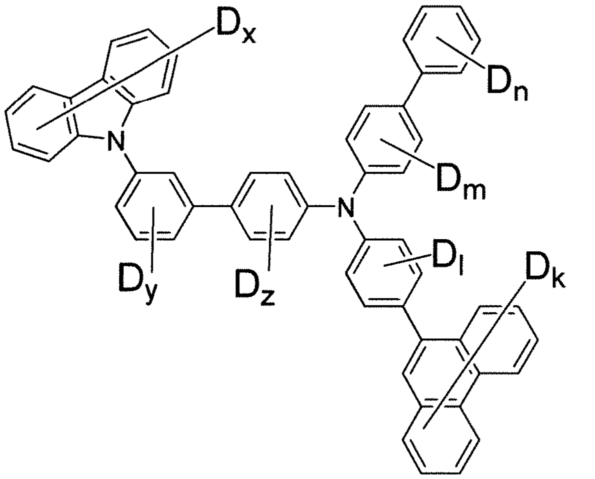

(1-11-7)

(In the formula (1-11-7), x + y + z + k + l + m + n = 1 to 38, and each R is omitted.)

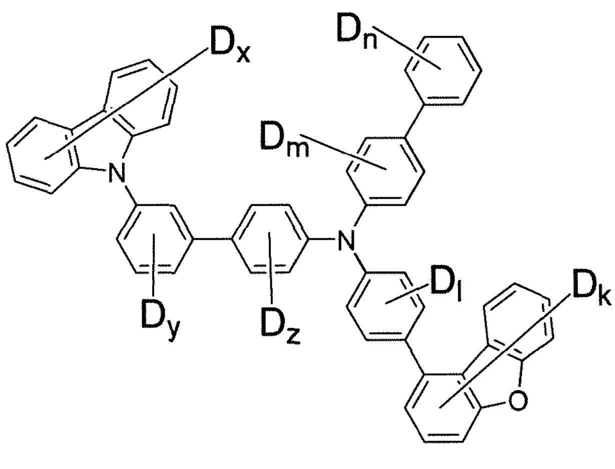

(1-11-8)

(In the formula (1-11-8), x + y + z + k + l + m + n = 1 to 36, and each R is omitted.)

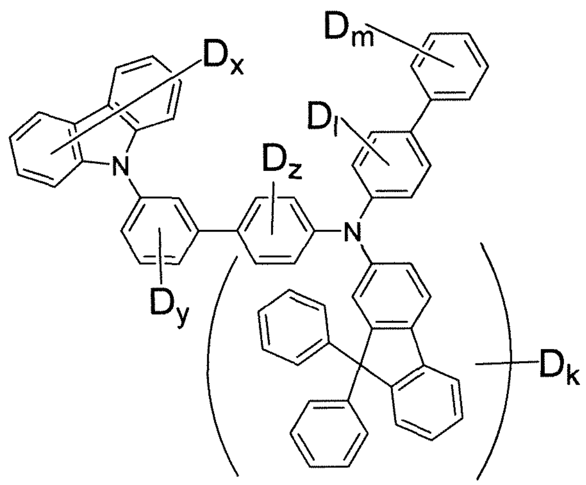

(1-11-9)

(In the formula (1-11-9), x + y + z + k + l + m = 1 to 43, and each R is omitted.)

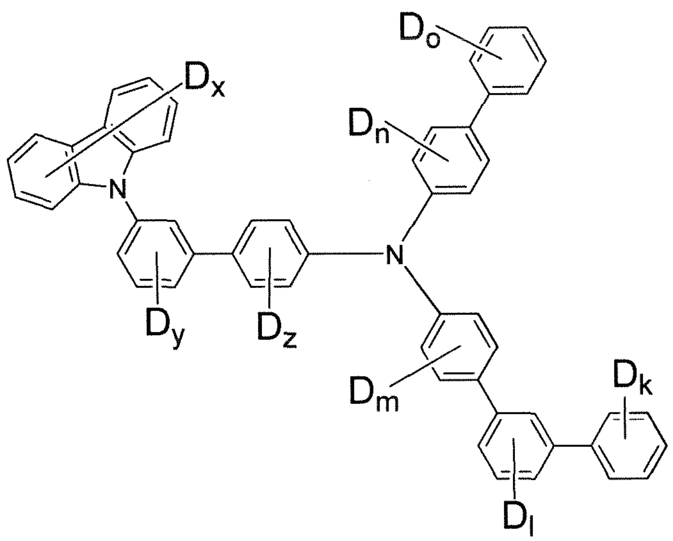

(1-11-10)

(In the formula (1-11-10), x + y + z + k + l + m + n + o = 1 to 38, and each R is omitted.)

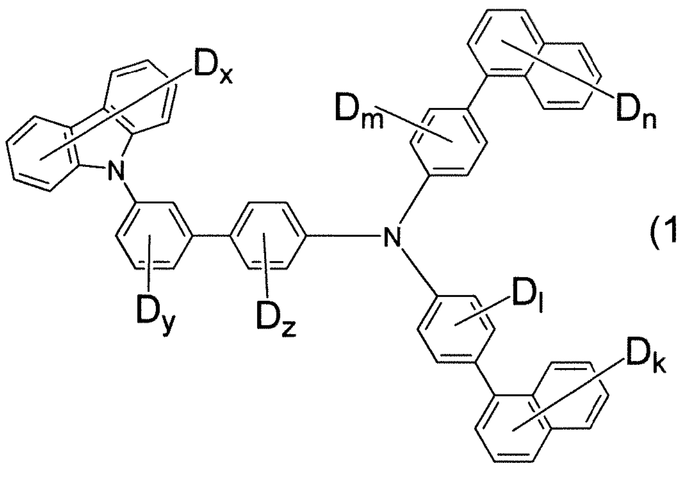

(1-11-11)

(In the formula (1-11-11), x + y + z + k + l + m + n = 1 to 38, and each R is omitted.)

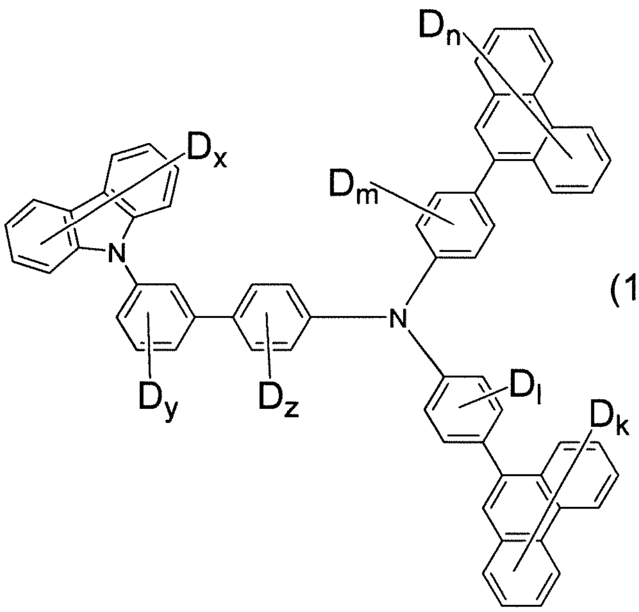

(1-11-12)

(In the formula (1-11-12), x + y + z + k + l + m + n = 1 to 42, and each R is omitted.)

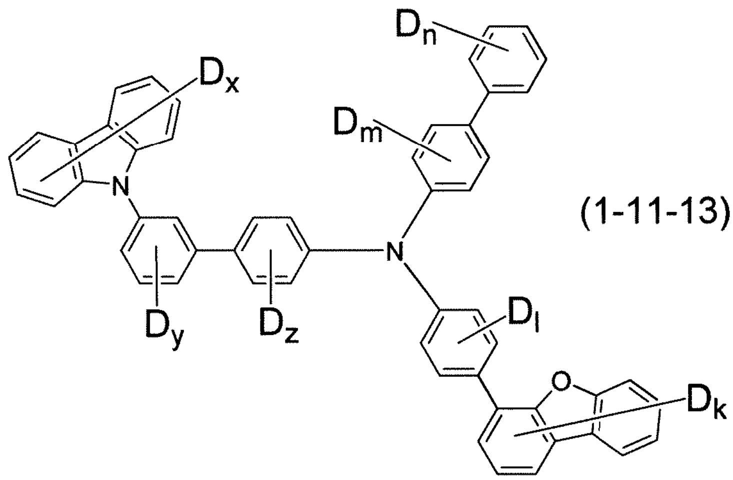

(1-11-13)

(In the formula (1-11-13), x + y + z + k + l + m + n = 1 to 36, and each R is omitted.)

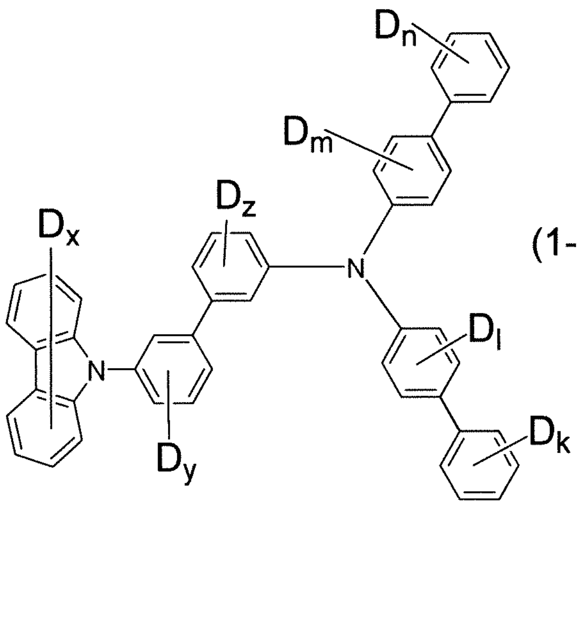

(1-11-14)

(In the formula (1-11-14), x + y + z + k + l + m + n = 1 to 34, and each R is omitted.)

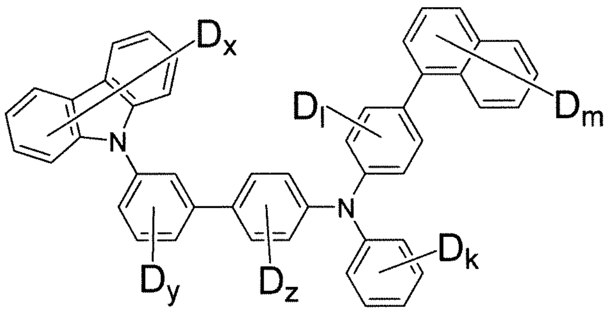

(1-11-15)

(In the formula (1-11-15), x + y + z + k + l + m = 1 to 32, and each R is omitted.)

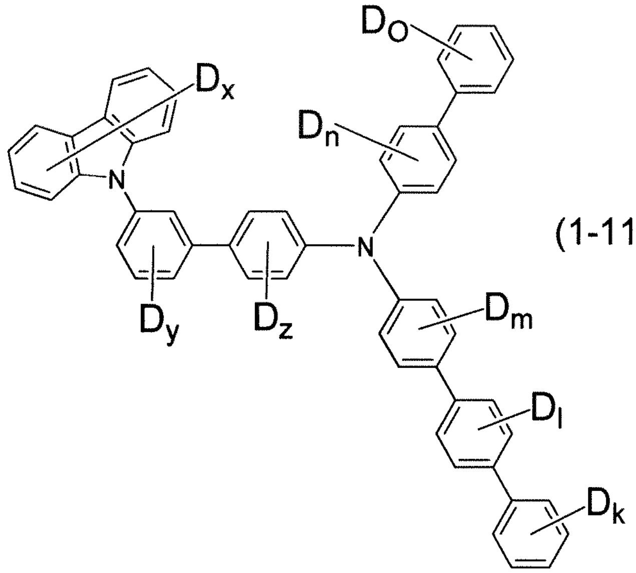

(1-11-16)

(In the formula (1-11-16), x + y + z + k + l + m + n + o = 1 to 38, and each R is omitted.)

Unless otherwise specified, the details of the substituent (arbitrary substituent) in the expression "substituted or unsubstituted" included in the definitions of the aforementioned formulae are the same as in the section of "Substituent for "Substituted or Unsubstituted"".

The inventive compound A can be readily produced by a person skilled in the art with reference to the following synthesis examples and the known synthesis methods.

<Inventive Compound B>

The compound according to one embodiment of the present invention is represented by the following formula (2).

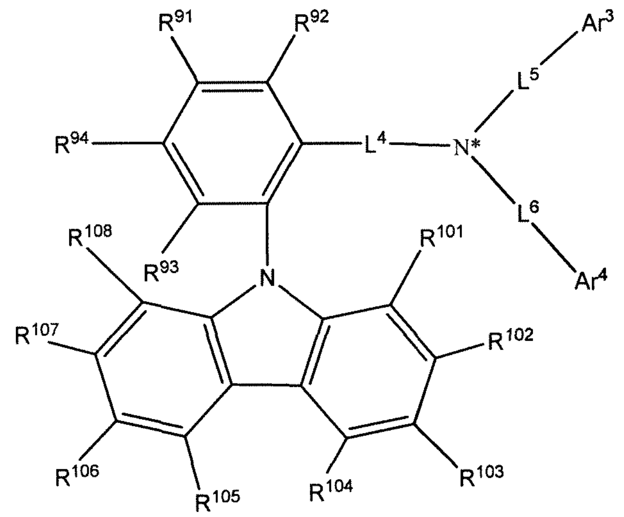

(2)

Hereinafter, symbols in the formula (2) and each formula included in the formula (2) described later will be described. Note that the same symbols have the same meaning.

In the formula (2), N* is a central nitrogen atom.

($Ar^3$, $Ar^4$)

In the formula (2), $Ar^3$ and $Ar^4$ are each independently a substituted or unsubstituted aryl group having 6 to 30 ring carbon atoms (excluding a phenyl group) or a substituted or unsubstituted monovalent heterocyclic group having 5 to 30 ring atoms.

(Aryl group represented by $Ar^3$ and $Ar^4$ (excluding phenyl group))

The details of the substituted or unsubstituted aryl group having 6 to 30 ring carbon atoms represented by $Ar^3$ and $Ar^4$ (excluding a phenyl group) are as described above in the section of "Substituents in Description".

The substituted or unsubstituted aryl group having 6 to 30 ring carbon atoms (excluding a phenyl group) represented by $Ar^3$ and $Ar^4$ is preferably a substituted or unsubstituted aryl group having 10 to 30 ring carbon atoms, and more preferably a biphenylyl group, a terphenylyl group, a naphthyl group, an acenaphthylenyl group, an anthryl group, a benzoanthryl group, an aceanthryl group, a phenanthryl group, a benzo[c]phenanthryl group, a phenalenyl group, or a fluorenyl group.

(Heterocyclic group represented by $Ar^3$ and $Ar^4$)

The details of the substituted or unsubstituted monovalent heterocyclic group having 5 to 30 ring atoms represented by $Ar^3$ and $Ar^4$ are as described above in the section of "Substituents in Description".

The unsubstituted monovalent heterocyclic group represented by $Ar^3$ and $Ar^4$ is preferably a heterocyclic group selected from a pyridyl group, a pyrimidinyl group, a triazinyl group, a quinolyl group, an isoquinolyl group, a quinazolinyl group, a benzimidazolyl group, a phenanthrolinyl group, a carbazolyl group (1-carbazolyl group, 2-carbazolyl group, 3-carbazolyl group, 4-carbazolyl group, or 9-carbazolyl group), a benzocarbazolyl group, a dibenzofuranyl group, a naphthobenzofuranyl group, a dibenzothiophenyl group, and a naphthobenzothiophenyl group, and more preferably a heterocyclic group selected from a pyridyl group, a dibenzofuranyl group, and a dibenzothiophenyl group.

($L^4$, $L^5$, $L^6$)

In the formula (2), $L^4$, $L^5$ and $L^6$ are each independently any one of a single bond, a substituted or unsubstituted arylene group having 6 to 30 ring carbon atoms, and a substituted or unsubstituted divalent heterocyclic group having 5 to 30 ring atoms.

(Arylene group represented by $L^4$, $L^5$, and $L^6$)

The details of the substituted or unsubstituted arylene group having 6 to 30 ring carbon atoms represented by

$L^4$, $L^5$, and $L^6$ are as described above in the section of "Substituents in Description".

The unsubstituted arylene group represented by $L^4$, $L^5$, and $L^6$ is preferably a phenylene group, a biphenylene group, or a terphenylene group.

(Heterocyclic group represented by $L^4$, $L^5$, and $L^6$)

The details of the substituted or unsubstituted divalent heterocyclic group having 5 to 30 ring atoms represented by $L^4$, $L^5$, and $L^6$ are as described above in the section of "Substituents in Description".

(Substituent of $Ar^3$, Substituent of $Ar^4$, Substituent of $L^4$, Substituent of $L^5$, and Substituent of $L^6$)

The substituent of $Ar^3$, the substituent of $Ar^4$, the substituent of $L^4$, the substituent of $L^5$, and the substituent of $L^6$ are each independently the same as the substituent of $Ar^1$, the substituent of $Ar^2$, the substituent of $L^1$, the substituent of $L^2$, and the substituent of $L^3$ in the formula (1) described above, and preferred embodiments thereof are also the same.

($R^{91}$ to $R^{94}$, $R^{101}$ to $R^{108}$)

In the formula (2), $R^{91}$ to $R^{94}$ and $R^{101}$ to $R^{108}$ are each independently a hydrogen atom or a substituent, and the substituent is a halogen atom; a nitro group; a cyano group; a substituted or unsubstituted alkyl group having 1 to 50 carbon atoms; a substituted or unsubstituted alkenyl group having 2 to 50 carbon atoms; a substituted or unsubstituted alkynyl group having 2 to 50 carbon atoms; a substituted or unsubstituted cycloalkyl group having 3 to 50 ring carbon atoms; a substituted or unsubstituted haloalkyl group having 1 to 50 carbon atoms; a substituted or unsubstituted alkoxy group having 1 to 50 carbon atoms; a substituted or unsubstituted haloalkoxy group having 1 to 50 carbon atoms; a substituted or unsubstituted alkylthio group having 1 to 50 carbon atoms; a substituted or unsubstituted aryl group having 6 to 50 ring carbon atoms; a substituted or unsubstituted aryloxy group having 6 to 50 ring carbon atoms; a substituted or unsubstituted arylthio group having 6 to 50 ring carbon atoms; a substituted or unsubstituted aralkyl group having 7 to 50 carbon atoms; a substituted or unsubstituted monovalent heterocyclic group having 5 to 50 ring atoms; or a mono-, di-, or tri-substituted silyl group having a substituent selected from a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, and a substituted or unsubstituted monovalent heterocyclic group having 5 to 50 ring atoms.

(Halogen atom)

The details of the halogen atom represented by $R^{91}$ to $R^{94}$ and $R^{101}$ to $R^{108}$ are as described above in the section of "Substituents in Description".

(Alkyl group)

The details of the substituted or unsubstituted alkyl group having 1 to 50 carbon atoms represented by $R^{91}$ to $R^{94}$ and $R^{101}$ to $R^{108}$ are as described above in the section of "Substituents in Description".

(Alkenyl group)

The details of the substituted or unsubstituted alkenyl group having 2 to 50 carbon atoms represented by $R^{91}$ to $R^{94}$ and $R^{101}$ to $R^{108}$ are as described above in the section of "Substituents in Description".

(Alkynyl group)

The details of the substituted or unsubstituted alkynyl group having 2 to 50 carbon atoms represented by $R^{91}$ to $R^{94}$ and $R^{101}$ to $R^{108}$ are as described above in the section of "Substituents in Description".

(Cycloalkyl group)

The details of the substituted or unsubstituted cycloalkyl group having 3 to 50 ring carbon atoms represented by $R^{91}$ to $R^{94}$ and $R^{101}$ to $R^{108}$ are as described above in the section of "Substituents in Description".

(Haloalkyl group)

The details of the substituted or unsubstituted haloalkyl group having 1 to 50 carbon atoms represented by $R^{91}$ to $R^{94}$ and $R^{101}$ to $R^{108}$ are as described above in the section of "Substituents in Description".

(Alkoxy group)

The details of the substituted or unsubstituted alkoxy group having 1 to 50 carbon atoms represented by $R^{91}$ to $R^{94}$ and $R^{101}$ to $R^{108}$ are as described above in the section of "Substituents in Description".

(Haloalkoxy group)

The substituted or unsubstituted haloalkoxy group having 1 to 50 carbon atoms represented by $R^{91}$ to $R^{94}$ and $R^{101}$ to $R^{108}$ is a group represented by -O(G12), and G12 is the aforementioned substituted or unsubstituted haloalkyl group.

(Alkylthio group)

The details of the substituted or unsubstituted alkylthio group having 1 to 50 carbon atoms represented by $R^{91}$ to $R^{94}$ and $R^{101}$ to $R^{108}$ are as described above in the section of "Substituents in Description".

(Aryl group)

The details of the substituted or unsubstituted aryl group having 6 to 50 ring carbon atoms represented by $R^{91}$ to $R^{94}$ and $R^{101}$ to $R^{108}$ are as described above in the section of "Substituents in Description".

(Aryloxy group)

The details of the substituted or unsubstituted aryloxy group having 6 to 50 ring carbon atoms represented by $R^{91}$ to $R^{94}$ and $R^{101}$ to $R^{108}$ are as described above in the section of "Substituents in Description".

(Arylthio group)

The details of the substituted or unsubstituted arylthio group having 6 to 50 ring carbon atoms represented by $R^{91}$ to $R^{94}$ and $R^{101}$ to $R^{108}$ are as described above in the section of "Substituents in Description".

(Aralkyl group)

The details of the substituted or unsubstituted aralkyl group having 7 to 50 carbon atoms represented by $R^{91}$ to $R^{94}$ and $R^{101}$ to $R^{108}$ are as described above in the section of "Substituents in Description".

(Heterocyclic group)

The details of the substituted or unsubstituted monovalent heterocyclic group having 5 to 50 ring carbon atoms represented by $R^{91}$ to $R^{94}$ and $R^{101}$ to $R^{108}$ are as described above in the section of "Substituents in Description".

(Substituted silyl group)

The details of the substituent of the mono-, di-, or tri-substituted silyl group represented by $R^{91}$ to $R^{94}$ and $R^{101}$ to $R^{108}$ are as described above in the section of "Substituents in Description".

(Structural Formula of Preferred Examples of Inventive Compound B)

Preferred examples of the inventive compound B include compounds represented by the following formulae (2-1) to (2-10). The compounds represented by the formulae (2-1) to (2-10) have at least one deuterium atom.

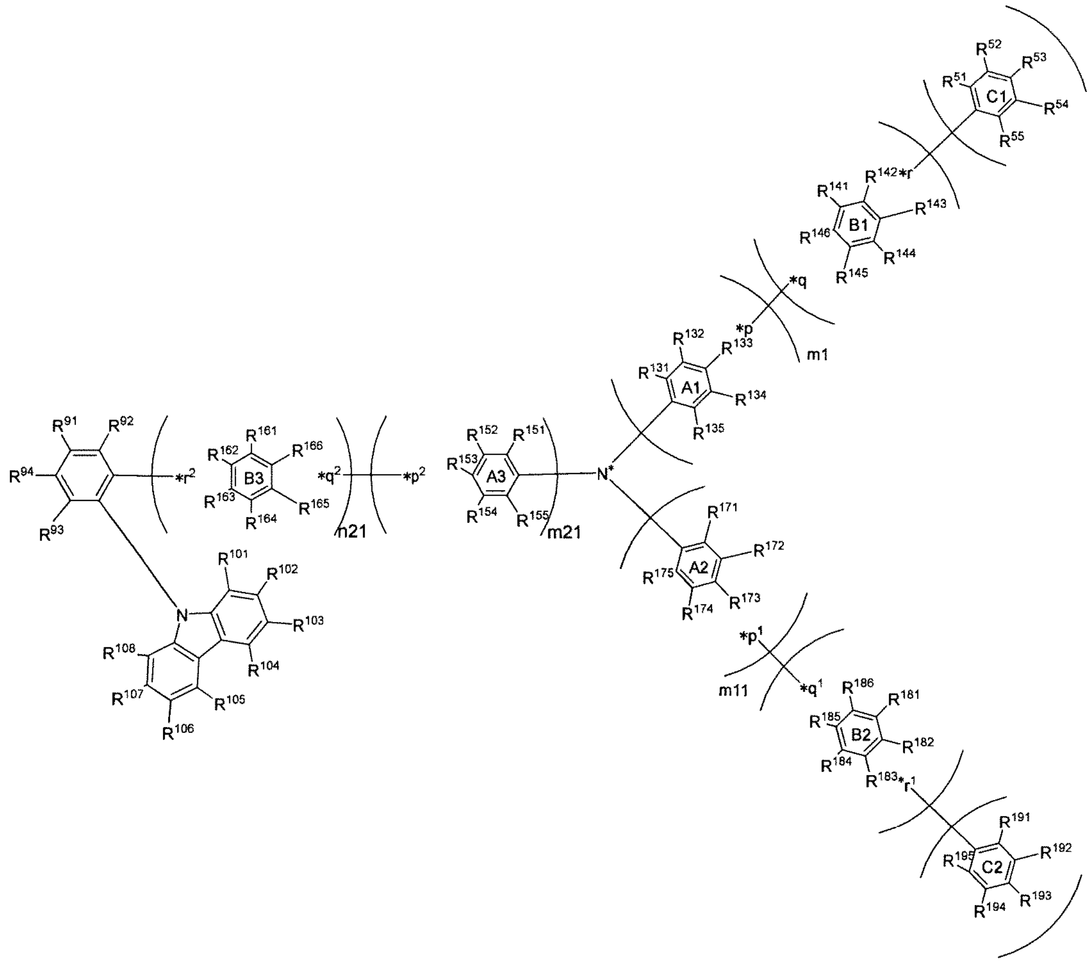

(2-1)

In the formula (2-1), $R^{91}$ to $R^{94}$ and $R^{101}$ to $R^{108}$ are as described above.

In the formula (2-1), $R^{151}$ to $R^{155}$, $R^{161}$ to $R^{166}$, $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{131}$ to $R^{135}$ is a single bond bonded to *p, one selected from $R^{141}$ to $R^{146}$ is a single bond bonded to *q and the other one selected from $R^{141}$ to $R^{146}$ is a single bond bonded to *r, one selected from $R^{171}$ to $R^{175}$ is a single bond bonded to $*p^1$, one selected from $R^{181}$ to $R^{186}$ is a single bond bonded to $*q^1$ and the other one selected from $R^{181}$ to $R^{186}$ is a single bond bonded to *r1.

In the formula (2-1), m1, m11, and m21 are each independently 0 or 1, n21 is 0 or 1, and

when m1 is 0, *p is bonded to a nitrogen atom N*,
when m11 is 0, $*p^1$ is bonded to a nitrogen atom N*,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-1), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{171}$ to $R^{175}$ that are not single bonds, adjacent two selected from $R^{181}$ to $R^{186}$ that are not single bonds, adjacent two selected from $R^{51}$ to $R^{55}$, and adjacent two selected from $R^{191}$ to $R^{195}$ are not independently bonded to each other and thus do not form a ring structure, and the benzene ring A1 and benzene ring B1, the benzene ring B1 and benzene ring C1, the benzene ring A2 and benzene ring B2, the benzene ring B2 and benzene ring C2, and the benzene ring A3 and benzene ring B3 are not crosslinked.

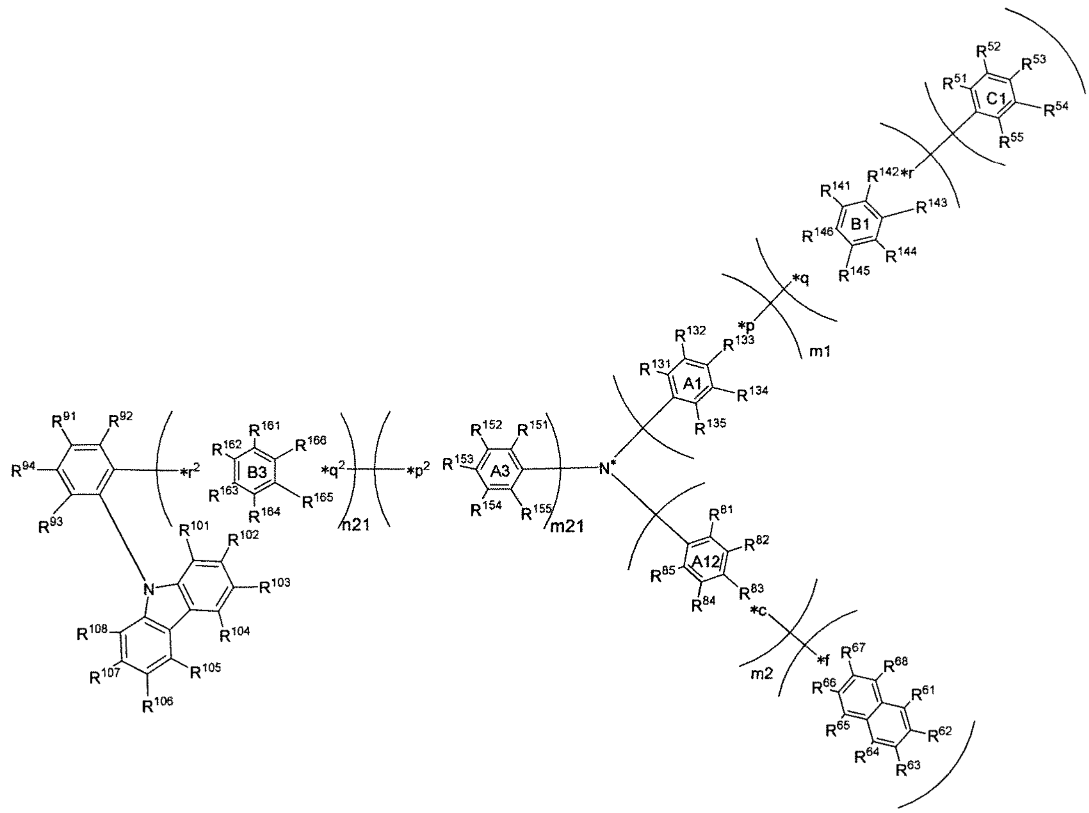

(2-2)

In the formula (2-2), $R^{91}$ to $R^{94}$ and $R^{101}$ to $R^{108}$ are as described above.

In the formula (2-2), $R^{151}$ to $R^{155}$, $R^{161}$ to $R^{166}$, $R^{131}$ to $R^{135}$, $R^{141}$ to $R^{146}$, $R^{81}$ to $R^{85}$, $R^{51}$ to $R^{55}$, and $R^{61}$ to $R^{68}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{131}$ to $R^{135}$ is a single bond bonded to *p, one selected from $R^{141}$ to $R^{146}$ is a single bond bonded to *q and the other one selected from $R^{141}$ to $R^{146}$ is a single bond bonded to *r, one selected from $R^{81}$ to $R^{85}$ is a single bond bonded to *c, and one selected from $R^{61}$ to $R^{68}$ is a single bond bonded to *f.

In the formula (2-2), m1, m2, and m21 are each independently 0 or 1, n21 is 0 or 1, and

when m1 is 0, *p is bonded to a nitrogen atom N*,
when m2 is 0, *c is bonded to a nitrogen atom N*,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-2), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{81}$ to $R^{85}$ that are not single bonds, adjacent two selected from $R^{51}$ to $R^{55}$, and adjacent two selected from $R^{61}$ to $R^{68}$ are not independently bonded to each other and thus do not form a ring structure, and the benzene ring A1 and benzene ring B1, the benzene ring B1 and benzene ring C1, and the benzene ring A3 and benzene ring B3 are not crosslinked.

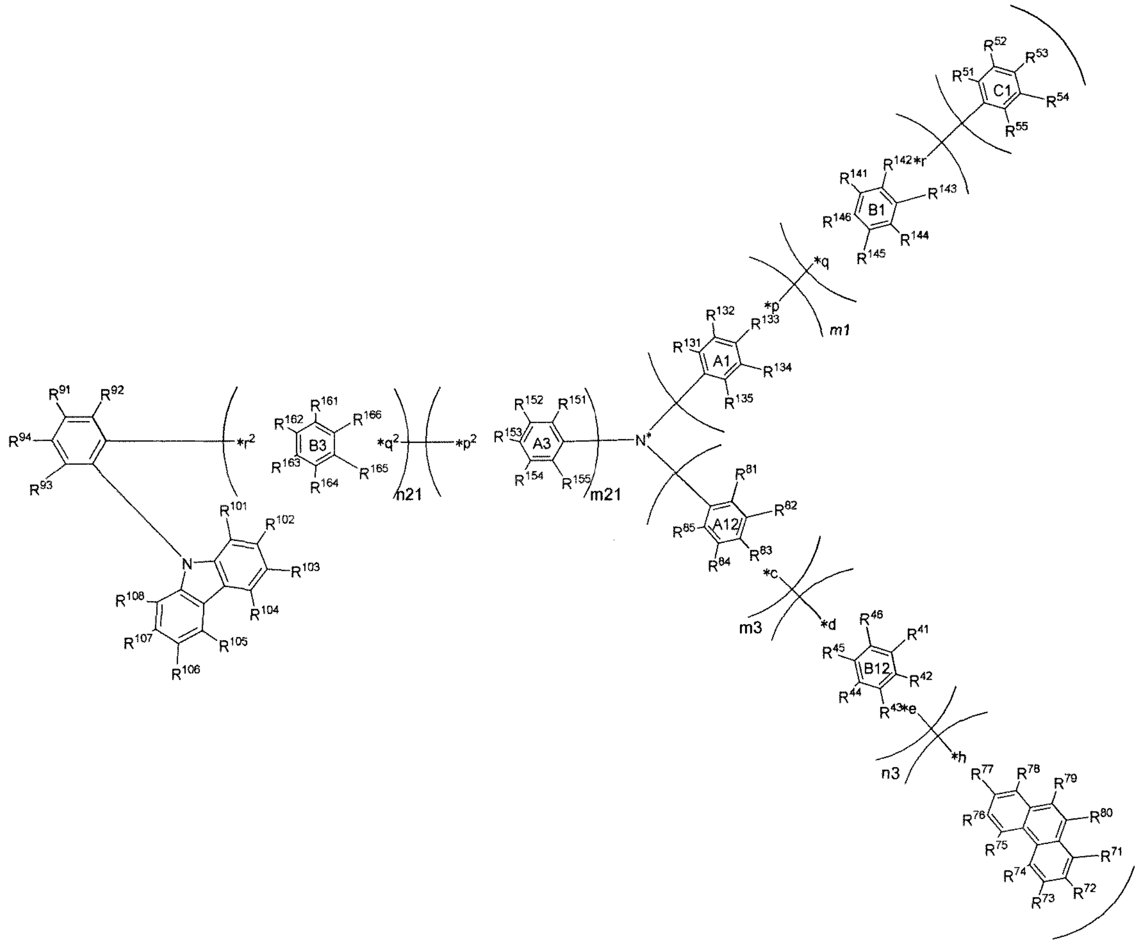

(2-3)

In the formula (2-3), $R^{91}$ to $R^{94}$ and $R^{101}$ to $R^{108}$ are as described above.

In the formula (2-3), $R^{151}$ to $R^{155}$, $R^{161}$ to $R^{166}$, $R^{131}$ to $R^{135}$, $R^{141}$ to $R^{146}$, $R^{81}$ to $R^{85}$, $R^{41}$ to $R^{46}$, $R^{51}$ to $R^{55}$, and $R^{71}$ to $R^{80}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{131}$ to $R^{135}$ is a single bond bonded to *p, one selected from $R^{141}$ to $R^{146}$ is a single bond bonded to *q and the other one selected from $R^{141}$ to $R^{146}$ is a single bond bonded to *r, one selected from $R^{81}$ to $R^{85}$ is a single bond bonded to *c, one selected from $R^{41}$ to $R^{46}$ is a single bond bonded to *d and the other one selected from $R^{41}$ to $R^{46}$ is a single bond bonded to *e, and one selected from $R^{71}$ to $R^{80}$ is a single bond bonded to *h.

In the formula (2-3), m1, m3, and m21 are each independently 0 or 1, n3 and n21 are each independently 0 or 1, and

when m1 is 0, *p is bonded to a nitrogen atom N*,
when m3 is 0 and n3 is 0, *e is bonded to a nitrogen atom N*,
when m3 is 0 and n3 is 1, *c is bonded to a nitrogen atom N*, and
when m3 is 1 and n3 is 0, one selected from $R^{81}$ to $R^{85}$ is a single bond bonded to *e,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-3), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{81}$ to $R^{85}$ that are not single bonds, adjacent two selected from $R^{41}$ to $R^{46}$ that are not single bonds, adjacent two selected from $R^{51}$ to $R^{55}$, and adjacent two selected from $R^{71}$ to $R^{80}$ are not independently bonded to each other and thus do not form a ring structure, and the

benzene ring A1 and benzene ring B1, the benzene ring B1 and benzene ring C1, the benzene ring A12 and benzene ring B12, and the benzene ring A3 and benzene ring B3 are not crosslinked.

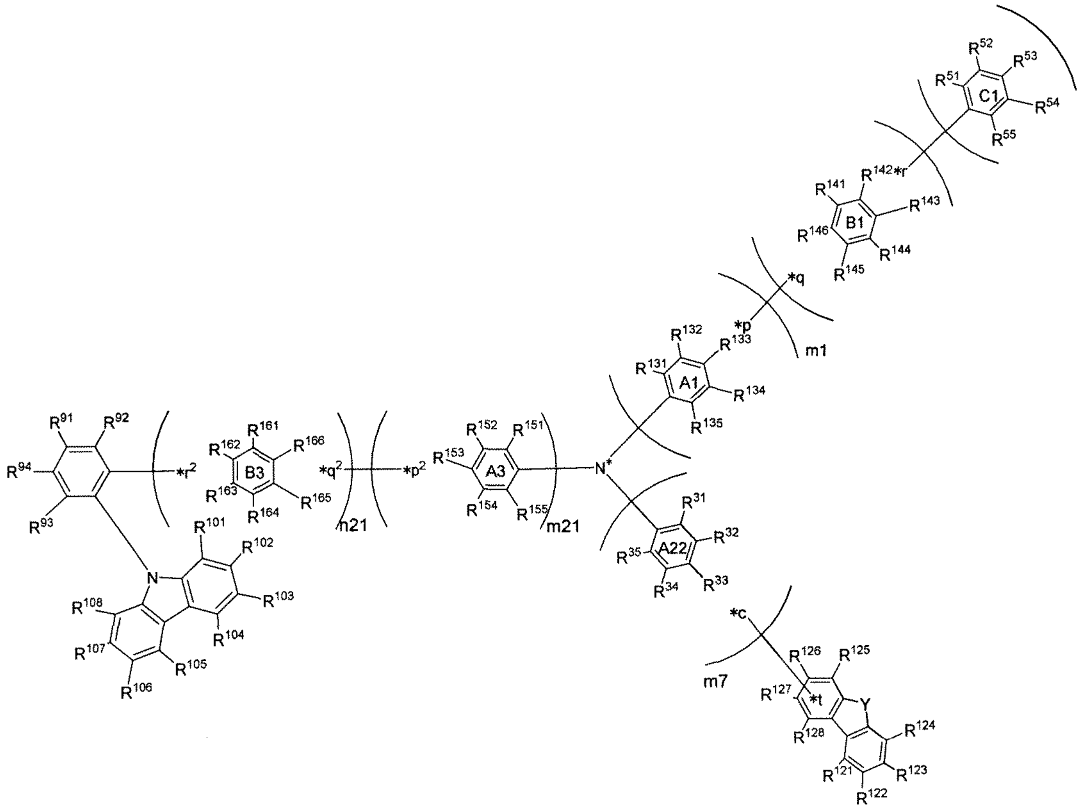

(2-4)

In the formula (2-4), $R^{91}$ to $R^{94}$ and $R^{101}$ to $R^{108}$ are as described above.

In the formula (2-4), $R^{151}$ to $R^{155}$, $R^{161}$ to $R^{166}$, $R^{131}$ to $R^{135}$, $R^{141}$ to $R^{146}$, $R^{31}$ to $R^{35}$, $R^{51}$ to $R^{55}$, and $R^{121}$ to $R^{128}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{131}$ to $R^{135}$ is a single bond bonded to *p, one selected from $R^{141}$ to $R^{146}$ is a single bond bonded to *q and the other one selected from $R^{141}$ to $R^{146}$ is a single bond bonded to *r, one selected from $R^{31}$ to $R^{35}$ is a single bond bonded to *c, and one selected from $R^{121}$ to $R^{128}$ is a single bond bonded to *t.

In the formula (2-4), m1, m7, and m21 are each independently 0 or 1, n21 is 0 or 1, and

when m1 is 0, *p is bonded to a nitrogen atom N*,
when m7 is 0, *c is bonded to a nitrogen atom N*,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-4), Y is an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group having 6 to 50 ring carbon atoms. In the case where both of $R^c$ and $R^d$ are aryl groups, $R^c$ and $R^d$ may be bonded to each other to form a spiro ring.

In the formula (2-4), adjacent two selected from $R^{131}$ to $R^{135}$ that are not single bonds, adjacent two selected from $R^{141}$ to $R^{146}$ that are not single bonds, adjacent two selected from $R^{151}$ to $R^{155}$ that are not single bonds, adjacent two selected from $R^{161}$ to $R^{166}$ that are not single bonds, adjacent two selected from $R^{31}$ to $R^{35}$ that are not single bonds, adjacent two selected from $R^{51}$ to $R^{55}$, and adjacent two selected from $R^{121}$ to $R^{124}$ and $R^{125}$ and $R^{128}$ are not independently bonded to each other and thus do not form a ring structure, and the benzene ring A1 and benzene ring B1, the benzene ring B1 and benzene ring C1, and the benzene ring A3 and benzene ring B3 are not crosslinked.

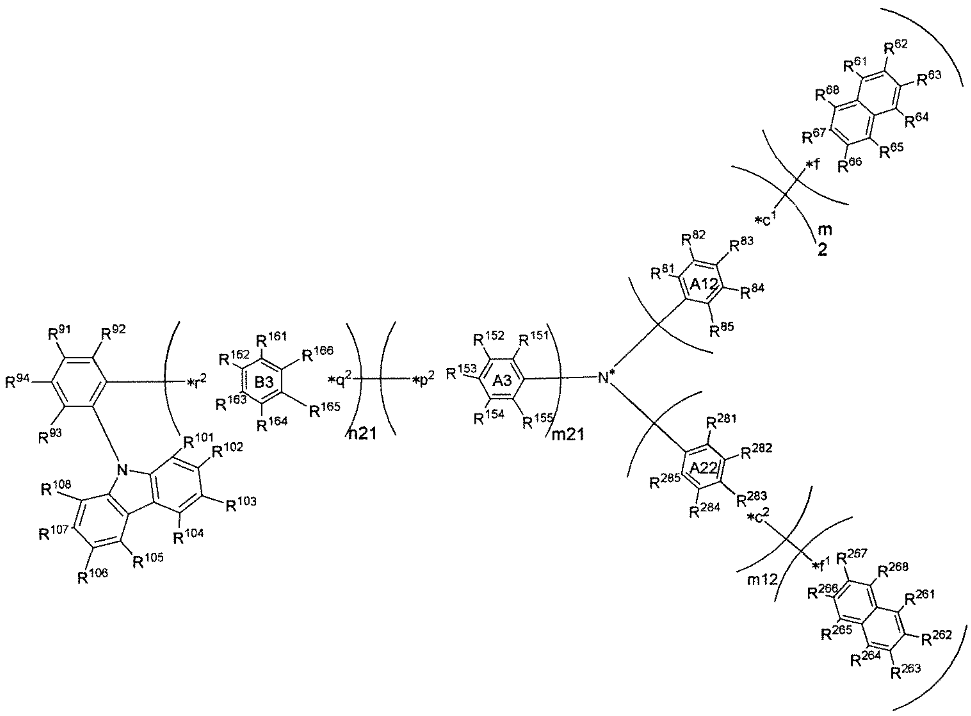

(2-5)

In the formula (2-5), $R^{91}$ to $R^{94}$ and $R^{101}$ to $R^{108}$ are as described above.

In the formula (2-5), $R^{151}$ to $R^{155}$, $R^{161}$ to $R^{166}$, $R^{81}$ to $R^{85}$, $R^{281}$ to $R^{285}$, $R^{61}$ to $R^{68}$, and $R^{261}$ to $R^{268}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{81}$ to $R^{85}$ is a single bond bonded to $*c^1$, one selected from $R^{281}$ to $R^{285}$ is a single bond bonded to $*c^2$, one selected from $R^{61}$ to $R^{68}$ is a single bond bonded to *f, and one selected from $R^{261}$ to $R^{268}$ is a single bond bonded to $*f^1$.

In the formula (2-5), m21 is 0 or 1, n21 is 0 or 1, and

when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-5), m2 is 0 or 1 and when m2 is 0, $*c^1$ is bonded to a nitrogen atom N*, and m12 is 0 or 1 and when m12 is 0, $*c^2$ is bonded to a nitrogen atom N*.

In the formula (2-5), adjacent two selected from $R^{81}$ to $R^{85}$ that are not single bonds, adjacent two selected from $R^{151}$ to $R^{155}$ that are not single bonds, adjacent two selected from $R^{161}$ to $R^{166}$ that are not single bonds, adjacent two selected from $R^{281}$ to $R^{285}$ that are not single bonds, adjacent two selected from $R^{61}$ to $R^{68}$, and adjacent two selected from $R^{261}$ to $R^{268}$ are not independently bonded to each other and thus do not form a ring structure, and the benzene ring A3 and benzene ring B3 are not crosslinked.

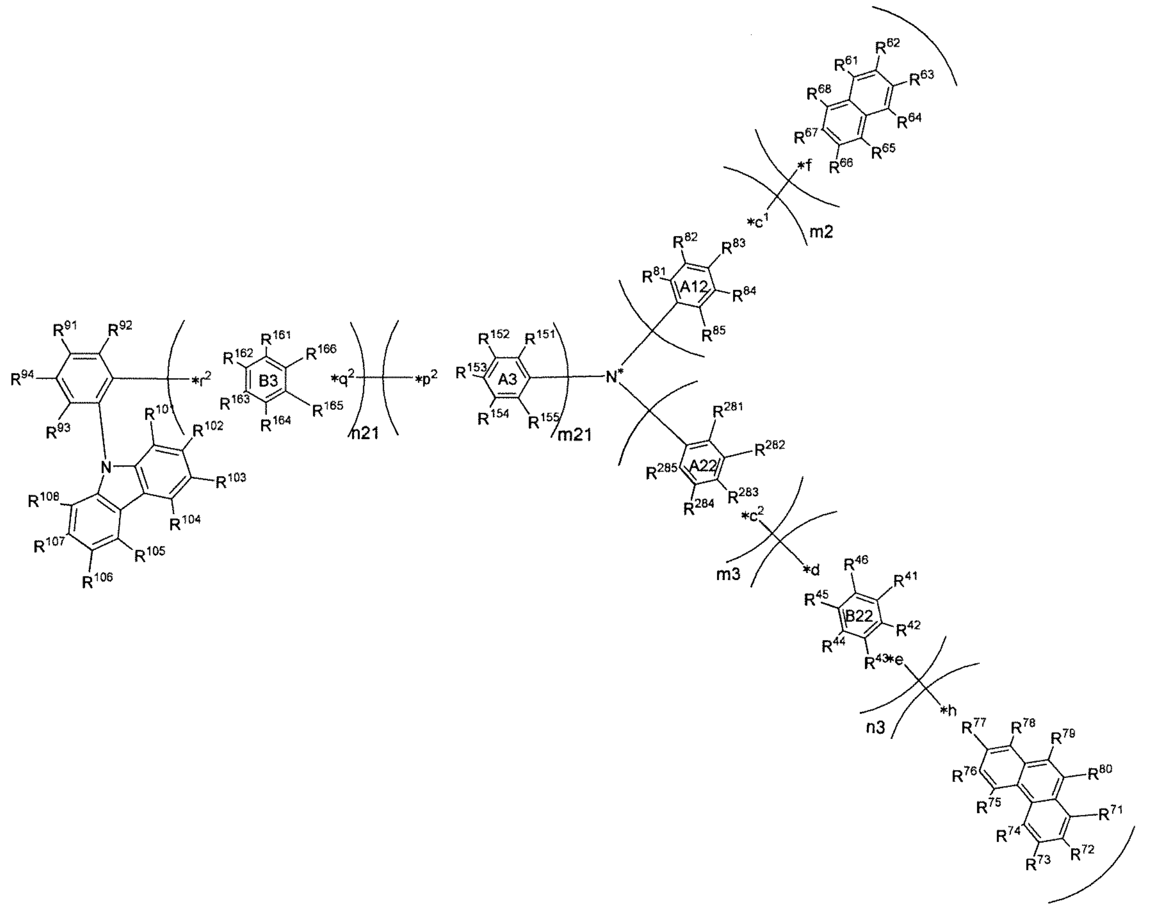

(2-6)

In the formula (2-6), $R^{91}$ to $R^{94}$ and $R^{101}$ to $R^{108}$ are as described above.

In the formula (2-6), $R^{151}$ to $R^{155}$, $R^{161}$ to $R^{166}$, $R^{81}$ to $R^{85}$, $R^{281}$ to $R^{285}$, $R^{41}$ to $R^{46}$, $R^{61}$ to $R^{68}$, and $R^{71}$ to $R^{80}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{81}$ to $R^{85}$ is a single bond bonded to $*c^1$, one selected from $R^{281}$ to $R^{285}$ is a single bond bonded to $*c^2$, one selected from $R^{41}$ to $R^{46}$ is a single bond bonded to *d and the other one selected from $R^{41}$ to $R^{46}$ is a single bond bonded to *e, one selected from $R^{61}$ to $R^{68}$ is a single bond bonded to *f, and one selected from $R^{71}$ to $R^{80}$ is a single bond bonded to *h.

In the formula (2-6), m21 and m3 are each independently 0 or 1, n21 and n3 are each independently 0 or 1, and

when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$,
when m3 is 0 and n3 is 0, *e is bonded to a nitrogen atom N*,
when m3 is 0 and n3 is 1, $*c^2$ is bonded to a nitrogen atom N*, and
when m3 is 1 and n3 is 0, one selected from $R^{281}$ to $R^{285}$ is a single bond bonded to *e,

In the formula (2-6), m2 is 0 or 1, and when m2 is 0, $*c^1$ is bonded to a nitrogen atom N*.

In the formula (2-6), adjacent two selected from $R^{81}$ to $R^{85}$ that are not single bonds, adjacent two selected from $R^{151}$ to $R^{155}$ that are not single bonds, adjacent two selected from $R^{161}$ to $R^{166}$ that are not single bonds, adjacent two selected from $R^{281}$ to $R^{285}$ that are not single bonds, adjacent two selected from $R^{41}$ to $R^{46}$ that are not single bonds, adjacent two selected from $R^{61}$ to $R^{68}$, and adjacent two selected from $R^{71}$ to $R^{80}$ are not independently bonded to each other and thus do not form a ring structure, and the benzene ring A22 and benzene ring B22, and the benzene ring A3 and benzene ring B3 are not crosslinked.

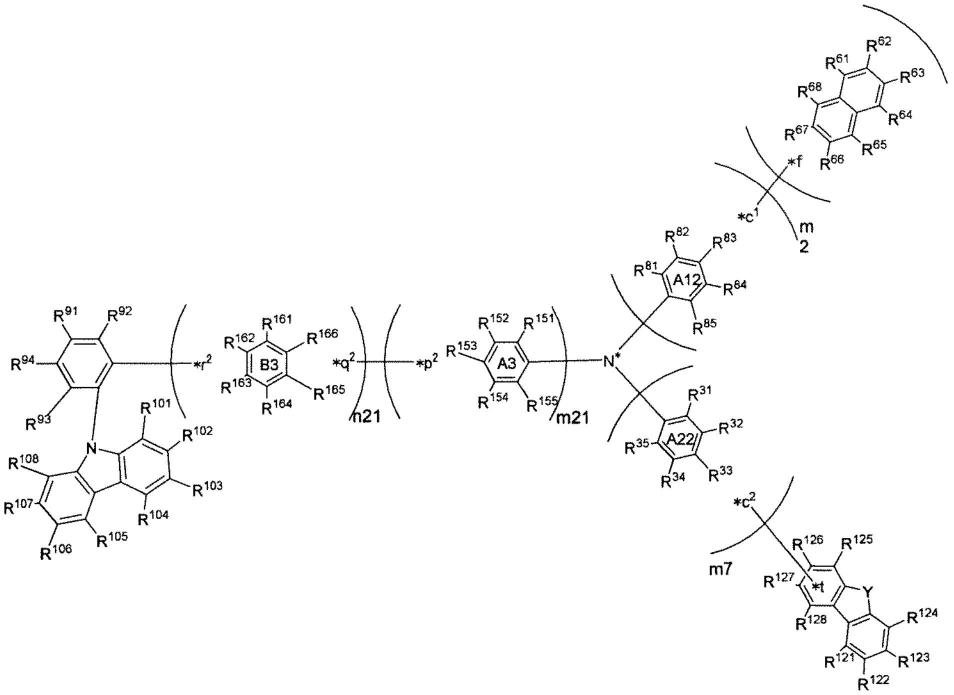

(2-7)

In the formula (2-7), $R^{91}$ to $R^{94}$ and $R^{101}$ to $R^{108}$ are as described above.

In the formula (2-7), $R^{151}$ to $R^{155}$, $R^{161}$ to $R^{166}$, $R^{81}$ to $R^{85}$, $R^{31}$ to $R^{35}$, $R^{61}$ to $R^{68}$, and $R^{121}$ to $R^{128}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{81}$ to $R^{85}$ is a single bond bonded to $*c^1$, one selected from $R^{31}$ to $R^{35}$ is a single bond bonded to $*c^2$, one selected from $R^{61}$ to $R^{65}$ is a single bond bonded to *f, and one selected from $R^{121}$ to $R^{128}$ is a single bond bonded to *t.

In the formula (2-7), m21 is 0 or 1, n21 is 0 or 1, and

when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-7), m2 is 0 or 1 and when m2 is 0, $*c^1$ is bonded to a nitrogen atom N*, and m7 is 0 or 1 and when m7 is 0, $*c^2$ is bonded to a nitrogen atom N*.

In the formula (2-7), Y is an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group having 6 to 50 ring carbon atoms. In the case where both of $R^c$ and $R^d$ are aryl groups, $R^c$ and $R^d$ may be bonded to each other to form a spiro ring.

In the formula (2-7), adjacent two selected from $R^{81}$ to $R^{85}$ that are not single bonds, adjacent two selected from $R^{151}$ to $R^{155}$ that are not single bonds, adjacent two selected from $R^{161}$ to $R^{166}$ that are not single bonds, adjacent two selected from $R^{31}$ to $R^{35}$ that are not single bonds, adjacent two selected from $R^{61}$ to $R^{68}$, and adjacent two selected from $R^{121}$ to $R^{128}$ are not independently bonded to each other and thus do not form a ring structure, and the benzene ring A3 and benzene ring B3 are not crosslinked.

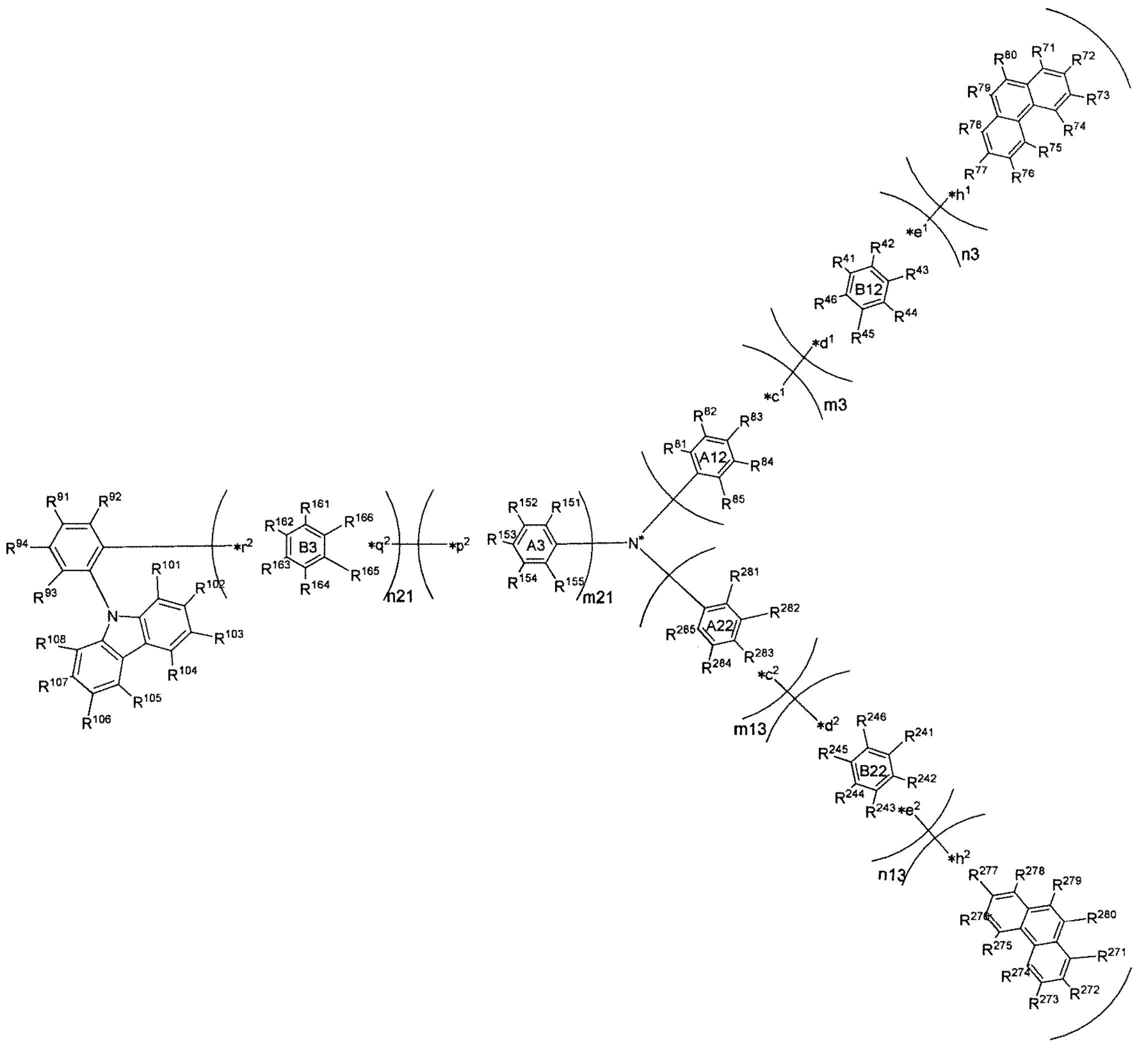

(2-8)

In the formula (2-8), $R^{91}$ to $R^{94}$ and $R^{101}$ to $R^{108}$ are as described above.

In the formula (2-8), $R^{151}$ to $R^{155}$, $R^{161}$ to $R^{166}$, $R^{81}$ to $R^{85}$, $R^{41}$ to $R^{46}$, $R^{281}$ to $R^{285}$, $R^{241}$ to $R^{246}$, $R^{71}$ to $R^{80}$, and $R^{271}$ to $R^{280}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{81}$ to $R^{85}$ is a single bond bonded to $*c^1$, one selected from $R^{41}$ to $R^{46}$ is a single bond bonded to $*d^1$ and the other one selected from $R^{41}$ to $R^{46}$ is a single bond bonded to $*e^1$, one selected from $R^{281}$ to $R^{285}$ is a single bond bonded to $*c^2$, one selected from $R^{241}$ to $R^{246}$ is a single bond bonded to $*d^2$ and the other one selected from $R^{241}$ to $R^{246}$ is a single bond bonded to $*e^2$, one selected from $R^{71}$ to $R^{80}$ is a single bond bonded to $*h^1$, and one selected from $R^{271}$ to $R^{280}$ is a single bond bonded to $*h^2$.

In the formula (2-8), m3, m13, and m21 are each independently 0 or 1, n3, n13, and n21 are each independently 0 or 1, and

when m3 is 0 and n3 is 0, $*e^1$ is bonded to a nitrogen atom N*,
when m3 is 0 and n3 is 1, $*c^1$ is bonded to a nitrogen atom N*, and
when m3 is 1 and n3 is 0, one selected from $R^{81}$ to $R^{85}$ is a single bond bonded to $*e^1$,
when m13 is 0 and n13 is 0, $*e^2$ is bonded to a nitrogen atom N*,
when m13 is 0 and n13 is 1, $*C^2$ is bonded to a nitrogen atom N*, and
when m13 is 1 and n13 is 0, one selected from $R^{281}$ to $R^{285}$ is a single bond bonded to $*e^2$,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-8), adjacent two selected from $R^{81}$ to $R^{85}$ that are not single bonds, adjacent two selected from $R^{41}$ to $R^{46}$ that are not single bonds, adjacent two selected from $R^{151}$ to $R^{155}$ that are not single bonds, adjacent two selected from $R^{161}$ to $R^{166}$ that are not single bonds, adjacent two selected from $R^{281}$ to $R^{285}$ that are not single bonds, adjacent two selected from $R^{241}$ to $R^{246}$, adjacent two selected from $R^{71}$ to $R^{80}$, and adjacent two selected from $R^{271}$ to $R^{280}$ are not independently bonded to each other and thus do not form a ring structure, and the benzene ring A12 and benzene ring B12, the benzene ring A22 and benzene ring B22, and the benzene ring A3 and benzene ring B3 are not crosslinked.

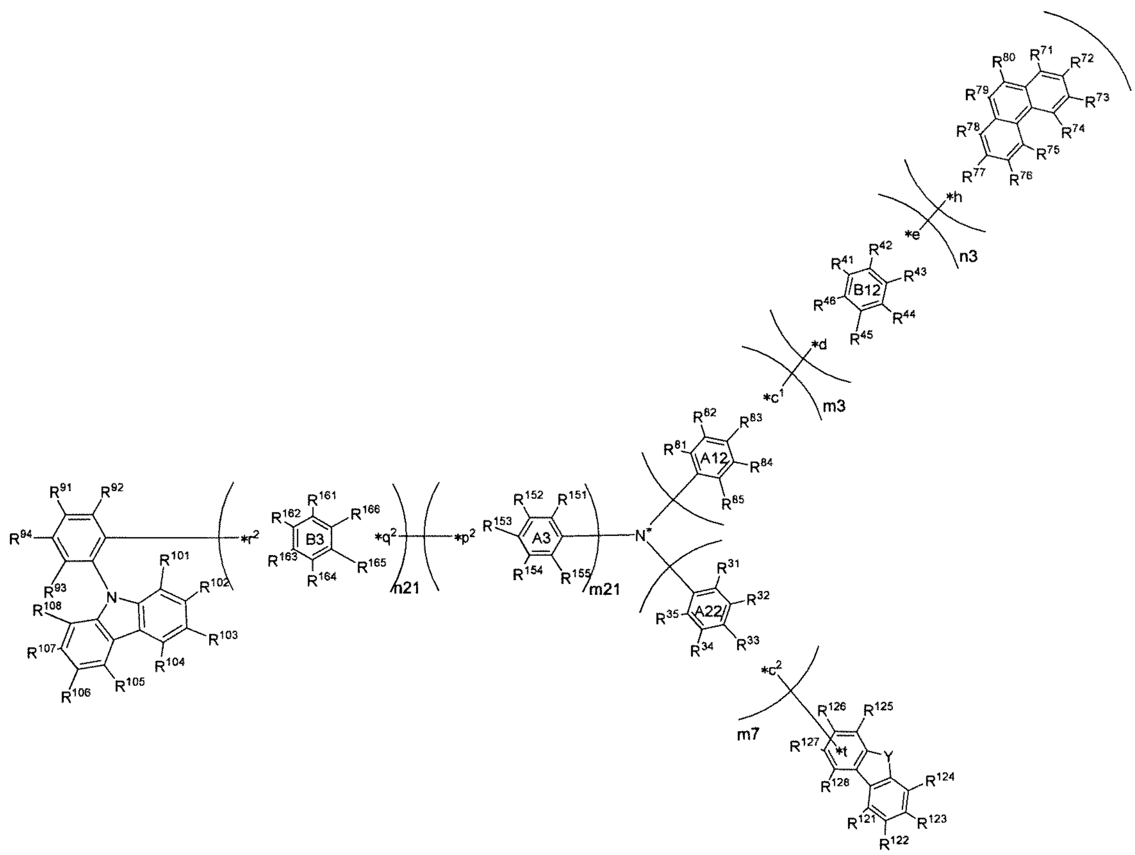

(2-9)

In the formula (2-9), $R^{91}$ to $R^{94}$ and $R^{101}$ to $R^{108}$ are as described above.

In the formula (2-9), $R^{151}$ to $R^{155}$, $R^{161}$ to $R^{166}$, $R^{81}$ to $R^{85}$, $R^{41}$ to $R^{46}$, $R^{31}$ to $R^{35}$, $R^{71}$ to $R^{80}$, and $R^{121}$ to $R^{128}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{166}$ is a single bond bonded to $*q^2$ and the other one selected from $R^{161}$ to $R^{166}$ is a single bond bonded to $*r^2$, one selected from $R^{8'}$ to $R^{85}$ is a single bond bonded to $*c^l$, one selected from $R^{41}$ to $R^{46}$ is a single bond bonded to *d and the other one selected from $R^{41}$ to $R^{46}$ is a single bond bonded to *e, one selected from $R^{31}$ to $R^{35}$ is a single bond bonded to $*C^2$, one selected from $R^{71}$ to $R^{80}$ is a single bond bonded to *h, and one selected from $R^{121}$ to $R^{128}$ is a single bond bonded to *t.

In the formula (2-9), m3 and m21 are each independently 0 or 1, n3 and n21 are each independently 0 or 1, and

when m3 is 0 and n3 is 0, *e is bonded to a nitrogen atom N*,
when m3 is 0 and n3 is 1, $*c^1$ is bonded to a nitrogen atom N*, and
when m3 is 1 and n3 is 0, one selected from $R^{81}$ to $R^{85}$ is a single bond bonded to *e,
when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-9), m7 is 0 or 1, and when m7 is 0, $*C^2$ is bonded to a nitrogen atom N*.

In the formula (2-9), Y is an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group

having 6 to 50 ring carbon atoms. In the case where both of $R^c$ and $R^d$ are aryl groups, $R^c$ and $R^d$ may be bonded to each other to form a spiro ring.

In the formula (2-9), adjacent two selected from $R^{81}$ to $R^{85}$ that are not single bonds, adjacent two selected from $R^{41}$ to $R^{46}$ that are not single bonds, adjacent two selected from $R^{151}$ to $R^{155}$ that are not single bonds, adjacent two selected from $R^{161}$ to $R^{166}$ that are not single bonds, adjacent two selected from $R^{31}$ to $R^{35}$ that are not single bonds, adjacent two selected from $R^{71}$ to $R^{80}$, and adjacent two selected from $R^{121}$ to $R^{128}$ are not independently bonded to each other and thus do not form a ring structure, and the benzene ring A12 and benzene ring B12, and the benzene ring A3 and benzene ring B3 are not crosslinked.

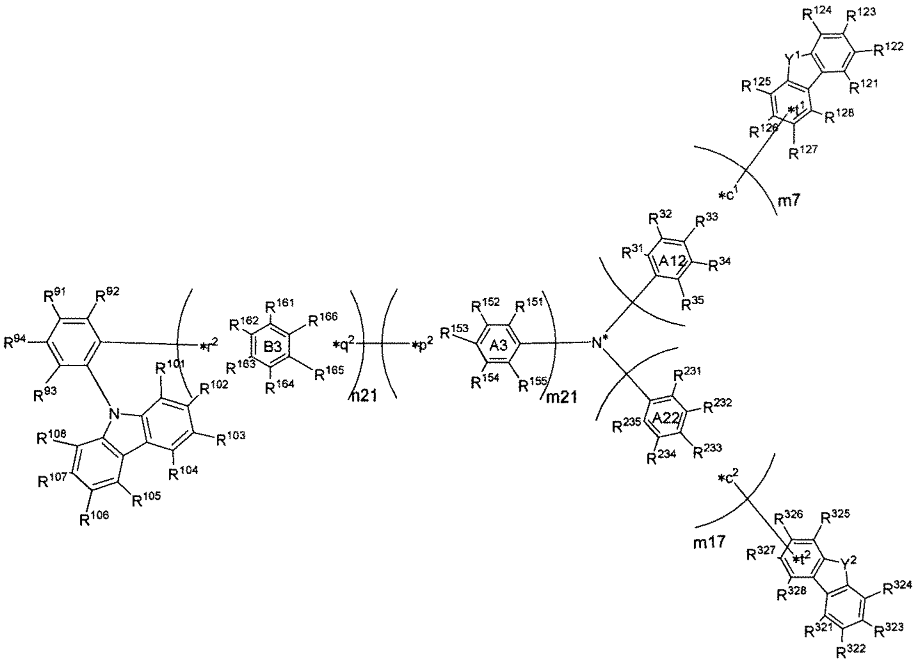

(2-10)

In the formula (2-10), $R^{91}$ to $R^{94}$ and $R^{101}$ to $R^{108}$ are as described above.

In the formula (2-10), $R^{151}$ to $R^{155}$, $R^{161}$ to $R^{166}$, $R^{31}$ to $R^{35}$, $R^{231}$ to $R^{235}$, $R^{121}$ to $R^{128}$, and $R^{321}$ to $R^{328}$ are each independently the same as $R^{131}$ to $R^{135}$, $R^{141}$ to $R^{146}$, $R^{171}$ to $R^{175}$, $R^{181}$ to $R^{186}$, $R^{51}$ to $R^{55}$, and $R^{191}$ to $R^{195}$ in the formula (1-1) described above, and preferred embodiments thereof are also the same, provided that one selected from $R^{151}$ to $R^{155}$ is a single bond bonded to $*p^2$, one selected from $R^{161}$ to $R^{16G}$ is a single bond bonded to $*q^2$ and the other one selected from $R^{161}$ to $R^{166}$ is a single bond bonded to $*r^2$, one selected from $R^{31}$ to $R^{35}$ is a single bond bonded to $*c^1$, one selected from $R^{231}$ to $R^{235}$ is a single bond bonded to $*C^2$, one selected from $R^{121}$ to $R^{128}$ is a single bond bonded to $*t^1$, and one selected from $R^{321}$ to $R^{328}$ is a single bond bonded to $*t^2$.

In the formula (2-10), m21 is each independently 0 or 1, n21 is each independently 0 or 1, and

when m21 is 0 and n21 is 0, $*r^2$ is bonded to a nitrogen atom N*,
when m21 is 0 and n21 is 1, $*p^2$ is bonded to a nitrogen atom N*, and
when m21 is 1 and n21 is 0, one selected from $R^{151}$ to $R^{155}$ is a single bond bonded to $*r^2$.

In the formula (2-10), m7 is 0 or 1 and when m7 is 0, $*c^1$ is bonded to a nitrogen atom N*, and m17 is 0 or 1 and when m17 is 0, $*C^2$ is bonded to a nitrogen atom N*.

In the formula (2-10), $Y^1$ and $Y^2$ are each independently an oxygen atom, a sulfur atom, or $CR^cR^d$, and $R^c$ and $R^d$ are each independently a substituted or unsubstituted alkyl group having 1 to 50 ring carbon atoms, or a substituted or unsubstituted aryl group having 6 to 50 ring carbon atoms. In the case where both of $R^c$ and $R^d$ are aryl groups, $R^c$ and $R^d$ may be bonded to each other to form a spiro ring.

In the formula (2-10), adjacent two selected from $R^{31}$ to $R^{35}$ that are not single bonds, adjacent two selected from $R^{151}$ to $R^{155}$ that are not single bonds, adjacent two selected from $R^{161}$ to $R^{166}$ that are not single bonds, adjacent two selected from $R^{231}$ to $R^{235}$ that are not single bonds, adjacent two selected from $R^{121}$ to $R^{128}$, and adjacent two selected from $R^{321}$ to $R^{328}$ are not independently bonded to each other and thus do not form a ring structure, and the benzene ring A3 and benzene ring B3 are not crosslinked.

(Deuterium Atom Contained in Inventive Compound B)

The inventive compound B contains at least one deuterium atom.

A deuterium atom may be intentionally introduced into the inventive compound A by using a deuterated compound as a part or the whole of the raw material compound.

Here, examples of the raw material compound in which a part or the whole thereof is deuterated include a compound forming a 9-carbazolyl group in the formula (2), a compound forming a phenylene group in the formula (2), a compound forming a linker ($L^4$, $L^5$, $L^6$) in the formula (2), and a compound forming a terminal ($Ar^3$, $Ar^4$) in the formula (2).

In the formula (2), it is preferable that at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, a hydrogen atom directly bonded to a monovalent heterocyclic group represented by $Ar^4$, a hydrogen atom represented by $R^{91}$ to $R^{94}$, and a hydrogen atom represented by $R^{101}$ to $R^{108}$ is a deuterium atom.

That is, in the following formula (2-11), the sum of a, b, c, d, e, f, and g is preferably 1 or more.

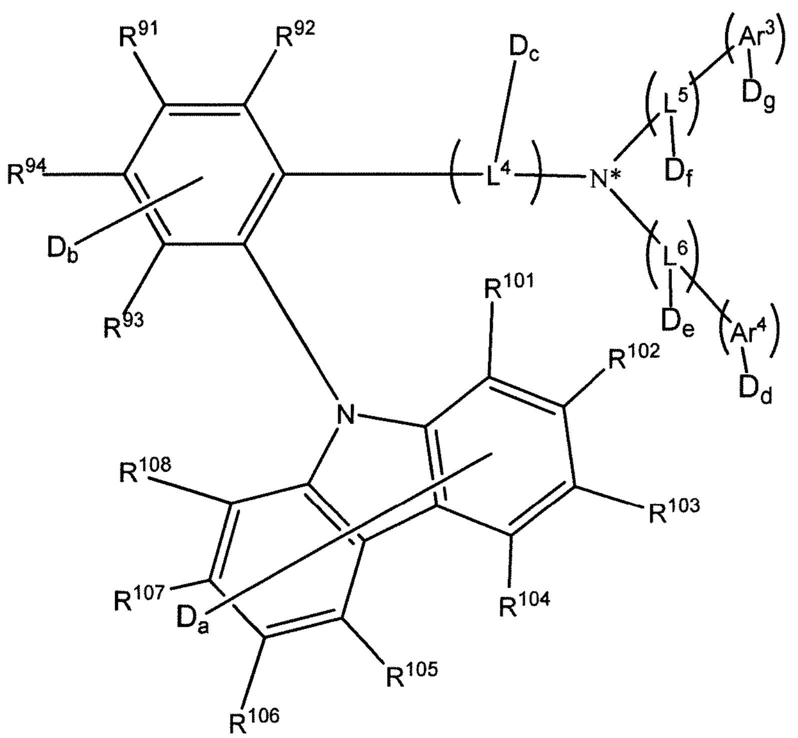

(2-11)

In the formula (2-11), "Da" represents that "a" of the hydrogen atoms represented by $R^{101}$ to $R^{108}$ indicates the number of deuterium atoms, "Db" represents that "b" of the hydrogen atoms represented by $R^{91}$ to $R^{94}$ indicates the number of deuterium atoms, "De" represents that "c" of the hydrogen atoms directly bonded to the arylene group or divalent heterocyclic group represented by $L^4$ indicates the number of deuterium atoms, "Dd" represents that "d" of the hydrogen atoms directly bonded to the aryl group or the hydrogen atoms directly bonded to the monovalent heterocyclic group represented by $Ar^4$ indicates the number of deuterium atoms, "De" represents that "e" of the hydrogen atoms directly bonded to the arylene group or divalent heterocyclic group represented by $L^6$ indicates the number of deuterium atoms, "Df" represents that "f' of the hydrogen atoms directly bonded to the arylene group or divalent heterocyclic group represented by $L^5$ indicates the number of deuterium atoms, and "Dg" represents that "g" of the hydrogen atoms directly bonded to the aryl group or monovalent heterocyclic group represented by $Ar^3$ indicates the number of deuterium atoms.

More preferably, in the formula (2), at least one deuterium atom contained in the inventive compound B is at least any one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom

directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, a hydrogen atom directly bonded to a monovalent heterocyclic group represented by $Ar^4$, a hydrogen atom represented by $R^{91}$ to $R^{94}$, and a hydrogen atom represented by $R^{101}$ to $R^{108}$ (that is, when $Ar^3$, $Ar^4$, $L^4$ to $L^6$, $R^{91}$ to $R^{94}$, and $R^{101}$ to $R^{108}$ are substituted in the formula (2), the substituents do not contain a deuterium atom).

In the formula (2), it is preferable that at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^4$, a hydrogen atom directly bonded to a monovalent heterocyclic group represented by $Ar^4$, a hydrogen atom represented by $R^{91}$ to $R^{94}$, and a hydrogen atom represented by $R^{101}$ to $R^{108}$ is a deuterium atom.

That is, in the formula (2-11), the sum of a, b, c, d, e, and f is preferably 1 or more.

More preferably, in the formula (2), at least one deuterium atom contained in the inventive compound B is at least any one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^4$, a hydrogen atom directly bonded to a monovalent heterocyclic group represented by $Ar^4$, a hydrogen atom represented by $R^{91}$ to $R^{94}$, and a hydrogen atom represented by $R^{101}$ to $R^{108}$ (that is, when $Ar^3$, $Ar^4$, $L^4$ to $L^6$, $R^{91}$ to $R^{94}$, and $R^{101}$ to $R^{108}$ are substituted in the formula (2), the substituents do not contain a deuterium atom, and the hydrogen atom directly bonded to the aryl group represented by $Ar^3$ and the hydrogen atom directly bonded to the monovalent heterocyclic group represented by $Ar^3$ do not contain a deuterium atom).

In the formula (2), it is more preferable that at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, a hydrogen atom directly bonded to a monovalent heterocyclic group represented by $Ar^4$, and a hydrogen atom represented by $R^{91}$ to $R^{94}$ is a deuterium atom.

That is, in the formula (2-11), the sum of b, c, d, e, f, and g is preferably 1 or more.

More preferably, in the formula (2), at least one deuterium atom contained in the inventive compound B is at least any one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, a hydrogen atom directly bonded to a monovalent heterocyclic group represented by $Ar^4$, and a hydrogen atom represented by $R^{91}$ to $R^{94}$ (that is, when $Ar^3$, $Ar^4$, $L^4$ to $L^6$, $R^{91}$ to $R^{94}$, and $R^{101}$ to $R^{108}$ are substituted in the formula (2), the substituents do not contain a deuterium atom, and the hydrogen atom represented by $R^{101}$ to $R^{108}$ does not contain a deuterium atom).

In the formula (2), it is preferable that at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, and a hydrogen atom represented by $R^{91}$ to $R^{94}$ is a deuterium atom.

That is, in the formula (2-11), the sum of b, c, e, and f is preferably 1 or more.

More preferably, in the formula (2), at least one deuterium atom contained in the inventive compound B is at least any one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, and a hydrogen atom represented by $R^{91}$ to $R^{94}$ (that is, when $Ar^3$, $Ar^4$, $L^4$ to $L^6$, $R^{91}$ to $R^{94}$, and

$R^{101}$ to $R^{108}$ are substituted in the formula (2), the substituents do not contain a deuterium atom, and the hydrogen atom directly bonded to the aryl group represented by $Ar^3$, the hydrogen atom directly bonded to the monovalent heterocyclic group represented by $Ar^3$, the hydrogen atom directly bonded to the aryl group represented by $Ar^4$, the hydrogen atom directly bonded to the monovalent heterocyclic group represented by $Ar^4$, and the hydrogen atom represented by $R^{101}$ to $R^{108}$ do not contain a deuterium atom).

In the formula (2), it is preferable that at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, and a hydrogen atom directly bonded to a monovalent heterocyclic group represented by $Ar^4$ is a deuterium atom.

That is, in the formula (2-11), the sum of c, d, e, f, and g is preferably 1 or more.

More preferably, in the formula (2), at least one deuterium atom contained in the inventive compound B is at least any one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, and a hydrogen atom directly bonded to a monovalent heterocyclic group represented by $Ar^4$ (that is, when $Ar^3$, $Ar^4$, $L^4$ to $L^6$, $R^{91}$ to $R^{94}$, and $R^{101}$ to $R^{108}$ are substituted in the formula (2), the substituents do not contain a deuterium atom, and the hydrogen atom represented by $R^{91}$ to $R^{94}$ and the hydrogen atom represented by $R^{191}$ to $R^{108}$ do not contain a deuterium atom).

In the formula (2), it is preferable that at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, and a hydrogen atom directly bonded to a divalent heterocyclic group represented by $L^6$ is a deuterium atom.

That is, in the formula (2-11), the sum of c, e, and f is preferably 1 or more.

More preferably, in the formula (2), at least one deuterium atom contained in the inventive compound B is at least any one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, and a hydrogen atom directly bonded to a divalent heterocyclic group represented by $L^6$ (that is, when $Ar^3$, $Ar^4$, $L^4$ to $L^6$, $R^{91}$ to $R^{94}$, and $R^{101}$ to $R^{108}$ are substituted in the formula (2), the substituents do not contain a deuterium atom, and the hydrogen atom directly bonded to the aryl group represented by $Ar^3$, the hydrogen atom directly bonded to the monovalent heterocyclic group represented by $Ar^3$, the hydrogen atom directly bonded to the aryl group represented by $Ar^4$, the hydrogen atom directly bonded to the monovalent heterocyclic group represented by $Ar^4$, the hydrogen atom represented by $R^{91}$ to $R^{94}$, and the hydrogen atom represented by $R^{101}$ to $R^{108}$ do not contain a deuterium atom).

In the formula (2), it is preferable that at least one of a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, and a hydrogen atom directly bonded to a monovalent heterocyclic group represented by $Ar^4$ is a deuterium atom. That is, in the formula (2-11), the sum of d, e, f, and g is preferably 1 or more.

More preferably, in the formula (2), at least one deuterium atom contained in the inventive compound B is at least any one of a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, and a hydrogen atom directly bonded to a monovalent heterocyclic group represented by $Ar^4$ (that is, when $Ar^3$, $Ar^4$, $L^4$ to $L^6$, $R^{91}$ to $R^{94}$, and $R^{101}$ to $R^{108}$ are substituted in the formula (2), the substituents do not contain a deuterium atom, and the hydrogen atom directly bonded to the arylene group represented by $L^4$, the hydrogen atom directly bonded to the divalent heterocyclic

group represented by $L^4$, the hydrogen atom represented by $R^{91}$ to $R^{94}$, and the hydrogen atom represented by $R^{101}$ to $R^{108}$ do not contain a deuterium atom).

In the formula (2), it is preferable that at least one of a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, and a hydrogen atom directly bonded to a divalent heterocyclic group represented by $L^6$ is a deuterium atom.

That is, in the formula (2-11), the sum of e and f is preferably 1 or more.

More preferably, in the formula (2), at least one deuterium atom contained in the inventive compound B is at least any one of a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, and a hydrogen atom directly bonded to a divalent heterocyclic group represented by $L^6$ (that is, when $Ar^3$, $Ar^4$, $L^4$ to $L^6$, $R^{91}$ to $R^{94}$, and $R^{101}$ to $R^{108}$ are substituted in the formula (2), the substituents do not contain a deuterium atom, and the hydrogen atom directly bonded to the arylene group represented by $L^4$, the hydrogen atom directly bonded to the divalent heterocyclic group represented by $L^4$, the hydrogen atom directly bonded to the aryl group represented by $Ar^3$, the hydrogen atom directly bonded to the monovalent heterocyclic group represented by $Ar^3$, the hydrogen atom directly bonded to the aryl group represented by $Ar^4$, the hydrogen atom directly bonded to the monovalent heterocyclic group represented by $Ar^4$, the hydrogen atom represented by $R^{91}$ to $R^{94}$, and the hydrogen atom represented by $R^{101}$ to $R^{108}$ do not contain a deuterium atom).

The deuteration rate of the inventive compound B depends on the deuteration rate of the raw material compound used. Even if a raw material having a predetermined deuteration rate is used, a naturally derived light hydrogen isotope can be contained at a certain ratio. Accordingly, the aspect of the deuteration rate of the inventive compound B shown below includes a ratio obtained by simply counting the number of deuterium atoms represented by the chemical formula in consideration of a trace amount of naturally derived isotope.

The deuteration rate of the inventive compound B is preferably 1% or more, more preferably 3% or more, still more preferably 5% or more, even more preferably 10% or more, and particularly preferably 50% or more.

The inventive compound B may be a mixture of a deuterated compound (i.e., a compound having deuterium atoms intentionally introduced thereto) and a non-deuterated compound, or a mixture of two or more compounds having different deuteration rates from each other. The deuteration rate of such a mixture (the proportion of the number of deuterium atoms with respect to the total number of hydrogen atoms in the inventive compound A contained in the mixture) is preferably 1% or more, more preferably 3% or more, still more preferably 5% or more, even more preferably 10% or more, particularly preferably 50% or more, and less than 100%.

In the inventive compound B, the proportion of the number of deuterium atoms with respect to the total number of hydrogen atoms in $L^4$ to $L^6$ and $R^{91}$ to $R^{94}$ is preferably 10% or more, more preferably 20% or more, still more preferably 30% or more, even more preferably 40% or more, and particularly preferably 50% or more.

Preferred examples of the inventive compound B include compounds represented by the following formulae (2-11-1) to (2-11-23).

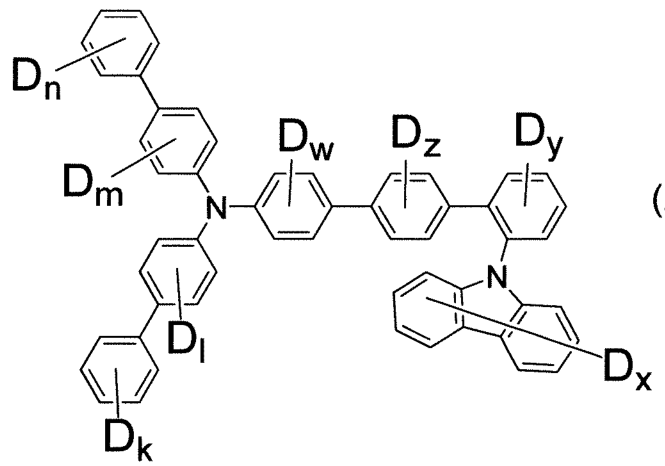

(2-11-1)

(In the formula (2-11-1), x + y + z + w + k + l + m + n = 1 to 38, and each R is omitted.)

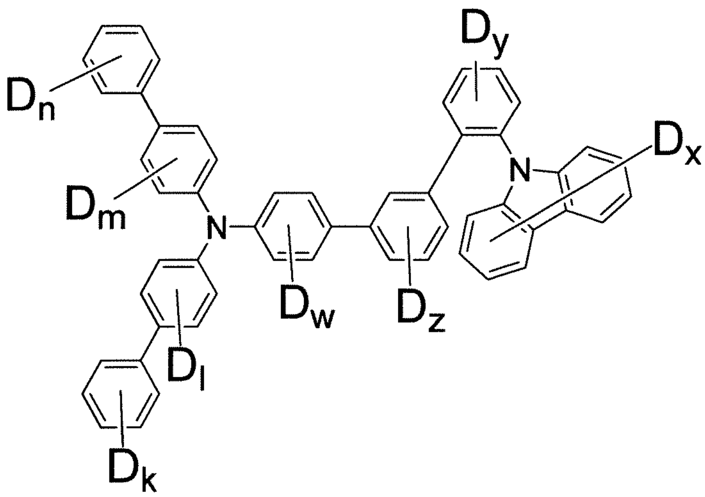

(2-11-2)

(In the formula (2-11-2), x + y + z + w + k + l + m + n = 1 to 38, and each R is omitted.)

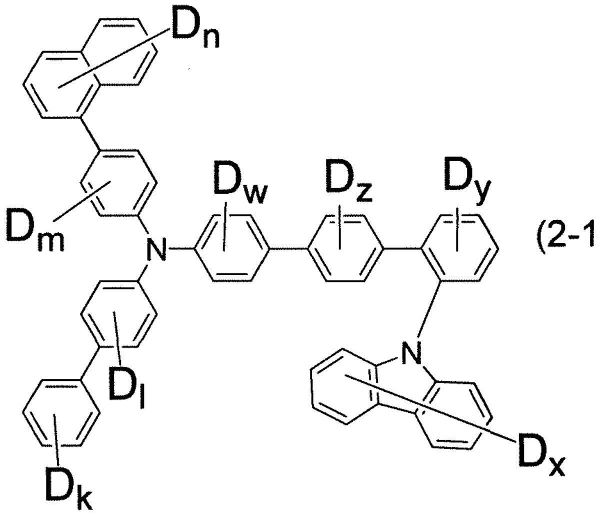

(2-11-3)

(In the formula (2-11-3), x + y + z + w + k + l + m + n = 1 to 40, and each R is omitted.)

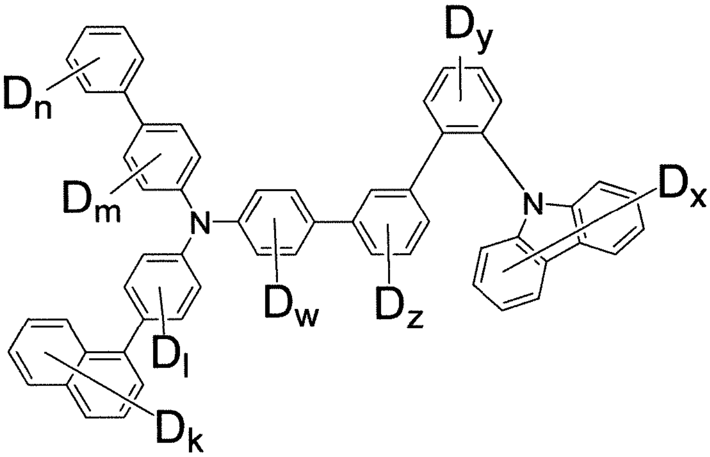

(2-11-4)

(In the formula (2-11-4), x + y + z + w + k + l + m + n = 1 to 40, and each R is omitted.)

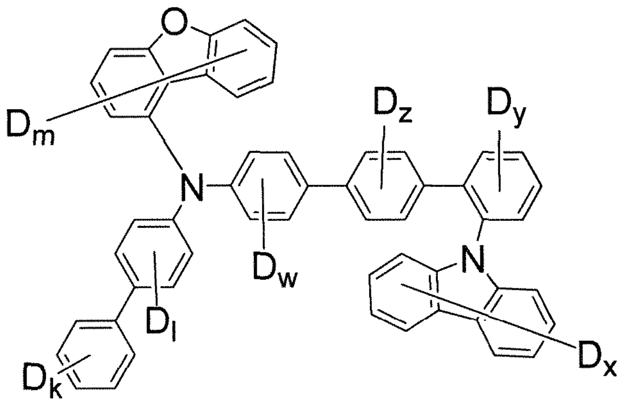

(2-11-5)

(In the formula (2-11-5), x + y + z + w + k + l + m = 1 to 36, and each R is omitted.)

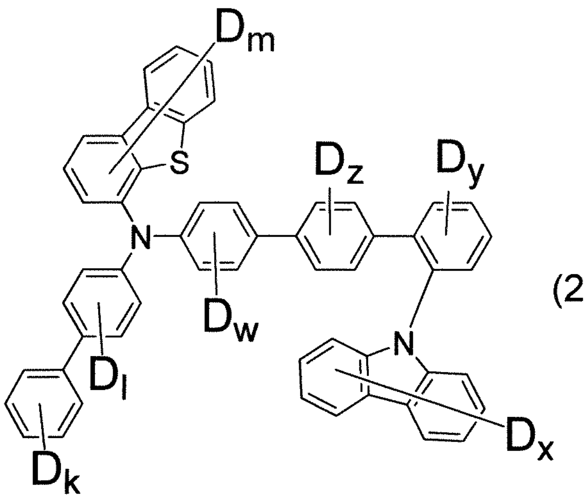

(2-11-6)

(In the formula (2-11-6), x + y + z + w + k + l + m = 1 to 36, and each R is omitted.)

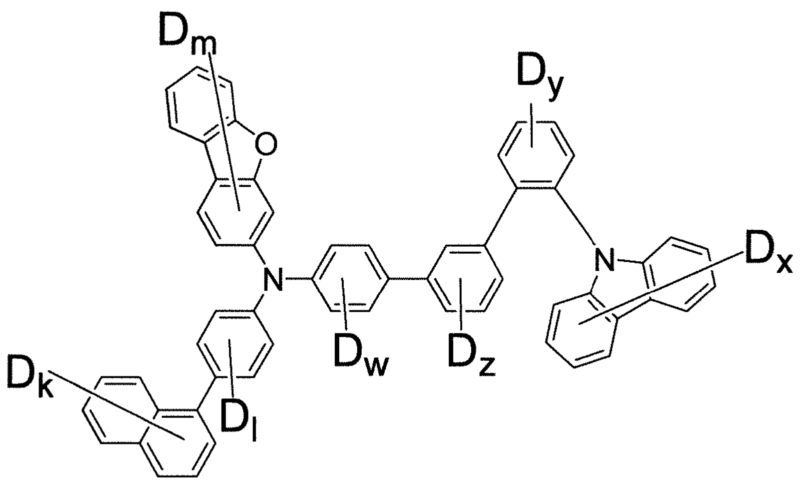

(2-11-7)

(In the formula (2-11-7), x + y + z + w + k + l + m = 1 to 38, and each R is omitted.)

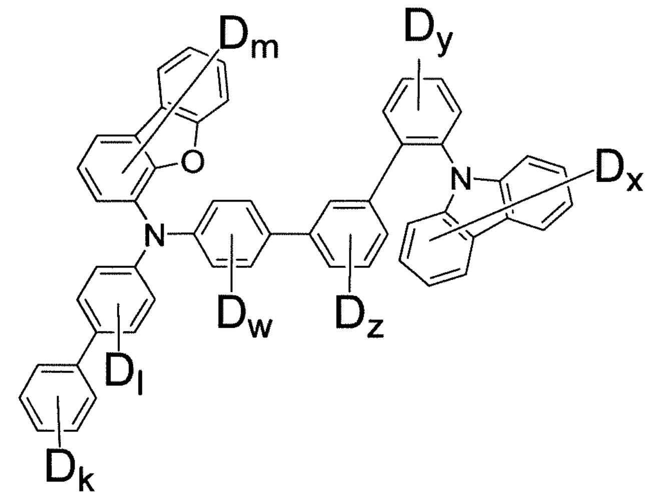

(2-11-8)

(In the formula (2-11-8), x + y + z + w + k + l + m = 1 to 36, and each R is omitted.)

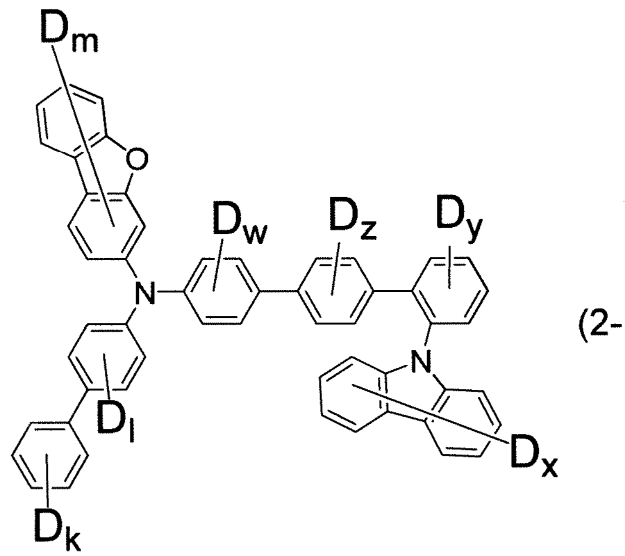

(2-11-9)

(In the formula (2-11-9), x + y + z + w + k + l + m = 1 to 36, and each R is omitted.)

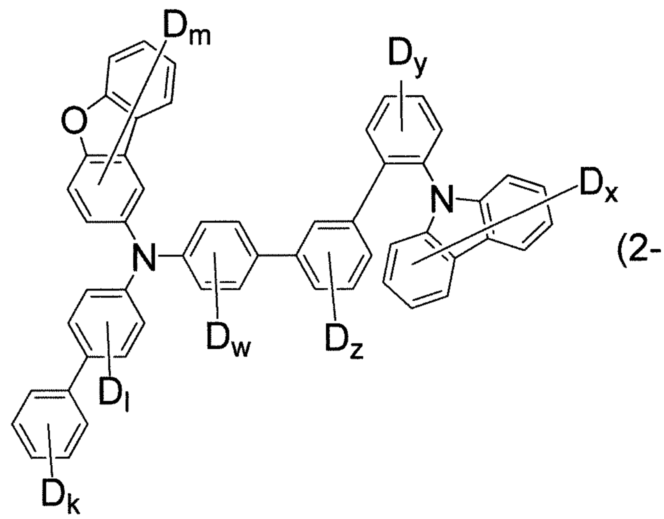

(2-11-10)

(In the formula (2-11-10), x + y + z + w + k + l + m = 1 to 36, and each R is omitted.)

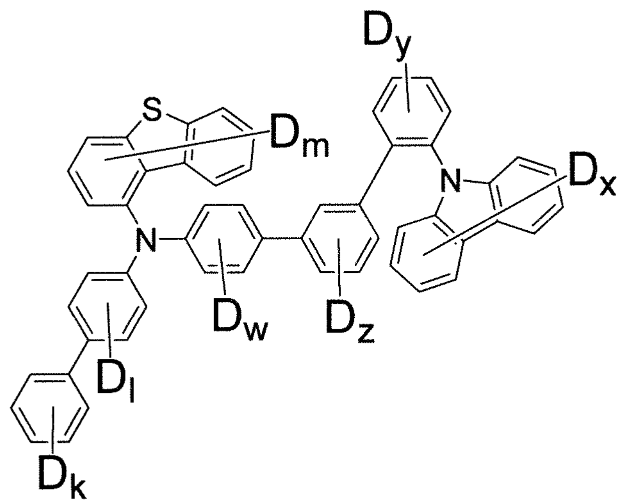

(2-11-11)

(In the formula (2-11-11), x + y + z + w + k + l + m = 1 to 36, and each R is omitted.)

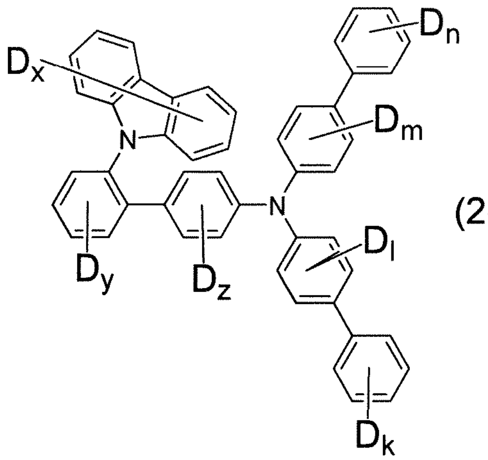

(2-11-12)

(In the formula (2-11-12), x + y + z + k + l + m + n = 1 to 34, and each R is omitted.)

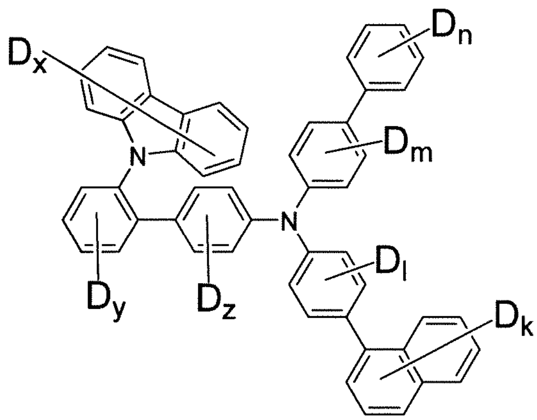

(2-11-13)

(In the formula (2-11-13), x + y + z + k + l + m + n = 1 to 36, and each R is omitted.)

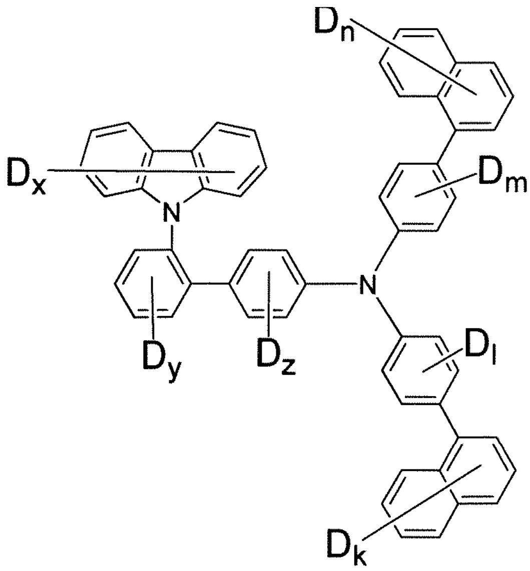

(2-11-14)

(In the formula (2-11-14), x + y + z + k + l + m + n = 1 to 38, and each R is omitted.)

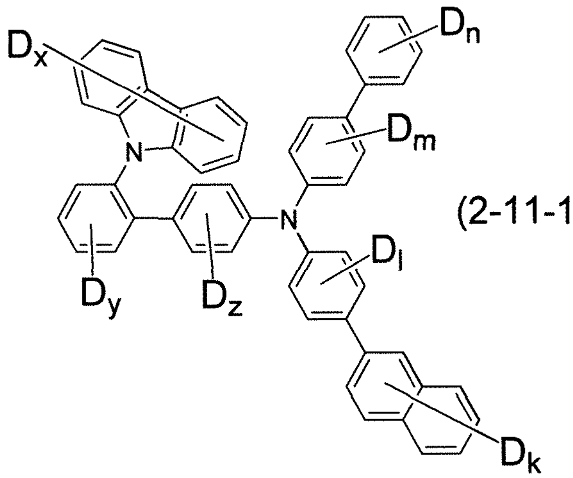

(2-11-15)

(In the formula (2-11-15), x + y + z + k + l + m + n = 1 to 36, and each R is omitted.)

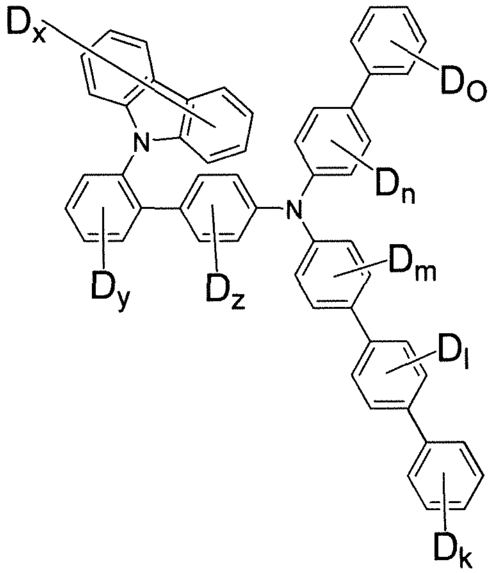

(2-11-16)

(In the formula (2-11-16), x + y + z + k + l + m + n + o = 1 to 38, and each R is omitted.)

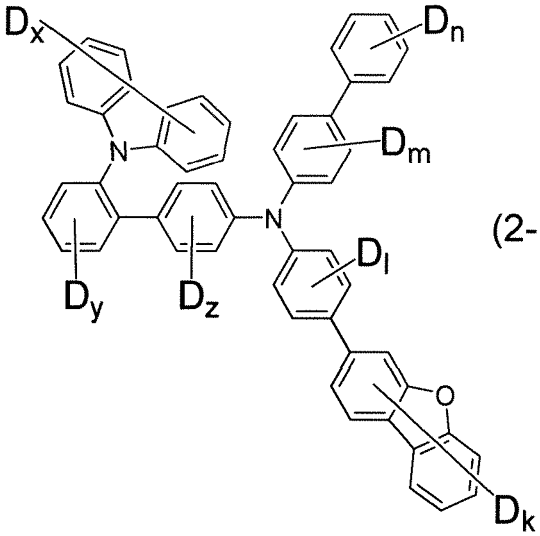

(2-11-17)

(In the formula (2-11-17), x + y + z + k + l + m + n = 1 to 36, and each R is omitted.)

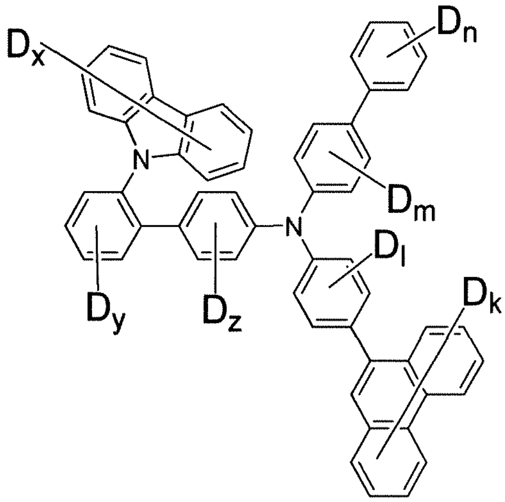

(2-11-18)

(In the formula (2-11-18), x + y + z + k + l + m + n = 1 to 38, and each R is omitted.)

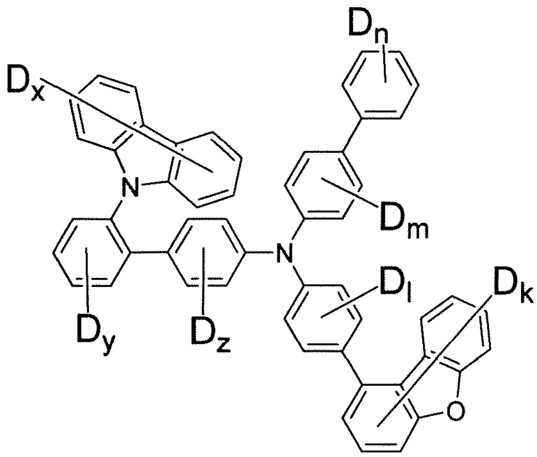

(2-11-19)

(In the formula (2-11-19), x + y + z + k + l + m + n = 1 to 36, and each R is omitted.)

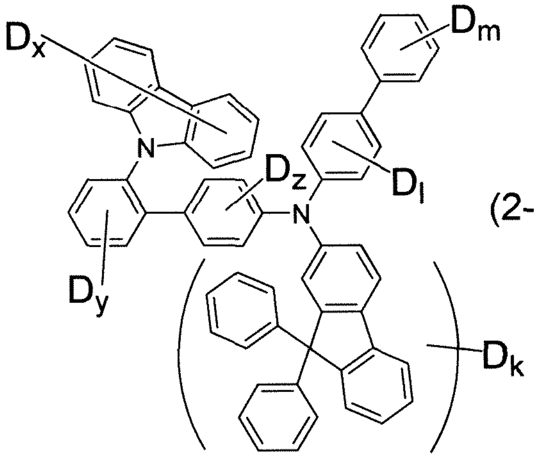

(2-11-20)

(In the formula (2-11-20), x + y + z + k + l + m = 1 to 42, and each R is omitted.)

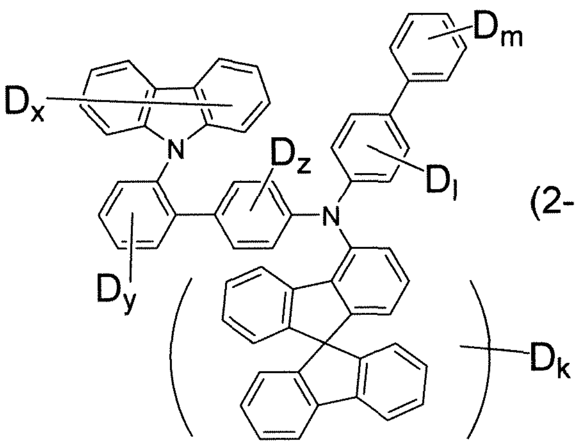

(2-11-21)

(In the formula (2-11-21), x + y + z + k + l + m = 1 to 40, and each R is omitted.)

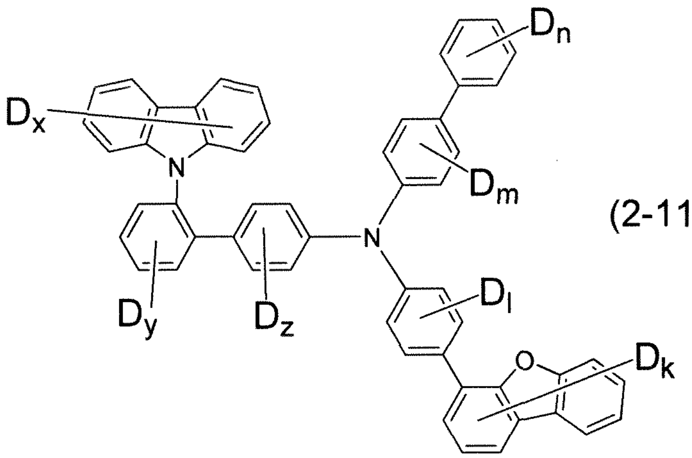

(2-11-22)

(In the formula (2-11-22), x + y + z + k + l + m + n = 1 to 36, and each R is omitted.)

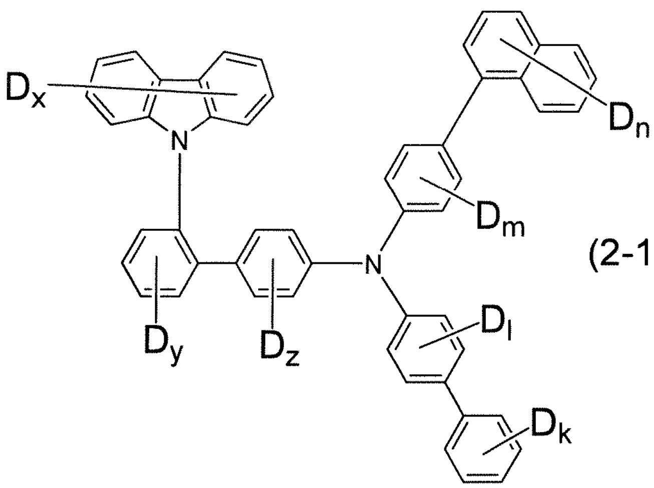

(2-11-23)

(In the formula (2-11-23), x + y + z + k + l + m + n = 1 to 36, and each R is omitted.)

Unless otherwise specified, the details of the substituent (arbitrary substituent) in the expression "substituted or unsubstituted" included in the definitions of the aforementioned formulae are the same as in the section of "Substituent for "Substituted or Unsubstituted"'".

The inventive compound B can be readily produced by a person skilled in the art with reference to the following

synthesis examples and the known synthesis methods.

Specific examples of the inventive compound will be described below, but the inventive compound is not limited to the following example compounds. In addition to the compounds described in the following exemplary compounds, the compounds of the present invention also include compounds in which some hydrogen atoms are not deuterated from the viewpoint of synthesis technology.

In the following specific examples, D represents a deuterium atom.

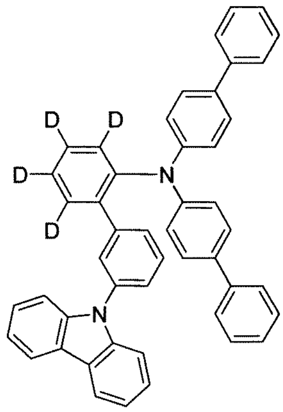
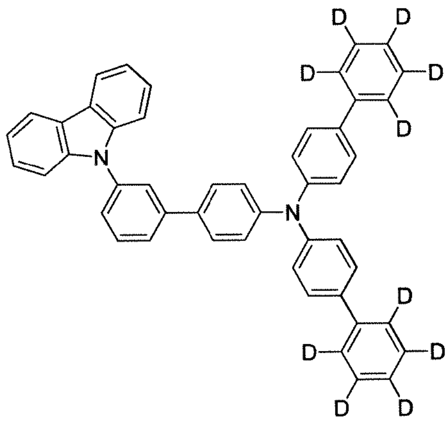
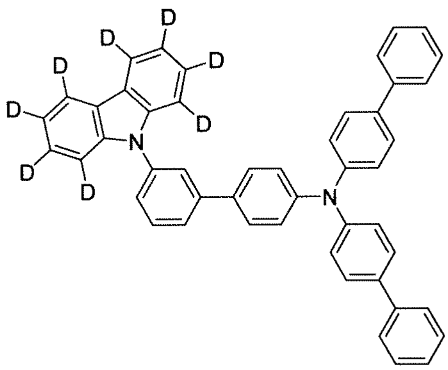
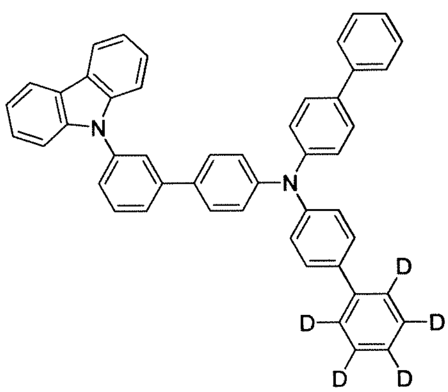
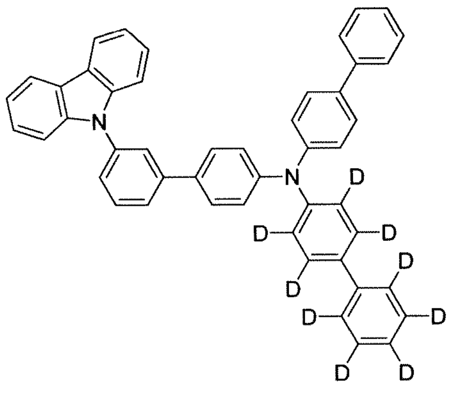
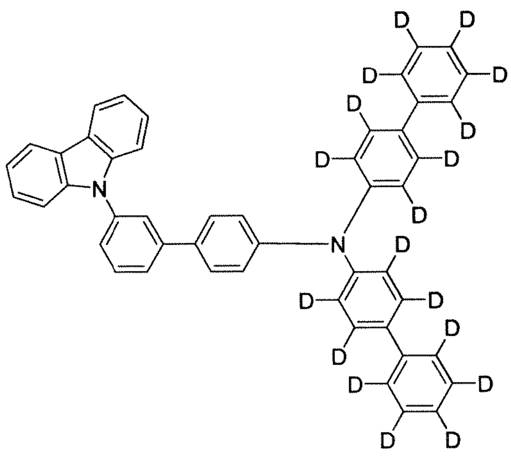

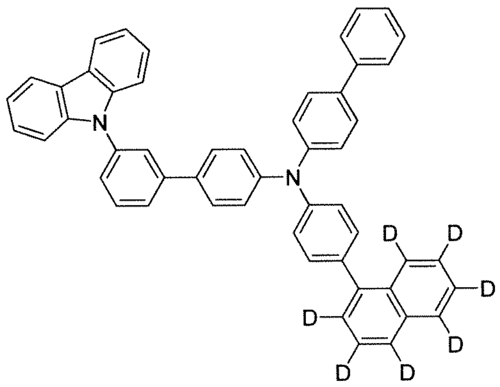

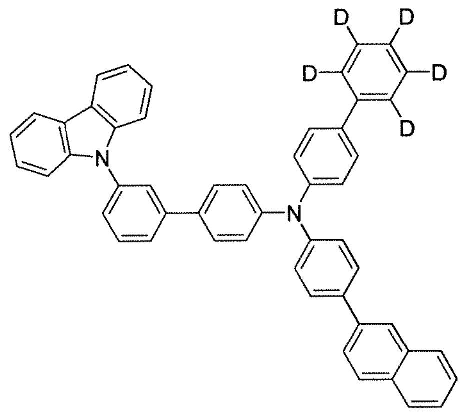

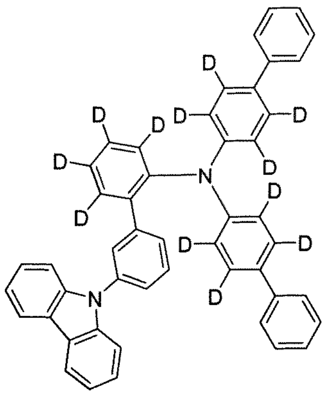

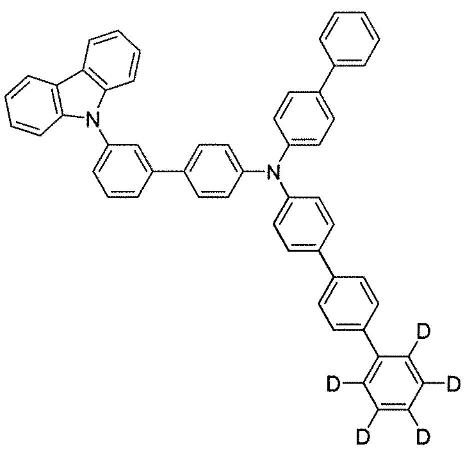

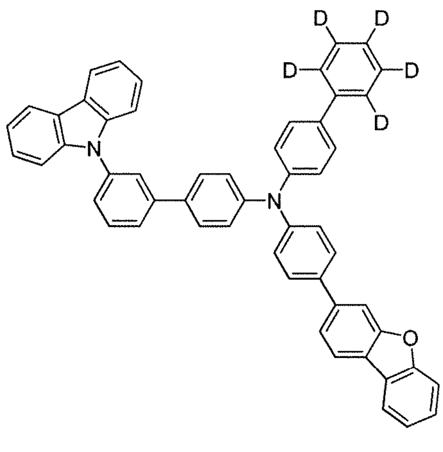

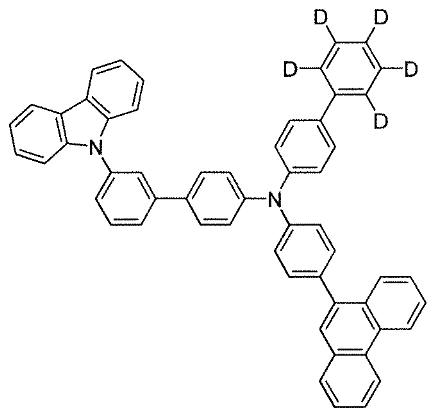

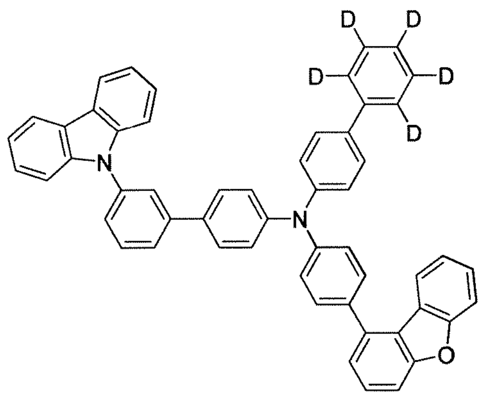

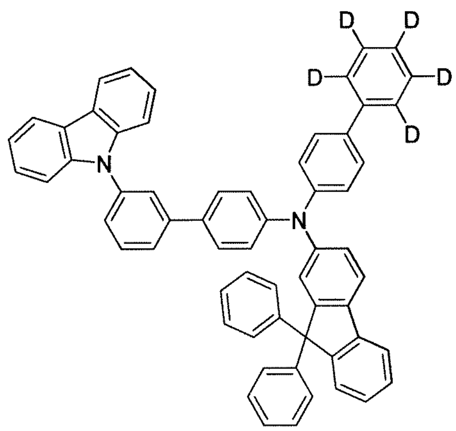

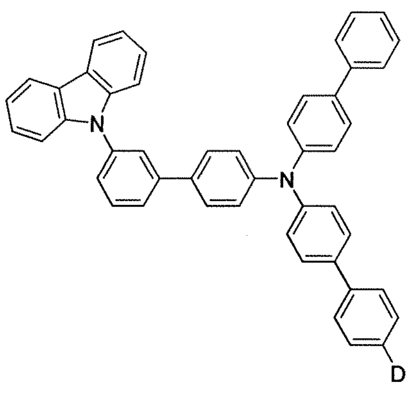

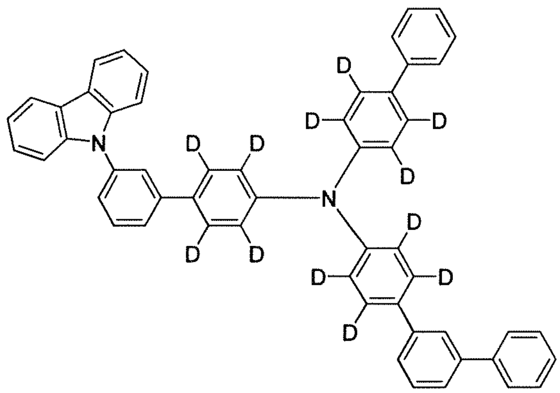

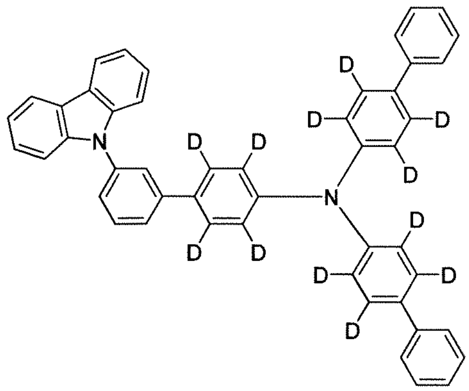

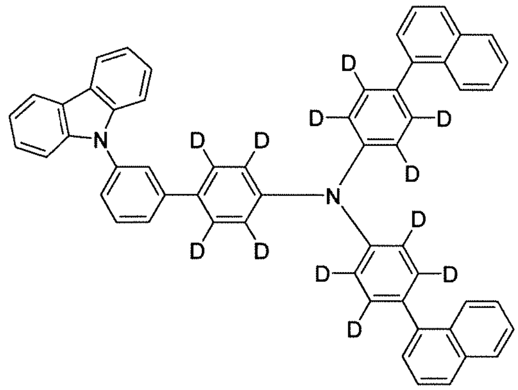

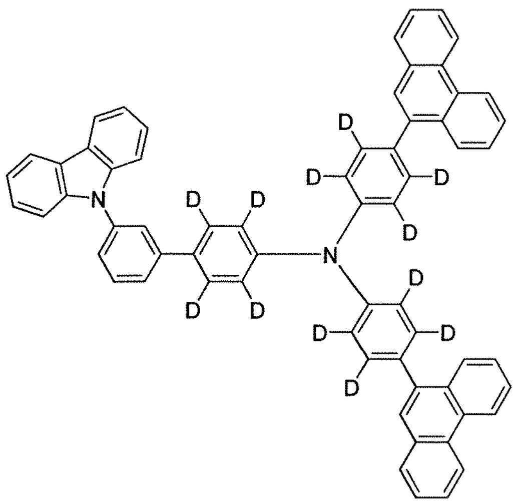

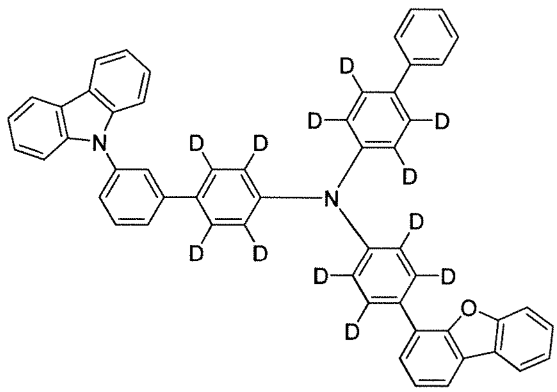

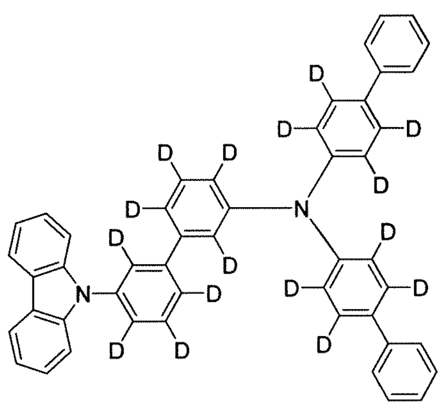

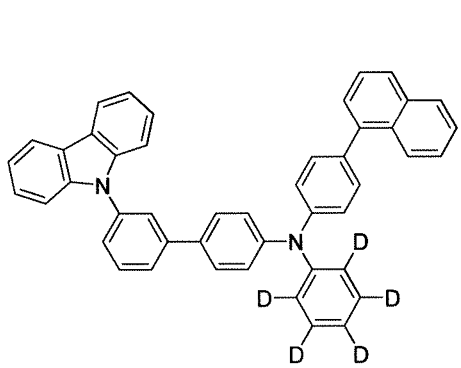

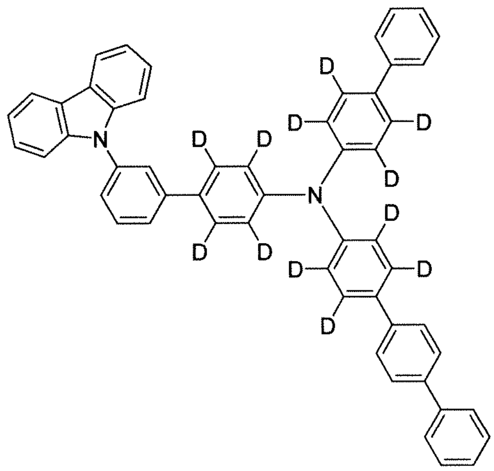

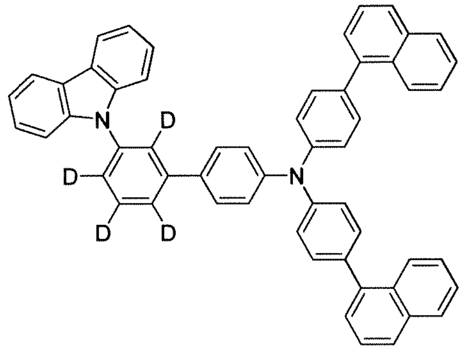

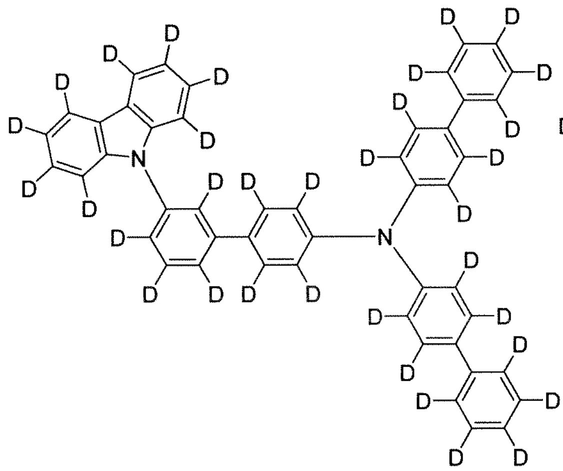

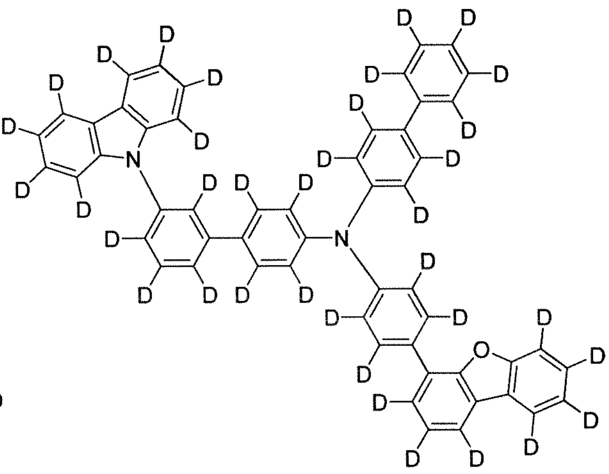

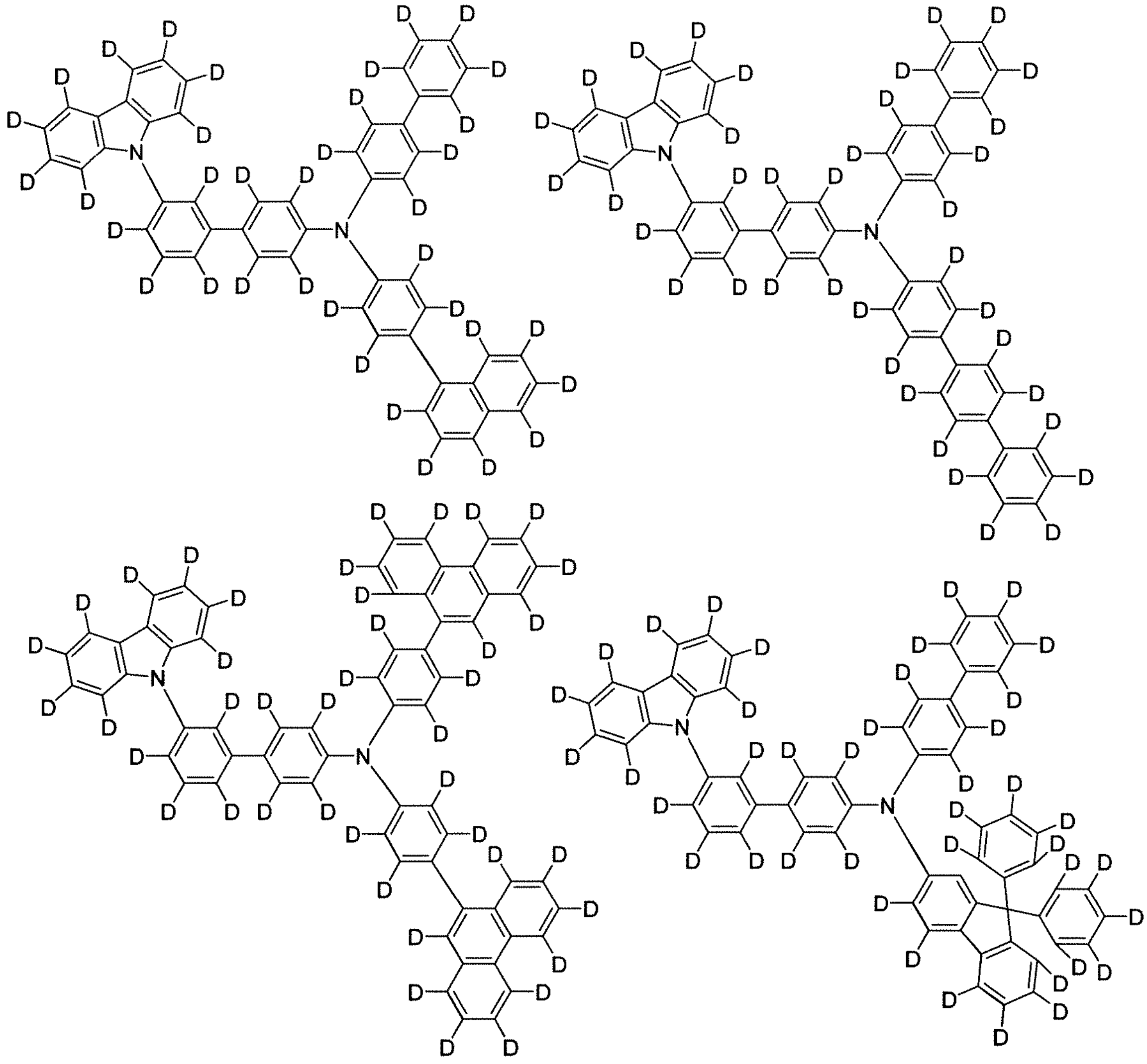

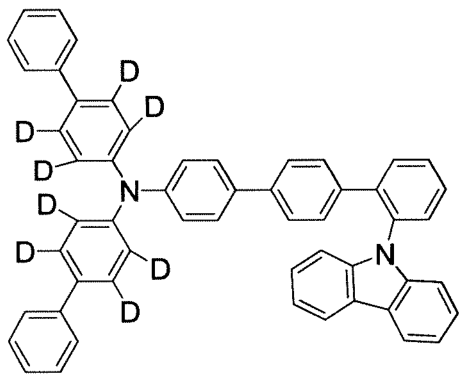

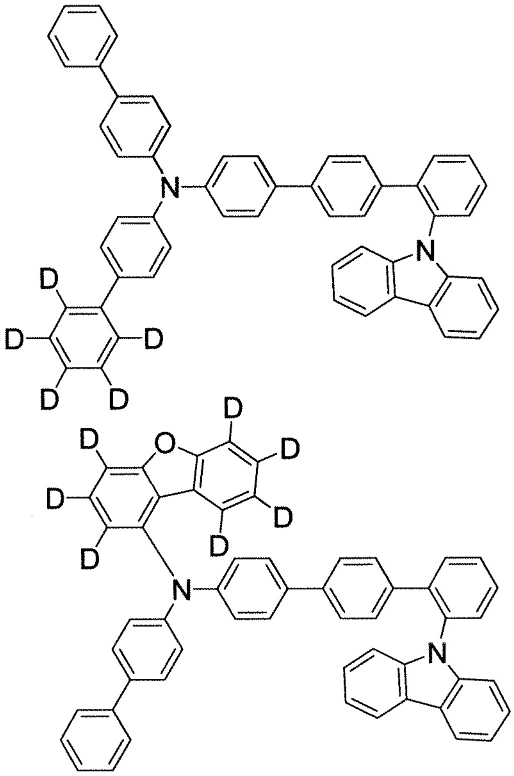

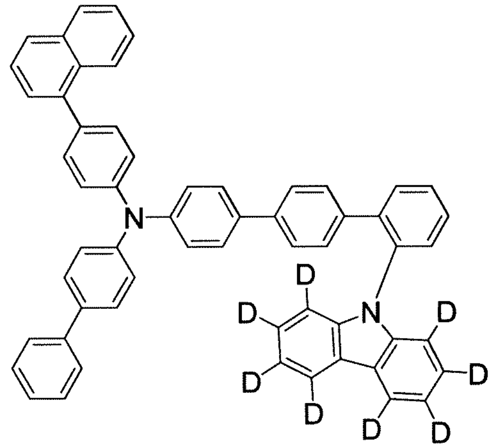

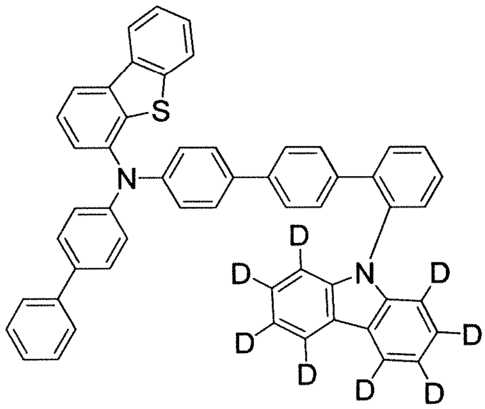

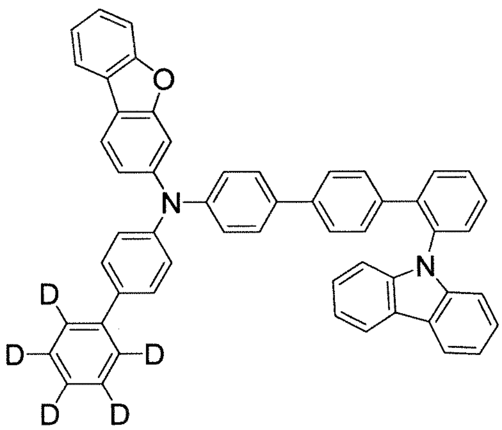

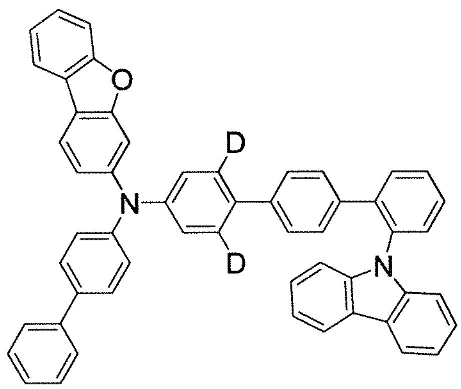

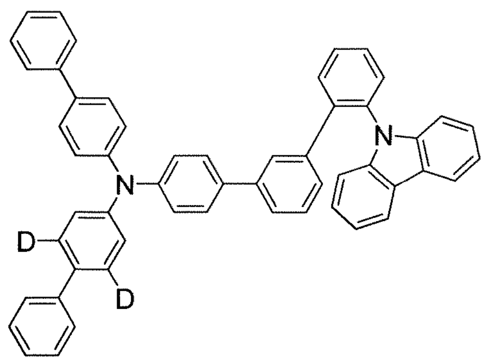

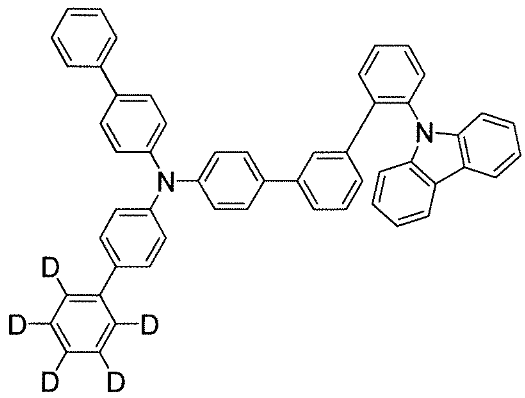

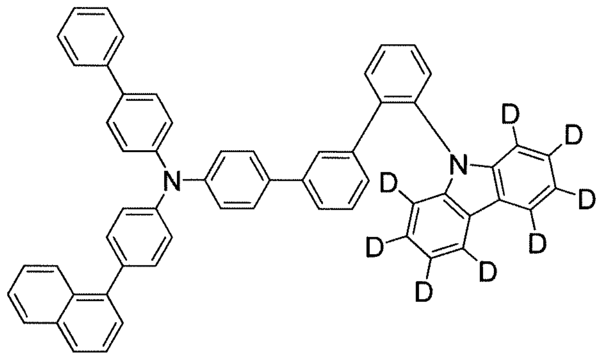

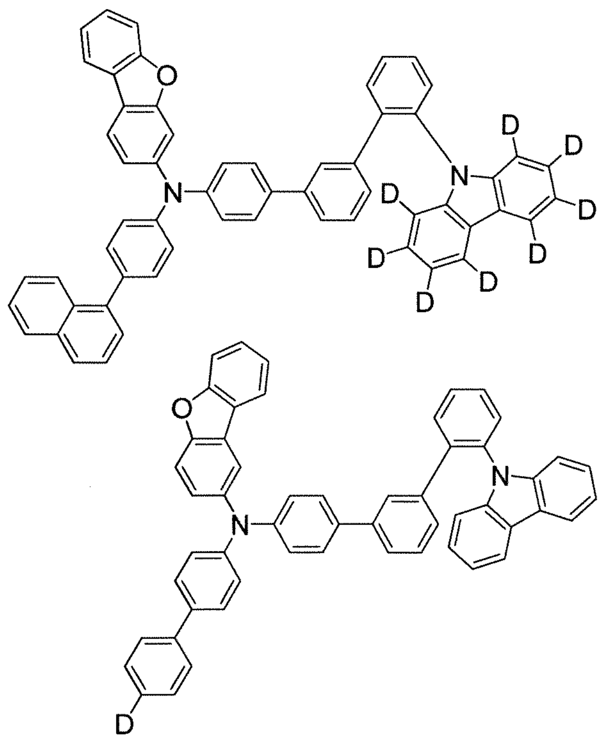

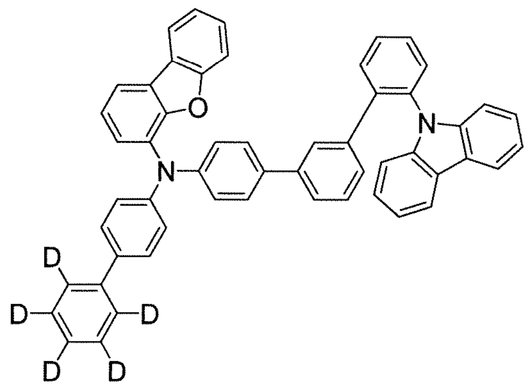

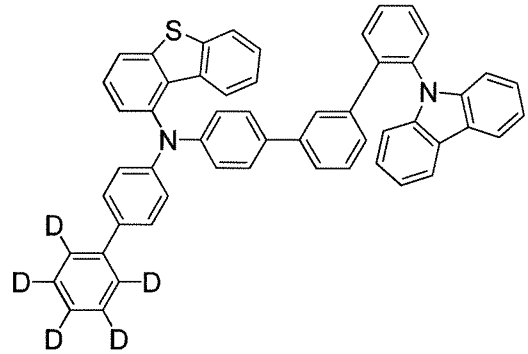

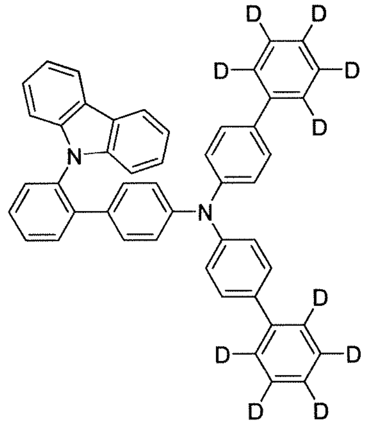

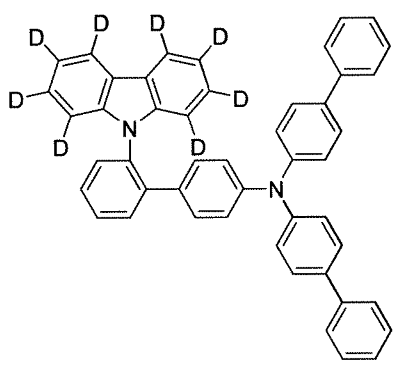

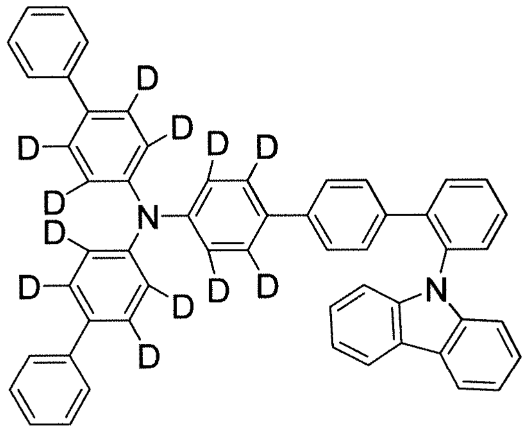

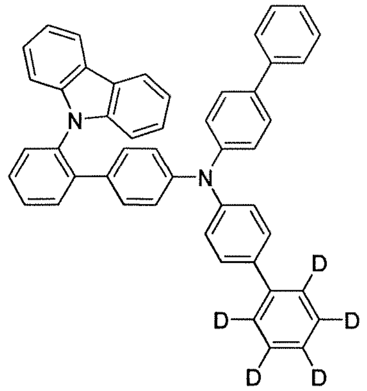

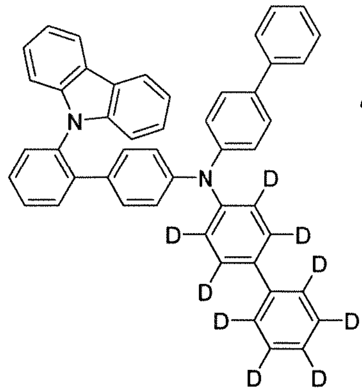

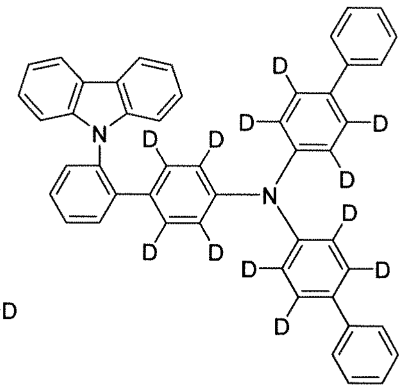

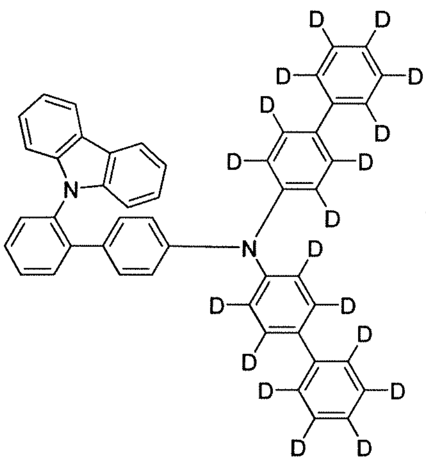

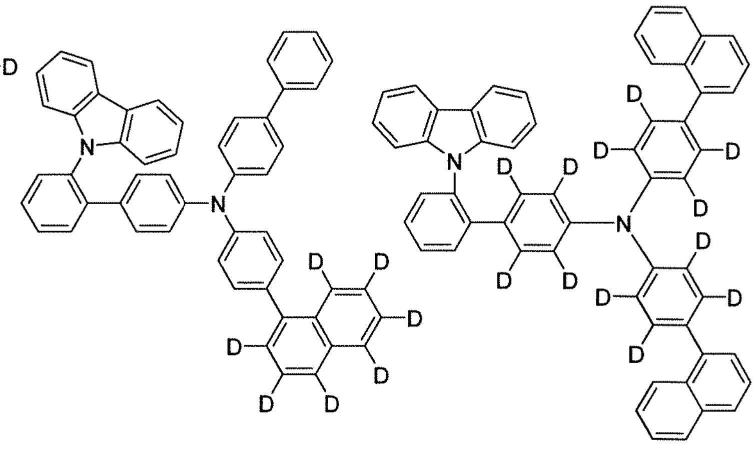

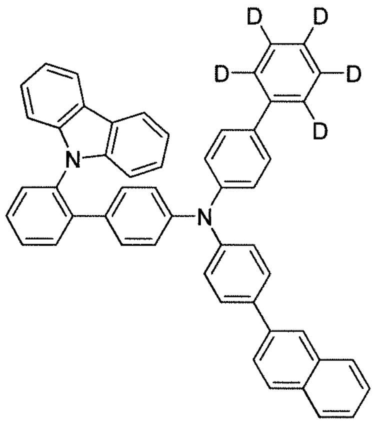

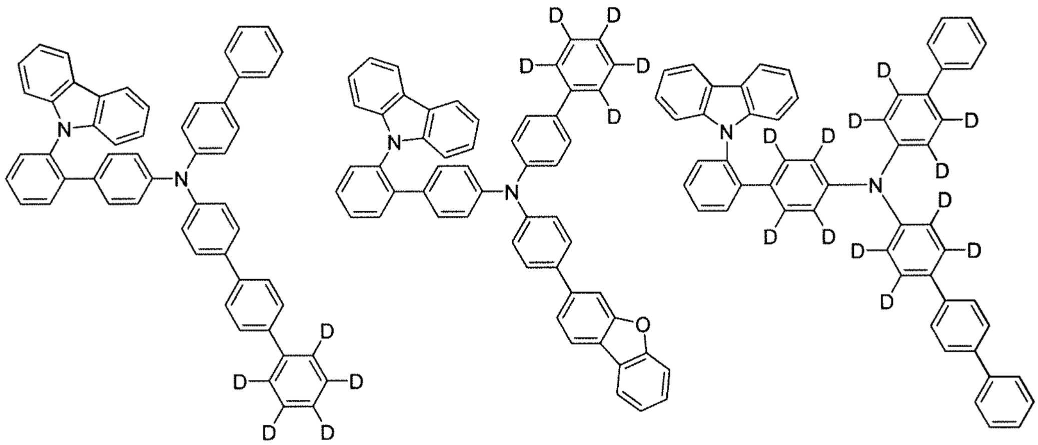

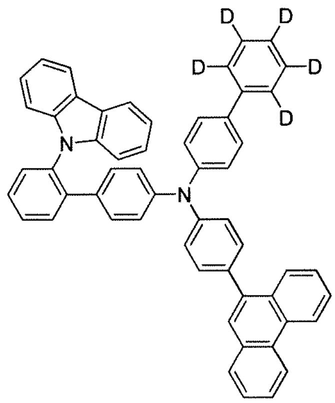

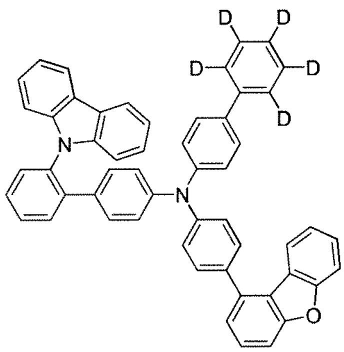

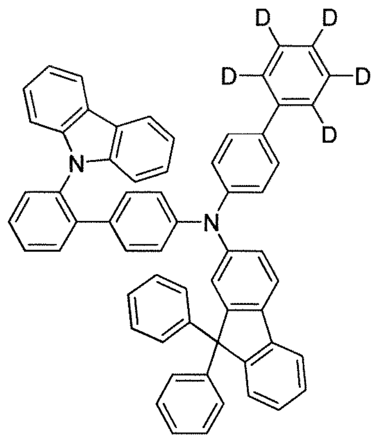

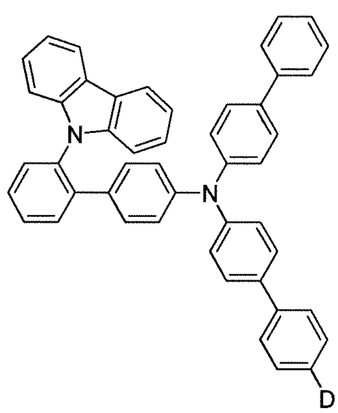

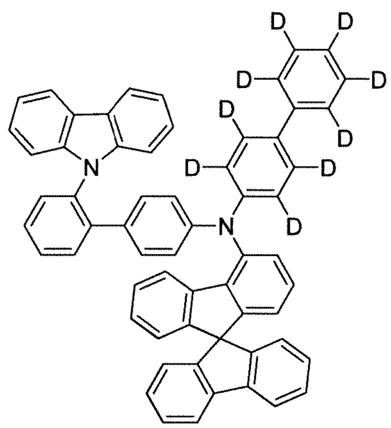

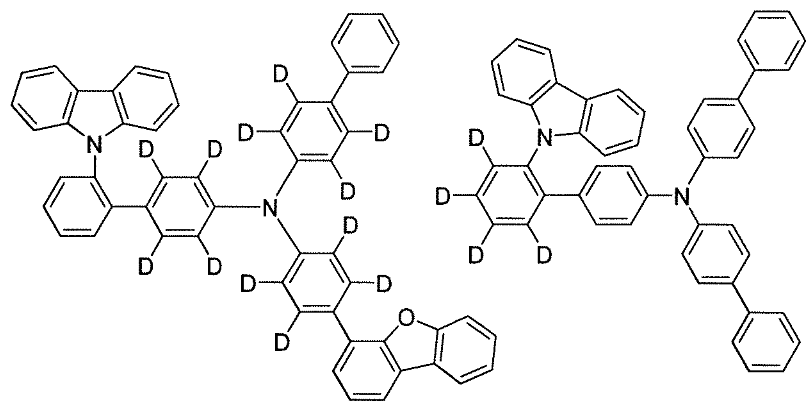

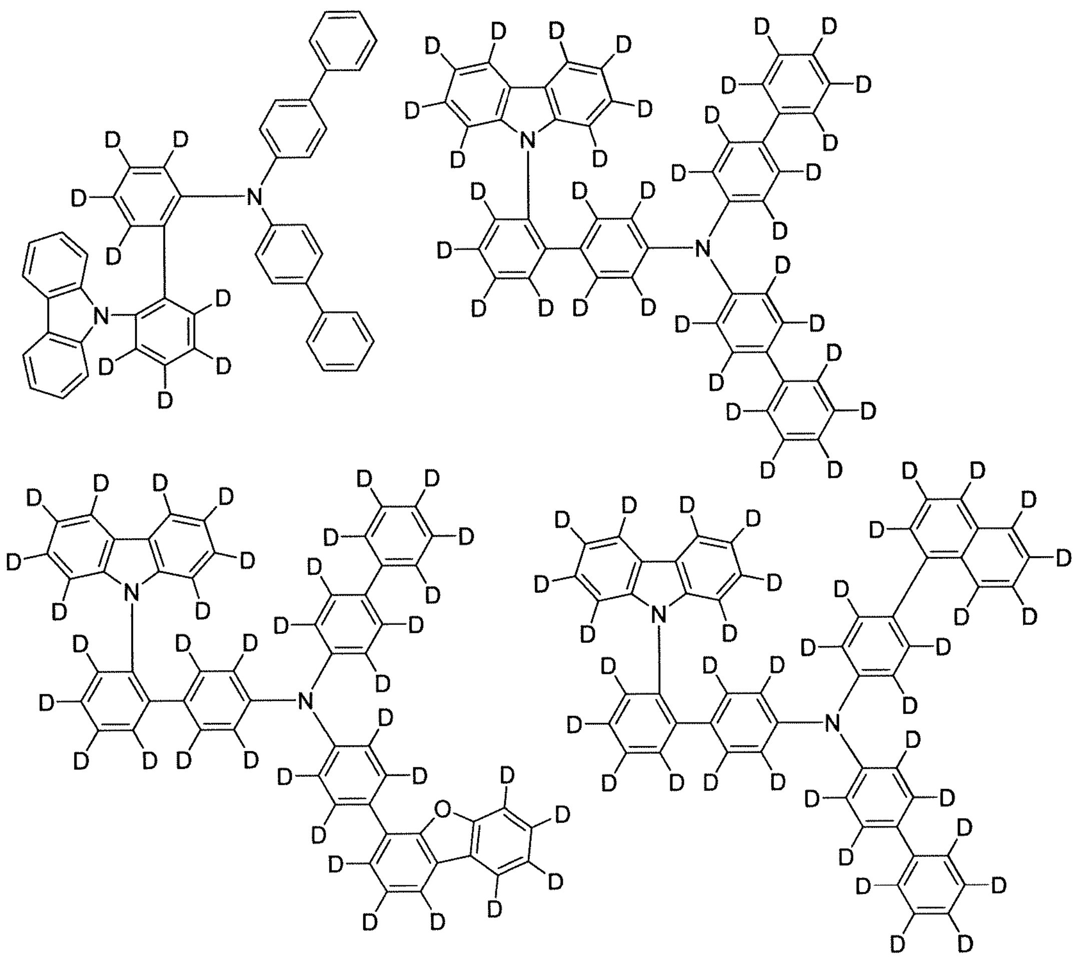

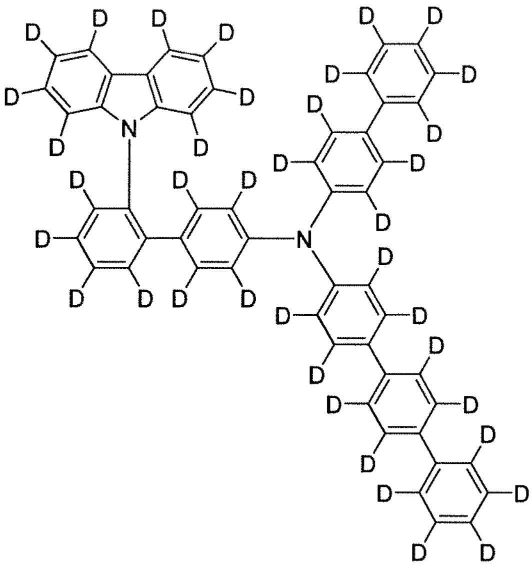

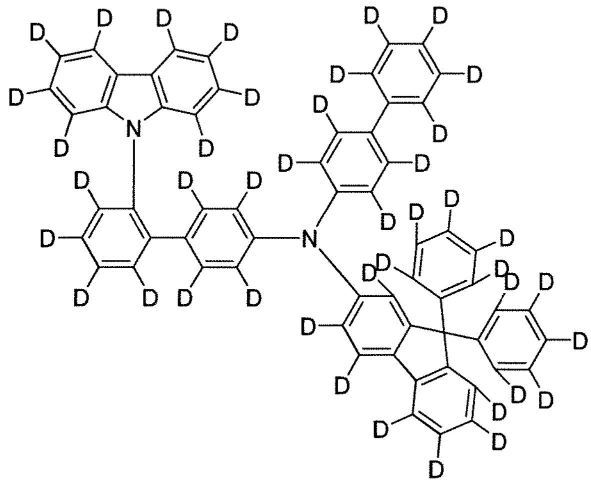

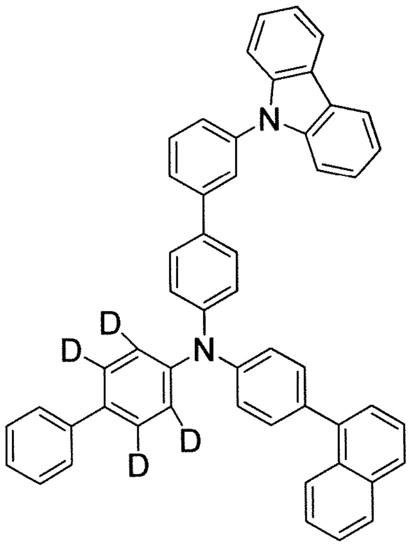

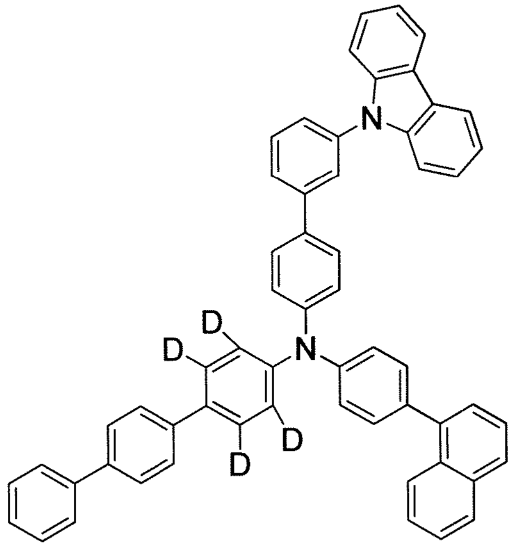

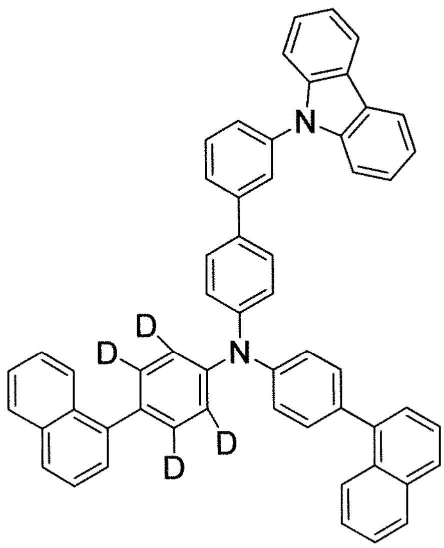

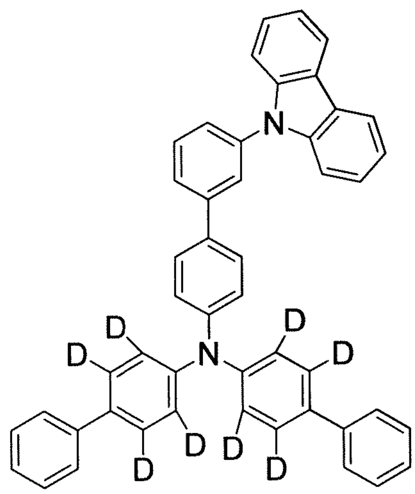

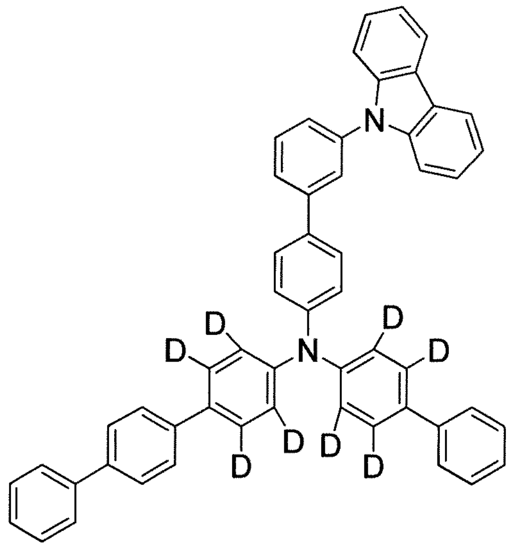

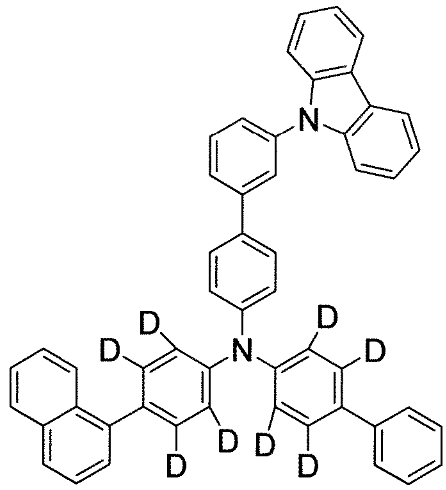

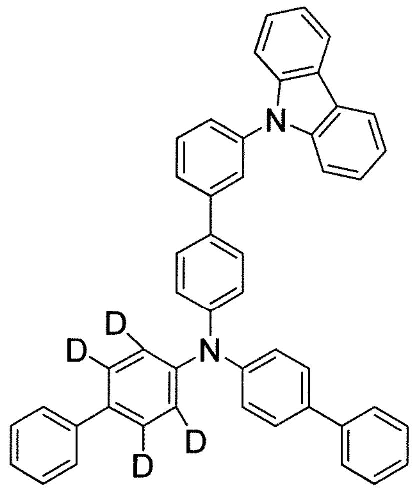

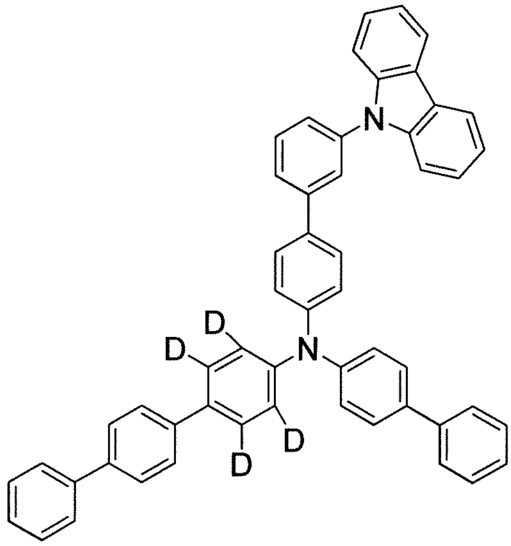

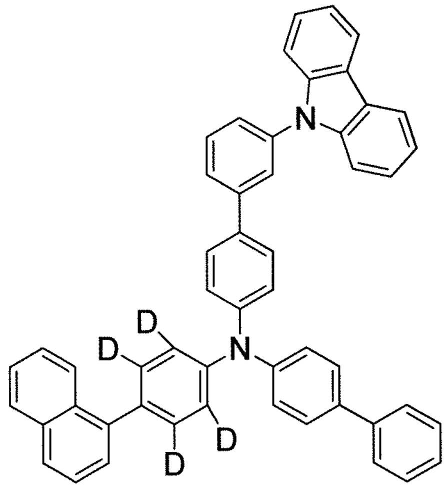

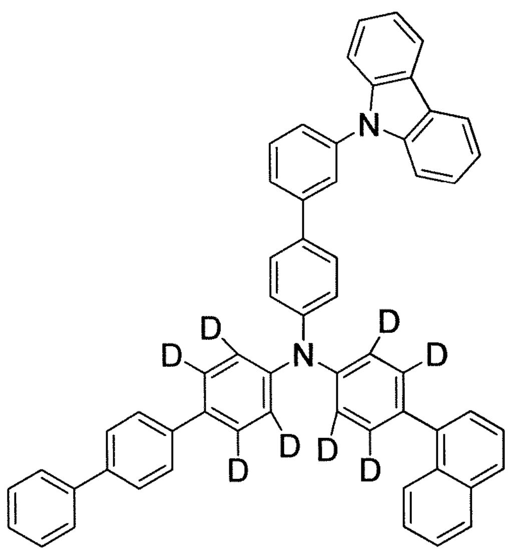

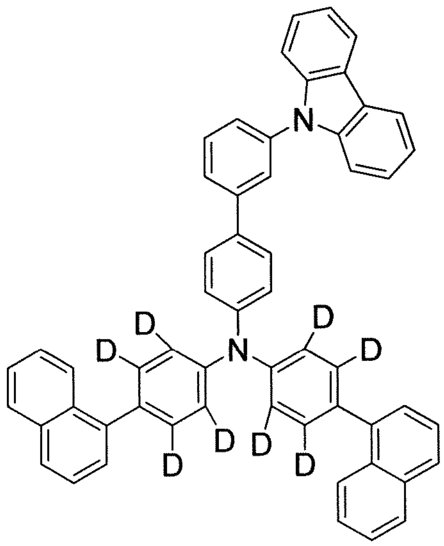

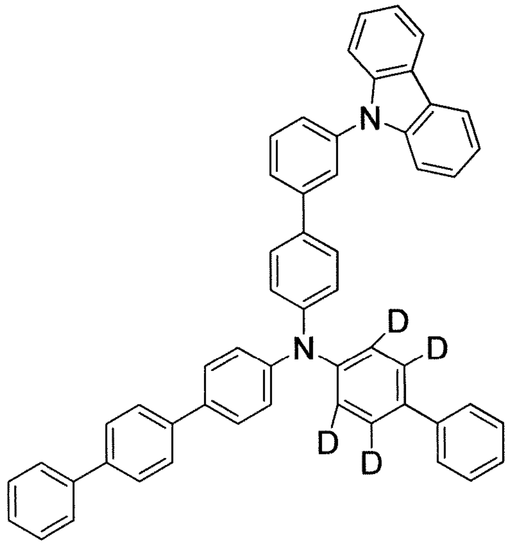

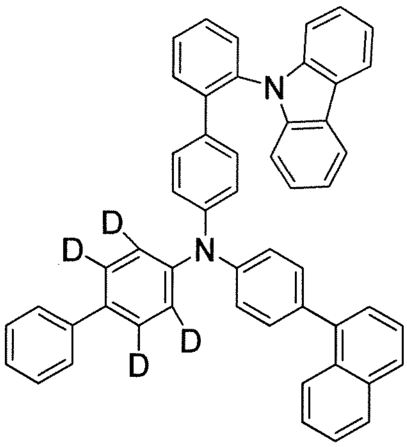

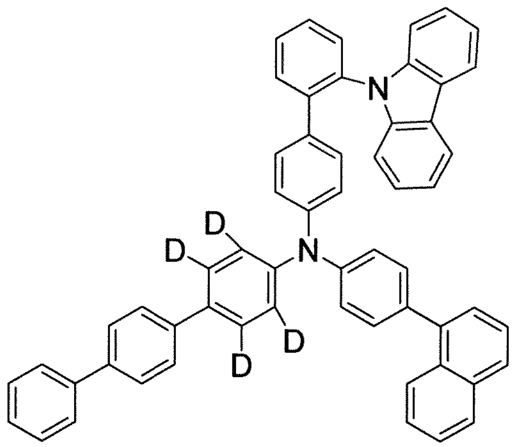

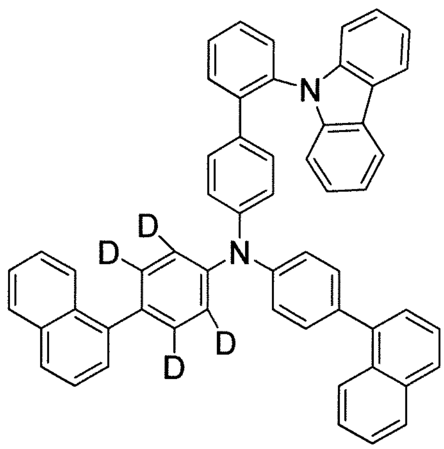

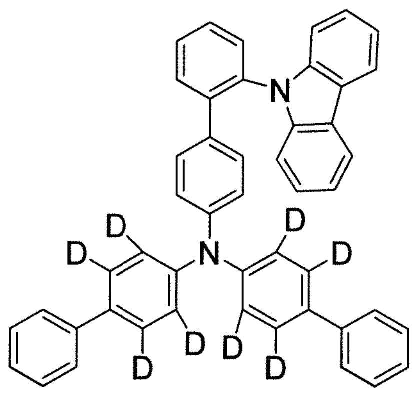

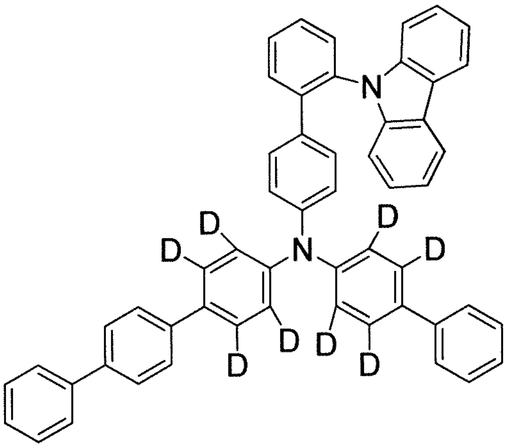

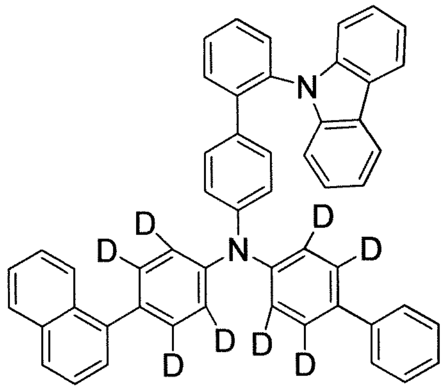

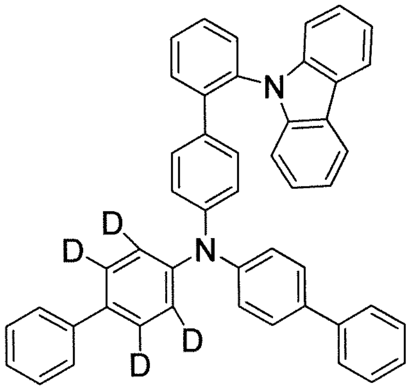

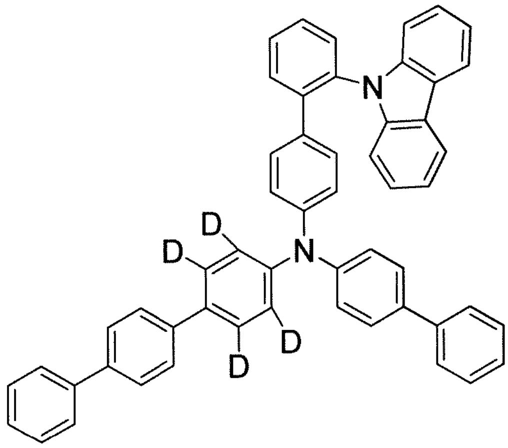

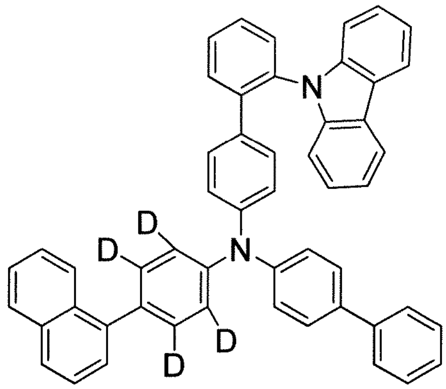

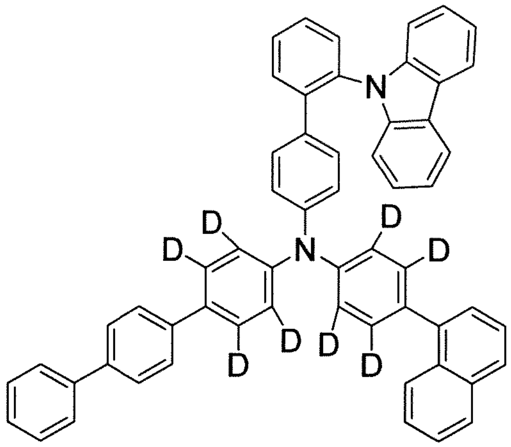

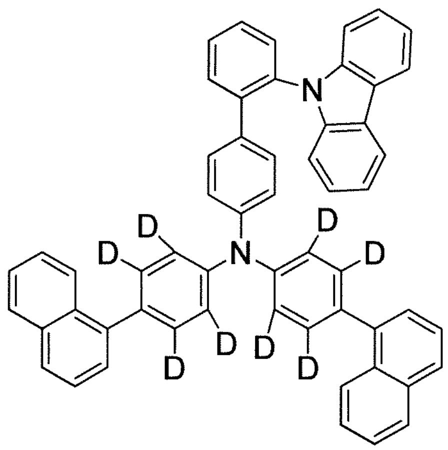

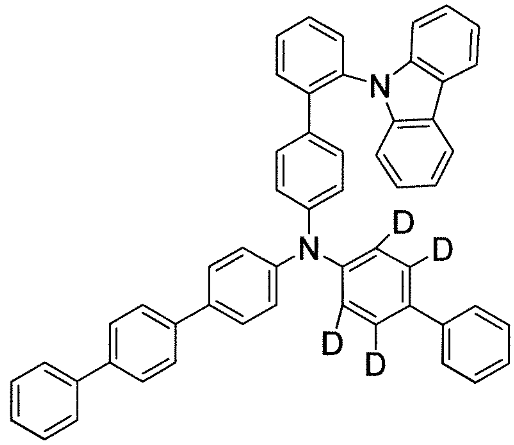

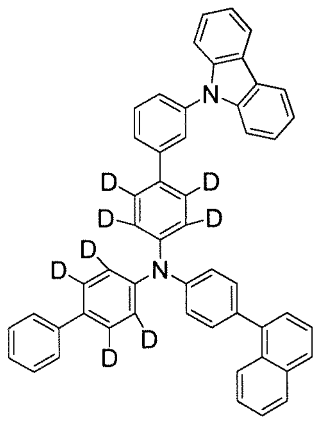

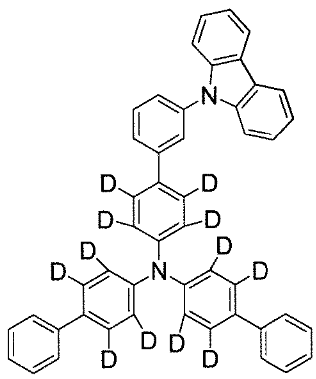

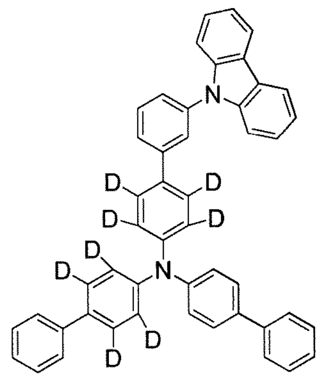

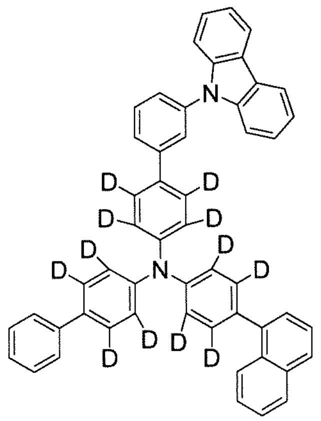

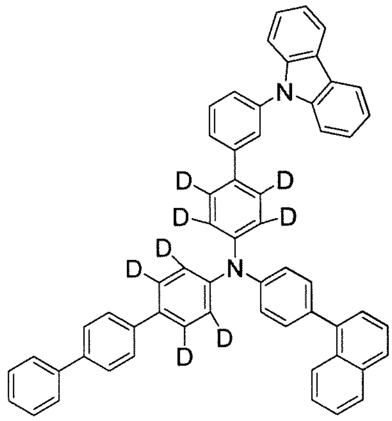

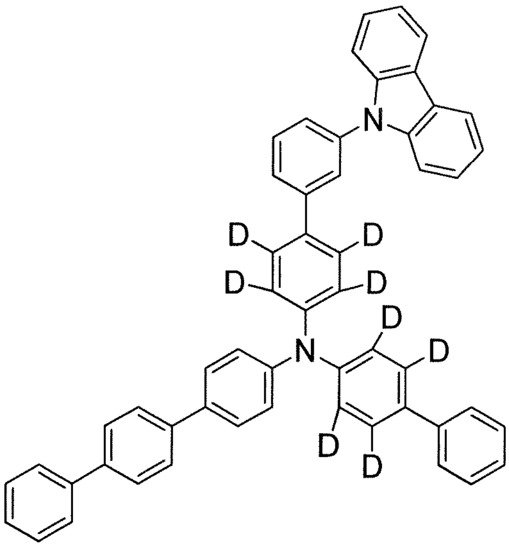

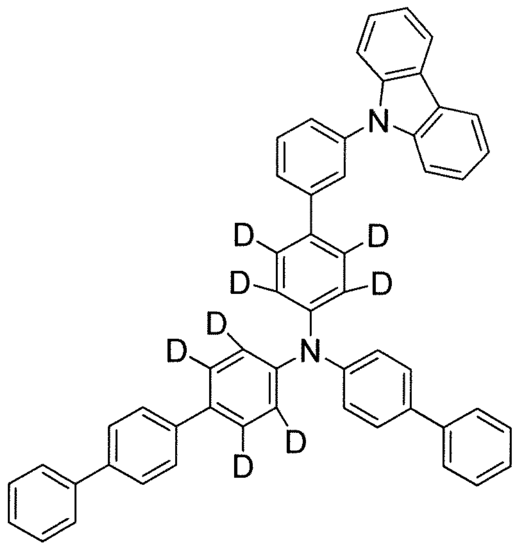

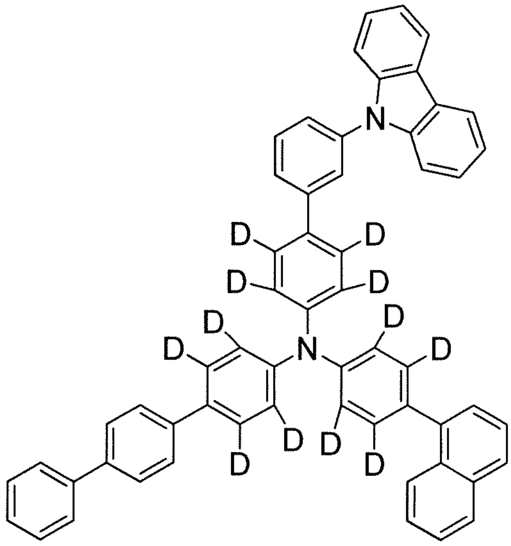

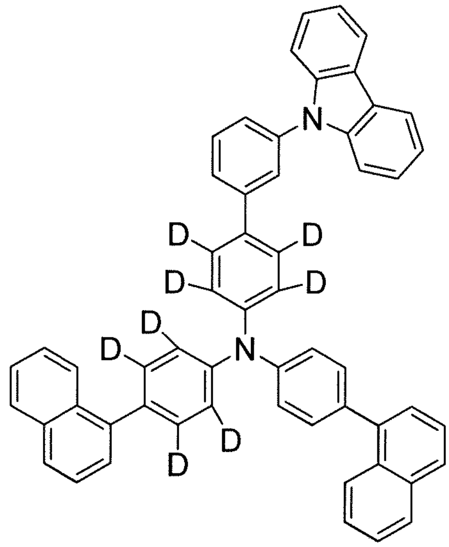

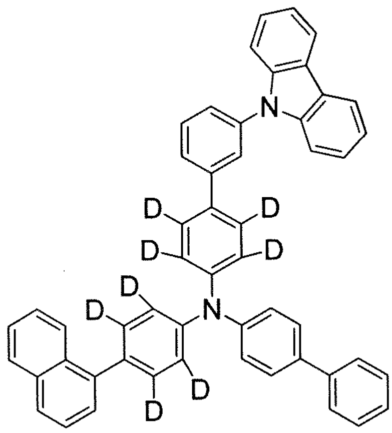

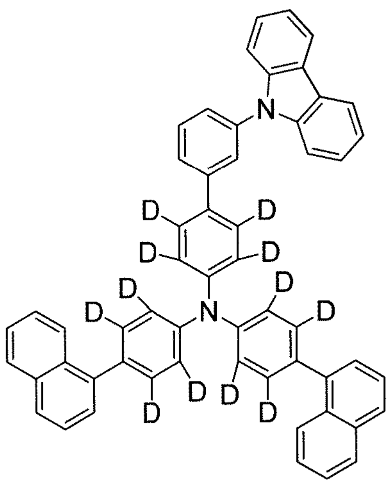

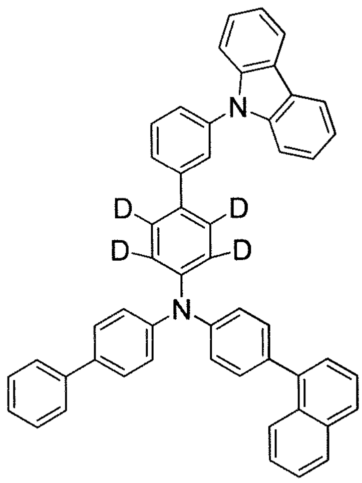

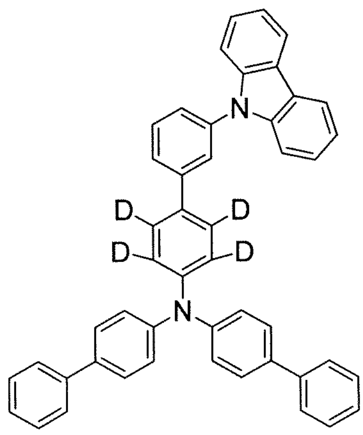

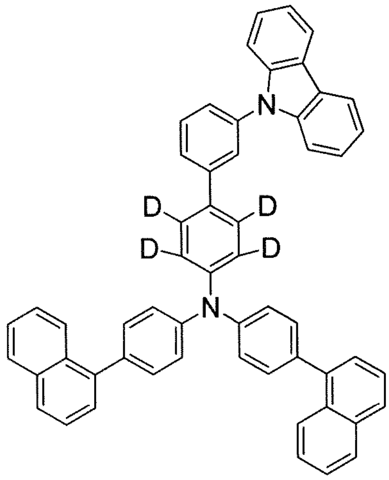

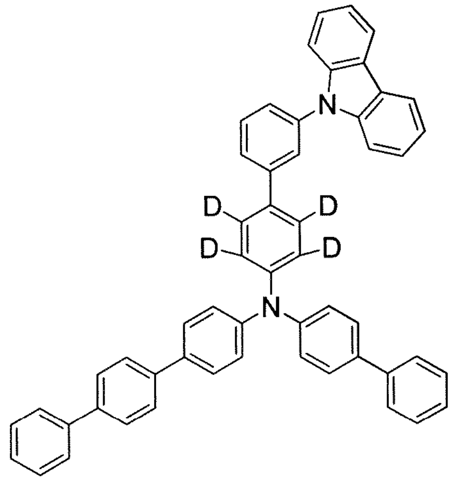

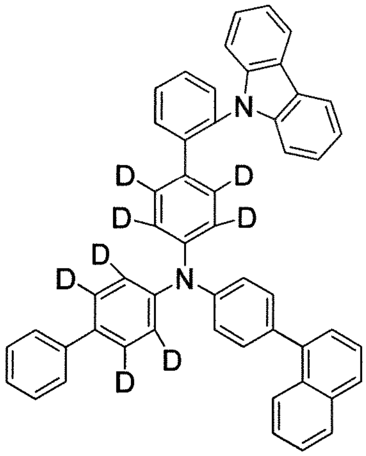

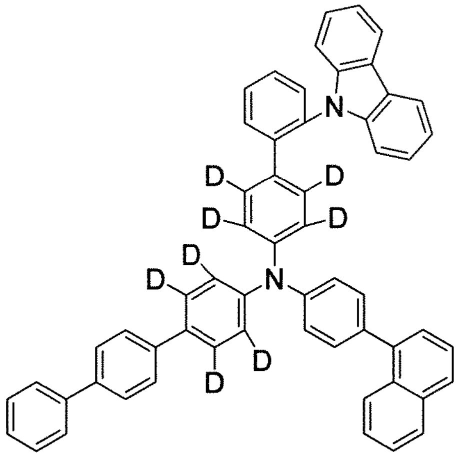

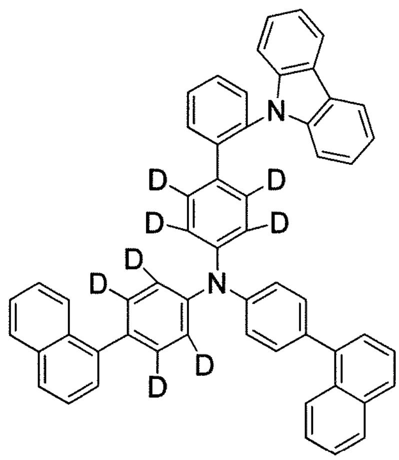

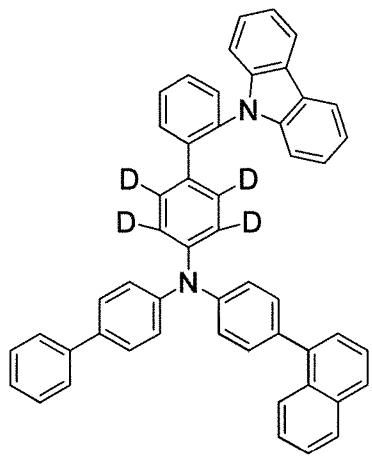

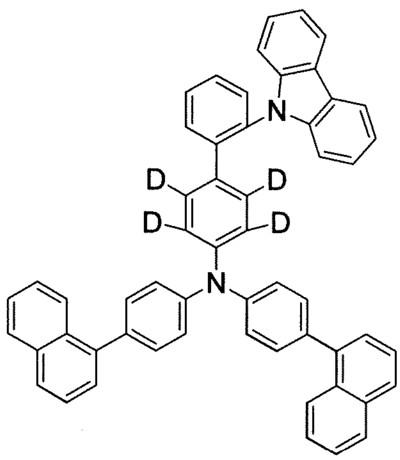

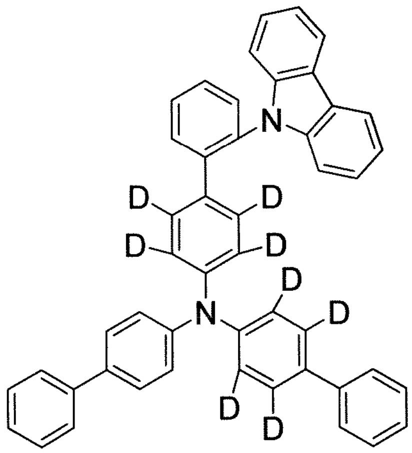

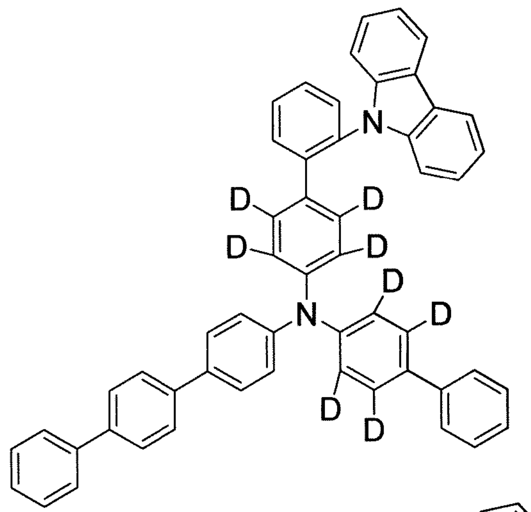

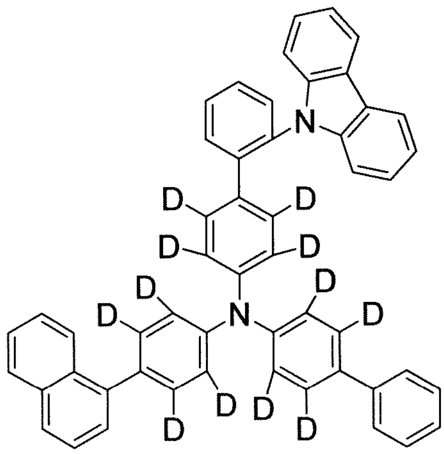

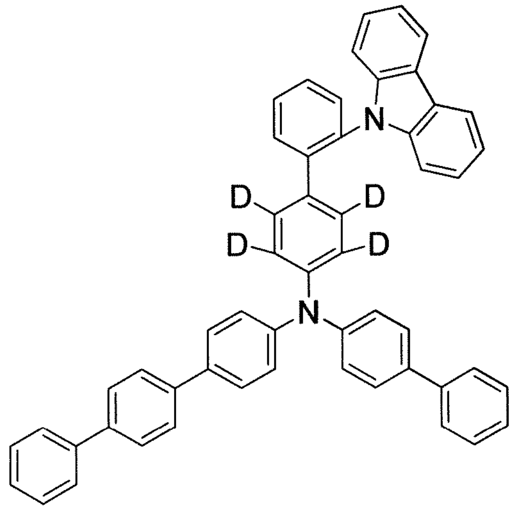

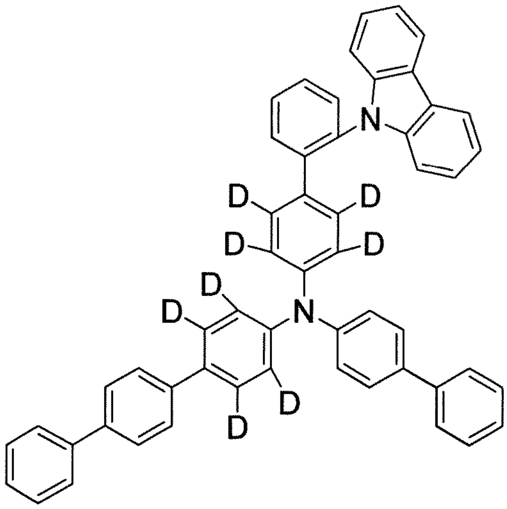

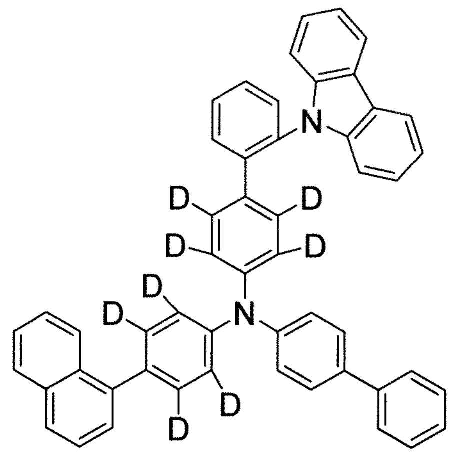

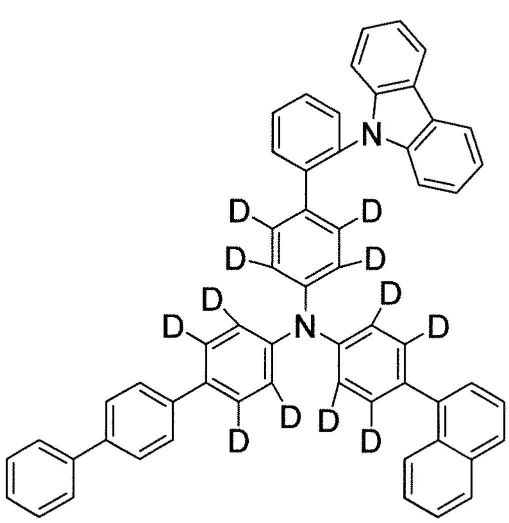

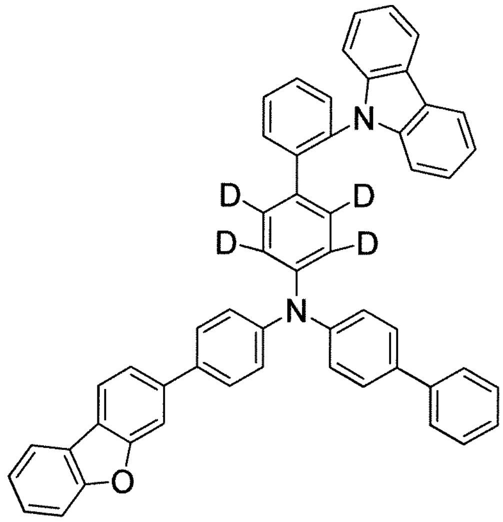

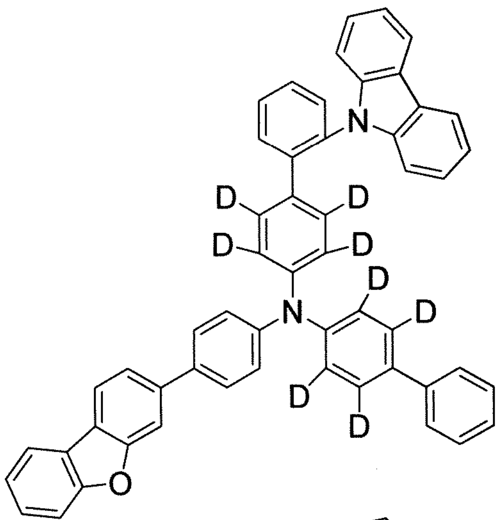

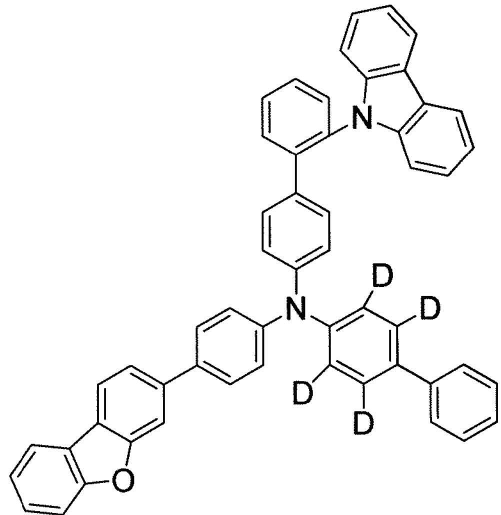

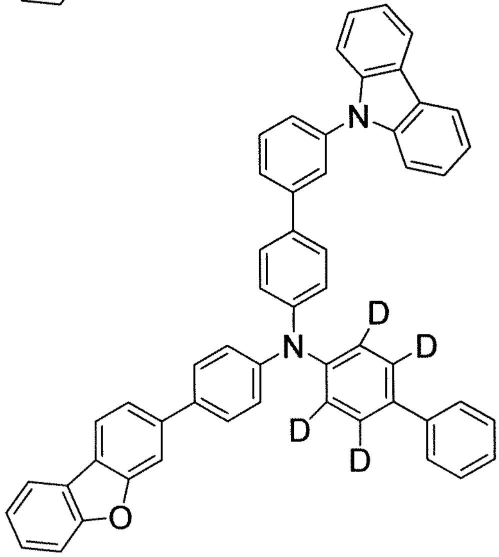

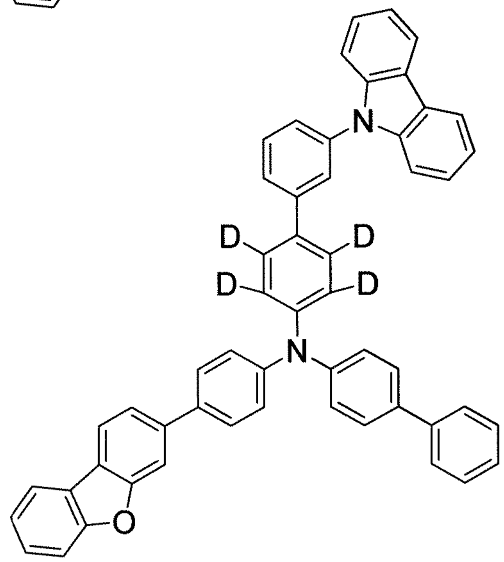

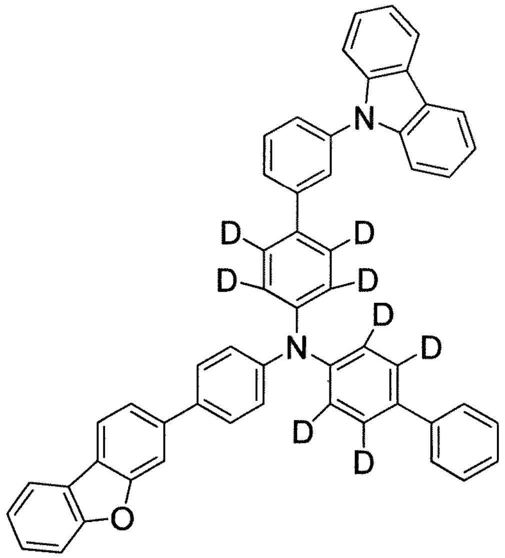

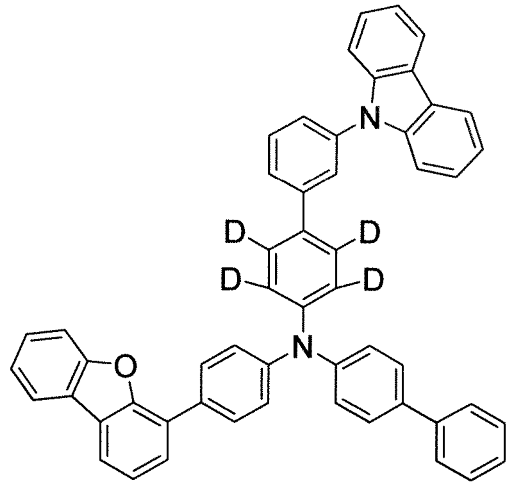

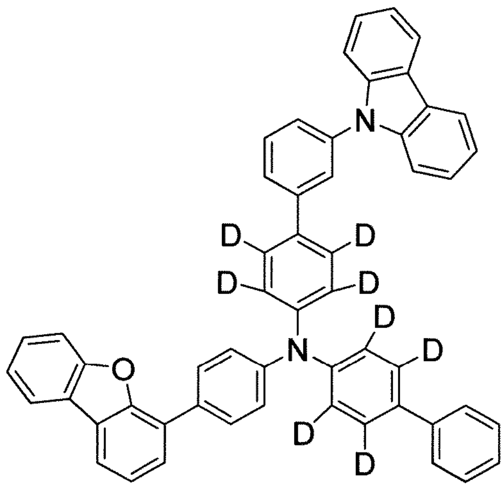

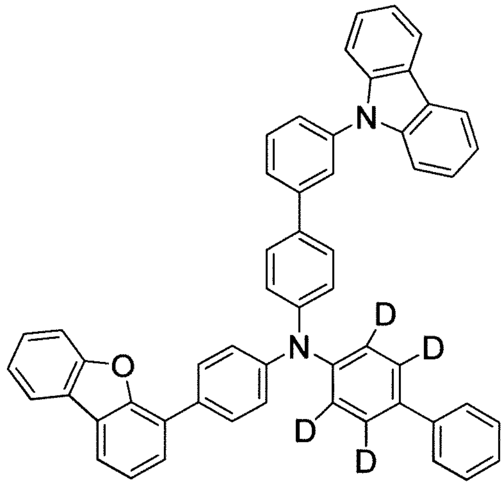

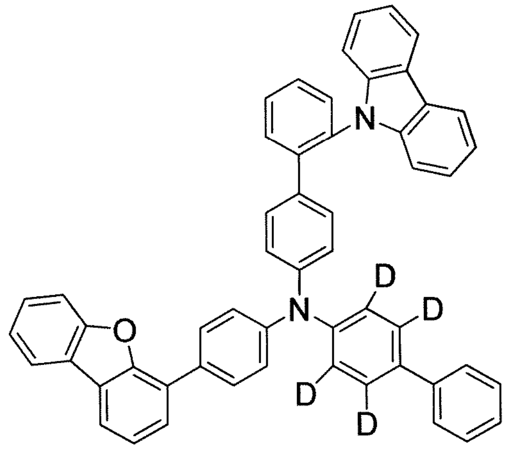

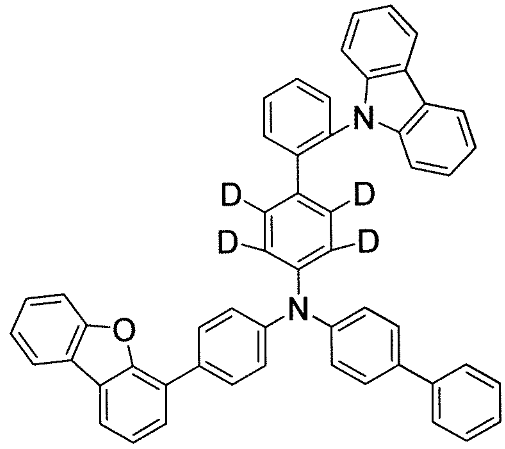

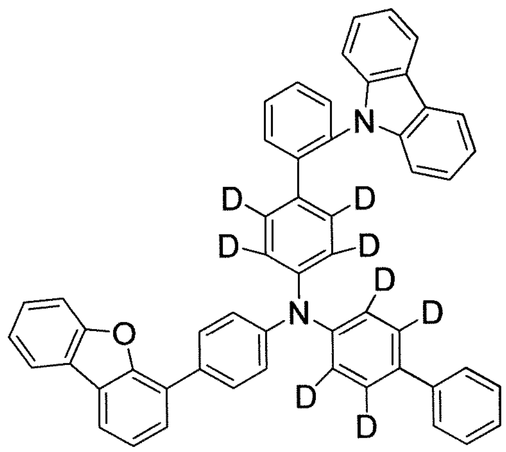

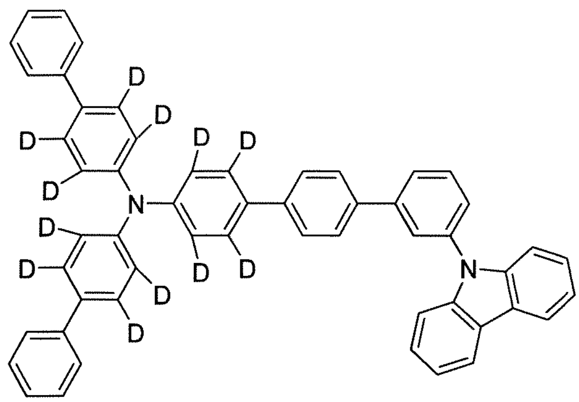

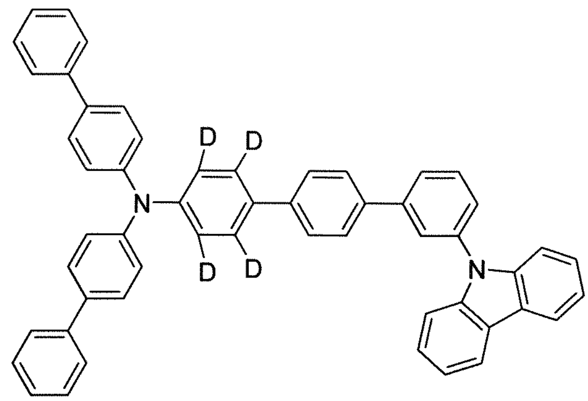

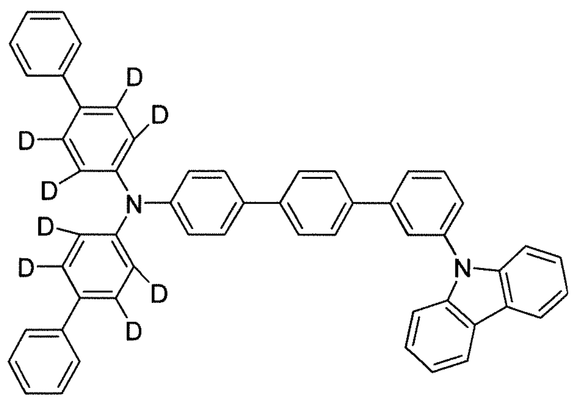

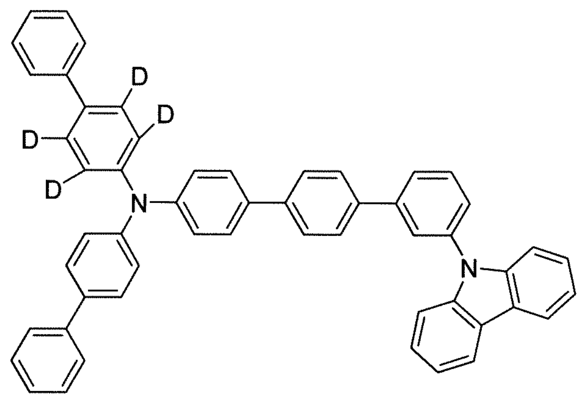

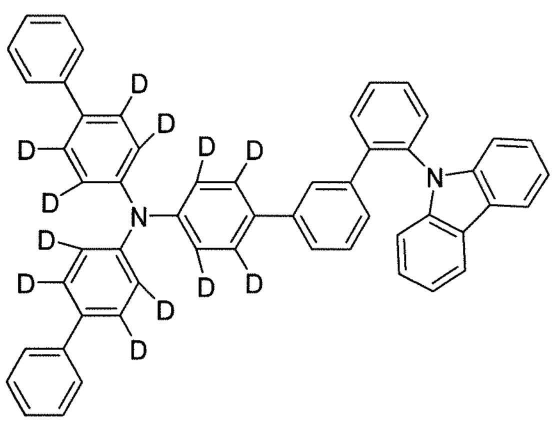

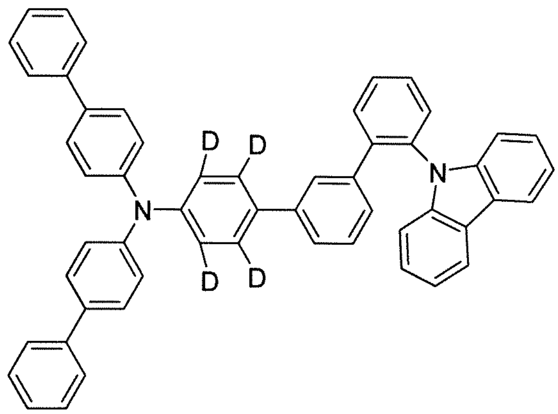

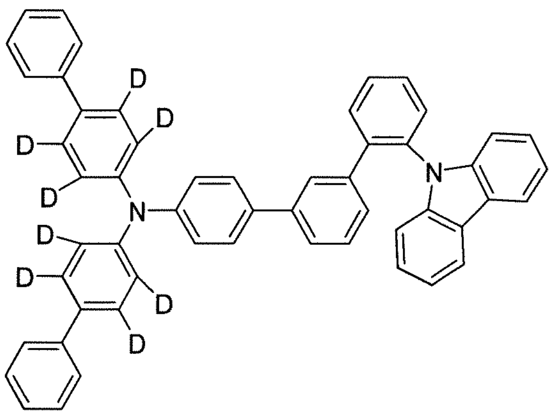

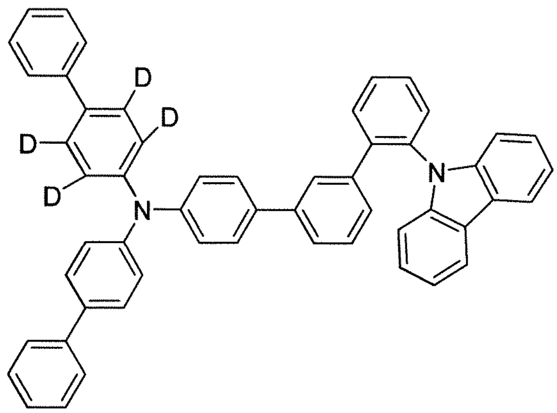

<Material for Organic EL devices>

The material for organic EL devices according to one embodiment of the present invention contains the inventive compound. The content of the inventive compound in the material for organic EL devices of the present invention may be 1% by mass or more (including 100%), and is preferably 10% by mass or more (including 100%), more preferably 50% by mass or more (including 100%), still more preferably 80% by mass or more (including 100%), and particularly preferably 90% by mass or more (including 100%). The material for organic EL devices according to one embodiment of the present invention is useful for the production of an organic EL device.

In one embodiment of the present invention, it is preferable to further contain a light hydrogen compound of the inventive compound. The light hydrogen compound refers to a compound in which all hydrogen atoms in the inventive compound are light hydrogen atoms.

The mixing molar ratio of the inventive compound to the light hydrogen compound of the inventive compound (inventive compound : light hydrogen compound) is preferably 10:90 to 90:10, more preferably 20:80 to 80:20, still more preferably 30:70 to 70:30, and particularly preferably 40:60 to 60:40.

In one embodiment of the present invention, it is preferable to contain at least two kinds of the inventive compounds.

In one embodiment of the present invention, the material for an organic electroluminescent element is a material for an organic electroluminescent element containing a first compound and a second compound, wherein the first compound is the inventive compound, the material for an organic electroluminescent element preferably contains 1% by mass or more of the first compound, and more preferably the first compound and the second compound are hole transporting layer materials.

The content of the first compound in the material for an organic electroluminescent element may be 1% by mass or more (including 100%), and is preferably 10% by mass or more (including 100%), more preferably 50% by mass or more (including 100%), still more preferably 80% by mass or more (including 100%), and particularly preferably 90% by mass or more (including 100%).

<Organic EL Device>

The organic EL device according to one embodiment of the present invention includes an anode, a cathode, and organic layers disposed between the anode and the cathode. The organic layers include a light emitting layer, and at least one layer of the organic layers contains the inventive compound.

Examples of the organic layer containing the inventive compound include a hole transporting zone (such as a hole injecting layer, a hole transporting layer, an electron blocking layer, and an exciton blocking layer) intervening between the anode and the light emitting layer, the light emitting layer, a space layer, and an electron transporting zone (such as an electron injecting layer, an electron transporting layer, and a hole blocking layer) intervening between the cathode and the light emitting layer, but are not limited thereto. The inventive compound is preferably used as a material for the hole transporting zone or the light emitting layer in a fluorescent or phosphorescent EL device, more preferably as a material for the hole transporting zone, still more preferably as a material for the hole injecting layer, the hole transporting layer, the electron blocking layer, or the exciton blocking layer, and particularly preferably as a material for the hole injecting layer or the hole transporting layer.

The organic EL device according to one embodiment of the present invention may be a fluorescent or phosphorescent light emission-type monochromatic light emitting device or a fluorescent/phosphorescent hybrid-type white light emitting device, and may be a simple type having a single light emitting unit or a tandem type having a plurality of light emitting units. Above all, the fluorescent light emission-type device is preferred. The "light emitting unit" referred to herein refers to a minimum unit that emits light through recombination of injected holes and electrons, which includes organic layers among which at least one layer is a light emitting layer.

For example, as a representative device configuration of the simple type organic EL device, the following device configuration may be exemplified.

(1) Anode/Light Emitting Unit/Cathode

The light emitting unit may be a multilayer type having a plurality of phosphorescent light emitting layers or fluorescent light emitting layers. In this case, a space layer may intervene between the light emitting layers for the purpose of preventing excitons generated in the phosphorescent light emitting layer from diffusing into the fluorescent light emitting layer. Representative layer configurations of the simple type light emitting unit are described below. Layers in parentheses are optional.

(a) (hole injecting layer/) hole transporting layer/fluorescent light emitting layer/electron transporting layer (/electron injecting layer)
(b) (hole injecting layer/) hole transporting layer/phosphorescent light emitting layer/electron transporting layer (/electron injecting layer)
(c) (hole injecting layer/) hole transporting layer/first fluorescent light emitting layer/second fluorescent light emitting layer/electron transporting layer (/electron injecting layer)
(d) (hole injecting layer/) hole transporting layer/first phosphorescent light emitting layer/second phosphorescent light emitting layer/electron transporting layer (/electron injecting layer)
(e) (hole injecting layer/) hole transporting layer/phosphorescent light emitting layer/space layer/fluorescent light emitting layer/electron transporting layer (/electron injecting layer)
(f) (hole injecting layer/) hole transporting layer/first phosphorescent light emitting layer/second phosphorescent light emitting layer/space layer/fluorescent light emitting layer/electron transporting layer (/electron injecting layer)
(g) (hole injecting layer/) hole transporting layer/first phosphorescent light emitting layer/space layer/second phosphorescent light emitting layer/space layer/fluorescent light emitting layer/electron transporting layer (/electron injecting layer)
(h) (hole injecting layer/) hole transporting layer/phosphorescent light emitting layer/space layer/first fluorescent light emitting layer/second fluorescent light emitting layer/electron transporting layer (/electron injecting layer)
(i) (hole injecting layer/) hole transporting layer/electron blocking layer/fluorescent light emitting layer/electron transporting layer (/electron injecting layer)
(j) (hole injecting layer/) hole transporting layer/electron blocking layer/phosphorescent light emitting layer/electron transporting layer (/electron injecting layer)
(k) (hole injecting layer/) hole transporting layer/exciton blocking layer/fluorescent light emitting layer/electron transporting layer (/electron injecting layer)
(l) (hole injecting layer/) hole transporting layer/exciton blocking layer/phosphorescent light emitting layer/electron transporting layer (/electron injecting layer)
(m) (hole injecting layer/) first hole transporting layer/second hole transporting layer/fluorescent light emitting layer/electron transporting layer (/electron injecting layer)

(n) (hole injecting layer/) first hole transporting layer/second hole transporting layer/phosphorescent light emitting layer/electron transporting layer (/electron injecting layer)

(o) (hole injecting layer/) first hole transporting layer/second hole transporting layer/fluorescent light emitting layer/first electron transporting layer/second electron transporting layer (/electron injecting layer)

(p) (hole injecting layer/) first hole transporting layer/second hole transporting layer/phosphorescent light emitting layer/first electron transporting layer/second electron transporting layer (/electron injecting layer)

(q) (hole injecting layer/) hole transporting layer/fluorescent light emitting layer/hole blocking layer/electron transporting layer (/electron injecting layer)

(r) (hole injecting layer/) hole transporting layer/phosphorescent light emitting layer/hole blocking layer/electron transporting layer (/electron injecting layer)

(s) (hole injecting layer/) hole transporting layer/fluorescent light emitting layer/exciton blocking layer/electron transporting layer (/electron injecting layer)

(t) (hole injecting layer/) hole transporting layer/phosphorescent light emitting layer/exciton blocking layer/electron transporting layer (/electron injecting layer)

The phosphorescent and fluorescent light emitting layers may emit emission colors different from each other, respectively. Specifically, in the light emitting unit (f), a layer configuration, such as (hole injecting layer/) hole transporting layer/first phosphorescent light emitting layer (red light emission)/second phosphorescent light emitting layer (green light emission)/space layer/fluorescent light emitting layer (blue light emission)/electron transporting layer, may be exemplified.

Further, an electron blocking layer may be properly provided between each light emitting layer and the hole transporting layer or the space layer. In addition, a hole blocking layer may be properly provided between each light emitting layer and the electron transporting layer. The employment of the electron blocking layer or the hole blocking layer allows to improve the emission efficiency by trapping electrons or holes within the light emitting layer and increasing the probability of charge recombination in the light emitting layer.

As a representative device configuration of the tandem type organic EL device, the following device configuration may be exemplified.

(2) Anode/First Light Emitting Unit/Intermediate Layer/Second Light Emitting Unit/Cathode

For example, each of the first light emitting unit and the second light emitting unit may be independently selected from the above-described light emitting units.

The intermediate layer is also generally referred to as an intermediate electrode, an intermediate conductive layer, a charge generation layer, an electron withdrawing layer, a connecting layer, or an intermediate insulating layer, and a known material configuration can be used, in which electrons are supplied to the first light emitting unit, and holes are supplied to the second light emitting unit.

Fig. 1 is a schematic illustration showing an example of the configuration of the organic EL device according to one embodiment of the present invention. The organic EL device 1 of this example includes a substrate 2, an anode 3, a cathode 4, and a light emitting unit 10 disposed between the anode 3 and the cathode 4. The light emitting unit 10 includes a light emitting layer 5. A hole transporting zone 6 (such as a hole injecting layer and a hole transporting layer) is provided between the light emitting layer 5 and the anode 3, and an electron transporting zone 7 (such as an electron injecting layer and an electron transporting layer) is provided between the light emitting layer 5 and the cathode 4. In addition, an electron blocking layer (which is not shown in the figure) may be provided on the side of the anode 3 of the light emitting layer 5, and a hole blocking layer (which is not shown in the figure) may be provided on the side of the cathode 4 of the light emitting layer 5. According to the configuration, electrons and holes are trapped in the light emitting layer 5, thereby enabling one to further increase the production efficiency of excitons in the light emitting layer 5.

Fig. 2 is a schematic illustration showing another configuration of the organic EL device according to one embodiment of the present invention. The organic EL device 11 of this example includes a substrate 2, an anode 3, a cathode 4, and a light emitting unit 20 disposed between the anode 3 and the cathode 4. The light emitting unit 20 includes a light emitting layer 5. The hole transporting zone disposed between the anode 3 and the light emitting layer 5 is formed by a hole injecting layer 6a, a first hole transporting layer 6b, and a second hole transporting layer 6c. In addition, the electron transporting zone disposed between the light emitting layer 5 and the cathode 4 is formed by a first electron transporting layer 7a and a second electron transporting layer 7b.

In the present invention, a host combined with a fluorescent dopant (a fluorescent emitting material) is referred to as a fluorescent host, and a host combined with a phosphorescent dopant is referred to as a phosphorescent host. The fluorescent host and the phosphorescent host are not distinguished from each other merely by the molecular structures thereof. Specifically, the phosphorescent host means a material that forms a phosphorescent light emitting layer containing a phosphorescent dopant, but does not mean unavailability as a material that forms a fluorescent light emitting layer. The same also applies to the fluorescent host.

<Substrate>

The substrate is used as a support of the organic EL device. Examples of the substrate include a plate of glass, quartz, and plastic. In addition, a flexible substrate may be used. Examples of the flexible substrate include a plastic substrate made of polycarbonate, polyarylate, polyether sulfone, polypropylene, polyester, polyvinyl fluoride, or polyvinyl chloride. In addition, an inorganic vapor deposition film can be used.

<Anode>

It is preferred that a metal, an alloy, an electrically conductive compound, or a mixture thereof which has a high work function (specifically 4.0 eV or more) is used for the anode formed on the substrate. Specific examples thereof include indium oxide-tin oxide (ITO: Indium Tin Oxide), indium oxide-tin oxide containing silicon or silicon oxide, indium oxide-zinc oxide, indium oxide containing tungsten oxide and zinc oxide, and graphene. Besides, examples thereof include gold (Au), platinum (Pt), nickel (Ni), tungsten (W), chromium (Cr), molybdenum (Mo), iron (Fe), cobalt (Co), copper (Cu), palladium (Pd), titanium (Ti), or nitrides of the metals (for example, titanium nitride).

These materials are usually deposited by a sputtering method. For example, through a sputtering method, it is possible to form indium oxide-zinc oxide by using a target in which 1 to 10 wt% of zinc oxide is added to indium oxide, and to form indium oxide containing tungsten oxide and zinc oxide by using a target containing 0.5 to 5 wt% of tungsten oxide and 0.1 to 1 wt% of zinc oxide with respect to indium oxide. Besides, the manufacturing may be performed by a vacuum vapor deposition method, a coating method, an inkjet method, a spin coating method, or the like.

The hole injecting layer formed in contact with the anode is formed by using a material that facilitates hole injection regardless of a work function of the anode, and thus, it is possible to use materials generally used as an electrode material (for example, metals, alloys, electrically conductive compounds, or mixtures thereof, elements belonging to Group 1 or 2 of the periodic table of the elements).

It is also possible to use elements belonging to Group 1 or 2 of the periodic table of the elements, which are materials having low work functions, that is, alkali metals, such as lithium (Li) and cesium (Cs), alkaline earth metals, such as magnesium (Mg), calcium (Ca), and strontium (Sr), and alloys containing these (such as MgAg and AlLi), and rare earth metals, such as europium (Eu) and ytterbium (Yb), and alloys containing these. When the anode is formed by using the alkali metals, the alkaline earth metals, and alloys containing these, a vacuum vapor deposition method or a sputtering method can be used. Further, when a silver paste or the like is used, a coating method, an inkjet method, or the like can be used.

<Hole Injecting Layer>

The hole injecting layer is a layer containing a material having a high hole injection capability (a hole injecting material) and is provided between the anode and the light emitting layer, or between the hole transporting layer, if exists, and the anode.

As the hole injecting material other than the inventive compound, molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, and the like can be used.

Examples of the hole injecting layer material also include aromatic amine compounds as low-molecular weight organic compounds, such as 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1).

High-molecular weight compounds (such as oligomers, dendrimers, and polymers) may also be used. Examples thereof include high-molecular weight compounds, such as poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). In addition, high-molecular weight compounds to which an acid is added, such as poly(3,4-ethylenedioxythiophene)/poly (styrene sulfonic acid) (PEDOT/PSS), and polyaniline/poly (styrenesulfonic acid) (PAni/PSS), can also be used.

Furthermore, it is also preferred to use an acceptor material, such as a hexaazatriphenylene (HAT) compound represented by formula (K).

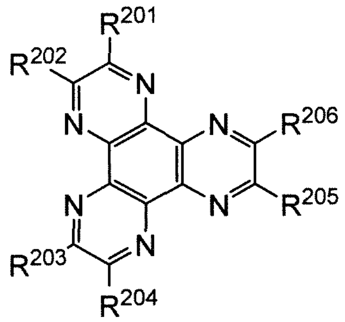

(K)

In the aforementioned formula, $R^{201}$ to $R^{206}$ each independently represent a cyano group, $-CONH_2$, a carboxy group, or $-COOR^{207}$ ($R^{207}$ represents an alkyl group having 1 to 20 carbon atoms or a cycloalkyl group having 3 to 20 carbon atoms). In addition, adjacent two selected from $R^{201}$ and $R^{202}$, $R^{203}$ and $R^{204}$, and $R^{205}$ and $R^{206}$ may be bonded to each other to form a group represented by $-CO-O-CO^-$.

Examples of $R^{207}$ include a methyl group, an ethyl group, an n-propyl group, an isopropyl group, an n-butyl group, an isobutyl group, a t-butyl group, a cyclopentyl group, and a cyclohexyl group.

<Hole Transporting Layer>

The hole transporting layer is a layer containing a material having a high hole transporting capability (a hole transporting material) and is provided between the anode and the light emitting layer, or between the hole injecting layer, if exists, and the light emitting layer. The inventive compound may be used alone or in combination with the following compounds in the hole transporting layer.

The hole transporting layer may have a single layer structure or a multilayer structure including two or more layers. For example, the hole transporting layer may have a two-layer structure including a first hole transporting layer (anode side) and a second hole transporting layer (cathode side). In one embodiment of the present invention, the hole transporting layer having a single layer structure is preferably disposed adjacent to the light emitting layer, and the hole transporting layer that is closest to the cathode in the multilayer structure, such as the second hole transporting layer in the two-layer structure, is preferably disposed adjacent to the light emitting layer. In another embodiment of the present invention, an electron blocking layer described later and the like may be disposed between the hole transporting layer having a single layer structure and the light emitting layer, or between the hole transporting layer that is closest to the light emitting layer in the multilayer structure and the light emitting layer.

In the hole transporting layer having a two-layer structure, the inventive compound may be contained in one of the first hole transporting layer and the second hole transporting layer, and may be contained in both of them.

In one embodiment of the present invention, it is preferred that the inventive compound is contained only in the first hole transporting layer, and in another embodiment thereof, it is preferred that the inventive compound is contained only in the second hole transporting layer, and in still another embodiment thereof, it is preferred that the inventive compound is contained in the first hole transporting layer and the second hole transporting layer.

As the hole transporting layer material other than the inventive compound, for example, an aromatic amine compound, a carbazole derivative, an anthracene derivative, and the like can be used.

Examples of the aromatic amine compound include 4,4'-bis[N-(1-naphthyl)-N-phenylamino]biphenyl (abbreviation: NPB) or N,N'-bis(3-methylphenyl)-N,N'-diphenyl-[1,1'-biphenyl]-4,4'-diamine (abbreviation: TPD), 4-phenyl-4'-(9-phenylfluoren-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The aforementioned compounds have a hole mobility of $10^{-6}$ $cm^2/Vs$ or more.

Examples of the carbazole derivative include 4,4'-di(9-carbazolyl)biphenyl (abbreviation: CBP), 9-[4-(9-carbazolyl)phenyl]-10-phenylanthracene (abbreviation: CzPA), and 9-phenyl-3-[4-(10-phenyl-9-anthryl)phenyl]-9H-carbazole (abbreviation: PCzPA).

Examples of the anthracene derivative include 2-t-butyl-9,10-di(2-naphthyl)anthracene (abbreviation: t-BuDNA), 9,10-di(2-naphthyl)anthracene (abbreviation: DNA), and 9,10-diphenylanthracene (abbreviation: DPAnth).

High-molecular weight compounds, such as poly(N-vinylcarbazole) (abbreviation: PVK) and poly(4-vinyltriphenylamine) (abbreviation: PVTPA), can also be used.

However, compounds other than those as mentioned above can also be used so long as they are compounds high in the hole transporting capability rather than in the electron transporting capability.

<Dopant Material of Light Emitting Layer>

The light emitting layer is a layer containing a material having a high light emitting property (a dopant material), and various materials can be used. For example, a fluorescent emitting material or a phosphorescent emitting material can be used as the dopant material. The fluorescent emitting material is a compound that emits light from a singlet excited state, and the phosphorescent emitting material is a compound that emits light from a triplet excited state.

Examples of a blue-based fluorescent emitting material that can be used for the light emitting layer include a pyrene derivative, a styrylamine derivative, a chrysene derivative, a fluoranthene derivative, a fluorene derivative, a diamine derivative, and a triarylamine derivative. Specific examples thereof include N,N'-bis[4-(9H-carbazole-9-yl)phenyl]-N,N'-diphenylstilbene-4,4'-diamine (abbreviation: YGA2S), 4-(9H-carbazole-9-yl)-4'-(10-phenyl-9-anthryl)triphenylamine (abbreviation: YGAPA), and 4-(10-phenyl-9-anthryl)-4'-(9-phenyl-9H-carbazole-3-yl)triphenylamine (abbreviation: PCBAPA).

Examples of a green-based fluorescent emitting material that can be used for the light emitting layer include an aromatic amine derivative. Specific examples thereof include N-(9,10-diphenyl-2-anthryl)-N,9-diphenyl-9H-carbazole-3-amine (abbreviation: 2PCAPA), N-[9,10-bis(1,1'-biphenyl-2-yl)-2-anthryl]-N,9-diphenyl-9H-carbazole-3-amine (abbreviation: 2PCABPhA), N-(9,10-diphenyl-2-anthryl)-N,N',N'-triphenyl-1,4-phenylenediamine (abbreviation: 2DPAPA), N-[9,10-bis(1,1'-biphenyl-2-yl)-2-anthryl]-N,N',N'-triphenyl-1,4-phenylenediamine (abbreviation: 2DPABPhA), N-[9,10-bis(1,1'-biphenyl-2-yl)]-N-[4-(9H-carbazole-9-yl)phenyl]-N-phenylanthracene-2-amine (abbreviation: 2YGABPhA), and N,N,9-triphenylanthracene-9-amine (abbreviation: DPhAPhA).

Examples of a red-based fluorescent emitting material that can be used for the light emitting layer include a tetracene derivative and a diamine derivative. Specific examples thereof include N,N,N',N'-tetrakis(4-methylphenyl)tetracene-5,11-diamine (abbreviation: p-mPhTD) and 7,14-diphenyl-N,N,N',N'-tetrakis(4-methylphenyl)acenaphtho[1,2-a]fluoranthene-3,10-diamine (abbreviation: p-mPhAFD).

Examples of a blue-based phosphorescent emitting material that can be used for the light emitting layer include a metal complex, such as an iridium complex, an osmium complex, and a platinum complex. Specific examples thereof include bis[2-(4',6'-difluorophenyl)pyridinato- N,C2']iridium(III)tetrakis(1-pyrazolyl)borate (abbreviation: FIr6), bis[2-(4',6'-difluorophenyl)pyridinato-N,C2']iridium(III)picolinate (abbreviation: FIrpic), bis[2-(3', 5'bistrifluoromethylphenyl)pyridinato-N, C2']iridium(III)picolinate (abbreviation: Ir(CF3ppy)2(pic)), and bis[2-(4',6'-difluorophenyl)pyridinato-N,C2']iridium(III)acetylacetonate (abbreviation: FIracac).

Examples of a green-based phosphorescent emitting material that can be used for the light emitting layer include an iridium complex. Examples thereof include tris(2-phenylpyridinato-N,C2')iridium(III) (abbreviation: Ir(ppy)3), bis(2-phenylpyridinato-N,C2')iridium(III)acetylacetonate (abbreviation: Ir(ppy)2(acac)), bis(1,2-diphenyl-1H-benzimidazolato)iridium(III)acetylacetonate (abbreviation: Ir(pbi)2(acac)), and bis(benzo[h]quinolinato)iridium(III)acetylacetonate (abbreviation: Ir(bzq)2(acac)).

Examples of a red-based phosphorescent emitting material that can be used for the light emitting layer include a metal complex, such as an iridium complex, a platinum complex, a terbium complex, and a europium complex. Specific examples thereof include organic metal complexes, such as bis[2-(2'-benzo[4,5-$\alpha$]thienyl)pyridinato-N,C3']iridium(III)acetylacetonate (abbreviation: Ir(btp)2(acac)), bis(1-phenylisoquinolinato-N,C2')iridium(III)acetylacetonate (abbreviation: Ir(piq)2(acac)), (acetylacetonate)bis[2,3-bis(4-fluorophenyl)quinoxalinato]iridium(III) (abbreviation: Ir(Fdpq)2(acac)), and 2,3,7,8,12,13,17,18-octaethyl-21H,23H-porphyrinplatinum(II) (abbreviation: PtOEP).

Rare earth metal complexes, such as tris(acetylacetonate) (monophenanthroline)terbium(III) (abbreviation: Tb(acac)3(Phen)), tris(1,3-diphenyl-1,3- propanedionate)(monophenanthroline)europium(III) (abbreviation: Eu(DBM)3(Phen)), and tris[1-(2-thenoyl)-3,3,3-trifluoroacetonate](monophenanthroline)europium(III) (abbreviation: Eu(TTA)3(Phen)), emit light from rare earth metal ions (electron transition between different multiplicities), and thus may be used as the phosphorescent emitting material.

<Host Material of Light Emitting Layer>

The light emitting layer may have a configuration in which the aforementioned dopant material is dispersed in another material (a host material). It is preferred to use a material having a lowest unoccupied molecular orbital level (LUMO level) higher than that of the dopant material and a highest occupied molecular orbital level (HOMO level) lower than that of the dopant material.

Examples of the host material include:

(1) a metal complex, such as an aluminum complex, a beryllium complex, and a zinc complex,

(2) a heterocyclic compound, such as an oxadiazole derivative, a benzimidazole derivative, and a phenanthroline derivative,

(3) a fused aromatic compound, such as a carbazole derivative, an anthracene derivative, a phenanthrene derivative,

a pyrene derivative, and a chrysene derivative, or
(4) an aromatic amine compound, such as a triarylamine derivative and a fused polycyclic aromatic amine derivative.

For example, metal complexes, such as tris(8-quinolinolato)aluminum(III) (abbreviation: Alq), tris(4-methyl-8-quinolinolato)aluminum(III) (abbreviation: Almq3), bis(10-hydroxybenzo[h]quinolinato)beryllium(II) (abbreviation: BeBq2), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolatolzinc(II) (abbreviation: ZnPBO), and bis[2-(2-benzothiazolyl)phenolato]zinc(II) (abbreviation: ZnBTZ);

heterocyclic compounds, such as 2-(4-biphenylyl)-5-(4-tert-butylphenyl)-1,3,4-oxadiazole (abbreviation: PBD), 1,3-bis[5-(p-tert-butylphenyl)-1,3,4-oxadiazole-2-yl]benzene (abbreviation: OXD-7), 3-(4-biphenylyl)-4-phenyl-5-(4-tert-butylphenyl)-1,2,4-triazole (abbreviation: TAZ), 2,2',2"-(1,3,5-benzenetriyl)tris(1-phenyl-1H-benzimidazole) (abbreviation: TPBI), bathophenanthroline (abbreviation: BPhen), and bathocuproine (abbreviation: BCP);
fused aromatic compounds, such as 9-[4-(10-phenyl-9-anthryl)phenyl]-9H-carbazole (abbreviation: CzPA), 3,6-diphenyl-9-[4-(10-phenyl-9-anthryl)phenyl]-9H-carbazole (abbreviation: DPCzPA), 9,10-bis(3,5-diphenylphenyl)anthracene (abbreviation: DPPA), 9,10-di(2-naphthyl)anthracene (abbreviation: DNA), 2-tert-butyl-9,10-di(2-naphthyl)anthracene (abbreviation: t-BuDNA), 9,9'-bianthryl(abbreviation: BANT), 9,9'-(stilbene-3,3'-diyl)diphenanthrene (abbreviation: DPNS), 9,9'-(stilbene-4,4'-diyl)diphenanthrene (abbreviation: DPNS2), 3,3',3"-(benzene-1,3,5-triyl)tripyrene (abbreviation: TPB3), 9,10-diphenylanthracene (abbreviation: DPAnth), and 6,12-dimethoxy-5,11-diphenylchrysene; and
aromatic amine compounds, such as N,N-diphenyl-9-[4-(10-phenyl-9-anthryl)phenyl]-9H-carbazole-3-amine (abbreviation: CzA1PA), 4-(10-phenyl-9-anthryl)triphenylamine (abbreviation: DPhPA), N,9-diphenyl-N-[4-(10-phenyl-9-anthryl)phenyl]-9H-carbazole-3-amine (abbreviation: PCAPA), N,9-diphenyl-N-{4-[4-(10-phenyl-9-anthryl)phenyl]phenyl}-9H-carbazole-3-amine (abbreviation: PCAPBA), N-(9,10-diphenyl-2-anthryl)-N,9-diphenyl-9H-carbazole-3-amine (abbreviation: 2PCAPA), 4,4'-bis[N-(1-naphthyl)-N-phenylamino]biphenyl (abbreviation: NPB or α-NPD), N,N'-bis(3-methylphenyl)-N,N'-diphenyl-[1,1'-biphenyl]-4,4'-diamine (abbreviation: TPD), 4,4'-bis[N-(9,9-dimethylfluorene-2-yl)-N-phenylamino]biphenyl (abbreviation: DFLDPBi), and 4,4'-bis[N-(spiro-9,9'-bifluorene-2-yl)-N-phenylamino]biphenyl (abbreviation: BSPB) can be used. A plurality of host materials may be used.

In particular, in the case of a blue fluorescent device, it is preferred to use the following anthracene compounds as the host material.

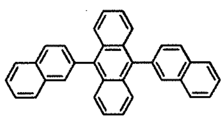 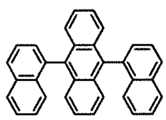 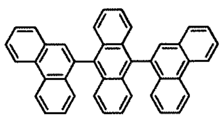 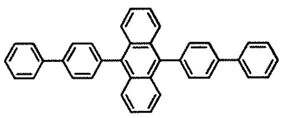

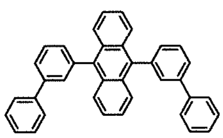 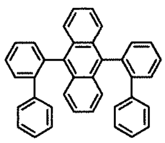 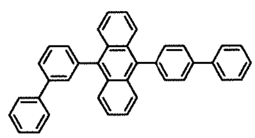 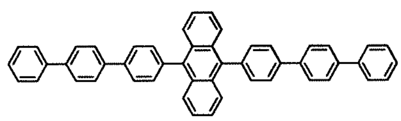

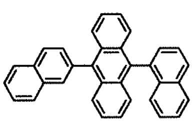 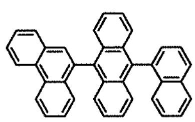 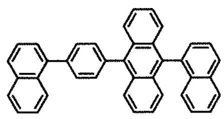 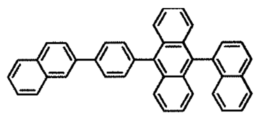

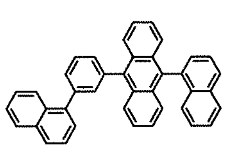 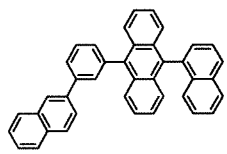 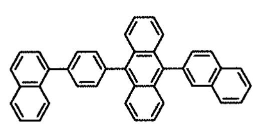 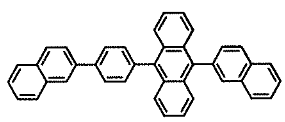

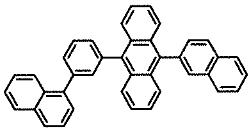
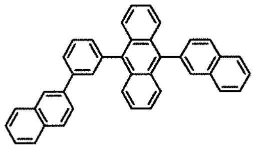
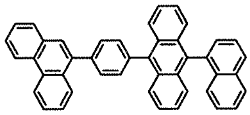
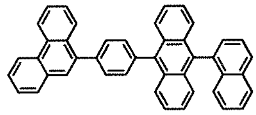
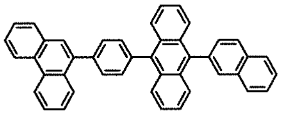
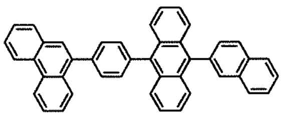
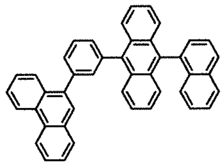
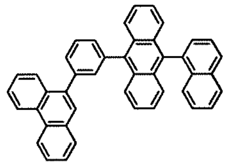
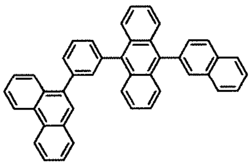
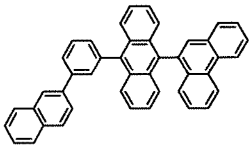
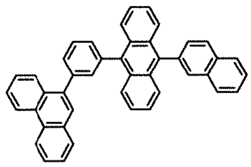
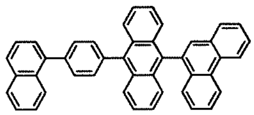
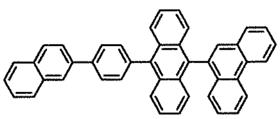
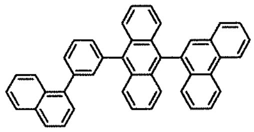
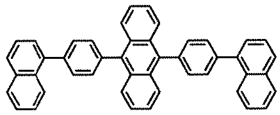
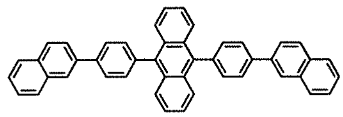
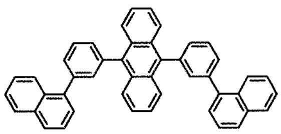
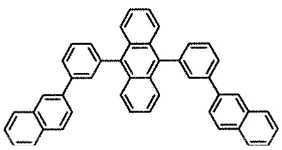
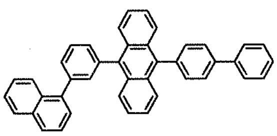
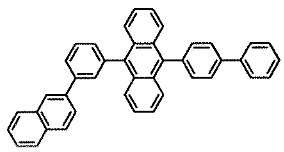
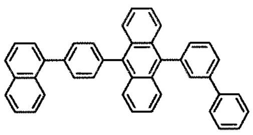
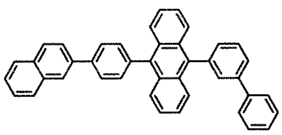
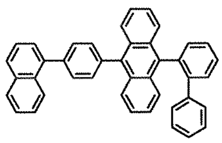
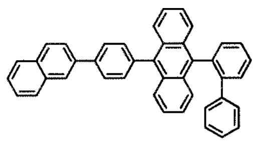
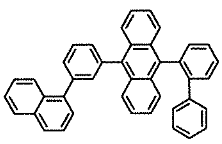
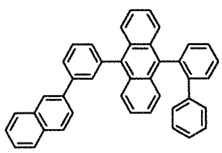
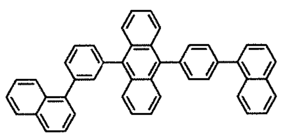
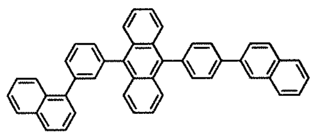
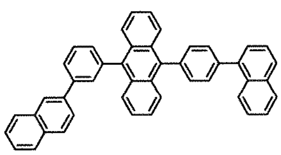
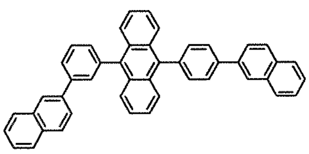
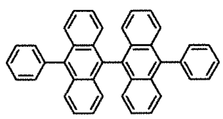
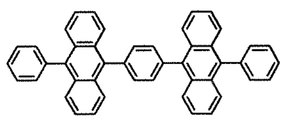
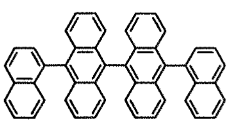
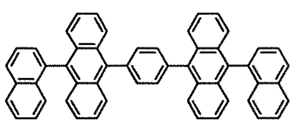
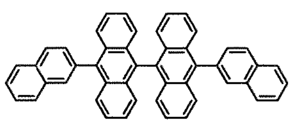
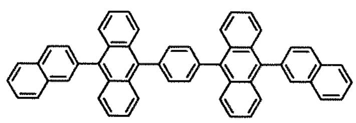

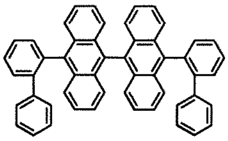 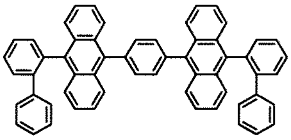 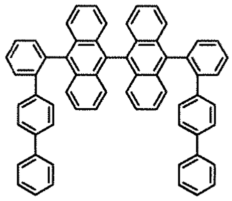 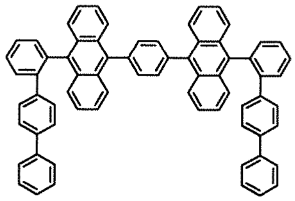

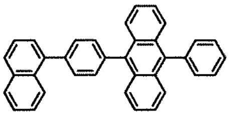 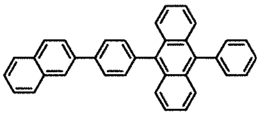 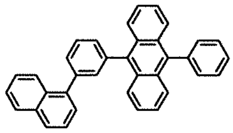 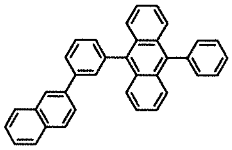

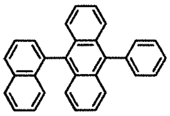 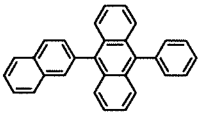 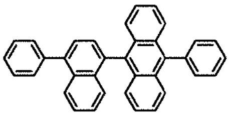 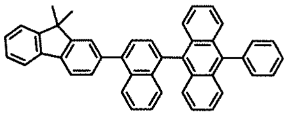

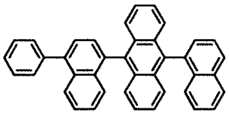 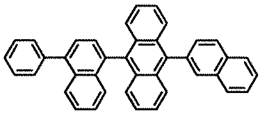 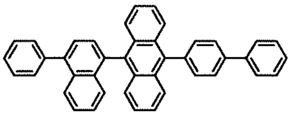 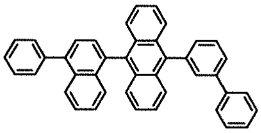

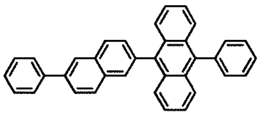 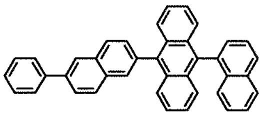 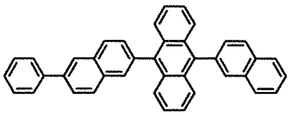 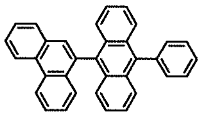

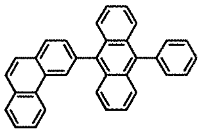 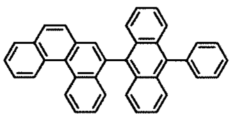 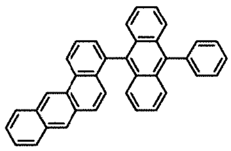 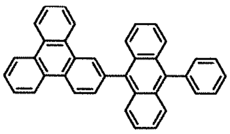

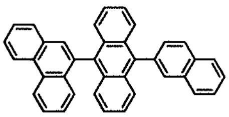 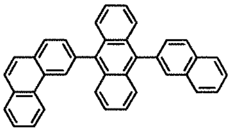 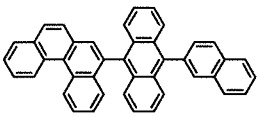 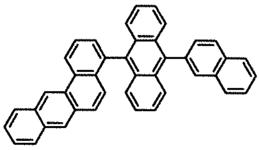

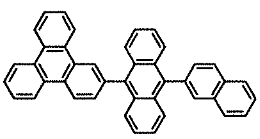 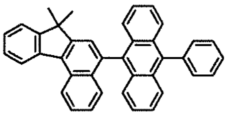 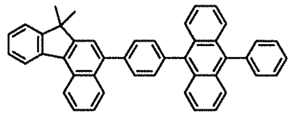 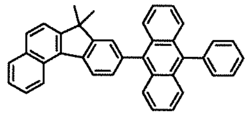

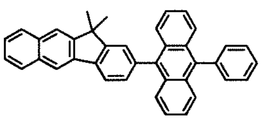 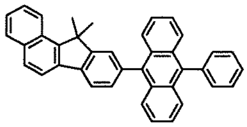 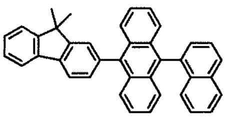 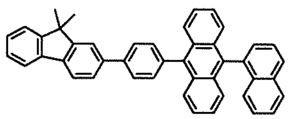

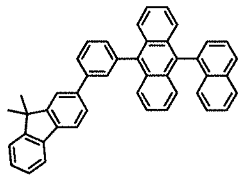
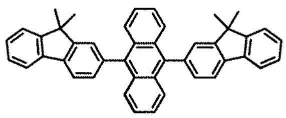
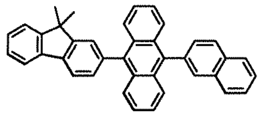
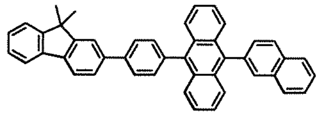

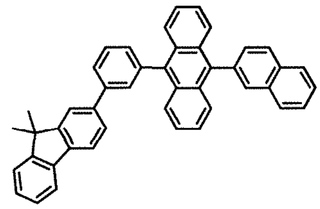

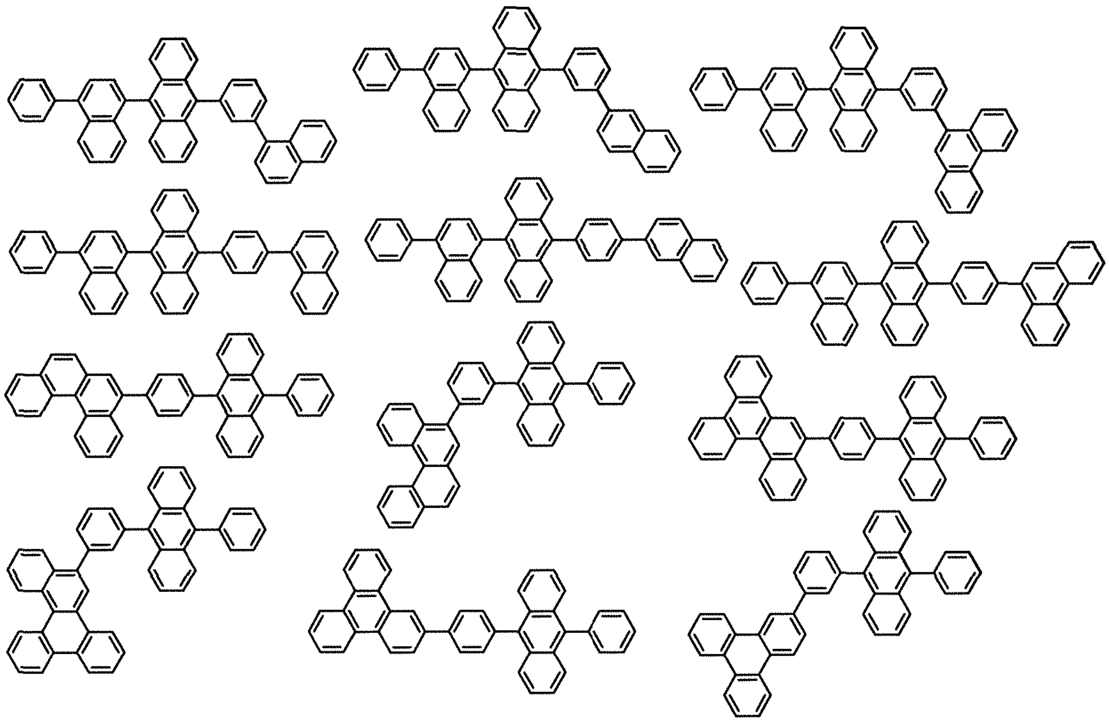

<Electron Transporting Layer>

The electron transporting layer is a layer containing a material having a high electron transporting capability (an electron transporting material) and is provided between the light emitting layer and the cathode, or between the electron injecting layer, if exists, and the light emitting layer.

The electron transporting layer may have a single layer structure or a multilayer structure including two or more layers. For example, the electron transporting layer may have a two-layer structure including a first electron transporting layer (anode side) and a second electron transporting layer (cathode side). In one embodiment of the present invention, the electron transporting layer having a single layer structure is preferably disposed adjacent to the light emitting layer, and the electron transporting layer that is closest to the anode in the multilayer structure, such as the first electron transporting layer in the two-layer structure, is preferably disposed adjacent to the light emitting layer. In another embodiment of the present invention, a hole blocking layer described later and the like may be disposed between the electron transporting layer having a single layer structure and the light emitting layer, or between the electron transporting layer that is closest to the light emitting layer in the multilayer structure and the light emitting layer.

For the electron transporting layer, for example, (1) a metal complex, such as an aluminum complex, a beryllium complex, and a zinc complex; (2) a heteroaromatic compound, such as an imidazole derivative, a benzimidazole derivative, an azine derivative, a carbazole derivative, and a phenanthroline derivative; and (3) a high-molecular weight compound can be used.

Examples of the metal complex include tris(8-quinolinolato)aluminum(III) (abbreviation: Alq), tris(4-methyl-8-quinolinolato)aluminum (abbreviation: Almq3), bis(10-hydroxybenzo[h]quinolinato)beryllium (abbreviation: $BeBq_2$), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolato]zinc(II) (abbreviation: ZnPBO), and bis[2-(2-benzothiazolyl)phenolato]zinc(II) (abbreviation: ZnBTZ).

Examples of the heteroaromatic compound include 2-(4-biphenylyl)-5-(4-tert-butylphenyl)-1,3,4-oxadiazole (abbreviation: PBD), 1,3-bis[5-(p-tert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzxazol-2-yl)stilbene (abbreviation: BzOs).

Examples of the high-molecular weight compound include poly[(9,9-dihexylfluorene-2,7-diyl)-co-(pyridine-3,5-diyl)] (abbreviation: PF-Py), and poly[(9,9-dioctylfluorene-2,7-diyl)-co-(2,2'-bipyridine-6,6'-diyl)] (abbreviation: PF-BPy).

The above-mentioned materials are materials having an electron mobility of $10^{-6}$ $cm^2/Vs$ or more. Materials other than those as mentioned above may also be used in the electron transporting layer so long as they are materials high in the electron transporting capability rather than in the hole transporting capability.

<Electron Injecting Layer>

The electron injecting layer is a layer containing a material having high electron injection capability. For the electron injecting layer, an alkali metal such as lithium (Li) and cesium (Cs), an alkaline earth metal such as magnesium (Mg), calcium (Ca), and strontium (Sr), a rare earth metal such as europium (Eu) and ytterbium (Yb), or a compound containing any of these metals can be used. Examples of the compounds include an alkali metal oxide, an alkali metal halide, an alkali metal-containing organic complex, an alkaline earth metal oxide, an alkaline earth metal halide, an alkaline earth metal-containing organic complex, a rare earth metal oxide, a rare earth metal halide, and a rare earth metal-containing organic complex. These compounds may be used as a mixture of a plurality thereof.

In addition, a material having an electron transporting capability, in which an alkali metal, an alkaline earth metal, or a compound thereof is contained, specifically Alq in which magnesium (Mg) is contained may be used. In this case, electron injection from the cathode can be more efficiently performed.

Otherwise, in the electron injecting layer, a composite material obtained by mixing an organic compound with an electron donor may be used. Such a composite material is excellent in the electron injection capability and the electron transporting capability because the organic compound receives electrons from the electron donor. In this case, the organic compound is preferably a material excellent in transporting received electrons, and specifically, examples thereof include a material constituting the aforementioned electron transporting layer (such as a metal complex and a heteroaromatic compound). As the electron donor, a material having an electron donation property for the organic compound may be used. Specifically, alkali metals, alkaline earth metals, and rare earth metals are preferred, and examples thereof include lithium, cesium, magnesium, calcium, erbium, and ytterbium. In addition, an alkali metal oxide or an alkaline earth metal oxide is preferred, and examples thereof include lithium oxide, calcium oxide, and barium oxide. In addition, a Lewis base, such as magnesium oxide, can also be used. In addition, an organic compound, such as tetrathiafulvalene (abbreviation: TTF), can also be used.

<Cathode>

It is preferred that a metal, an alloy, an electrically conductive compound, or a mixture thereof which has a low work function (specifically 3.8 eV or less) is used for the cathode. Specific examples of such a cathode material include elements belonging to group 1 or 2 of the periodic table of the elements, that is, alkali metals, such as lithium (Li) and cesium (Cs), alkaline earth metals, such as magnesium (Mg), calcium (Ca), and strontium (Sr), and alloys containing these (such as MgAg, and AlLi), and rare earth metals, such as europium (Eu), and ytterbium (Yb) and alloys containing these.

When the cathode is formed by using the alkali metals, the alkaline earth metals, and the alloys containing these, a vacuum vapor deposition method or a sputtering method can be employed. In addition, when a silver paste or the like is used, a coating method, an inkjet method, of the like can be employed.

By providing the electron injecting layer, the cathode can be formed using various conductive materials, such as Al, Ag, ITO, graphene, and indium oxide-tin oxide containing silicon or silicon oxide regardless of the magnitude of a work function. Such a conductive material can be deposited by using a sputtering method, an inkjet method, a spin coating method, or the like.

<Insulating Layer>

The organic EL device applies an electric field to an ultrathin film, and thus, pixel defects are likely to occur

due to leaks or short-circuiting. In order to prevent this, an insulating layer formed of an insulating thin film layer may be inserted between a pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. A mixture or a laminate of these may also be used.

<Space Layer>

The space layer is, for example, a layer provided between a fluorescent light emitting layer and a phosphorescent light emitting layer for the purpose of preventing excitons generated in the phosphorescent light emitting layer from diffusing into the fluorescent light emitting layer, or adjusting a carrier balance, in the case where the fluorescent light emitting layer and the phosphorescent light emitting layer are stacked. The space layer can also be provided among the plurality of phosphorescent light emitting layers.

Since the space layer is provided between the light emitting layers, a material having both an electron transporting capability and a hole transporting capability is preferred. Also, one having a triplet energy of 2.6 eV or more is preferred in order to prevent triplet energy diffusion in the adjacent phosphorescent light emitting layer. Examples of the material used for the space layer include the same as those used for the hole transporting layer as described above.

<Blocking Layer>

The blocking layer such as the electron blocking layer, the hole blocking layer, or the exciton blocking layer may be provided adjacent to the light emitting layer. The electron blocking layer is a layer that prevents electrons from leaking from the light emitting layer to the hole transporting layer, and the hole blocking layer is a layer that prevents holes from leaking from the light emitting layer to the electron transporting layer. The exciton blocking layer has a function of preventing excitons generated in the light emitting layer from diffusing into the surrounding layers, and trapping the excitons within the light emitting layer.

Each layer of the organic EL device may be formed by a conventionally known vapor deposition method, a coating method, or the like. For example, formation can be performed by a known method using a vapor deposition method such as a vacuum vapor deposition method, or a molecular beam vapor deposition method (MBE method), or a coating method using a solution of a compound for forming a layer, such as a dipping method, a spin-coating method, a casting method, a bar-coating method, and a roll-coating method.

The film thickness of each layer is not particularly limited, but is typically 5 nm to 10 μm, and more preferably 10 nm to 0.2 pm because in general, when the film thickness is too small, defects such as pinholes are likely to occur, and conversely, when the film thickness is too large, a high driving voltage is required and the efficiency decreases.

Embodiments of the organic EL device of the present invention include, for example, a first embodiment in which the second hole transporting layer contains the compound of the present invention and the first hole transporting layer does not contain the compound of the present invention; a second embodiment in which both the first hole transporting layer and the second hole transporting layer contain the compound of the present invention; and a third embodiment in which the first hole transporting layer contains the compound of the present invention and the second hole transporting layer does not contain the compound of the present invention.

<Electronic Devices>

The organic EL device can be suitably used for electronic devices, such as display components of an organic EL panel module and the like, display devices of a television, a mobile phone, a personal computer, and the like, and light emitting devices of lightings and vehicular lamps.

Examples

The following is a description of the implementation of Examples. The present invention is not limited to these Examples.

Structures of Compound Inv1 and Compound Inv7 as the inventive compound A represented by the formula (1) used in the production of the organic EL devices of Examples 1 and 2 are shown below.

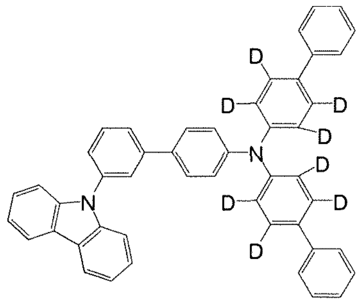

Compound Inv1

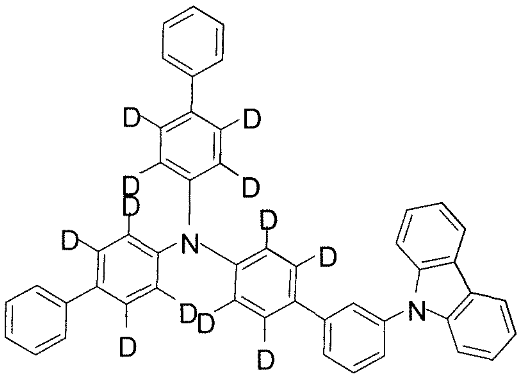

Compound Inv7

Structure of Comparative Compound Refl used in the production of the organic EL device of Comparative Example 1 is shown below.

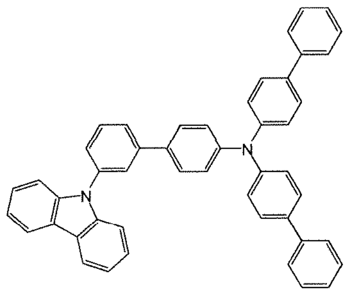

Comparative Compound Ref1

Structures of other compounds used in the production of the organic EL devices of Examples 1 and 2 and Comparative Example 1 are shown below.

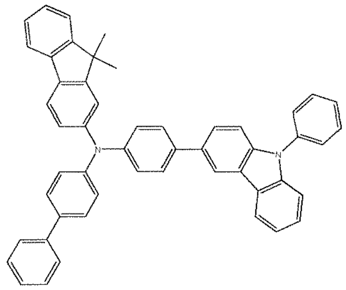

HTA

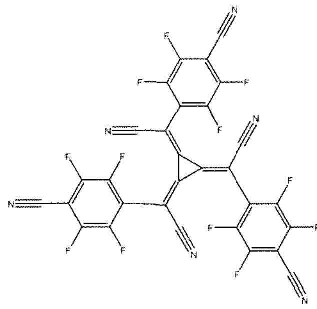

HA

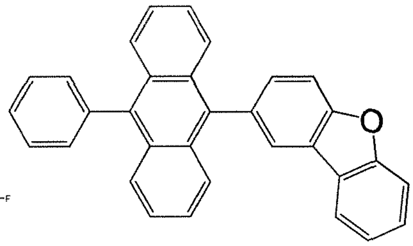

BH

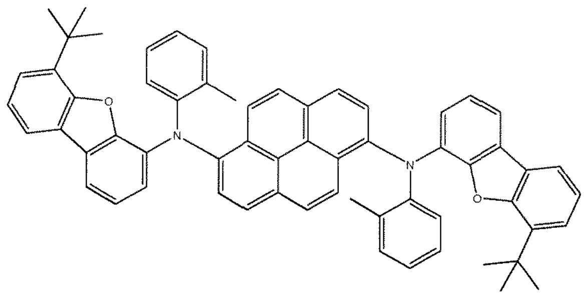

BD

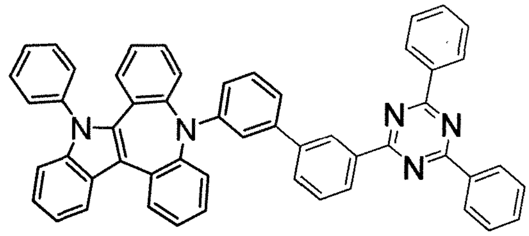

ETA

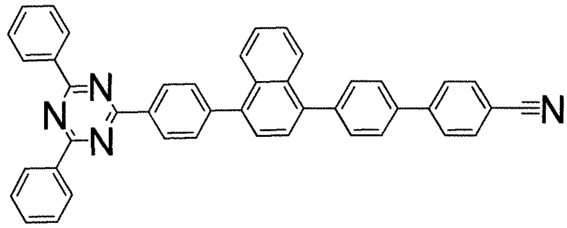

ETB

<Production of Organic EL Device>

Organic EL devices were produced and evaluated as follows.

(Example 1)

A glass substrate of 25 mm × 75 mm × 1.1 mm provided with an ITO transparent electrode (anode) (manufactured by GEOMATEC Co., Ltd.) was ultrasonically cleaned in isopropyl alcohol for 5 minutes and then subjected to UV ozone cleaning for 30 minutes. The film thickness of the ITO was 130 nm.

The cleaned glass substrate provided with the ITO transparent electrode was mounted on a substrate holder of a vacuum vapor deposition apparatus, and firstly, Compound HTA and Compound HA were vapor co-deposited on the surface having the transparent electrode formed thereon, so as to cover the transparent electrode, thereby forming a hole injecting layer with a film thickness of 10 nm. The mass ratio of Compound HTA and Compound HA (HTA:HA) was 97:3.

Subsequently, on this hole injecting layer, Compound HTA was vapor deposited to form a first hole transporting layer with a film thickness of 80 nm.

Subsequently, on this first hole transporting layer, Compound Inv1 was vapor deposited to form a second hole transporting layer with a film thickness of 10 nm.

Subsequently, on this second hole transporting layer, Compound BH (host material) and Compound BD (dopant

material) were vapor co-deposited to form a light emitting layer with a film thickness of 25 nm. The mass ratio of Compound BH and Compound BD (BH:BD) was 96:4.

Subsequently, on this light emitting layer, Compound ETA was vapor deposited to form a first electron transporting layer with a film thickness of 5 nm.

Subsequently, on this first electron transporting layer, Compound ETB and Liq were vapor co-deposited to form a second electron transporting layer with a film thickness of 20 nm. The mass ratio of Compound ETB and Liq (ETB:Liq) was 50:50.

Subsequently, on this second electron transporting layer, LiF was vapor deposited to form an electron injecting electrode with a film thickness of 1 nm.

Then, on this electron injecting electrode, metal Al was vapor deposited to form a metal cathode with a film thickness of 50 nm.

The layer configuration of the organic EL device of Example 1 thus obtained is shown below.

ITO (130) / HTA:HA = 97:3 (10) / HTA (80) / Compound Inv1 (10) / BH:BD = 96:4 (25) / ETA (5) / ETB:Liq = 50:50 (20) / LiF (1) / Al (50)

In the layer configuration, the numeral in parentheses indicates the film thickness (nm), and the ratio is a mass ratio.

[Device Lifetime (LT97) Measurement]

The resulting organic EL device was driven with direct current at a current density of 50 mA/$cm^2$, and the period of time (hour) until the luminance was reduced to 97% of the initial luminance was measured, and was defined as LT97 (97% lifetime). The results are shown in Table 1.

(Example 2)

An organic EL device was produced in the same manner as in Example 1 except that the second hole transporting layer material was changed to Compound Inv7 as shown in Table 1 below, and the LT97 was measured. The results are shown in Table 1.

(Comparative Example 1)

An organic EL device was produced in the same manner as in Example 1 except that the second hole transporting layer material was changed to Comparative Compound Refl as shown in Table 1 below, and the LT97 was measured. The results are shown in Table 1.

Table 1

| | Second hole transporting layer material | LT97 (hour) @50 mA/$cm^2$ |
|---|---|---|
| Example 1 | Compound Inv1 | 95 |
| Example 2 | Compound Inv7 | 103 |
| Comparative Example 1 | Comparative Compound Ref1 | 90 |

As is clear from the results shown in Table 1, the monoamines (Compound Inv1, Compound Inv7) satisfying the requirements of the present invention provide an organic EL device having a significantly improved device lifetime as compared with the monoamine (Comparative Compound Refl) not satisfying the requirements of the present invention.

Structures of Compound Inv1, Compound Inv3, Compound Inv7, Compound Inv9, Compound Inv10, Compound Inv11, and Compound Inv13 and structures of Compound Inv4 and Compound Inv12, as the inventive compound A represented by the formula (1) used in the production of the organic EL devices of Examples 3 to 11 are shown below.

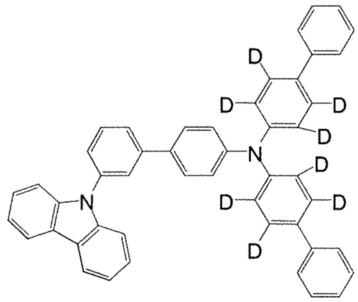

Compound Inv1

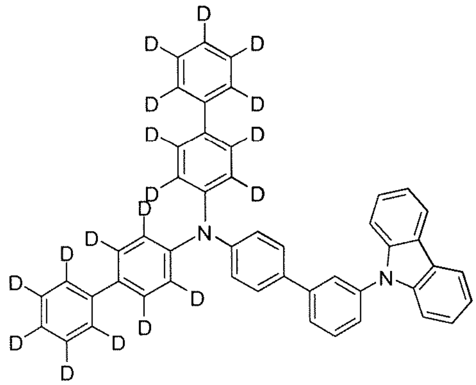

Compound Inv3

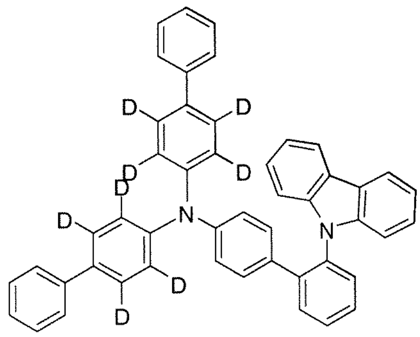

Compound Inv4

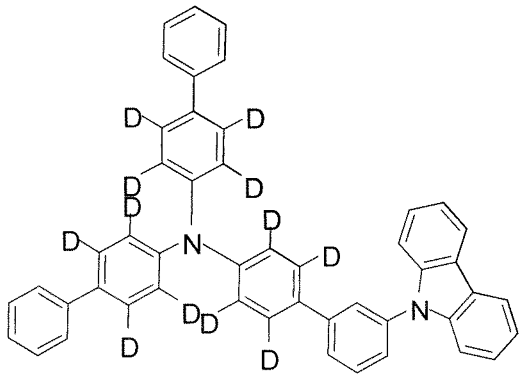

Compound Inv7

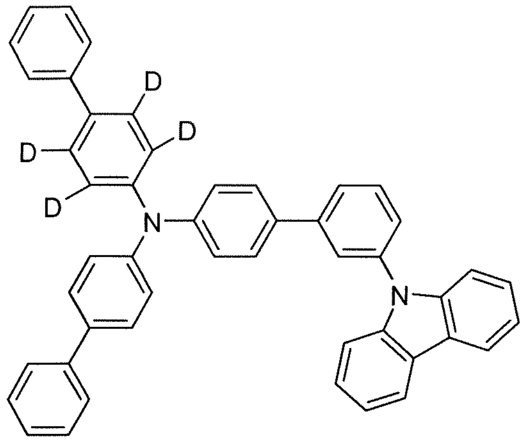

Compound Inv9

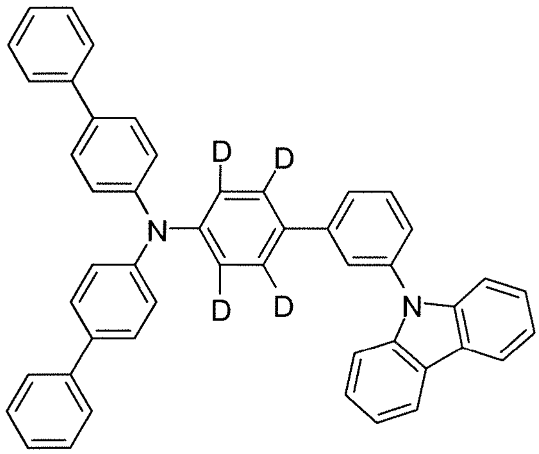

Compound Inv10

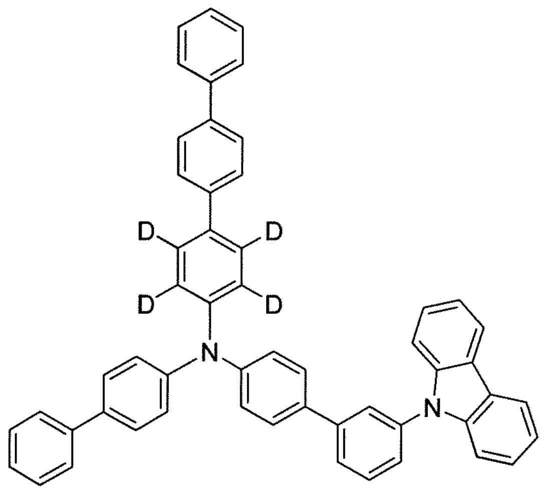

Compound Inv11

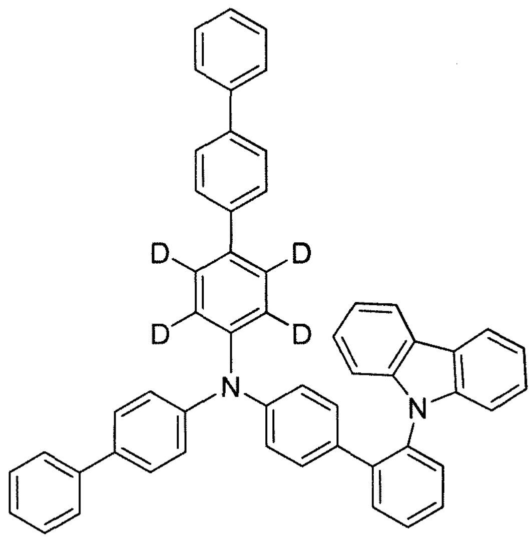

Compound Inv12

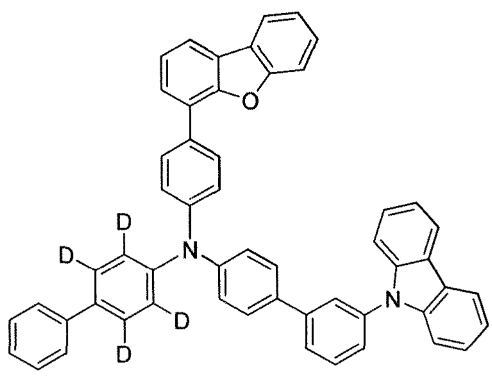

Compound Inv13

Structures of Comparative Compound Ref1 and Comparative Compound Ref2 used in the production of the

organic EL devices of Comparative Examples 2 and 3 are shown below.

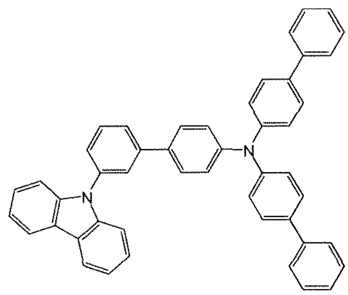

Comparative Compound Ref1

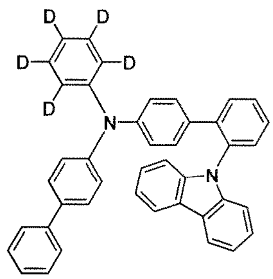

Comparative Compound Ref2

Structures of other compounds used in the production of the organic EL devices of Examples 3 to 11 and Comparative Examples 2 and 3 are shown below.

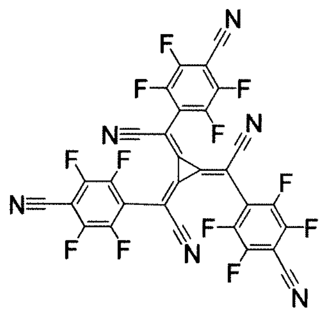

HA

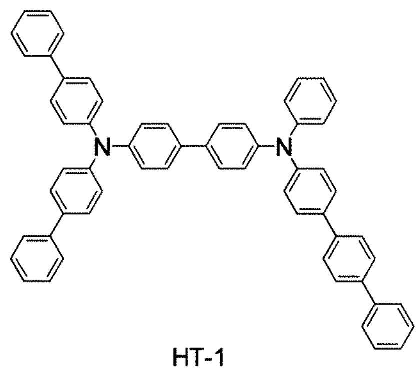

HT-1

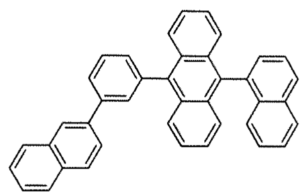

BH-1

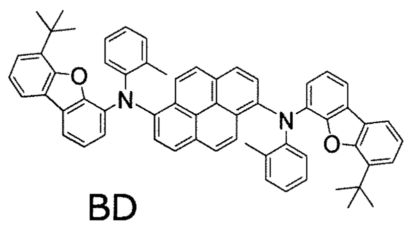

BD

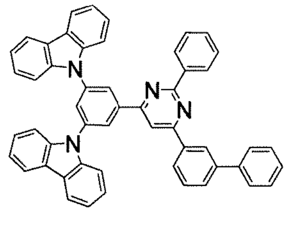

ET-1

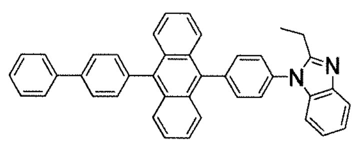

ET-2

<Production of Organic EL Device>

Organic EL devices were produced and evaluated as follows.

(Example 3)

A glass substrate of 25 mm × 75 mm × 1.1 mm provided with an ITO transparent electrode (anode) (manufactured by GEOMATEC Co., Ltd.) was ultrasonically cleaned in isopropyl alcohol for 5 minutes and then subjected to UV ozone cleaning for 30 minutes. The film thickness of the ITO was 130 nm.

The cleaned glass substrate provided with the ITO transparent electrode was mounted on a substrate holder of a vacuum vapor deposition apparatus, and firstly, Compound HT-1 and Compound HA were vapor co-deposited on the surface having the transparent electrode formed thereon, so as to cover the transparent electrode, thereby forming a hole injecting layer with a film thickness of 10 nm. The mass ratio of Compound HT-1 and Compound HA (HT-1:HA) was 97:3.

Subsequently, on this hole injecting layer, Compound HT-1 was vapor deposited to form a first hole transporting layer with a film thickness of 80 nm.

Subsequently, on this first hole transporting layer, Compound Inv1 was vapor deposited to form a second hole transporting layer with a film thickness of 10 nm.

Subsequently, on this second hole transporting layer, Compound BH-1 (host material) and Compound BD (dopant material) were vapor co-deposited to form a light emitting layer with a film thickness of 25 nm. The mass ratio of Compound BH-1 and Compound BD (BH-1:BD) was 96:4.

Subsequently, on this light emitting layer, Compound ET-1 was vapor deposited to form a first electron transporting layer with a film thickness of 10 nm.

Subsequently, on this first electron transporting layer, Compound ET-2 was vapor deposited to form a second electron transporting layer with a film thickness of 15 nm.

Subsequently, on this second electron transporting layer, LiF was vapor deposited to form an electron injecting electrode with a film thickness of 1 nm.

Then, on this electron injecting electrode, metal Al was vapor deposited to form a metal cathode with a film thickness of 50 nm.

The layer configuration of the organic EL device of Example 1 thus obtained is shown below.

ITO (130) / HT-1:HA = 97:3 (10) / HT-1 (80) / Compound Inv1 (10) / BH-1:BD = 96:4 (25) / ET-1 (10) / ET-2 (15) / LiF (1) / Al (50)

In the layer configuration, the numeral in parentheses indicates the film thickness (nm), and the ratio is a mass ratio.

[Device Lifetime (LT95) Measurement]

The resulting organic EL device was driven with direct current at a current density of 50 mA/cm$^2$, and the period of time (hour) until the luminance was reduced to 95% of the initial luminance was measured, and was defined as LT95 (95% lifetime). The results are shown in Table 1.

(Examples 4 to 11)

Each organic EL device was produced in the same manner as in Example 3 except that the second hole transporting layer material was changed to Compound Inv3, Compound Inv4, Compound Inv7, Compound Inv9, Compound Inv10, Compound Inv11, Compound Inv12, or Compound Inv13 as shown in Table 1 below, and the LT95 was measured. The results are shown in Table 2.

(Comparative Examples 2 and 3)

Each organic EL device was produced in the same manner as in Example 1 except that the second hole transporting layer material was changed to Comparative Compound Refl or Comparative Compound Ref2 as shown in Table 2 below, and the LT95 was measured. The results are shown in Table 2.

Table 2

| | Second hole transporting layer material | LT95 (hour) @50 mA/cm$^2$ |
|---|---|---|
| Example 3 | Compound Inv1 | 156 |

(continued)

| | Second hole transporting layer material | LT95 (hour) @50 mA/cm$^2$ |
|---|---|---|
| Example 4 | Compound Inv3 | 160 |
| Example 5 | Compound Inv4 | 157 |
| Example 6 | Compound Inv7 | 164 |
| Example 7 | Compound Inv9 | 153 |
| Example 8 | Compound Inv10 | 155 |
| Example 9 | Compound Inv11 | 162 |
| Example 10 | Compound Inv12 | 157 |
| Example 11 | Compound Inv13 | 155 |
| Comparative Example 2 | Comparative Compound Ref1 | 139 |
| Comparative Example 3 | Comparative Compound Ref2 | 130 |

As is clear from the results shown in Table 2, the monoamines (Compound Inv1, Compound Inv3, Compound Inv4, Compound Inv7, Compound Inv9, Compound Inv10, Compound Inv11, Compound Inv12, Compound Inv13) satisfying the requirements of the present invention provide an organic EL device having a significantly improved device lifetime as compared with the monoamines (Comparative Compound Refl, Comparative Compound Ref2) not satisfying the requirements of the present invention.

<Synthesis of Compounds>

Compounds Inv1 to Inv15 synthesized in Synthesis Examples 1 to 8 are shown below.

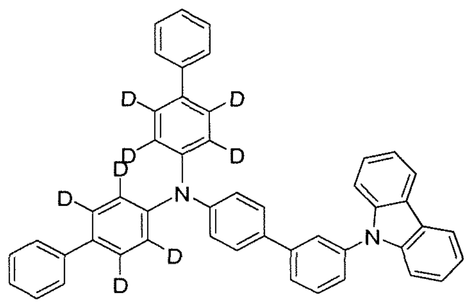

Compound Inv1

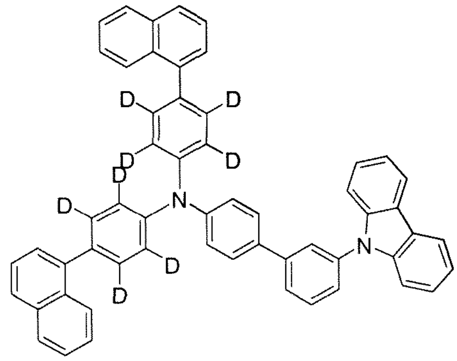

Compound Inv2

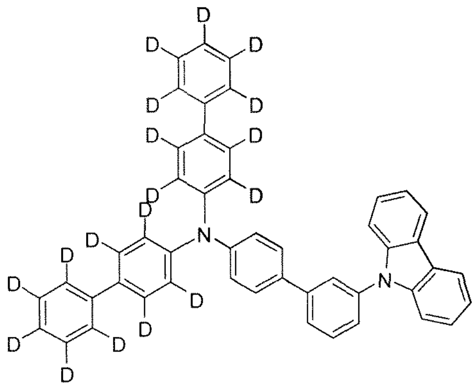

Compound Inv3

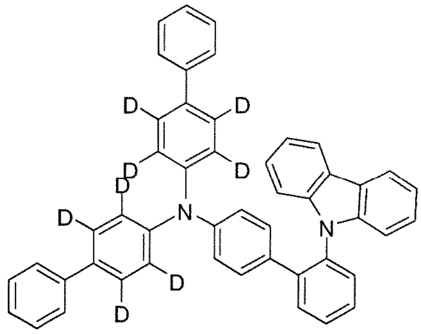

Compound Inv4

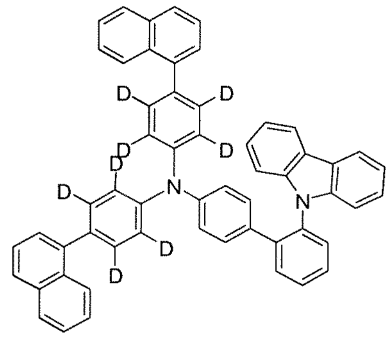

Compound Inv5

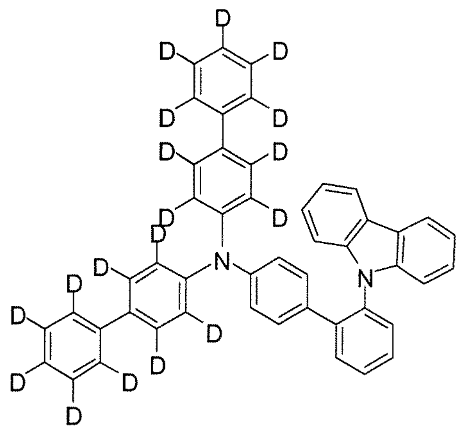

Compound Inv6

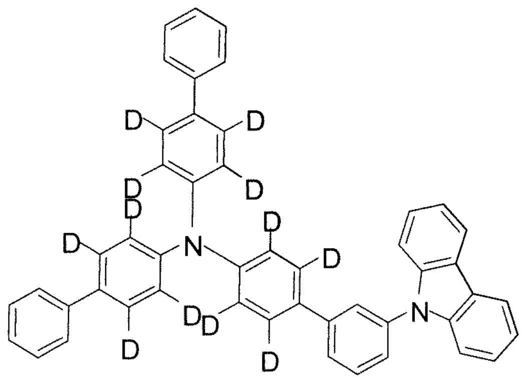

Compound Inv7

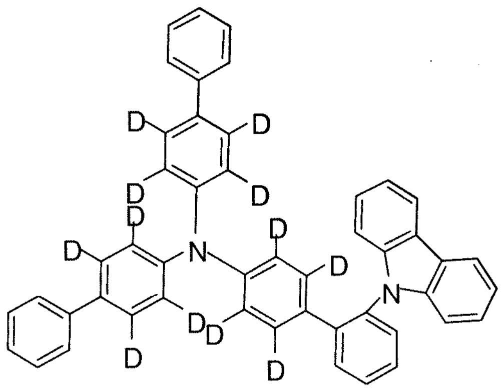

Compound Inv8

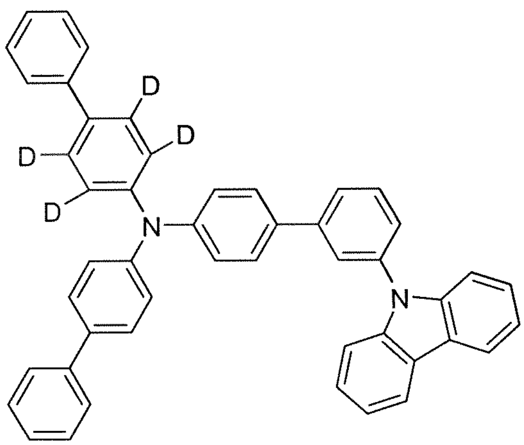

Compound Inv9

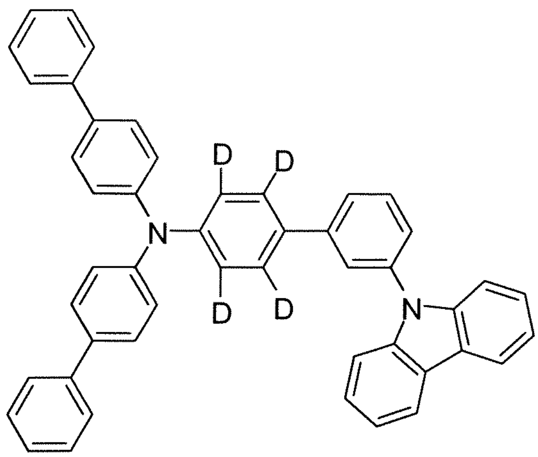

Compound Inv10

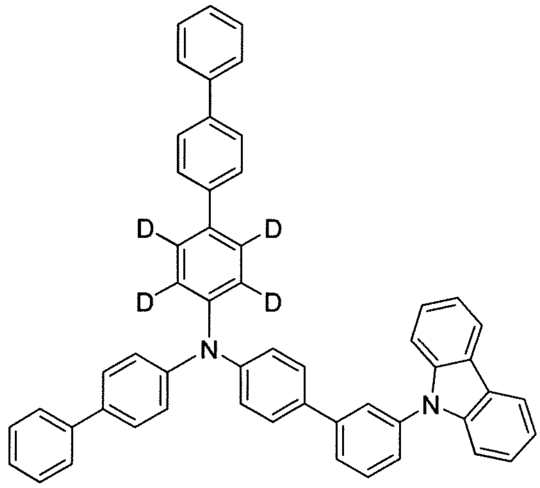

Compound Inv11

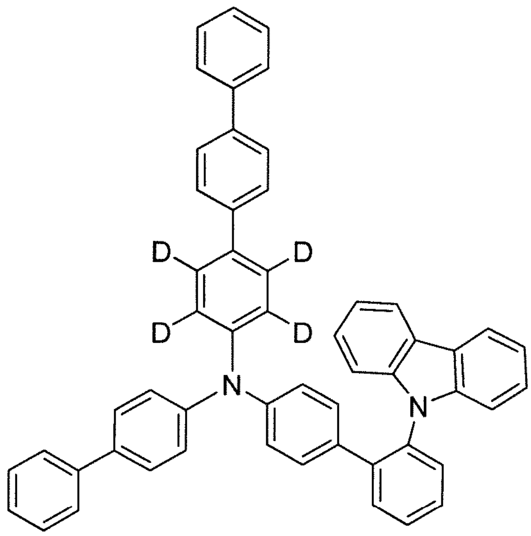

Compound Inv12

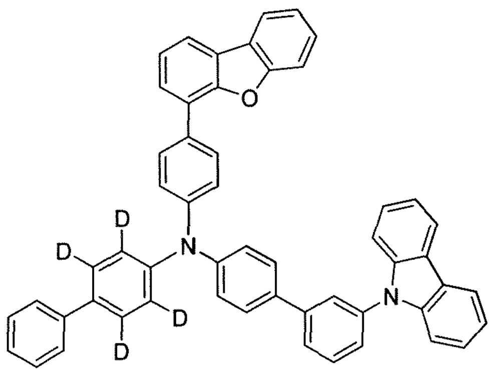

Compound Inv13

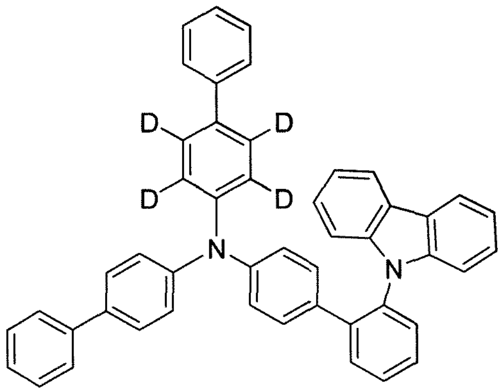

Compound Inv14

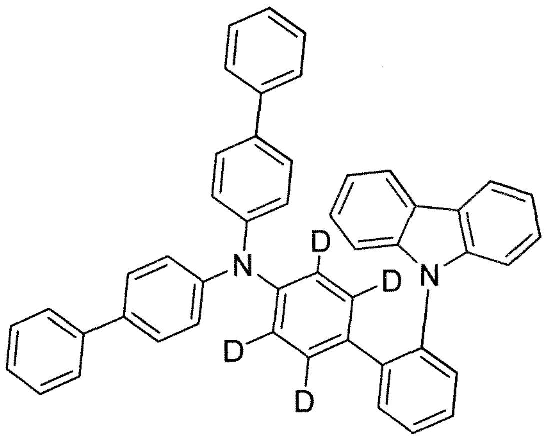

Compound Inv15

(Intermediate Synthesis Example 1: Synthesis of Intermediate A)

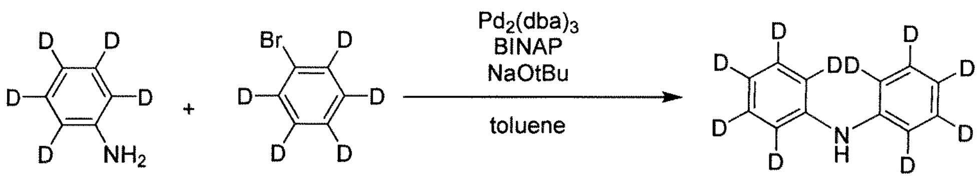


Intermediate A

Under an argon atmosphere, aniline-2,3,4,5,6-d5 (2.19 g, 22.33 mmol), bromobenzene-d5 (3.29 g, 20.3 mmol), tris(dibenzylideneacetone)dipalladium(0) (372 mg, 0.41 mmol), BINAP (506 mg, 0.812 mmol), sodium-t-butoxide (2.15 g, 22.33 mmol), and toluene (200 mL) were added, and the mixture was heated and stirred at 100°C for 3 hours. After allowing to cool, the mixture was filtered, and the resulting residue was purified by column chromatography to obtain Intermediate A (3.59 g). The yield was 99%.

(Intermediate Synthesis Example 2: Synthesis of Intermediate C)

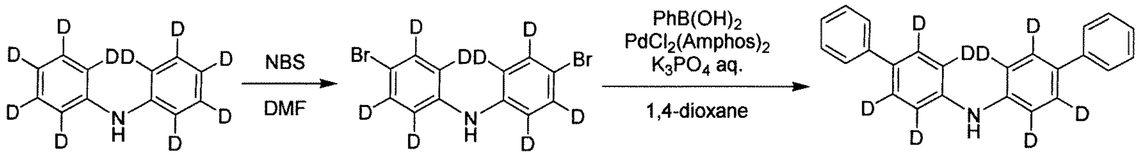


Intermediate A Intermediate B Intermediate C

Under an argon atmosphere, Intermediate A (2.9 g, 16.18 mmol) and DMF (55 mL) were mixed, and N-bromosuccinimide (5.76 g, 32.4 mmol) was added thereto at 0°C. Water and ethyl acetate were added for extraction, and the obtained organic layer was distilled off under reduced pressure to obtain Intermediate B. Intermediate B was subjected to the next reaction without purification.

Under an argon atmosphere, Intermediate B (6.41 g, 19.12 mmol), phenylboronic acid (5.83 g, 47.8 mmol), bis(di-t-butyl(4-dimethylaminophenyl)phosphine)dichloropalladium(II) (406 mg, 0.574 mmol), and 1,4-dioxane (100 mL) were mixed, and a potassium phosphate aqueous solution was added thereto. After heating and stirring at 110°C for 5 hours and allowing to cool, the mixture was filtered and purified by column chromatography and recrystallization to obtain Intermediate C (3.9 g). The yield was 62% (2 steps).

(Synthesis Example 1: Synthesis of Compound Inv1)

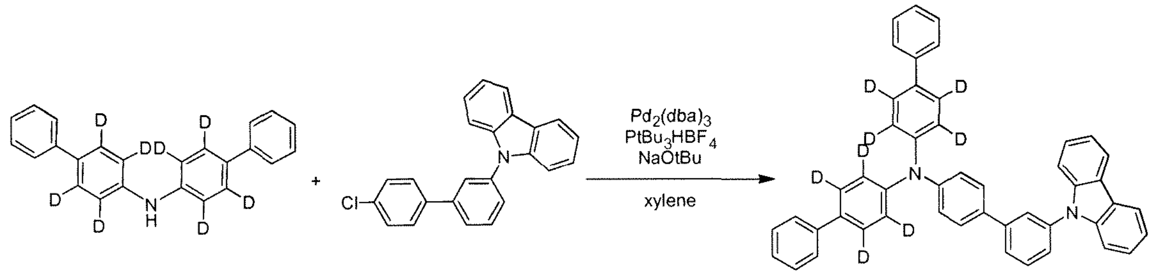


Intermediate C Intermediate D Compound Inv1

Under an argon atmosphere, Intermediate C (2.9 g, 8.8 mmol), Intermediate D (2.83 g, 8.0 mmol) synthesized by a method known in the literature, tris(dibenzylideneacetone)dipalladium(0) (147 mg, 0.16 mmol), sodium-t-butoxide (1.07 g, 11.2 mmol), and xylene (50 mL) were added, and the mixture was heated and stirred at 140°C for 1 hour. After allowing to cool, the mixture was filtered and purified by column chromatography to synthesize Compound Inv1 (3.6 g). The yield was 70%. As a result of mass spectral analysis, the obtained product was found to be Compound Inv1, and had a molecular weight of 646.86 and m/e = 646.

(Synthesis Example 2: Synthesis of Compound Inv2)

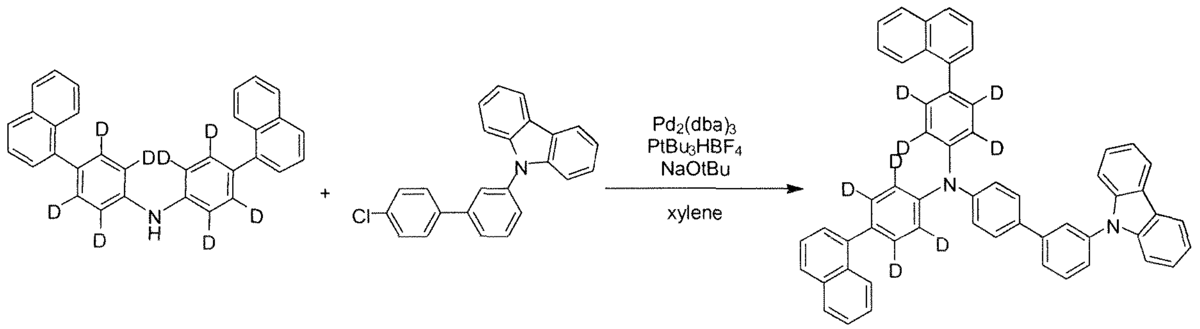


Intermediate E Intermediate D Compound Inv2

Intermediate E was obtained in the same manner as in the synthesis of Intermediate C, except that 1-naphthaleneboronic acid was used instead of phenylboronic acid. Further, Compound Inv2 was synthesized in the same manner as in the synthesis of Compound Inv1, except that Intermediate E was used instead of Intermediate C. As a result of mass spectral analysis, the obtained product was found to be Compound Inv2, and had a molecular weight of 746.98 and m/e = 746.

(Synthesis Example 3: Synthesis of Compound Inv3)

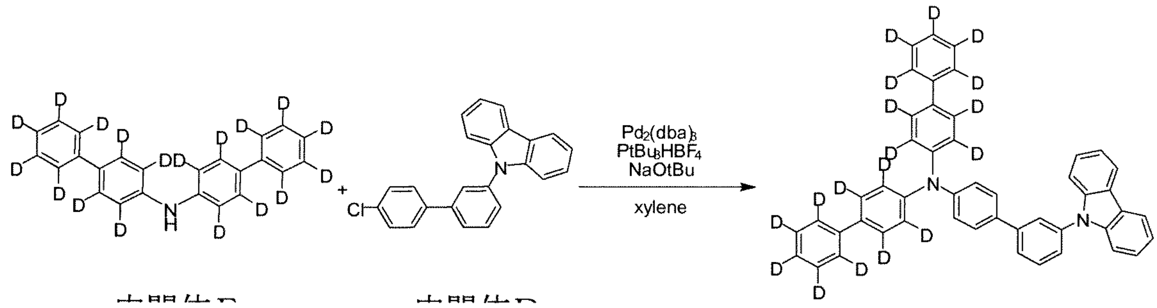


Intermediate F Intermediate D Compound Inv3

Intermediate F was obtained in the same manner as in the synthesis of Intermediate C, except that phenylboronic acid-d5 was used instead of phenylboronic acid. Further, Compound Inv3 was synthesized in the same manner as in the synthesis of Compound Inv1, except that Intermediate F was used instead of Intermediate C. As a result of mass spectral analysis, the obtained product was found to be Compound Inv3, and had a molecular weight of 656.92 and m/e = 656.

(Synthesis Example 4: Synthesis of Compound Inv4)

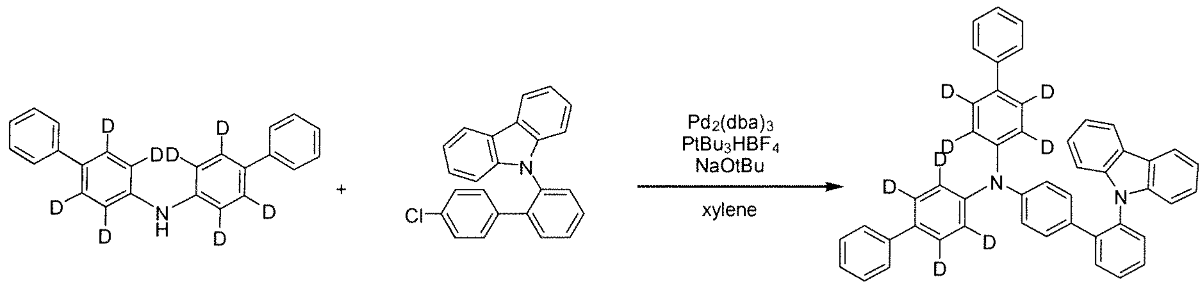


Intermediate C Intermediate G Compound Inv4

Compound Inv4 was synthesized in the same manner as in the synthesis of Compound Inv1, except that Intermediate G synthesized by a method known in the literature was used instead of Intermediate D. As a result of mass spectral analysis, the obtained product was found to be Compound Inv4, and had a molecular weight of 646.86 and m/e = 646.

(Synthesis Example 5: Synthesis of Compound Inv5)

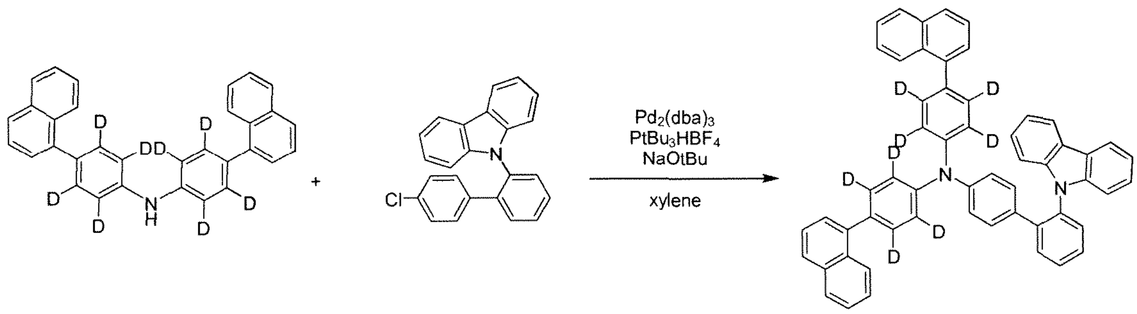


Intermediate E Intermediate G Compound Inv5

Compound Inv5 was synthesized in the same manner as in the synthesis of Compound Inv2, except that Intermediate G was used instead of Intermediate D. As a result of mass spectral analysis, the obtained product was found to be Compound Inv5, and had a molecular weight of 746.98 and m/e = 746.

(Synthesis Example 6: Synthesis of Compound Inv6)

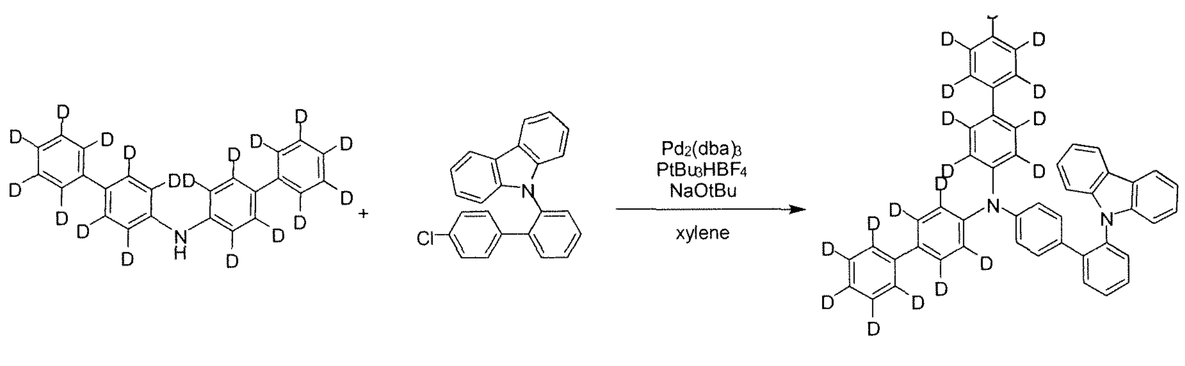


Intermediate F Intermediate G Compound Inv6

Compound Inv6 was synthesized in the same manner as in the synthesis of Compound Inv3, except that Intermediate G was used instead of Intermediate D. As a result of mass spectral analysis, the obtained product was found to be Compound Inv6, and had a molecular weight of 656.92 and m/e = 656.

(Intermediate Synthesis Example 3: Synthesis of Intermediate I)

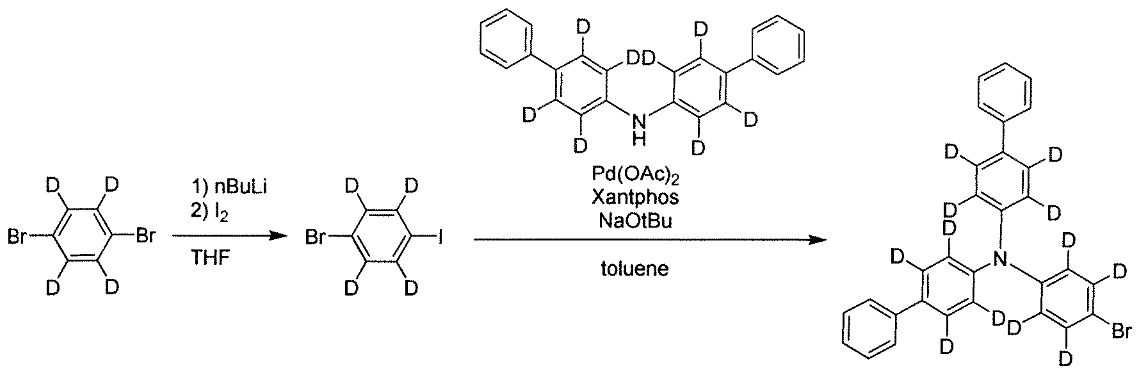


Intermediate I

Under an argon atmosphere, 1,4-dibromobenzene-2,3,5,6-d4 (2.5 g, 10.42 mmol) and THF (105 mL) were mixed, and n-butyllithium (1.59 M, 6.55 mL) was added dropwise thereto at -78°C. Thereafter, a mixture of iodine (3.97 g, 15.63 mmol) and THF (25 mL) was added dropwise thereto at -78°C, followed by stirring for 30 minutes. Thereafter, water and sodium thiosulfate aqueous solution were added, the temperature was raised to room temperature, extraction was carried out with dichloromethane, and the obtained organic layer was distilled off under reduced pressure to obtain 1-bromo-4-iodobenzene-2,3,5,6-d4.

Under an argon atmosphere, Intermediate C (4.12 g, 12.5 mmol), 1-bromo-4-iodobenzene-2,3,5,6-d4 (4.3,g, 15.0,mmol), palladium (II) acetate (56 mg, 0.25 mmol), 4,5-bis(diphenylphosphino)-9,9-dimethylxanthene (145 mg, 0.25 mmol), sodium-t-butoxide (1.68 g, 17.5 mmol), and toluene (120 mL) were mixed, and the mixture was heated and stirred at 100°C for 3 hours. After allowing to cool, the mixture was filtered, and the resulting residue was suspended and washed with methanol to obtain Intermediate I. The yield was 98% (2 steps).

(Intermediate Synthesis Example 4: Synthesis of Intermediate J)

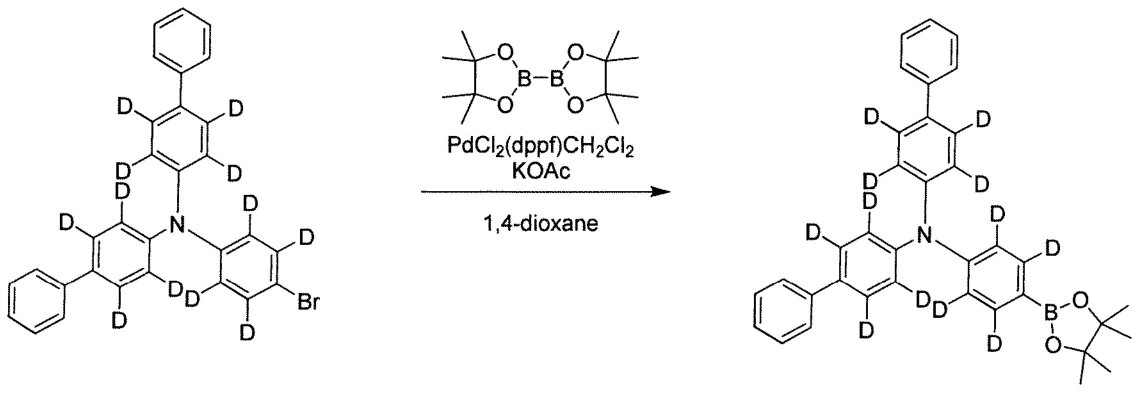


Intermediate I

Intermediate J

Under an argon atmosphere, Intermediate I (4.5 g, 9.21 mmol), bis(pinacolato)diboron (2.81 g, 11.05 mmol), [1,1'-bis(diphenylphosphino)ferrocene] palladium (II) dichloride dichloromethane adduct (226 mg, 0.28 mmol), potassium

acetate (2.71 g, 27.6 mmol), and 1,4-dioxane (47 mL) were mixed, and the mixture was heated and stirred at 110°C for 4 hours. After allowing to cool, water and ethyl acetate were added and extracted. The obtained organic layer was distilled off under reduced pressure to obtain Intermediate J.

(Synthesis Example 7: Synthesis of Compound Inv7)

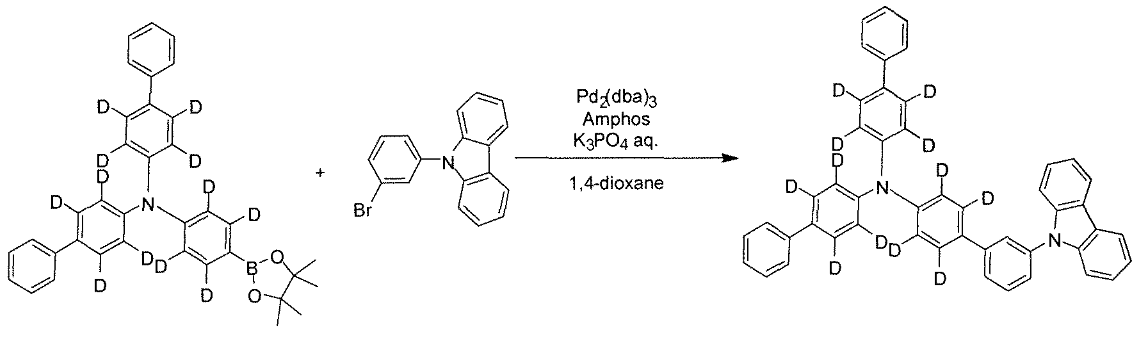


Intermediate J    Compound Inv7

Under an argon atmosphere, Intermediate J (4.93 g, 9.21 mmol), 9-(3-bromophenyl)carbazole (4.45 g, 13.82 mmol), tris(dibenzylideneacetone)dipalladium(0) (169 mg, 0.18 mmol), [4-(N,N-dimethylamino)phenyl]di-t-butylphosphine (196 mg, 0.74 mmol), potassium phosphate (5.87 g, 27.6 mmol), 1,4-dioxane. (75 mL), and water (15 mL) were mixed, and the mixture was heated and stirred at 110°C for 4 hours. After allowing to cool, the mixture was extracted with toluene, and the obtained organic layer was distilled off under reduced pressure. The residue was purified by column chromatography and recrystallization to synthesize Compound Inv7. The yield was 40% (2 steps). As a result of mass spectral analysis, the obtained product was found to be Compound Inv7, and had a molecular weight of 650.89 and m/e = 650.

(Synthesis Example 8: Synthesis of Compound Inv8)

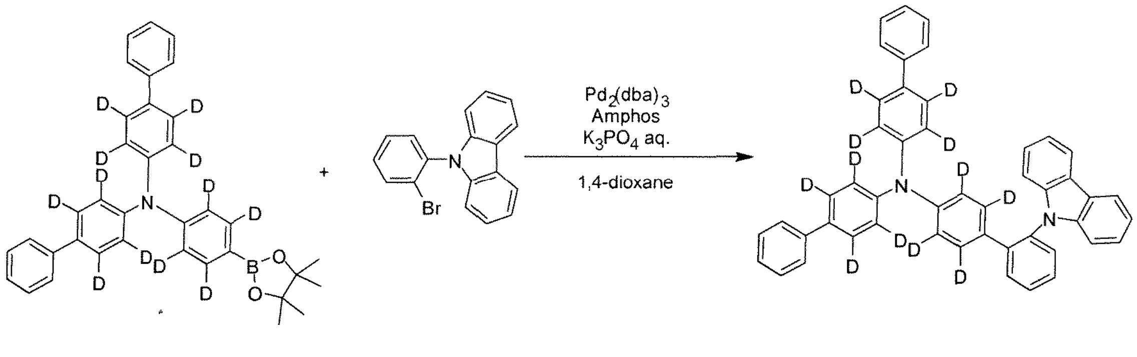


Intermediate J    Compound Inv8

Compound Inv8 was synthesized in the same manner as in the synthesis of Compound Inv7, except that 9-(2-bromophenyl)carbazole was used instead of 9-(3-bromophenyl)carbazole. As a result of mass spectral analysis, the obtained product was found to be Compound Inv8, and had a molecular weight of 650.89 and m/e = 650.

(Synthesis Example 9: Synthesis of Compound Inv9)

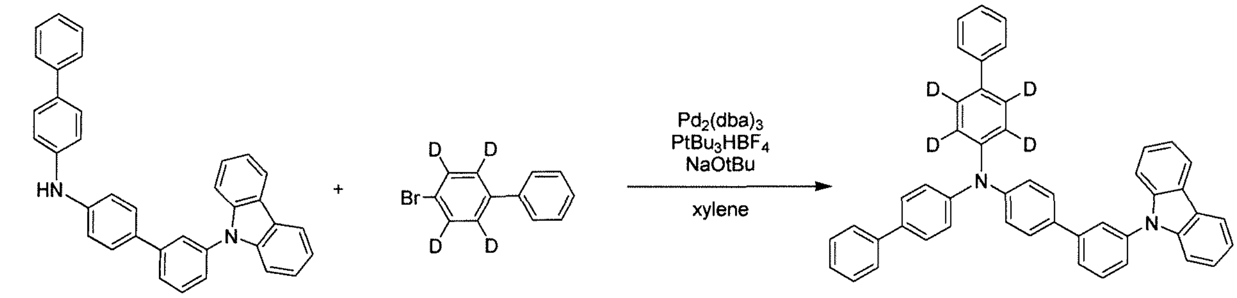


Intermediate K Intermediate L Compound Inv9

Compound Inv9 was synthesized in the same manner as in the synthesis of Compound Inv1, except that Intermediate K was used instead of Intermediate C and Intermediate L was used instead of Intermediate D in the synthesis of Compound Inv1. As a result of mass spectral analysis, the obtained product was found to be Compound Inv9, and had a molecular weight of 642.84 and m/e = 642.

(Intermediate Synthesis Example 5: Synthesis of Intermediate O)

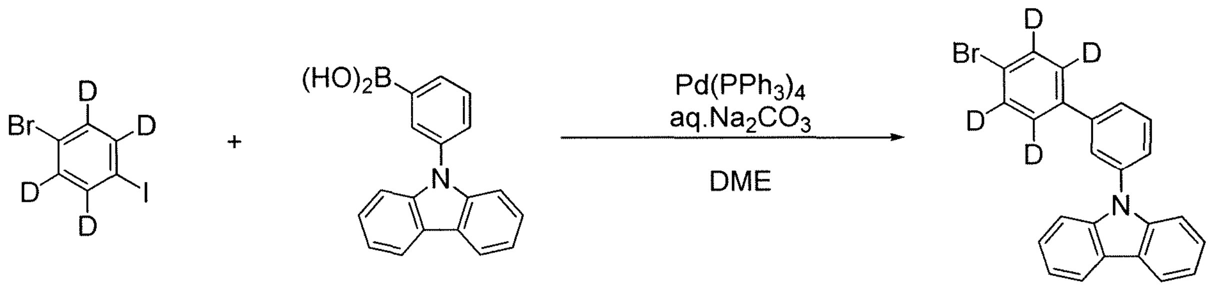


Intermediate O

Under an argon atmosphere, a mixture of 1,4-dibromobenzene-2,3,5,6-d4 (2.5 g, 10.4 mmol), (3-(9H-carbazol-9-yl)phenyl)boronic acid (3.59 g, 12.5 mmol), tetrakis(triphenylphosphine)palladium(0) (0.358 g, 0.31 mmol), 2M sodium carbonate aqueous solution (10.4 mL, 20.8 mmol), and DME (50 mL) was heated and stirred at 70°C for 24 hours. The reaction solution was cooled to room temperature, concentrated under reduced pressure, and the resulting residue was purified by silica gel column chromatography to obtain a white solid of 3.18 g. The yield was 76%.

(Synthesis Example 10: Synthesis of Compound Inv10)

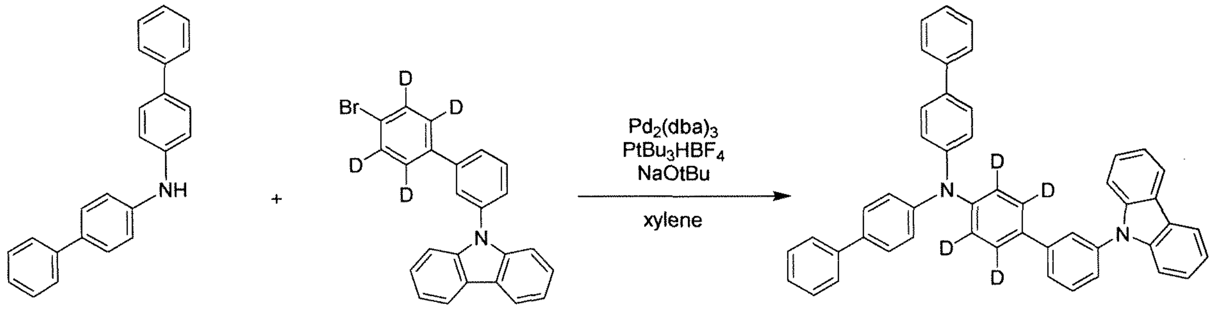


Intermediate O Compound Inv10

Compound Inv10 was synthesized in the same manner as in the synthesis of Compound Inv1, except that N-[1,1'-biphenyl]-4-yl-[1,1'-biphenyl]-4-amine was used instead of Intermediate C and Intermediate O was used instead of Intermediate D in the synthesis of Compound Inv1. As a result of mass spectral analysis, the obtained product was found to be Compound Inv10, and had a molecular weight of 642.84 and m/e = 642.

(Intermediate Synthesis Example 6: Synthesis of Intermediate Q)

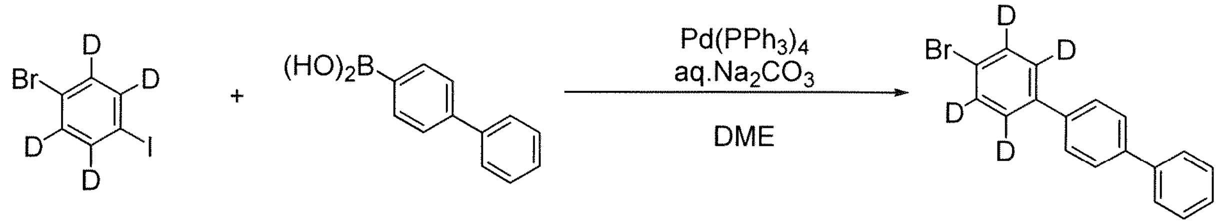


Intermediate Q

Intermediate Q was obtained in the same manner as in the synthesis of Intermediate O, except that 4-biphenylboronic acid was used instead of (3-(9H-carbazol-9-yl)phenyl)boronic acid in the synthesis of Intermediate O.

(Synthesis Example 11: Synthesis of Compound Inv11)

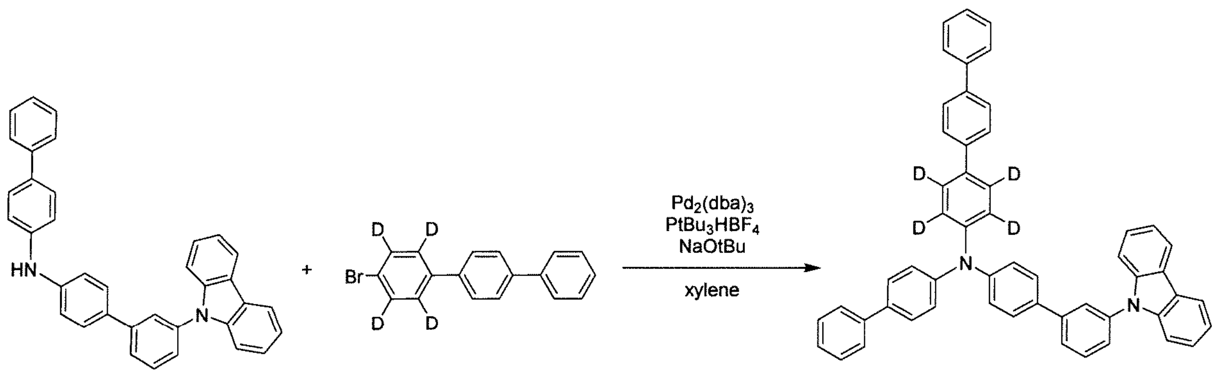


Intermediate K Intermediate Q Compound Inv11

Compound Inv11 was synthesized in the same manner as in the synthesis of Compound 1, except that Intermediate K was used instead of Intermediate C and Intermediate Q was used instead of Intermediate D in the synthesis of Compound 1. As a result of mass spectral analysis, the obtained product was found to be Compound Inv11, and had a molecular weight of 718.94 and m/e = 718.

(Synthesis Example 12: Synthesis of Compound Inv12)

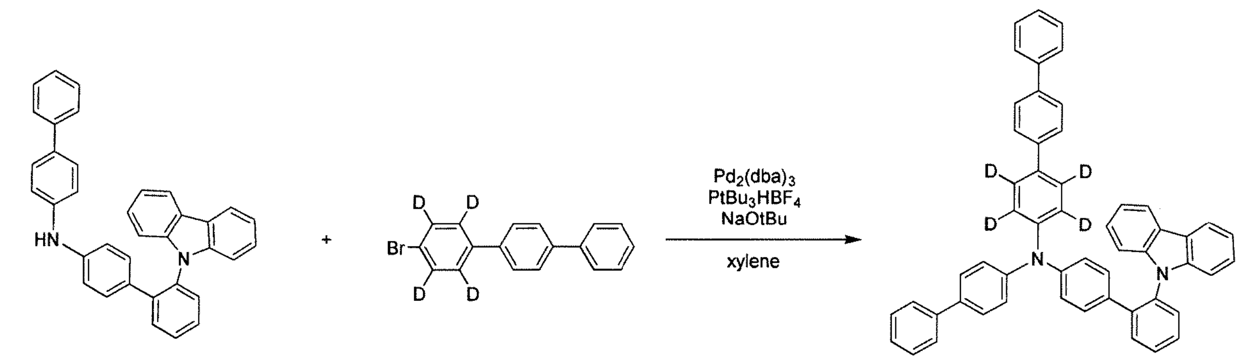


Intermediate M Intermediate Q Compound Inv12

Compound Inv12 was synthesized in the same manner as in the synthesis of Compound 1, except that Intermediate M was used instead of Intermediate C and Intermediate Q was used instead of Intermediate D in the synthesis of Compound 1. As a result of mass spectral analysis, the obtained product was found to be Compound Inv12, and had a molecular weight of 718.94 and m/e = 718.

(Synthesis Example 13: Synthesis of Compound Inv13)

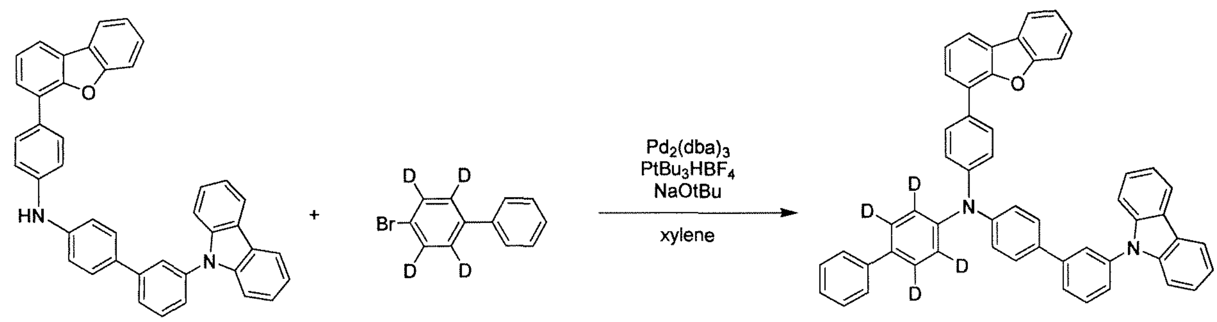


Intermediate R Intermediate L Compound Inv13

Compound Inv13 was synthesized in the same manner as in the synthesis of Compound 1, except that Intermediate R was used instead of Intermediate C and Intermediate L was used instead of Intermediate D in the synthesis of Compound 1. As a result of mass spectral analysis, the obtained product was found to be Compound Inv13, and had a molecular weight of 732.92 and m/e = 732.

(Synthesis Example 14: Synthesis of Compound Inv14)

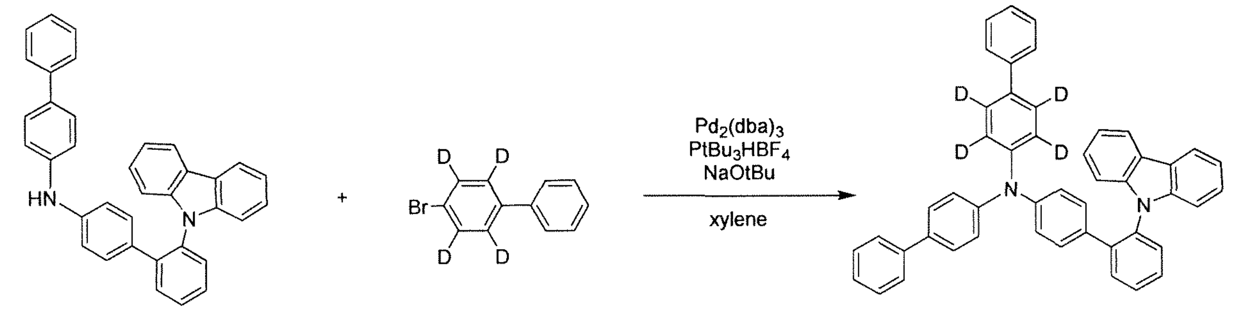


Intermediate M Intermediate L Compound Inv14

Compound Inv14 was synthesized in the same manner as in the synthesis of Compound 1, except that Intermediate M was used instead of Intermediate C and Intermediate L was used instead of Intermediate D in the synthesis of Compound 1. As a result of mass spectral analysis, the obtained product was found to be Compound Inv14, and had a molecular weight of 642.84 and m/e = 642.

(Intermediate Synthesis Example 7: Synthesis of Intermediate P)

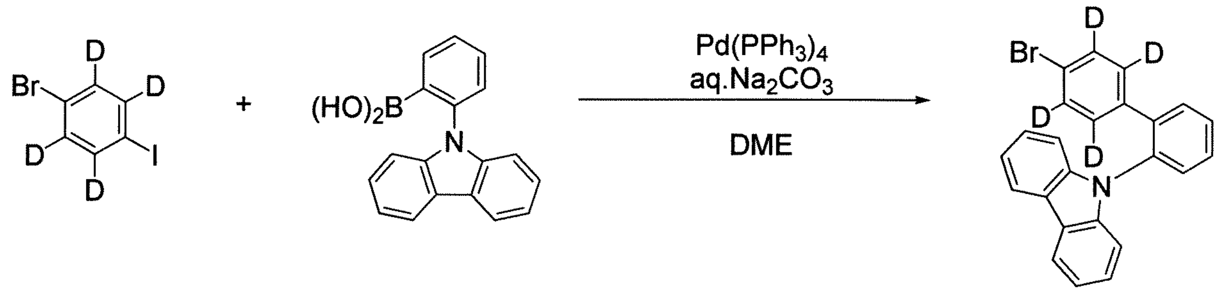


Intermediate P

Intermediate P was obtained in the same manner as in the synthesis of Intermediate O, except that (2-(9H-carbazol-9-yl)phenyl)boronic acid was used instead of (3-(9H-carbazol-9-yl)phenyl)boronic acid in the synthesis of Intermediate O.

(Synthesis Example 15: Synthesis of Compound Inv15)

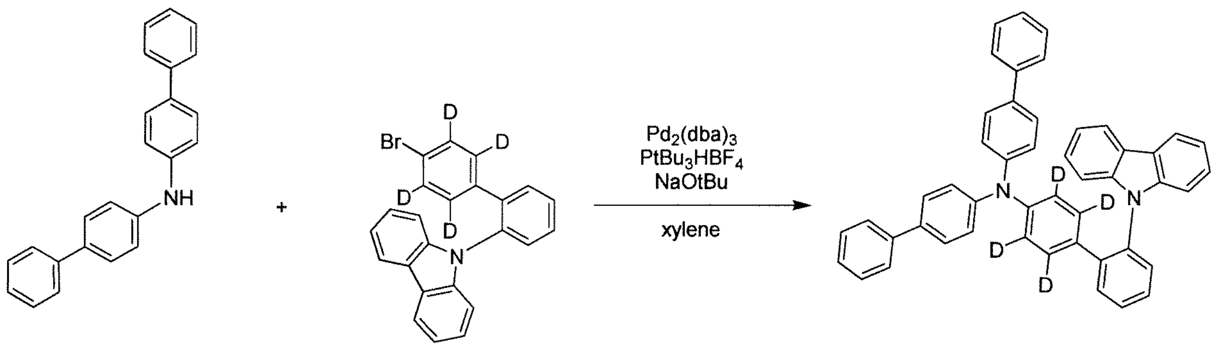


Intermediate P

Compound Inv15

Compound Inv15 was synthesized in the same manner as in the synthesis of Compound 1, except that N-[1,1'-biphenyl]-4-yl-[1,1'-biphenyl]-4-amine was used instead of Intermediate C and Intermediate P was used instead of Intermediate D in the synthesis of Compound 1. As a result of mass spectral analysis, the obtained product was found to be Compound Inv15, and had a molecular weight of 642.84 and m/e = 642.

Reference Signs List

- 1, 11: Organic EL device
- 2: Substrate
- 3: Anode
- 4: Cathode
- 5: Light emitting layer
- 6: Hole transporting zone (hole transporting layer)
- 6a: Hole injecting layer
- 6b: First hole transporting layer
- 6c: Second hole transporting layer
- 7: Electron transporting zone (electron transporting layer)
- 7a: First electron transporting layer
- 7b: Second electron transporting layer
- 10, 20: Light emitting unit

## Claims

**1.** A compound represented by the following formula (1), which has at least one deuterium atom:

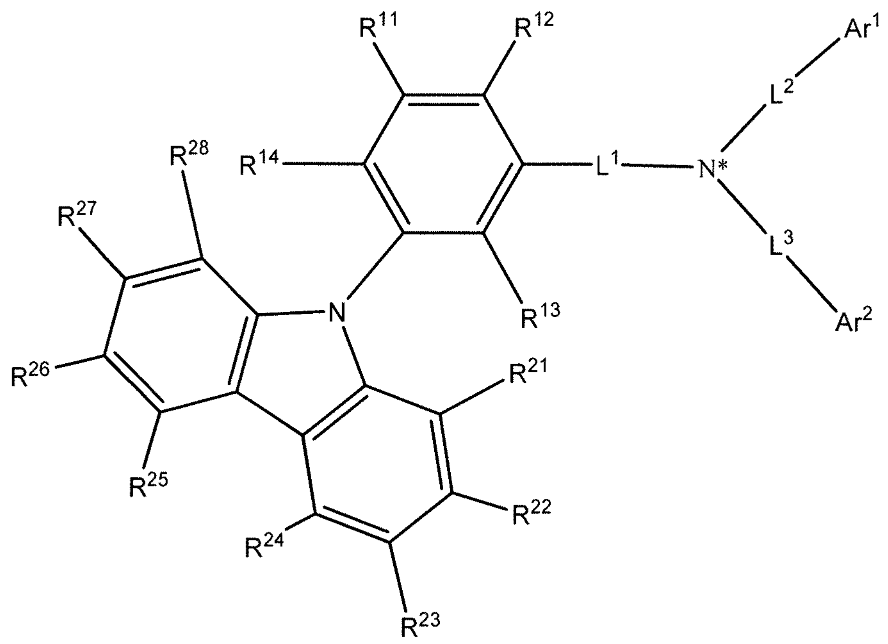

(1)

wherein

N* is a central nitrogen atom;
$Ar^1$ and $Ar^2$ are each independently a substituted or unsubstituted aryl group having 6 to 16 ring carbon atoms or a substituted or unsubstituted monovalent heterocyclic group having 5 to 30 ring atoms;
when both of $Ar^1$ and $Ar^2$ are the substituted or unsubstituted aryl groups having 6 to 16 ring carbon atoms, the total number of carbon atoms of the two substituted or unsubstituted aryl groups having 6 to 16 ring carbon atoms is 12 to 38;
$L^1$ is any one of a single bond, a substituted or unsubstituted arylene group having 6 to 30 ring carbon atoms, and a substituted or unsubstituted divalent heterocyclic group having 5 to 30 ring atoms;
the substituent of $L^1$ is a substituted or unsubstituted alkyl group having 1 to 50 carbon atoms, or a substituted or unsubstituted aryl group having 6 to 50 ring carbon atoms;
$L^2$ and $L^3$ are each independently any one of a single bond, a substituted or unsubstituted non-fused arylene group having 6 to 30 ring carbon atoms, and a substituted or unsubstituted divalent heterocyclic group having 5 to 30 ring atoms;
$Ar^1$ and $L^2$ are not crosslinked;
$Ar^2$ and $L^3$ are not crosslinked; and
$R^{11}$ to $R^{14}$ and $R^{21}$ to $R^{28}$ are each independently a hydrogen atom or a substituent, and the substituent is a substituted or unsubstituted alkyl group having 1 to 50 carbon atoms or a substituted or unsubstituted aryl group having 6 to 50 ring carbon atoms.

2. The compound according to claim 1, wherein each of the substituted or unsubstituted aryl groups having 6 to 16 ring carbon atoms independently consists of a benzene ring.

3. The compound according to claim 1 or 2, wherein the substituent of $Ar^1$ and the substituent of $Ar^2$ are each independently a halogen atom; a nitro group; a cyano group; an unsubstituted alkyl group having 1 to 50 carbon atoms; an unsubstituted alkenyl group having 2 to 50 carbon atoms; an unsubstituted alkynyl group having 2 to 50 carbon atoms; an unsubstituted cycloalkyl group having 3 to 50 ring carbon atoms; an unsubstituted haloalkyl group having 1 to 50 carbon atoms; an unsubstituted alkoxy group having 1 to 50 carbon atoms; an unsubstituted haloalkoxy group having 1 to 50 carbon atoms; an unsubstituted alkylthio group having 1 to 50 carbon atoms; an unsubstituted aryl group having 6 to 50 ring carbon atoms; an unsubstituted aryloxy group having 6 to 50 ring carbon atoms; an unsubstituted arylthio group having 6 to 50 ring carbon atoms; an unsubstituted aralkyl group having 7 to 50 carbon atoms; an unsubstituted monovalent heterocyclic group having 5 to 50 ring atoms; or a mono-, di-, or tri-substituted silyl group having a substituent selected from an unsubstituted alkyl group having 1 to 50 carbon atoms, an unsubstituted cycloalkyl group having 3 to 50 ring carbon atoms, an unsubstituted aryl group having 6 to 50 ring carbon atoms, and an unsubstituted monovalent heterocyclic group having 5 to 50 ring atoms.

**4.** The compound according to any one of claims 1 to 3, wherein the at least one deuterium atom is at least one of a hydrogen atom directly bonded to an arylene group represented by $L^1$, a hydrogen atom directly bonded to a divalent heterocyclic group represented by $L^1$, a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, and a hydrogen atom directly bonded to a divalent heterocyclic group represented by $L^3$.

**5.** The compound according to any one of claims 1 to 3, wherein the at least one deuterium atom is at least one of a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, a hydrogen atom directly bonded to a divalent heterocyclic group represented by $L^3$, a hydrogen atom directly bonded to an aryl group represented by $Ar^1$, a hydrogen atom directly bonded to a monovalent heterocyclic group represented by $Ar^1$, a hydrogen atom directly bonded to an aryl group represented by $Ar^2$, and a hydrogen atom directly bonded to a monovalent heterocyclic group represented by $Ar^2$.

**6.** The compound according to claim 4 or 5, wherein the at least one deuterium atom is at least one of a hydrogen atom directly bonded to a non-fused arylene group represented by $L^2$, a hydrogen atom directly bonded to a divalent heterocyclic group represented by $L^2$, a hydrogen atom directly bonded to a non-fused arylene group represented by $L^3$, and a hydrogen atom directly bonded to a divalent heterocyclic group represented by $L^3$.

**7.** The compound according to any one of claims 1 to 6, wherein the compound represented by formula (1) has a deuteration rate of 10% or more.

**8.** The compound according to claim 7, wherein the compound represented by formula (1) has a deuteration rate of 50% or more.

**9.** The compound according to any one of claims 1 to 8, wherein the proportion of the number of deuterium atoms with respect to the total number of hydrogen atoms in $L^1$ to $L^3$ and $R^{11}$ to $R^{14}$ is 10% or more.

**10.** A compound represented by the following formula (2), which has at least one deuterium atom:

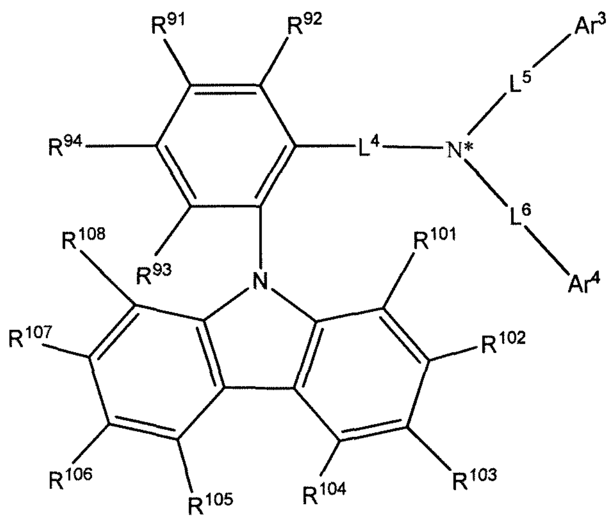

(2)

wherein

N* is a central nitrogen atom;
$Ar^3$ and $Ar^4$ are each independently a substituted or unsubstituted aryl group having 6 to 30 ring carbon atoms (excluding a phenyl group) or a substituted or unsubstituted monovalent heterocyclic group having 5 to 30 ring atoms;
$L^4$, $L^5$ and $L^6$ are each independently any one of a single bond, a substituted or unsubstituted arylene group having 6 to 30 ring carbon atoms, and a substituted or unsubstituted divalent heterocyclic group having 5 to 30

ring atoms; and
$R^{91}$ to $R^{94}$ and $R^{101}$ to $R^{108}$ are each independently a hydrogen atom or a substituent, and the substituent is a halogen atom; a nitro group; a cyano group; a substituted or unsubstituted alkyl group having 1 to 50 carbon atoms; a substituted or unsubstituted alkenyl group having 2 to 50 carbon atoms; a substituted or unsubstituted alkynyl group having 2 to 50 carbon atoms; a substituted or unsubstituted cycloalkyl group having 3 to 50 ring carbon atoms; a substituted or unsubstituted haloalkyl group having 1 to 50 carbon atoms; a substituted or unsubstituted alkoxy group having 1 to 50 carbon atoms; a substituted or unsubstituted haloalkoxy group having 1 to 50 carbon atoms; a substituted or unsubstituted alkylthio group having 1 to 50 carbon atoms; a substituted or unsubstituted aryl group having 6 to 50 ring carbon atoms; a substituted or unsubstituted aryloxy group having 6 to 50 ring carbon atoms; a substituted or unsubstituted arylthio group having 6 to 50 ring carbon atoms; a substituted or unsubstituted aralkyl group having 7 to 50 carbon atoms; a substituted or unsubstituted monovalent heterocyclic group having 5 to 50 ring atoms; or a mono-, di-, or tri-substituted silyl group having a substituent selected from a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, and a substituted or unsubstituted monovalent heterocyclic group having 5 to 50 ring atoms.

**11.** The compound according to claim 10, wherein the substituted or unsubstituted aryl group having 6 to 30 ring carbon atoms (excluding a phenyl group) is a substituted or unsubstituted aryl group having 10 to 30 ring carbon atoms.

**12.** The compound according to claim 10 or 11, wherein the at least one deuterium atom is at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, a hydrogen atom directly bonded to a monovalent heterocyclic group represented by $Ar^4$, a hydrogen atom represented by $R^{91}$ to $R^{94}$, and a hydrogen atom represented by $R^{101}$ to $R^{108}$.

**13.** The compound according to claim 12, wherein the at least one deuterium atom is at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to an divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^4$, a hydrogen atom directly bonded to a monovalent heterocyclic group represented by $Ar^4$, a hydrogen atom represented by $R^{91}$ to $R^{94}$, and a hydrogen atom represented by $R^{101}$ to $R^{108}$.

**14.** The compound according to claim 12, wherein the at least one deuterium atom is at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, a hydrogen atom directly bonded to a monovalent heterocyclic group represented by $Ar^4$, and a hydrogen atom represented by $R^{91}$ to $R^{94}$.

**15.** The compound according to claim 12, wherein the at least one deuterium atom is at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, and a hydrogen atom represented by $R^{91}$ to $R^{94}$.

**16.** The compound according to claim 12, wherein the at least one deuterium atom is at least one of a hydrogen atom directly bonded to an arylene group represented by $L^4$, a hydrogen atom directly bonded to a divalent heterocyclic

group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, and a hydrogen atom directly bonded to a monovalent heterocyclic group represented by $Ar^4$.

**17.** The compound according to claim 16, wherein the at least one deuterium atom is at least one of a hydrogen atom directly bonded to an arylene group represented by $L^4$, a hydrogen atom directly bonded to a divalent heterocyclic group represented by $L^4$, a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, and a hydrogen atom directly bonded to a divalent heterocyclic group represented by $L^6$.

**18.** The compound according to claim 16, wherein the at least one deuterium atom is at least one of a hydrogen atom directly bonded to an arylene group represented by $L^5$, a hydrogen atom directly bonded to a divalent heterocyclic group represented by $L^5$, a hydrogen atom directly bonded to an arylene group represented by $L^6$, a hydrogen atom directly bonded to a divalent heterocyclic group represented by $L^6$, a hydrogen atom directly bonded to an aryl group represented by $Ar^3$, a hydrogen atom directly bonded to a monovalent heterocyclic group represented by $Ar^3$, a hydrogen atom directly bonded to an aryl group represented by $Ar^4$, and a hydrogen atom directly bonded to a monovalent heterocyclic group represented by $Ar^4$.

**19.** The compound according to claim 17 or 18, wherein the at least one deuterium atom is at least one of a hydrogen atom directly bonded to a non-fused arylene group represented by $L^5$, a hydrogen atom directly bonded to a divalent heterocyclic group represented by $L^5$, a hydrogen atom directly bonded to an arylene group represented by $L^6$, and a hydrogen atom directly bonded to a divalent heterocyclic group represented by $L^6$.

**20.** The compound according to any one of claims 10 to 19, wherein the compound represented by formula (2) has a deuteration rate of 10% or more.

**21.** The compound according to claim 20, wherein the compound represented by formula (2) has a deuteration rate of 50% or more.

**22.** The compound according to any one of claims 10 to 21, wherein the proportion of the number of deuterium atoms with respect to the total number of hydrogen atoms in $L^4$ to $L^6$ and $R^{91}$ to $R^{94}$ is 10% or more.

**23.** A material for an organic electroluminescent element, comprising the compound according to any one of claims 1 to 22.

**24.** The material for an organic electroluminescent element according to claim 23, further comprising a light hydrogen compound of the compound according to any one of claims 1 to 22, wherein the light hydrogen compound is a compound in which all hydrogen atoms are light hydrogen atoms.

**25.** The material for an organic electroluminescent element according to claim 23, comprising at least two compounds according to any one of claims 1 to 22.

**26.** The material for an organic electroluminescent element according to claim 23, comprising a first compound and a second compound, wherein the first compound is the compound according to any one of claims 1 to 22, and the material for an organic electroluminescent element contains 1% by mass or more of the first compound.

**27.** The material for an organic electroluminescent element according to claim 26, wherein the first compound and the second compound are hole transporting layer materials.

**28.** An organic electroluminescent element comprising a cathode, an anode, and organic layers intervening between the cathode and the anode, the organic layers including a light emitting layer, at least one layer of the organic layers containing the compound according to any one of claims 1 to 22.

**29.** The organic electroluminescent element according to claim 28, wherein the organic layer includes a hole transporting zone between the anode and the light emitting layer, and the hole transporting zone includes the compound according

to any one of claims 1 to 22.

**30.** The organic electroluminescent element according to claim 28, wherein the hole transporting zone includes a first hole transporting layer on an anode side and a second hole transporting layer on a cathode side, and at least one of the first hole transporting layer and the second hole transporting layer includes the compound according to any one of claims 1 to 22.

**31.** The organic electroluminescent element according to any one of claims 28 to 30, wherein the light emitting layer contains a fluorescent dopant material.

**32.** The organic electroluminescent element according to any one of claims 28 to 30, wherein the light emitting layer contains a phosphorescent dopant material.

**33.** An electronic device comprising the organic electroluminescent element according to any one of claims 28 to 32.

[Fig. 1]

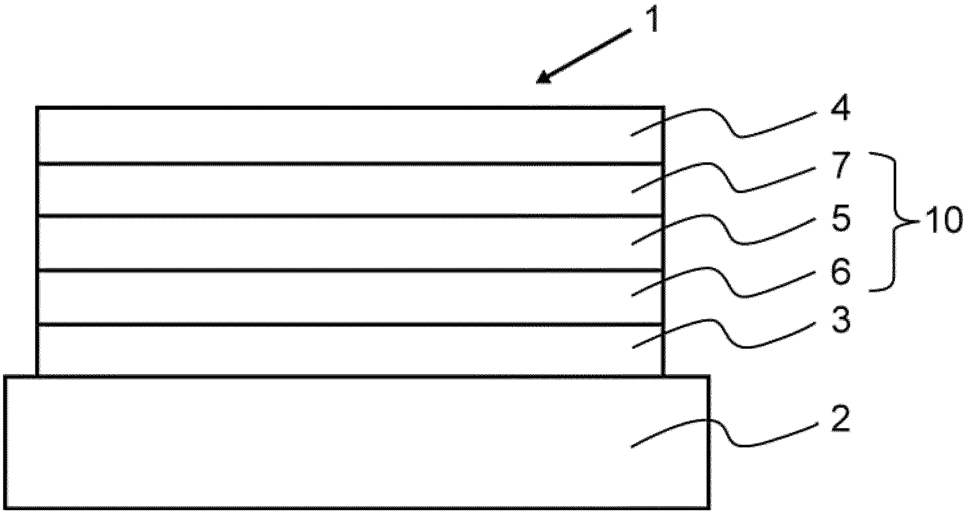


[Fig. 2]

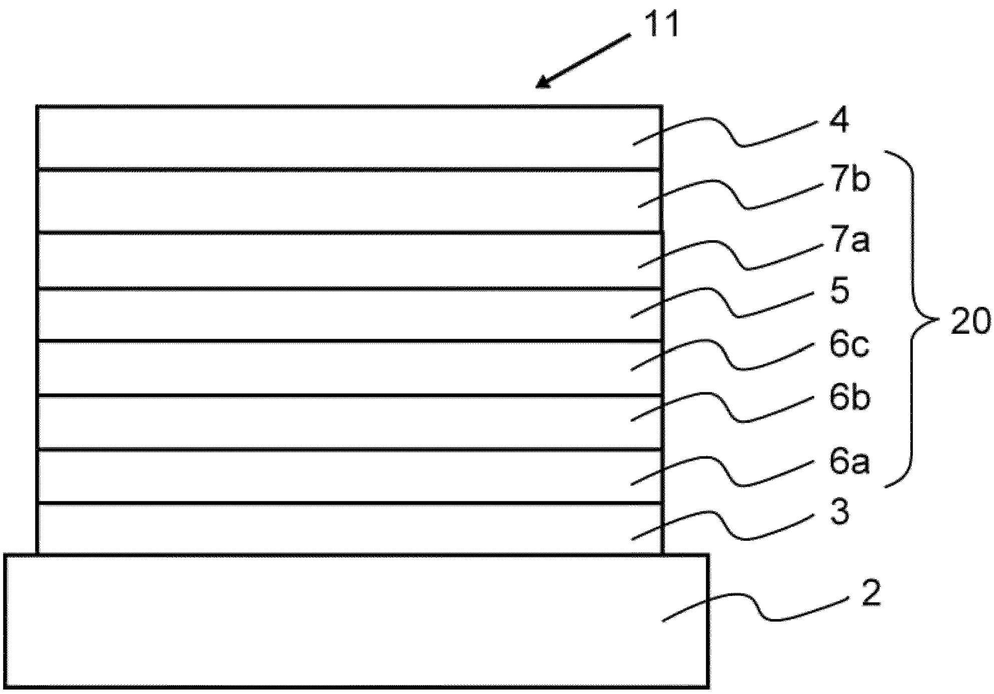

# INTERNATIONAL SEARCH REPORT

International application No.
**PCT/JP2021/035741**

**A. CLASSIFICATION OF SUBJECT MATTER**

***C07D 209/86***(2006.01)i; ***C07D 405/12***(2006.01)i; ***H01L 51/50***(2006.01)i
FI: C07D209/86 CSP; H05B33/14 A; H05B33/22 D; C07D405/12

According to International Patent Classification (IPC) or to both national classification and IPC

**B. FIELDS SEARCHED**

Minimum documentation searched (classification system followed by classification symbols)

C07D209/86; C07D405/12; H01L51/50

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | KR 10-2020-0088704 A (MATERIAL SCIENCE CO. LTD.) 23 July 2020 (2020-07-23) claims, paragraphs [0077]-[0102], compounds 9, 28, 76, 80, examples | 1-9, 23, 26, 28, 32-33 |
| X | KR 10-2015-0116337 A (DUK SAN NEOLUX CO. LTD.) 15 October 2015 (2015-10-15) claims, paragraphs [0013], [0087], compound P-53, examples, in particular, I-1 | 1-3, 5, 7, 23, 26-33 |
| Y | claims, compounds described in paragraphs [0013], [0085]-[0093], P-52, P-54, P-57, P-63 to P-65, P-71 to P-72, P-80, P-109 to P-110, P-112, P-114, P-116, P-121, P-123 to P-124, P-126 to P-127, P-134 to P-135, examples | 1-33 |
| X | WO 2018/012780 A1 (DUK SAN NEOLUX CO. LTD.) 18 January 2018 (2018-01-18) claims, in particular, claim 13, compound 1-36, paragraphs [0028], [0297], examples | 1-3, 7, 23, 26, 28, 32-33 |
| Y | document 3, claims, compounds 1-19 to 1-31, 1-34 to 1-37, paragraphs [0028], [0297], examples | 1-33 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 November 2021** | **07 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/035741**

**C. DOCUMENTS CONSIDERED TO BE RELEVANT**

| Category* | Citation of document, with indication, where appropriate, of the relevant passages | Relevant to claim No. |
|---|---|---|
| X | JP 2020-527550 A (LG CHEM, LTD.) 10 September 2020 (2020-09-10) claims, paragraphs [0010], [0031], page 25, line 2 from the top, compound on the right, page 26, line 2 from the top, compound on the right, line 2 from the top, compound on the right, page 27, line 4 from the top, compound on the left, page 28, line 2 from the top, compound on the left, page 28, line 3 from the top, compound on the right, examples, in particular, examples 2-12 to 2-24 | 1, 3-33 |
| Y | JP 2013-505982 A (E. I. DU PONT DE NEMOURS AND COMPANY) 21 February 2013 (2013-02-21) claims, paragraphs [0002], [0073], examples | 1-33 |
| Y | JP 2017-125087 A (SUMITOMO CHEMICAL CO) 20 July 2017 (2017-07-20) claims, paragraphs [0006]-[0007], [0017], examples | 1-33 |
| X | US 2018/0090688 A1 (LG CHEM, LTD.) 29 March 2018 (2018-03-29) claims, paragraphs [0018], compounds in paragraph [0076] in the vertical direction, 1st, 3-5th, 9-12th, 14th, 15-16th, 18-23th, page 22, 2nd compound in the left column, 2nd to 3rd compounds in the right colum, page 23, compounds, page 24, 1st to 3rd compounds in the left column, 2nd to 3rd compounds in the right column, paragraph [0133], examples, table 2 | 10-33 |
| P, X | WO 2021/066351 A1 (LG CHEM, LTD.) 08 April 2021 (2021-04-08) claims, paragraph [0122], examples | 1-3, 5, 23, 26, 28, 31-33 |
| P, X | WO 2021/162293 A1 (LG CHEM, LTD.) 19 August 2021 (2021-08-19) claims, paragraph [0193], examples | 10-23, 26-29, 31-33 |

Form PCT/ISA/210 (second sheet) (January 2015)

**INTERNATIONAL SEARCH REPORT**
**Information on patent family members**

International application No.
**PCT/JP2021/035741**

| Patent document cited in search report | Publication date (day/month/year) | Patent family member(s) | Publication date (day/month/year) |
|---|---|---|---|
| KR 10-2020-0088704 A | 23 July 2020 | (Family: none) | |
| KR 10-2015-0116337 A | 15 October 2015 | (Family: none) | |
| WO 2018/012780 A1 | 18 January 2018 | US 2019/0229271 A1<br>claims, compounds 1-36, paragraphs [0019], [0102], examples<br>US 2019/0300535 A1<br>WO 2018/012781 A1<br>KR 10-2018-0008286 A<br>KR 10-2018-0008291 A<br>CN 109476596 A | |
| JP 2020-527550 A | 10 September 2020 | US 2020/0106017 A1<br>claims, paragraphs [0022], [0044], [0054], examples<br>WO 2019/093649 A1<br>EP 3620449 A1<br>KR 10-2019-0053768 A<br>TW 201922686 A<br>CN 110799486 A | |
| JP 2013-505982 A | 21 February 2013 | US 2011/0095273 A1<br>claims, paragraph [0067], examples<br>US 2012/0187382 A1<br>WO 2011/040939 A1<br>WO 2010/075421 A2<br>EP 2379671 A2<br>EP 2483366 A1<br>TW 201035281 A<br>TW 201111326 A<br>KR 10-2011-0106397 A<br>TW 201040243 A<br>CN 102341475 A<br>CN 102510889 A<br>KR 10-2012-0091144 A<br>KR 10-2015-0061040 A | |
| JP 2017-125087 A | 20 July 2017 | (Family: none) | |
| US 2018/0090688 A1 | 29 March 2018 | WO 2017/052261 A1<br>KR 10-2017-0036641 A<br>TW 201722915 A<br>CN 107531627 A | |
| WO 2021/066351 A1 | 08 April 2021 | (Family: none) | |
| WO 2021/162293 A1 | 19 August 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011024451 A
- KR 1020110064222 A
- US 6110307 B
- WO 2013122082 A
- WO 2014007022 A
- US 20140138633 A1
- KR 1020140103697 A
- KR 1020150031892 A
- KR 1020150116337 A
- US 20180090688 A1
- WO 2017118238 A
- KR 1020170084917 A
- US 20190189927 A1
- US 20200106017 A1
- KR 1020180078177 A
- KR 101978651 B
- KR 1020190084880 A